# EUROPÄISCHE PATENTSCHRIFT

(11) **EP 1 210 335 B1**
(45) Veröffentlichungstag und Bekanntmachung des Hinweises auf die Patenterteilung: **14.09.2005**
(21) Anmeldenummer: 00951526.3
(22) Anmeldetag: 17.08.2000
(51) Int. Cl.: C07D 235/14, C07D 401/12, C07D 405/10, C07D 409/10, C07D 405/14, C07D 409/14, C07D 401/10, C07D 403/10, C07D 401/14

(54) **AMINOCARBONYL-SUBSTITUIERTE BENZIMIDAZOLDERIVATE, VERFAHREN ZU IHRER HERSTELLUNG UND IHRE VERWENDUNG ALS ARZNEIMITTEL**
AMINOCARBONYL-SUBSTITUTED BENZIMIDAZOLE DERIVATIVES, METHOD FOR PRODUCING SAME AND THE USE THEREOF AS MEDICAMENTS
DERIVES DE BENZIMIDAZOL SUBSTITUES AMINOCABONYLE, LEUR PROCEDE DE PREPARATION ET LEUR UTILISATION COMME PRODUIT PHARMACEUTIQUE

(30) Priorität: 20.08.1999 DE 19939463
(43) Veröffentlichungstag der Anmeldung: 05.06.2002
(73) Patentinhaber: Boehringer Ingelheim Pharma GmbH & Co.KG, 55218 Ingelheim am Rhein (DE)
(72) Erfinder: ANDERSKEWITZ, Ralf, D-55411 Bingen (DE); BRAUN, Christine, CH-6512 Giubiasco (CH); BRIEM, Hans, D-28279 Bremen (DE); DISSE, Bernd, D-55127 Mainz (DE); HOENKE, Christoph, D-55218 Ingelheim am Rhein (DE); JENNEWEIN, Hans-Michael, D-65193 Wiesbaden (DE); SPECK, Georg, D-55218 Ingelheim am Rhein (DE)
(74) Vertreter: Kompter, Hans-Michael
(86) Internationale Anmeldenummer: PCT/EP2000/008037
(87) Internationale Veröffentlichungsnummer: WO 2001/014342

(56) Entgegenhaltungen:
- WO-A-98/37075

## Beschreibung

Die Erfindung betrifft aminocarbonyl-substituierte Benzimidazolderivate der allgemeinen Formel (I) 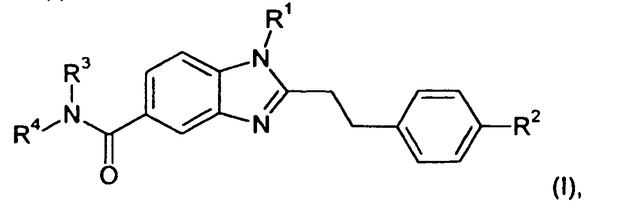 worin die Reste R¹, R², R³ und R⁴ die in den Ansprüchen und in der Beschreibung genannten Bedeutungen haben können, deren Prodrugs, Verfahren zu deren Herstellung sowie die Verwendung von Benzimidazolderivaten als Arzneimittel, insbesondere als Arzneimittel mit Tryptase-inhibierender Wirkung.

### Hintergrund der Erfindung

Benzimidazolderivate sind als Wirkstoffe mit wertvollen pharmazeutischen Eigenschaften aus dem Stand der Technik bekannt. So offenbart die Internationale Patentanmeldung WO 98/37075 neben anderen bicyclischen Heterocyclen auch Benzimidazole, die sich aufgrund einer thrombinhemmenden Wirkung zur Vorbeugung und Behandlung venöser und arterieller thrombotischer Erkrankungen wirksam einsetzen lassen.

Anders als der vorstehend beschriebenen und im Stand der Technik bereits bekannten Verwendung von Benzimidazolderivaten, liegt der vorliegenden Erfindung die Aufgabe zugrunde, neue Tryptase-Inhibitoren bereitzustellen, die aufgrund ihrer Tryptase-inhibierenden Eigenschaften zur Vorbeugung und Behandlung entzündlicher und/oder allergischer Erkrankungen eingesetzt werden können.

### Detaillierte Beschreibung der Erfindung

Überraschenderweise wurde gefunden, daß aminocarbonyl-substituierte Benzimidazolderivate der allgemeinen Formel (I) 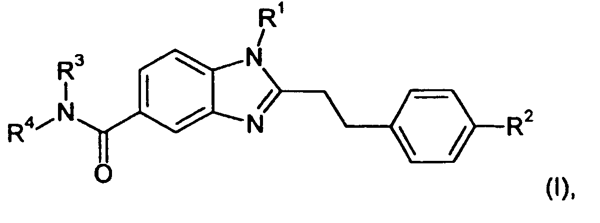 worin die Reste R¹, R², R³ und R⁴ die nachstehend genannten Bedeutungen tragen können, eine Tryptase-inhibierende Wirkung aufweisen und erfindungsgemäß zur Vorbeugung und Behandlung von Erkrankungen Verwendung finden können, in denen Tryptase-Inhibitoren einen therapeutischen Nutzen entfalten können.

Dementsprechend zielt die vorliegende Erfindung auf die erfindungsgemäße Verwendung von Verbindungen der allgemeinen Formel (I) 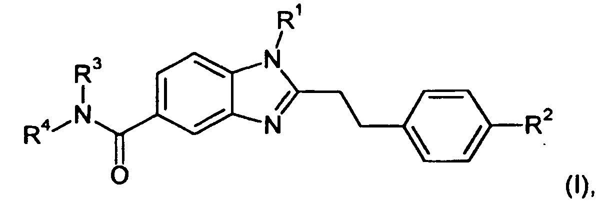 zur Herstellung eines Arzneimittels zur Vorbeugung und/oder Behandlung von Krankheiten, in denen Tryptase-Inhibitoren einen therapeutischen Nutzen entfalten können, worin
- R¹: C₁-C₁₀-Alkyl, welches gegebenenfalls ein-, zwei- oder dreifach durch eine oder mehrere der Gruppen C₁-C₄-Alkoxy, Phenoxy, Hydroxyphenoxy, C₁-C₄-Alkoxy-phenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiert sein kann, oder
Phenyl-C₁-C₄-alkyl, wobei der Phenylring gegebenenfalls ein-, zwei-, oder dreifach durch einen oder mehrere der Reste C₁-C₄-Alkyl, CF₃, Fluor, Chlor, Brom, COOH oder COO-C₁-C₄-Alkyl substituiert sein kann, oder
ein über eine Einfachbindung oder über eine C₁-C₄-Alkylenbrücke verknüpfter 5 oder 6 gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, gegebenenfalls durch C₁-C₄-Alkyl substituiertes Phenyl oder gegebenenfalls durch C₁-C₄-Alkyl substituiertes Benzyl substituiert sein kann oder an den über zwei benachbarte Kohlenstoffatome gegebenenfalls ein Benzolring ankondensiert sein kann;
- R²: -C(=NH)NH₂ oder -CH₂-NH₂;
- R³ und R⁴: gleich oder verschieden,
Wasserstoff, C₁-C₆-Alkyl, welches ein- oder zweifach durch eine oder mehrere der Gruppen COOH, COO-C₁-C₄-Alkyl, C₃-C₆-Cycloalkyl, -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert sein kann, oder
Phenyl-C₁-C₄-alkyl, wobei die C₁-C₄-Alkylenbrücke gegebenfalls durch Phenyl, COOH oder COO-C₁-C₄-Alkyl substituiert sein kann und wobei der Phenylring gegebenenfalls ein-, zwei-, oder dreifach, direkt oder über eine C₁-C₄-Alkylenbrücke durch einen oder mehrere der Reste C₁-C₄-Alkyl, C₁-C₄-Alkoxy, CF₃, Fluor, Chlor, Brom, COOH, COO-C₁-C₄-Alkyl, -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert sein kann, oder
ein direkt oder über eine C₁-C₄-Alkylenbrücke verknüpfter 5-, 6- oder 7-gliedriger, gesättigter oder ungesättigter Heterocyclus, der ein, zwei, drei oder vier Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, Phenyl oder Benzyl substituiert sein kann oder an den über zwei benachbarte Kohlenstoffatome gegebenenfalls ein Benzolring arikondensiert sein kann, oder
C₃-C₈-Cycloalkyl, Naphthyl oder Phenyl, welches gegebenenfalls ein-, zwei-, oder dreifach durch einen oder mehrere der Reste C₁-C₄-Alkyl, C₁-C₄-Alkoxy, Phenyloxy, Benzyloxy, CF₃, Fluor, Chlor, Brom, COOH oder COO-C₁-C₄-Alkyl substituiert sein kann, oder
oder
R³ und R⁴ bilden gemeinsam mit dem Stickstoffatom einen 5-, 6- oder 7-gliedrigen, gesättigten oder ungesättigten Heterocyclus, der ein oder zwei weitere Heteroatome ausgewählt aus der Gruppe Sauerstoff, Stickstoff oder Schwefel enthalten kann und der gegebenenfalls durch einen oder mehrere der Reste C₁-C₄-Alkyl, C₅-C₆-Cycloalkyl, Benzyl, das gegebenenfalls durch C₁-C₄-Alkyl substituiert sein kann, Pyridyl oder Phenyl, das gegebenenfalls durch C₁-C₄-Alkyl-, C₁-C₄-Alkoxy- oder Hydroxy substituiert ist, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß bevorzugt ist die vorstehend genannte Verwendung von Verbindungen der allgemeinen Formel (I) zur Herstellung eines Arzneimittels zur Vorbeugung und/oder Behandlung entzündlicher und/oder allergischer Erkrankungen. Besonders bevorzugt ist die eingangs genannte Verwendung der Verbindungen der allgemeinen Formel (I) zur Herstellung eines Arzneimittels zur Vorbeugung und/oder Behandlung von Asthma bronchiale, allergischer Rhinitis, allegischer Conjunctivitis, atopischer Dermatitis, Urticaria, allergischer Otitis, allergischer Magen-Darmerkrankungen, Morbus Crohn, Colitis ulcerosa, anaphylaktischer Schock, septischer Schock, Schocklunge (ARDS) und Arthritis. Ferner ist von Interesse die eingangs genannte Verwendung der Verbindungen der allgemeinen Formel (I) zur Herstellung eines Arzneimittels zur Vorbeugung und/oder Behandlung von Fibrosen wie Lungenfibrose, fibrosierende Alveolitis und Narbenbildung, von Kollagneosen wie Lupus erythematodes und Sklerodermie sowie von Arteriosklerose, Psoriasis und Neoplasien.

Eine weitere Aufgabe der vorliegenden Erfindung ist, neue Verbindungen bereitzustellen, die eine Tryptase-inhibierende Wirkung aufweisen und zur Vorbeugung und Behandlung von Erkrankungen, in denen Tryptase-Inhibitoren einen therapeutischen Nutzen entfalten können, Verwendung finden können.

Vorstehende Aufgabe wird gelöst durch die nachfolgend beschriebenen erfindungsgemäßen Verbindungen der allgemeinen Formel (I). Die vorliegende Erfindung betrifft folglich ferner Aminocarbonyl-substituierte Benzimidazolderivate der allgemeinen Formel (I) 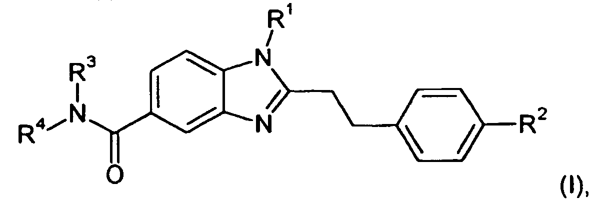 worin
- R¹: C₁-C₁₀-Alkyl, welches gegebenenfalls ein-, zwei- oder dreifach durch eine oder mehrere der Gruppen C₁-C₄-Alkoxy, Phenoxy-, C₁-C₄-Alkoxy-phenoxy, Hydroxyphenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiert sein kann, oder
Phenyl-C₁-C₄-alkyl, wobei der Phenylring gegebenenfalls ein-, zwei-, oder dreifach durch einen oder mehrere der Reste C₁-C₄-Alkyl, CF₃, Fluor, Chlor, Brom, COOH oder COO-C₁-C₄-Alkyl substituiert sein kann, oder
ein über eine Einfachbindung oder über eine C₁-C₄-Alkylenbrücke verknüpfter 5 oder 6 gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, gegebenenfalls durch C₁-C₄-Alkyl substituiertes Phenyl oder gegebenenfalls durch C₁-C₄-Alkyl substituiertes Benzyl substituiert sein kann oder an den über zwei benachbarte Kohlenstoffatome gegebenenfalls ein Benzolring ankondensiert sein kann;
- R²: -C(=NH)NH₂ oder -CH₂-NH₂;
- R³: C₁-C₆-Alkyl, welches ein- oder zweifach durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Benzyl, wobei der Phenylring ein- oder zweifach, direkt oder über eine C₁-C₄-Alkylenbrücke durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Phenyl-C₂-C₄-alkyl, wobei die C₂-C₄-Alkylenbrücke gegebenfalls durch Phenyl, COOH oder COO-C₁-C₄-Alkyl substituiert sein kann und wobei der Phenylring gegebenenfalls ein- oder zweifach, direkt oder über eine C₁-C₄-Alkylenbrücke durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert sein kann, oder
ein über eine C₁-C₄-Alkylenbrücke verknüpfter 5 oder 6 gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, Phenyl oder Benzyl substituiert sein kann;
- R⁴: Wasserstoff, C₁-C₆-Alkyl, welches ein- oder zweifach durch eine oder mehrere der Gruppen COOH, COO-C₁-C₄-Alkyl oder C₃-C₆-Cycloalkyl substituiert sein kann, oder
Phenyl-C₁-C₄-alkyl, wobei die C₁-C₄-Alkylenbrücke gegebenfalls durch Phenyl substituiert sein kann und wobei der Phenylring gegebenenfalls ein-, zwei-, oder dreifach durch einen oder mehrere der Reste C₁-C₄-Alkyl, C₁-C₄-Alkoxy, CF₃, Fluor, Chlor, Brom, COOH oder COO-C₁-C₄-Alkyl substituiert sein kann, oder
C₃-C₈-Cycloalkyl, Naphthyl oder Phenyl, welches gegebenenfalls ein-, zwei-, oder dreifach durch einen oder mehrere der Reste C₁-C₄-Alkyl, C₁-C₄-Alkoxy, Phenyloxy, Benzyloxy, CF₃, Fluor, Chlor, Brom, COOH oder COO-C₁-C₄-Alkyl substituiert sein kann, oder
ein über eine C₁-C₄-Alkylenbrücke verknüpfter 5-,6- oder 7-gliedriger, gesättigter oder ungesättigter Heterocyclus, der ein Heteroatom ausgewählt aus der Gruppe Sauerstoff, Stickstoff oder Schwefel enthält und der gegebenenfalls ein-, zwei- oder dreifach durch einen oder mehrere der Reste C₁-C₄-Alkyl, Phenyl oder Benzyl substituiert sein kann oder an den über zwei benachbarte Kohlenstoffatome gegebenenfalls ein Benzolring ankondensiert sein kann,
oder
R³ und R⁴ bilden gemeinsam mit dem Stickstoffatom einen 5-, 6- oder 7-gliedrigen, gesättigten oder ungesättigten Heterocyclus, der ein oder zwei weitere Heteroatome ausgewählt aus der Gruppe Sauerstoff, Stickstoff oder Schwefel enthält und der gegebenenfalls durch einen oder mehrere der Reste C₁-C₄-Alkyl, Benzyl, das gegebenenfalls durch C₁-C₄-Alkyl-substituiert ist, C₅-C₆-Cycloalkyl, Pyridyl oder Phenyl, das gegebenenfalls einen Rest ausgewählt aus der Gruppe C₁-C₄-Alkyl, C₁-C₄-Alkoxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Bevorzugt sind Verbindungen der allgemeinen Formel (I), worin
- R¹: unsubstituiertes C₁-C₁₀-Alkyl, oder
ein- oder zweifach durch C₁-C₄-Alkoxy, Phenoxy-, C₁-C₄-Alkoxy-phenoxy, Hydroxyphenoxy, C₃-C₆-Cycloalkyl, -NH₂, -NH(C₁-C₄-Alkyl), -N(C₁-C₄-Alkyl)2, -NH-CO-(C₁-C₄-Alkyl), -CO-NH₂, -CO-NH-(C₁-C₄-Alkyl) oder -NH-CO-Benzyl substituiertes C₁-C₄-Alkyl, oder
Phenyl-C₁-C₃-alkyl, wobei der Phenylring gegebenenfalls ein- oder zweifach durch C₁-C₄-Alkyl, CF₃, Fluor, Chlor, Brom, COOH oder COO-C₁-C₄-Alkyl substituiert sein kann, oder
ein über eine C₁-C₃-Alkylenbrücke verknüpfter 5-, 6- oder 7- gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls ein- oder zweifach durch einen oder mehrere der Reste Methyl, Ethyl, Propyl, Phenyl, Methylphenyl- oder Benzyl substituiert sein kann oder an den über zwei benachbarte Kohlenstoffatome gegebenenfalls ein Benzolring ankondensiert sein kann;
- R²: -C(=NH)NH₂ oder -CH₂-NH₂;
- R³: C₁-C₆-Alkyl, welches ein- oder zweifach durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Benzyl, wobei der Phenylring direkt oder über eine C₁-C₄-Alkylenbrücke durch eine der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Phenyl-C₂-C₄-alkyl, wobei die C₂-C₄-Alkylenbrücke gegebenfalls durch Phenyl, COOH oder COO-C₁-C₄-Alkyl substituiert sein kann und
wobei der Phenylring direkt oder über eine C₁-C₄-Alkylenbrücke durch eine der Gruppen -NH₂, -NH(C₁-C₄-Alkyl), -N(C₁-C₄-Alkyl)₂, -NHPhenyl, -N(Phenyl)₂,
-NHBenzyl, -N(Benzyl)₂, -C(=NH)NH₂ oder -NH-C(=NH)NH₂ substituiert sein kann, oder
ein über eine C₁-C₄-Alkylenbrücke verknüpfter 5-, 6- oder 7-gliedriger, gesättigter oder ungesättigter Heterocyclus, der ein oder zwei Heteroatome ausgewählt aus der Gruppe Sauerstoff oder Stickstoff enthalten kann und der gegebenenfalls ein- oder zweifach durch einen oder mehrere der Reste Methyl, Ethyl, Propyl, Phenyl oder Benzyl substituiert sein kann;
- R⁴: Wasserstoff, C₁-C₄-Alkyl, welches durch eine der Gruppen COOH, COO-C₁-C₄-Alkyl oder C₃-C₆-Cycloalkyl substituiert sein kann, oder
Phenyl-C₁-C₄-alkyl, wobei die C₁-C₄-Alkylenbrücke gegebenfalls durch Phenyl substituiert sein kann und wobei der Phenylring gegebenenfalls ein- oder zweifach durch einen oder mehrere der Reste C₁-C₄-Alkyl, C₁-C₄-Alkoxy, CF₃, Fluor, Chlor, Brom, COOH oder COO-C₁-C₄-Alkyl substituiert sein kann, oder
C₃-C₈-Cycloalkyl, Naphthyl oder Phenyl, welches gegebenenfalls ein- oder zweifach durch einen oder mehrere der Reste C₁-C₄-Alkyl, C₁-C₄-Alkoxy, Phenyloxy, Benzyloxy, CF₃, Fluor, Chlor, Brom, COOH oder COO-C₁-C₄-Alkyl substituiert sein kann, oder
ein über eine C₁-C₄-Alkylenbrücke verknüpfter 5-, 6- oder 7-gliedriger, gesättigter oder ungesättigter Heterocyclus, der ein Heteroatom ausgewählt aus der Gruppe Sauerstoff, Stickstoff oder Schwefel enthält und der gegebenenfalls ein- oder zweifach durch einen oder mehrere der Reste Methyl, Ethyl, Propyl, Phenyl oder Benzyl substituiert sein kann oder an den über zwei benachbarte Kohlenstoffatome gegebenenfalls ein Benzolring ankondensiert sein kann,
oder
R³ und R⁴ bilden gemeinsam mit dem Stickstoffatom einen 6- oder 7-gliedrigen, gesättigten oder ungesättigten Heterocyclus, der ein oder zwei weitere Heteroatome ausgewählt aus der Gruppe Sauerstoff oder Stickstoff enthält und der gegebenenfalls durch einen oder mehrere der Reste Methyl, Ethyl, Propyl, Benzyl, Cyclopentyl, Cyclohexyl, Pyridyl oder Phenyl, das gegebenenfalls einen Rest ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind Verbindungen der allgemeinen Formel (I), worin
- R¹: unsubstituiertes C₁-C₁₀-Alkyl, oder durch C₁-C₄-Alkoxy, Phenoxy, C₁-C₄-Alkoxy-phenoxy, Hydroxyphenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiertes C₁-C₄-Alkyl, oder
Phenyl-C₁-C₃-alkyl, wobei der Phenylring gegebenenfalls ein- oder zweifach durch C₁-C₄-Alkyl, CF₃, Fluor, Chlor, Brom, COOH oder COO-C₁-C₄-Alkyl substituiert sein kann, oder
ein über eine C₁-C₃-Alkylenbrücke verknüpfter, gegebenenfalls ein- oder zweifach durch einen oder mehrere der Reste Methyl, Ethyl, Propyl, Phenyl, Methylphenyl- oder Benzyl substituierter Heterocyclus, ausgewählt aus der Gruppe Pyrrol, Pyrrolin, Pyrrolidin, Pyridin, Piperidin, Pyrimidin, Piperazin, Morpholin, Thiomorpholin, Imidazol, Imidazolin, Imidazolidin, Pyrazol, Pyrazolin, Pyrazolidin, Triazol, Furan, Tetrahydrofuran, α-Pyran, γ-Pyran, Dioxolan, Tetrahydropyran, Dioxan, Thiophen, Dihydrothiophen, Thiolan, Dithiolan,
Oxazol, Isoxazol, Thiazol, Isothiazol, Oxadiazol, Benzodioxol, Benzimidazol, Benzthiophen, Benzfuran oder Indol;
- R²: -C(=NH)NH₂ oder -CH₂-NH₂;
- R³: C₁-C₃-Alkyl, das durch -NH₂, -NH(C₁-C₃-Alkyl), -N(C₁-C₃-Alkyl)₂, -NHPhenyl, -N(Phenyl)₂, -NHBenzyl, -N(Benzyl)₂, -NH-CO-(C₁-C₃-Alkyl), -C(=NH)NH₂ oder -NH-C(=NH)NH₂ substituiert ist, oder Benzyl, wobei der Phenylring direkt oder über eine Methylenbrücke durch eine der Gruppen -NH₂, -NH(C₁-C₃-Alkyl), -N(C₁-C₄-Alkyl)₂, -NHPhenyl, -N(Phenyl)₂, -NHBenzyl, -N(Benzyl)₂, -NH-CO-(C₁-C₃-Alkyl), -C(=NH)NH₂ oder -NH-C(=NH)NH₂ substituiert ist, oder
Phenyl-C₂-C₃-alkyl, wobei die C₂-C₃-Alkylenbrücke gegebenfalls durch Phenyl, COOH oder COO-C₁-C₃-Alkyl substituiert sein kann und
wobei der Phenylring direkt oder über eine Methylenbrücke durch eine der Gruppen -NH₂, -NH(C₁-C₃-Alkyl), -N(C₁-C₃-Alkyl)₂, -NHPhenyl, -N(Phenyl)₂, -NHBenzyl, -N(Benzyl)₂, -C(=NH)NH₂ oder -NH-C(=NH)NH₂ substituiert sein kann, oder
ein über eine C₁-C₃-Alkylenbrücke verknüpfter, gegebenenfalls ein- oder zweifach durch einen oder mehrere der Reste Methyl, Ethyl, Propyl, Phenyl oder Benzyl substituierter Heterocyclus, ausgewählt aus der Gruppe Pyrrol, Pyrrolin, Pyrrolidin, Pyridin, Piperidin, Pyrimidin, Piperazin, Morpholin, Diazepan, Imidazol, Imidazolin, Imidazolidin, Pyrazol, Pyrazolin, Pyrazolidin, Furan, Tetrahydrofuran, α-Pyran, γ-Pyran, Dioxolan, Tetrahydropyran, Dioxan, Oxazol oder Isoxazol;
- R⁴: Wasserstoff, C₁-C₄-Alkyl, welches durch eine der Gruppen COOH, COO-C₁-C₃-Alkyl oder C₃-C₆-Cycloalkyl substituiert sein kann, oder
Phenyl-C₁-C₃-alkyl, wobei die C₁-C₃-Alkylenbrücke gegebenfalls durch Phenyl substituiert sein kann und wobei der Phenylring gegebenenfalls durch C₁-C₃-Alkyl, C₁-C₃-Alkoxy, CF₃, Fluor, Chlor, Brom, COOH oder COO-C₁-C₃-Alkyl substituiert sein kann, oder
C₃-C₈-Cycloalkyl, Naphthyl oder Phenyl, welches gegebenenfalls ein- oder zweifach durch einen oder mehrere der Reste C₁-C₃-Alkyl, C₁-C₃-Alkoxy, Phenyloxy, Benzyloxy, CF₃, Fluor, Chlor, Brom, COOH oder COO-C₁-C₃-Alkyl substituiert sein kann, oder
ein über eine C₁-C₃-Alkylenbrücke verknüpfter, gegebenenfalls ein- oder zweifach durch einen oder mehrere der Reste Methyl, Ethyl, Propyl, Phenyl, Methylphenyl- oder Benzyl substituierter Heterocyclus, ausgewählt aus der Gruppe Pyrrol, Pyrrolin, Pyrrolidin, Pyridin, Piperidin, Pyrimidin, Piperazin, Morpholin, Thiomorpholin, Imidazol, Imidazolin, Imidazolidin, Pyrazol,
Pyrazolin,-Pyrazolidin, Triazol, Furan, Tetrahydrofuran, α-Pyran, γ-Pyran, Dioxolan, Tetrahydropyran, Dioxan, Thiophen, Dihydrothiophen, Thiolan, Dithiolan,
Oxazol, Isoxazol, Thiazol, Isothiazol, Oxadiazol, Benzodioxol, Benzimidazol, Benzthiophen, Benzofuran oder Indol;
oder
R³ und R⁴ bilden gemeinsam mit dem Stickstoffatom einen 6- oder 7-gliedrigen, gesättigten Heterocyclus, der ein oder zwei weitere Stickstoff-Heteroatome enthält und der gegebenenfalls durch einen oder mehrere der Reste Methyl, Ethyl, Propyl, Benzyl, Cyclopentyl, Cyclohexyl, Pyridyl oder Phenyl, das gegebenenfalls einen Rest ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von besonderer Bedeutung sind Verbindungen der allgemeinen Formel (I), worin
- R¹: Methyl, Ethyl, Propyl, Butyl, Pentyl, Hexyl, Heptyl, Octyl, Nonyl oder Decyl, oder
ein Methyl-, Ethyl- oder Propyl-Rest, der durch Methoxy, Ethoxy, Cyclopropyl, Cyclopentyl, Cyclohexyl, Phenyl, Methoxyphenoxy, -NH₂, -NH(C₁-C₄-Alkyl), -N(C₁-C₄-Alkyl)₂, -NH-CO-Methyl, -CO-NH₂, -CO-NH-Methyl oder -NH-CO-Benzyl substituiert ist, oder
Benzyl, das ein- oder zweifach durch Methyl, Ethyl, Propyl, CF_{3,} Fluor, Chlor, Brom, COOH, COOMe oder COOEt substituiert ist, oder
Phenylethyl, daß ein- oder zweifach durch Methyl, Ethyl, Propyl, CF₃, Fluor, Chlor, Brom, COOH, COOMe oder COOEt substituiert ist, oder
ein über eine Methylen-, Ethylen oder Propylenbrücke verknüpfter, gegebenenfalls ein- oder zweifach durch einen oder mehrere der Reste Methyl, Ethyl, Propyl, Phenyl, Methylphenyl- oder Benzyl substituierter Heterocyclus, ausgewählt aus der Gruppe Pyrrol, Pyrrolidin, Pyridin, Piperidin, Piperazin, Morpholin, Furan, Tetrahydrofuran, Thiophen, Benzodioxol oder Benzimidazol;
- R²: -C(=NH)NH₂ oder -CH₂-NH₂;
- R³: ein Methyl-, Ethyl- oder Propyl-Rest, der durch -NH₂, -NH(C₁-C₃-Alkyl), -N(C₁-C₃-Alkyl)₂, -NHPhenyl, -N(Phenyl)₂, -NHBenzyl, -N(Benzyl)₂, -NH-CO-(C₁-C₃-Alkyl), -NH-CO-Benzyl (?) oder -C(=NH)NH₂ substituiert ist, oder
Benzyl, das direkt oder über eine Methylenbrücke durch eine der Gruppen -NH₂, -NH(C₁-C₃-Alkyl), -N(C₁-C₄-Alkyl)₂, -NHPhenyl, -N(Phenyl)₂, -NHBenzyl, -N(Benzyl)₂, -NH-CO-(C₁-C₃-Alkyl) oder -C(=NH)NH₂ substituiert ist, oder
Phenyl-C₂-C₃-alkyl, wobei die C₂-C₃-Alkylenbrücke gegebenfalls durch Phenyl, COOH oder COO-C₁-C₃-Alkyl substituiert sein kann und
wobei der Phenylring direkt oder über eine Methylenbrücke durch eine der Gruppen -NH₂, -NH(C₁-C₃-Alkyl), -N(C₁-C₃-Alkyl)₂, -NHPhenyl, -N(Phenyl)₂, -NHBenzyl, -N(Benzyl)₂ oder -C(=NH)NH₂ substituiert sein kann, oder
ein über eine Methylen-, Ethylen oder Propylenbrücke verknüpfter, gegebenenfalls ein- oder zweifach durch Methyl, Ethyl, Propyl, Phenyl oder Benzyl substituierter Heterocyclus, ausgewählt aus der Gruppe Pyrrol, Pyrrolidin, Pyridin, Piperidin, Piperazin, Morpholin, Diazepan, Furan, Tetrahydrofuran, Thiophen, Benzodioxol oder Benzimidazol;
- R⁴: Wasserstoff oder ein Methyl-, Ethyl-, Propyl- oder Butyl-Rest, der durch eine der Gruppen COOH, COOMe, COOEt, Cyclopropyl, Cyclopentyl oder Cyclohexyl substituiert sein kann, oder
Benzyl, das gegebenfalls durch Methyl, Ethyl, Propyl, Methoxy, Ethoxy, CF₃, Fluor, Chlor, Brom, COOH, COOMe oder COOEt substituiert sein kann, oder Phenylethyl, Phenylpropyl, Diphenylpropyl;
Cyclopropyl, Cyclopentyl, Cyclohexyl, Cyclooctyl, Naphthyl oder Phenyl, das gegebenenfalls durch Methyl, Ethyl, Propyl, Methoxy, Ethoxy, Phenyloxy, Benzyloxy, CF₃, Fluor, Chlor, Brom, COOH, COOMe oder COOEt substituiert sein kann, oder
ein über eine Methylen-, Ethylen oder Propylenbrücke verknüpfter, gegebenenfalls ein- oder zweifach durch Methyl, Ethyl, Propyl, Phenyl oder Benzyl substituierter Heterocyclus, ausgewählt aus der Gruppe Pyrrol, Pyrrolidin, Pyridin, Piperidin, Piperazin, Morpholin, Furan, Tetrahydrofuran, Thiophen, Chinolin, Isochinolin, Benzodioxol oder Benzimidazol; oder
R³ und R⁴ bilden gemeinsam mit dem Stickstoffatom einen Piperazin- oder Diazepan-Ring, der gegebenenfalls durch einen der Reste Methyl, Ethyl, Propyl, Cyclopentyl, Cyclohexyl, Pyridyl, Benzyl oder Phenyl, das gegebenenfalls einen Rest ausgewählt aus der Gruppe Methyl, Methoxy Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß bevorzugt sind ferner Verbindungen der allgemeinen Formel (I), worin
- R¹: Methyl, Ethyl, Propyl, Pentyl oder n-Decyl,
oder
ein Methyl-, Ethyl- oder Propyl-Rest, der durch Methoxy, Ethoxy, Cyclopropyl, Cyclopentyl, Cyclohexyl, Phenyl oder Methoxyphenoxy substituiert ist, oder
Benzyl, das ein- oder zweifach durch Methyl, CF₃, COOH, COOMe oder COOEt substituiert ist, oder
ein über eine Methylenbrücke verknüpftes Tetrahydrofuran;
- R²: -C(=NH)NH₂ oder -CH₂-NH₂;
- R³: ein Ethyl- oder Propyl-Rest, der durch -NH₂, -NHMe, -NMe₂, -NHEt, -NEt₂, -NHPhenyl, -N(Phenyl)₂, -NHBenzyl, -N(Benzyl)₂ oder -C(=NH)NH₂ substituiert
ist, oder
Benzyl, das durch eine der Gruppen -NH₂, -CH₂-NH₂, -NMe₂, -NHMe, -NEt₂, -NHEt, -NH-CO-Me, -CH₂-NH-CO-Me oder -C(=NH)NH₂ substituiert ist, oder
Phenylethyl, wobei die Ethylenbrücke gegebenfalls durch COOH, COOMe oder COOEt substituiert sein kann und wobei der Phenylring durch eine der Gruppen -NH₂, -CH₂-NH₂, -NMe₂, -NHMe, -NEt₂, -NHEt, -NH-CO-Me, -CH₂-NH-CO-Me oder -C(=NH)NH₂ substituiert ist, oder
Phenylpropyl, Diphenylpropyl oder Pyridylmethyl;
- R⁴: Wasserstoff oder ein Methyl-, Ethyl-, Propyl- oder Butyl-Rest, der durch eine der Gruppen COOH, COOMe, COOEt, Cyclopropyl, Cyclopentyl oder Cyclohexyl substituiert sein kann, oder
Benzyl, das gegebenfalls durch Methyl, Ethyl, Propyl, Methoxy, Ethoxy, CF₃, Fluor, Chlor, Brom, COOH, COOMe oder COOEt substituiert sein kann, oder Phenyl-ethyl, Phenylpropyl, Diphenylpropyl, oder
Cyclopentyl, Cyclohexyl, Cyclooctyl, Naphthyl oder Phenyl, das gegebenenfalls durch Methyl, Ethyl, Methoxy, Ethoxy, Phenyloxy oder CF₃ substituiert sein kann,oder
ein über eine Methylenbrücke verknüpftes Pyridin oder Chinolin, oder
R³ und R⁴ bilden gemeinsam mit dem Stickstoffatom einen Piperazin- oder Diazepan-Ring, der gegebenenfalls durch einen der Reste Cyclopentyl, Cyclohexyl, Pyridyl, Benzyl oder Phenyl, das gegebenenfalls einen der Reste ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß besonders bevorzugt sind Verbindungen der allgemeinen Formel (I), worin
- R¹: Methyl, Ethyl, Propyl, Pentyl, Phenylethyl, Phenylpropyl, Cyclopropylmethyl, Tetrahydrofuranylmethyl oder Benzyl, das ein- oder zweifach durch CF₃, COOH, COOMe oder COOEt substituiert ist;
- R²: -C(=NH)NH₂ oder -CH₂-NH₂, bevorzugt -C(=NH)NH₂;
- R³: ein Ethyl- oder Propyl-Rest, der durch -NH₂, -NHMe, -NMe₂, -NHEt, -NEt₂, -NHPhenyl, -N(Phenyl)₂, -NHBenzyl, -N(Benzyl)₂ oder -C(=NH)NH₂ substituiert
ist, oder
Benzyl, das durch eine der Gruppen -NH₂, -CH₂-NH₂, -NMe₂, -NHMe, -NEt₂, -NHEt, -NH-CO-Me, -CH₂-NH-CO-Me oder -C(=NH)NH₂ substituiert ist, oder
Phenylethyl, wobei die Ethylenbrücke durch COOH, COOMe oder COOEt substituiert ist und wobei der Phenylring eine der Gruppen -NH₂, -CH₂-NH₂, -NMe₂, -NHMe, -NEt₂, -NHEt, -NH-CO-Me, -CH₂-NH-CO-Me oder -C(=NH)NH₂ trägt, oder
Phenylpropyl, Diphenylpropyl oder Pyridylmethyl;
- R⁴: Wasserstoff oder ein Methyl-, Ethyl-, Propyl- oder Butyl-Rest, der durch eine der Gruppen COOH, COOMe, COOEt oder Cyclohexyl substituiert sein kann, oder
Phenyl, das gegebenenfalls durch Methyl, Ethyl, Methoxy, Ethoxy, Phenyloxy oder CF₃ substituiert sein kann, oder
Benzyl, Phenylethyl, Phenylpropyl, Diphenylpropyl, Cyclohexyl, Cyclooctyl oder Naphthyl, oder
ein über eine Methylenbrücke verknüpftes Pyridin oder Chinolin, oder
R³ und R⁴ bilden gemeinsam mit dem Stickstoffatom einen Piperazin- oder Diazepan-Ring, der gegebenenfalls durch einen der Reste Cyclopentyl, Cyclohexyl, Pyridyl, Benzyl oder Phenyl, das gegebenenfalls einen Rest ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Von besonderer Bedeutung sind erfindungsgemäß Verbindungen der allgemeinen Formel (I), worin
- R¹: Methyl;
- R²: -C(=NH)NH₂ oder -CH₂-NH₂, bevorzugt -C(=NH)NH₂;
- R³: Ethyl, das durch -NH₂, -NMe₂, -NHPhenyl, -NHBenzyl, -N(Benzyl)₂, Pyrrolidin, Piperidin, Diazepan oder -C(=NH)NH₂ substituiert ist, Benzyl, das durch eine der Gruppen -CH₂-NH₂, -NMe₂ oder -C(=NH)NH₂ substituiert ist, Phenylethyl, wobei die Ethylenbrücke durch COOH, COOMe oder COOEt substituiert ist und wobei der Phenylring eine der Gruppen -CH₂-NH-CO-Me oder -C(=NH)NH₂ trägt, Diphenylpropyl oder Pyridylmethyl;
- R⁴: Wasserstoff oder ein Methyl- oder Ethyl-Rest, der gegebenfalls durch eine der Gruppen COOH oder COOEt substituiert sein kann, Propyl, Butyl oder Phenyl, das gegebenenfalls durch Methyl, Ethyl, Methoxy, Ethoxy, Phenyloxy oder CF₃ substituiert sein kann, Benzyl, Phenylethyl, Phenylpropyl, Diphenylpropyl, Cyclohexyl, Cyclooctyl, Naphthyl, Pyridylmethyl oder Chinolinylmethyl oder
R³ und R⁴ bilden gemeinsam mit dem Stickstoffatom einen Piperazin- oder Diazepan-Ring, der durch einen der Reste Benzyl, Cyclopentyl, Cyclohexyl oder Phenyl, das gegebenenfalls einen Rest ausgewählt aus der Gruppe Methyl, Ethoxy, Propyloxy oder Hydroxy trägt, substituiert ist, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß bedeutsam sind ferner Verbindungen der allgemeinen Formel (I), worin
- R¹: Methyl;
- R²: -C(=NH)NH₂;
- R³: Ethyl, das durch -NH₂, -NHPhenyl, -NHBenzyl, -N(Benzyl)₂, Pyrrolidin, Piperidin, Diazepan oder -C(=NH)NH₂ substituiert ist, Benzyl, das durch -C(=NH)NH₂ substituiert ist oder Diphenylpropyl;
- R⁴: Wasserstoff, Methyl, Propyl, Butyl, Benzyl oder Phenyl, das gegebenenfalls durch Ethyl oder Phenyloxy substituiert sein kann, Phenylethyl, Cyclohexyl oder Cyclooctyl, oder
R³ und R⁴ bilden gemeinsam mit dem Stickstoffatom einen Piperazin-Ring, der durch einen Rest ausgewählt aus der Gruppe Cyclopentyl, Cyclohexyl, Phenyl, Methylphenyl, Ethoxyphenyl oder Propoxyphenyl substituiert ist, oder einen Diazepan-Ring, der durch Methylphenyl substituiert ist,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Von besonderer Bedeutung sind ferner die erfindungsgemäßen Verbindungen der allgemeinen Formel (I), worin
- R¹: Methyl;
- R²: -C(=NH)NH₂;
- R³: Ethyl, das durch -NH₂, -NHBenzyl, -N(Benzyl)₂, Pyrrolidin, Piperidin, Diazepan oder -C(=NH)NH₂ substituiert ist, Benzyl, das durch -C(=NH)NH₂ substituiert ist oder Diphenylpropyl;
- R⁴: Wasserstoff, Methyl, Butyl oder Phenyl, das gegebenenfalls durch Ethyl oder Phenyloxy substituiert sein kann, Phenylethyl, Cyclohexyl oder Cyclooctyl, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Als erfindungsgemäß besonders bevorzugte Verbindungen seien beispielsweise genannt:
- 2-[2-(4-Amidinophenyl)-ethyl]-1-methyl-benzimidazol-5-yl-carbonsäure-N-(2-amidinoethyl)-N-(2-phenylethyl)-amid;
- 2-[2-(4-Amidinophenyl)-ethyl]-1-methyl-benzimidazol-5-yl-carbonsäure- N-[2-N',N'-dibenzylamino)ethyl]-N-phenyl-amid;
- 2-[2-(4-Amidinophenyl)-ethyl]-1-methyl-benzimidazol-5-yl-carbonsäure- N-(2-amidinoethyl)-N-(3-phenoxy-phenyl)-amid;
- 2-[2-(4-Amidinophenyl)-ethyl]-1-methyl-benzimidazol-5-yl-carbonsäure- N-(2-amidinoethyl)-N-phenyl-amid;
- 2-[2-(4-Amidinophenyl)-ethyl]-1-methyl-benzimidazol-5-yl-carbonsäure- N-(2-amidinoethyl)-N-cyclooctyl-amid;
- 2-[2-(4-Amidinophenyl)-ethyl]-1-methyl-benzimidazol-5-yl-carbonsäure- N-(2-amidinoethyl)-N-(3-ethyl-phenyl)-amid;
- 2-[2-(4-Amidinophenyl)-ethyl]-1-methyl-benzimidazol-5-yl-carbonsäure- [N-(2-(N'-benzylamino)-ethyl)-N-cyclohexyl-amid];
- 2-[2-(4-Amidinophenyl)-ethyl]-1-methyl-benzimidazol-5-yl-carbonsäure-[N-(2-amino-ethyl)-N-cyclohexyl-amid];
- 2-[2-(4-Amidinophenyl)-ethyl]-1-methyl-benzimidazol-5-yl-carbonsäure- N-(3,3-diphenylpropyl)-amid;
- 2-[2-(4-Amidinophenyl)-ethyl]-1-methyl-benzimidazol-5-yl-carbonsäure- N-(4-amidino-benzyl)-N-methyl-amid;
- 2-[2-(4-Amidinophenyl)-ethyl]-1-methyl-benzimidazol-5-yl-carbonsäure- N-(2-amidinoethyl)-N-iso-butyl-amid;
- 2-{2-[4-(Amino-hydroximino-methyl)phenyl]-ethyl}-1-methyl-benzimidazol-5-yl-carbonsäure-[N'-(cyclohexyl)-piperazid];
- 2-[2-(4-Amidinophenyl)-ethyl]-1-methyl-benzimidazol-5-yl-carbonsäure- [N'-(2-methyl-phenyl)-diazepid];

Neben den vorstehend genannten Verbindungen der allgemeinen Formel (I) zielt die vorliegende Erfindung ferner auf Verbindungen, die aufgrund einer in vivo abspaltbaren Funktionalität erst nach ihrer Einnahme durch den Patienten vom Organismus in die therapeutisch wirksamen Verbindungen der allgemeinen Formel (I) überführt werden. Solche Verbindungen werden als Prodrugs bezeichnet. Ein weiterer Aspekt der vorliegenden Erfindung zielt entsprechend auf Prodrugs der allgemeinen Formel (II) 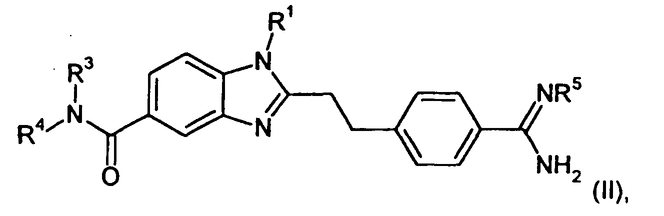 worin
- R¹ und R⁴: die vorstehend genannten Bedeutungen aufweisen können und
- R³: die vorstehend genannten Bedeutungen aufweisen kann oder C₁-C₄-Alkyl bedeutet, welches durch einen Rest ausgewählt aus der Gruppe -C(=NOH)NH₂, -C(=NCOO-C₁-C₄-alkyl)NH₂ oder
-C(=NCOO-C₁-C₄-alkyl-Phenyl)NH₂ substituiert ist;
- R⁵: Hydroxy, -COO-C₁-C₈-Alkyl oder -COO-C₁-C₄-Alkyl-Phenyl,
wobei in der vorstehend genannten Gruppe der Phenylring jeweils durch C₁-C₄-Alkyl oder C₁-C₄-Alkoxy substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Bevorzugt sind Prodrugs der allgemeinen Formel (II), worin
- R¹, R³ und R⁴: die vorstehend genannten Bedeutungen aufweisen können und
- R⁵: Hydroxy, Methoxycarbonyl, Ethoxycarbonyl, Propyloxycarbonyl, Butyloxycarbonyl, Pentyloxycarbonyl, Hexyloxycarbonyl, Benzyloxycarbonyl, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Ein weiterer Aspekt der vorliegenden Erfindung zielt auf Verbindungen der allgemeinen Formel (III) 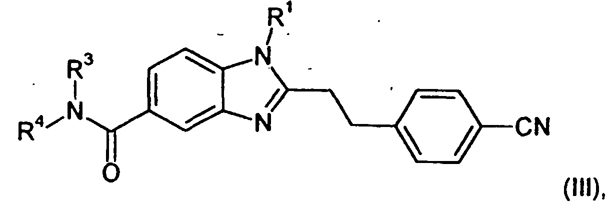 in der die Reste R¹, R³ und R⁴ die vorstehende Bedeutung aufweisen können.

Die Verbindungen der allgemeinen Formel (III), stellen wertvolle Zwischenprodukte zur Herstellung der erfindungsgemäßen aminocarbonyl-substituierten Benzimidazol-Derivate der allgemeinen Formel (I) sowie der der erfindungsgemäßen Prodrugs der allgemeinen Formel (II) dar.

Als Alkylgruppen (auch soweit sie Bestandteil anderer Reste sind) werden verzweigte und unverzweigte Alkylgruppen mit 1 bis 10 Kohlenstoffatomen bevorzugt 1 - 6, besonders bevorzugt 1-4 Kohlenstoffatomen betrachtet, beispielsweise werden genannt: Methyl, Ethyl, Propyl, Butyl, Pentyl, Hexyl, Heptyl und Octyl. Sofern nicht anders genannt, sind von den vorstehend genannten Bezeichnungen Propyl, Butyl, Pentyl, Hexyl, Heptyl und Octyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc. Gegebenfalls werden zur Bezeichnung der vorstehend genannten Alkylreste auch gängige Abkürzungen wie Me für Methyl, Et für Ethyl etc. verwendet.

Als Cycloalkylreste mit 3 - 8 Kohlenstoffatomen werden beispielsweise Cyclopropyl, Cyclobutyl, Cyclopentyl, Cyclohexyl, Cycloheptyl oder Cyxclooctyl bezeichnet. Als Halogen wird im allgemeinen Fluor, Chlor, Brom oder Jod bezeichnet.

Als Beispiele für N-verknüpfte cyclische Reste der allgemeinen Formel NR³R⁴ werden genannt: Pyrrol, Pyrrolin, Pyrrolidin, 2-Methylpyrrolidin, 3-Methylpyrrolidin, Piperidin, Piperazin, Morpholin, Thiomorpholin, Imidazol, Imidazolin, Imidazolidin, Pyrazol, Pyrazolin, Pyrazolidin oder Diazepan, die wie in den Definitionen angegeben substituiert sein können.

Als 5- ,6- oder 7-gliedrige, gesättigte oder ungesättigte Heterocyclen, die als Heteroatome Stickstoff, Sauerstoff oder Schwefel enthalten können, werden, soweit in den Definitionen nicht anders beschrieben beispielsweise Furan, Tetrahydrofuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Pyrazolidin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Diazepan, Oxazol, Isoxazol, Oxazin, Thiazol, Isothiazol, Thiadiazol, Oxadiazol, Pyrazolidin genannt, wobei der Heterocyclus wie in den Definitionen angegeben substituiert sein kann.

"=O" bedeutet ein über eine Doppelbindung verknüpftes Sauerstoffatom.

Die Synthese aminocarbonyl-substituierter Benzimidazol-Derivate der Formel (I) sowie die der Prodrugs der allgemeinen Formel (II) gelingt in Anlehnung an aus dem Stand der Technik bekannte synthetische Zugänge. Diesbezüglich sei beispielsweise auf die bereits eingangs genannte Internationale Patentanmeldung WO 98/37075 verwiesen, auf die an dieser Stelle inhaltlich Bezug genommen wird.

Ein möglicher Zugang zu den erfindungsgemäßen Verbindungen in Anlehnung an und unter Verwendung von konventionellen chemischen Synthesemethoden ist schematisch im Folgenden dargestellt (Schema 1).

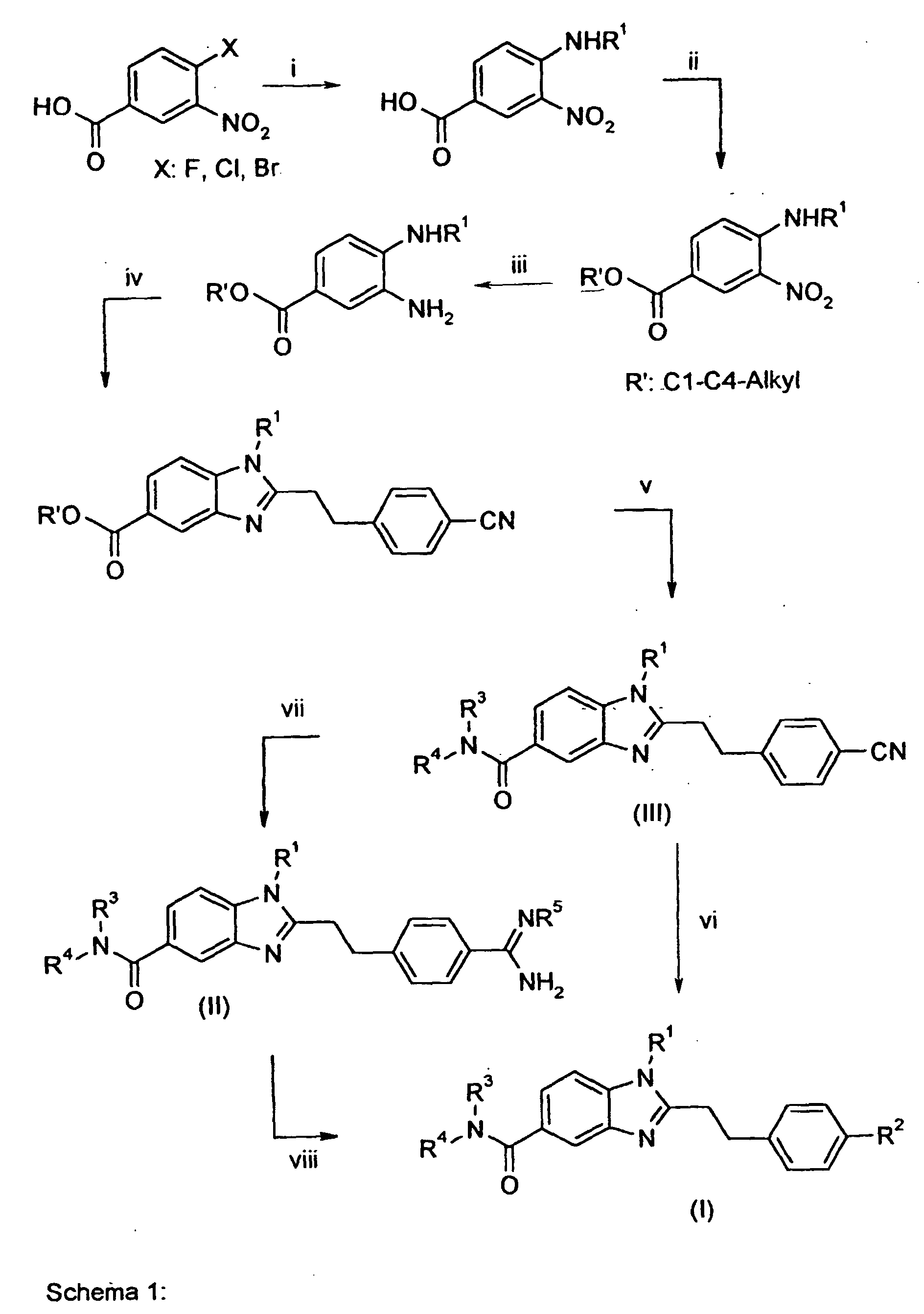

In einem ersten Syntheseschritt (Stufe i, Schema 1) gelingt ausgehend von 4-Halogen-3-nitro-benzoesäure-derivaten durch Aminolyse mit geeignet substituierten primären Aminen die Synthese von 4-Amino-3-nitrobenzoesäure-derivaten. Die Umsetzung erfolgt in geeigneten organischen Lösemitteln wie beispielsweise Dimethylsulfoxid, N,N-Dimethylformamid, N-Methylpyrrolidon oder gegebenenfalls auch in Wasser bei Raumtemperetur oder in einem Temperaturbereich von 30-80°C, bevorzugt 40-50°C. Die so erhaltenen Aminobenzoesäure-Verbindungen werden über konventionelle Standardverfahren in die entsprechenden Alkylester, vorzugsweise in die entsprechenden Methylester oder Ethylester überführt (Stufe ii, Schema 1). Die Reduktion der Nitro-gruppe zu den Diaminobenzoesäurealkylestern gelingt vorzugsweise durch katalytische Hydrierungen gemäß Stufe iii (Schema 1). Als Katalysator kommt vorzugsweise Palladium in Betracht. Besonders bevorzugt ist als Katalysator Palladium auf Kohle (5%). Durch Umsetzung der so erhaltenen Diaminobenzoesäureester mit p-Cyanophenylpropionsäure in Gegenwart dehydratisierender Reagentien wird gemäß Stufe v (Schema 1) der Benzimidazolheterocyclus gebildet. Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan durchgeführt. Als dehydratisierende Mittel kommen beispielsweise in Betracht Chlorameisensäureisobutylester, Orthokohlensäuretetraethylester, Orthoessigsäuretrimethylester, 2,2-Dimethoxypropan, Tetramethoxysilan, Phosphoroxychlorid, Thionylchlorid, Trimethylchlorsilan, Phosphortrichlorid, Phosphorpentoxid, 1,2-Dihydro-2-ethoxy-chinolin-1-carbonsäureethylester (EEDQ), 1,2-Dihydro-2-*i*-propyloxy-chinolin-1-carbonsäure-*i*-propylester (IIDQ), N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff. Gegebenenfalls kann sich der Zusatz einer Base wie Pyridin, 4-Dimethylaminopyridin, N-Methyl-morpholin oder Triethylamin als zweckmäßig erweisen. Die Umsetzung erfolgt üblicherweise bei Temperaturen zwischen 0 und 150°C, vorzugsweise bei Temperaturen zwischen 0 und 110°C.

Die gemäß Stufe v (Schema 1) erhältlichen Benzimidazolderivate der allgemeinen Formel (III) sind entweder direkt aus den vorstehend genannten Benzimidazolcarbonsäureestern zugänglich oder werden über die entsprechenden Carbonsäuren oder Carbonsäurehalogenide erhalten.

Werden die gemäß Stufe iv (Schema 1) erhaltenen Carbonsäureester unter Standardbedingungen (protisches organisches Lösemittel wie beispielsweise Methanol, Ethanol oder Isopropanol, gegebenenfalls in Gegenwart von Wasser in Anwesenheit von Basen wie Hydroxiden oder Carbonaten der Alkali- und Erdalkalimetalle) verseift, führt dies zu den entsprechenden freien Carbonsäuren. Üblicherweise wird diese Verseifung bei Temperaturen zwischen 0-40°C, bevorzugt bei 10-30°C durchgeführt. Gegebenenfalls kann die Synthese aber auch bei erhöhter Temperatur (>50°C bis Rückflußtemperatur) durchgeführt werden.
Erfindungsgemäß bevorzugt ist als Lösemittel ein Methanol-Wasser-gemisch. Als Base gelangt bevorzugt Natriumhydroxid zur Anwendung. Die Umsetzung der so erhaltenen Säure mit den Aminen H-NR³R⁴ zu den Verbindungen der allgemeinen Formel (III) wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder in dem entsprechenden Amin H-NR³R⁴, gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N, N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Die Synthese der Verbindungen der allgemeinen Formel (III) ausgehend von den gemäß Schema 1 (Stufe iv) erhaltenen Carbonosäureestern oder von den entsprechenden Carbonsäurechloriden wird entweder in dem entsprechenden Amin H-NR³R⁴ als Lösungsmittel, oder mit dem Amin H-NR³R⁴ in Gegenwart eines Lösemittels wie Methylenchlorid, Ether oder Ethylacetat und vorzugsweise in Gegenwart einer tertiären organische Base wie Triethylamin, N-Ethyl-diisopropylamin oder N-Methyl-morpholin bei Temperaturen zwischen 0 und 150°C, vorzugsweise bei Temperaturen zwischen 40 und 100°C, durchgeführt.

Eine Verbindung der allgemeinen Formel (I) erhält man beispielsweise durch Behandlung einer Verbindung der allgemeinen Formel (III, Schema 1, Stufe vi) mit einem entsprechenden Alkohol wie Methanol, Ethanol, n-Propanol, Isopropanol oder Benzylalkohol gegebenenfalls im Gemisch mit einem anderen organischen Lösungsmittel wie beispielsweise Chloroform, Nitrobenzol oder Toluol in Gegenwart einer Säure wie Salzsäure oder durch Umsetzung eines entsprechenden Amids mit einem Trialkyloxoniumsalz wie Triethyloxonium-tetrafluorborat in einem Lösemittel wie Methylenchlorid, Tetrahydrofuran oder Dioxan bei Temperaturen zwischen -10 und 50°C, vorzugsweise jedoch bei 0-20°C. Alternativ dazu lassen sich die Verbindungen der allgemeinen Formel (I) erhalten durch Umsetzung einer Verbindung der allgemeinen Formel (III, Schema 1, Stufe vi) mit Schwefelnukleophilen wie z.B. Schwefelwasserstoff, Ammonium- bzw. Natriumsulfid, Natriumhydrogensulfid, Kohlenstoffdisulfid, Thioacetamid oder Bistrimethylsilylthioether gegebenenfalls in Gegenwart von Basen wie Triethylamin, Ammoniak, Natriumhydrid oder Natriumalkoholat in Lösungsmitteln wie Methanol, Ethanol, Wasser, Tetrahydrofuran, Pyridin , Dimethylformamid oder 1,3-Dimethyl-imidazolidin-2-on bei 20-100 °C und anschließende Behandlung mit einem geeigneten Methylierungsmittel wie z.B. Methyliodid oder Dimethylsulfat in einem Lösungsmittel wie Acetonitril oder Aceton bei Temperaturen zwischen -10 und 50°C, vorzugsweise jedoch bei 0-20°C und anschließende Behandlung mit Ammoniak, Ammoniumcarbonat oder Ammoniumchlorid in einem geeigneten Alkohol, wie beispielsweise Methanol, Ethanol, Isopropanol etc. bei Temperaturen zwischen -10 und 50°C, vorzugsweise jedoch bei 0-20°C.

Ferner sind die erfindungsgemäßen Verbindungen der allgemeinen Formel (I) zugänglich durch Behandlung einer Verbindung der allgemeinen Formel (III) mit Lithiumhexamethyldisilazid in einem geeigneten organischen Lösungsmittel wie z.B. Tetrahydrofuran bei Temperaturen zwischen -20 und 50 °C, vorzugsweise jedoch bei 0-20 °C und anschließende Hydrolyse mit verdünnter Salzsäure bei 0-5 °C.
Ein weiterer alternativer Zugang zu Verbindungen der allgemeinen Formel (I) gelingt durch Behandlung einer Verbindung der allgemeinen Formel (III) mit Ammoniumchlorid und Trimethylaluminium in einem geeigneten organischen Lösungsmittel wie z.B. Toluol bei Temperaturen zwischen 20 und 150 °C, vorzugsweise jedoch bei 110 °C.

Eine Verbindung der allgemeinen Formel (II) erhält man beispielsweise durch Behandlung einer Verbindung der allgemeinen Formel (III, Schema 1, Stufe vii) mit Hydroxylamin in Gegenwart von Carbonaten oder Alkoholaten der Alkali- oder Erdalkalimetalle in Lösemitteln wie Methanol, Ethanol, n-Propanol oder Isopropanol gegebenenfalls im Gemisch mit Dioxan oder Tetrahydrofuran. Die Alkoholate können dargestellt werden aus den jeweiligen Alkalimetallen oder Metallhydriden und dem entsprechenden Alkohol. Die Reaktion wird vorzugsweise bei 20-100°C, besonders bevorzugt bei der Siedetemperatur des verwendeten Lösemittels durchgeführt.
Verbindungen der allgemeinen Formel (II) sind alternativ zugänglich durch Behandlung einer Verbindung der allgemeinen Formel (III, Schema 1, Stufe vii) mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösemittel wie Methylenchlorid, Tetrahydrofuran oder Dioxan bei Temperaturen zwischen -10 und 50°C, vorzugsweise jedoch bei 0-20°C und anschließende Behandlung mit Hydroxylamin in Gegenwart von Basen in einem geeigneten Alkohol, wie Methanol, Ethanol, Isopropanol etc. bei Temperaturen zwischen -10 und 50°C, vorzugsweise jedoch bei 0-20°C.

Eine Verbindung der allgemeinen Formel (I) erhält man beispielsweise durch Behandlung einer Verbindung der allgemeinen Formel (II, Schema 1, Stufe viii) mit Wasserstoff in Gegenwart von Hydrierkatalysatoren wie Raney-Nickel oder Rhodium/Aluminiumoxid in Wasser oder Methanol gegebenenfalls unter Zusatz von Säuren wie Salzsäure oder Methansulfonsäure oder durch Behandlung mit Wasserstoff in Gegenwart von Palladium/Kohle in Essigsäure/Essigsäureanhydrid bei 20-50 °C und 1-5 bar Wasserstoffdruck, bevorzugt bei Raumtemperatur und Normaldruck,

Acyl- oder Alkoxycarbonyl-Prodrugs der Verbindung mit der allgemeinen Formel (I) erhält man durch Umsetzung der Verbindungen der allgemeinen Formel (I) mit den entsprechenden Säurechloriden in Gegenwart von Basen wie z.B. Triethylamin, N-Methylmorpholin, Diethylisopropylamin oder DBU in einem geeigneten Lösungsmittel wie Methylenchlorid, Chloroform, Tetrahydrofuran, Acetonitril, Dimethylformamid oder Dimethylsulfoxid.

Alternativ zu vorstehend genannter Methodik können die erfindungsgemäßen Verbindungen auch an einem polymeren Träger über einen Festphasensynthetischen Zugang erhalten werden. Der synthetische Zugang über Festphasensynthese, wie er in exemplarischer und den Kern der Erfindung als nicht beschränkend anzusehenden Art und Weise in Schema 2 skizziert ist, ist vor allem für solche erfindungsgemäßen Verbindungen von Interesse, in denen der Rest R³ endständig Amino-substituiert ist.

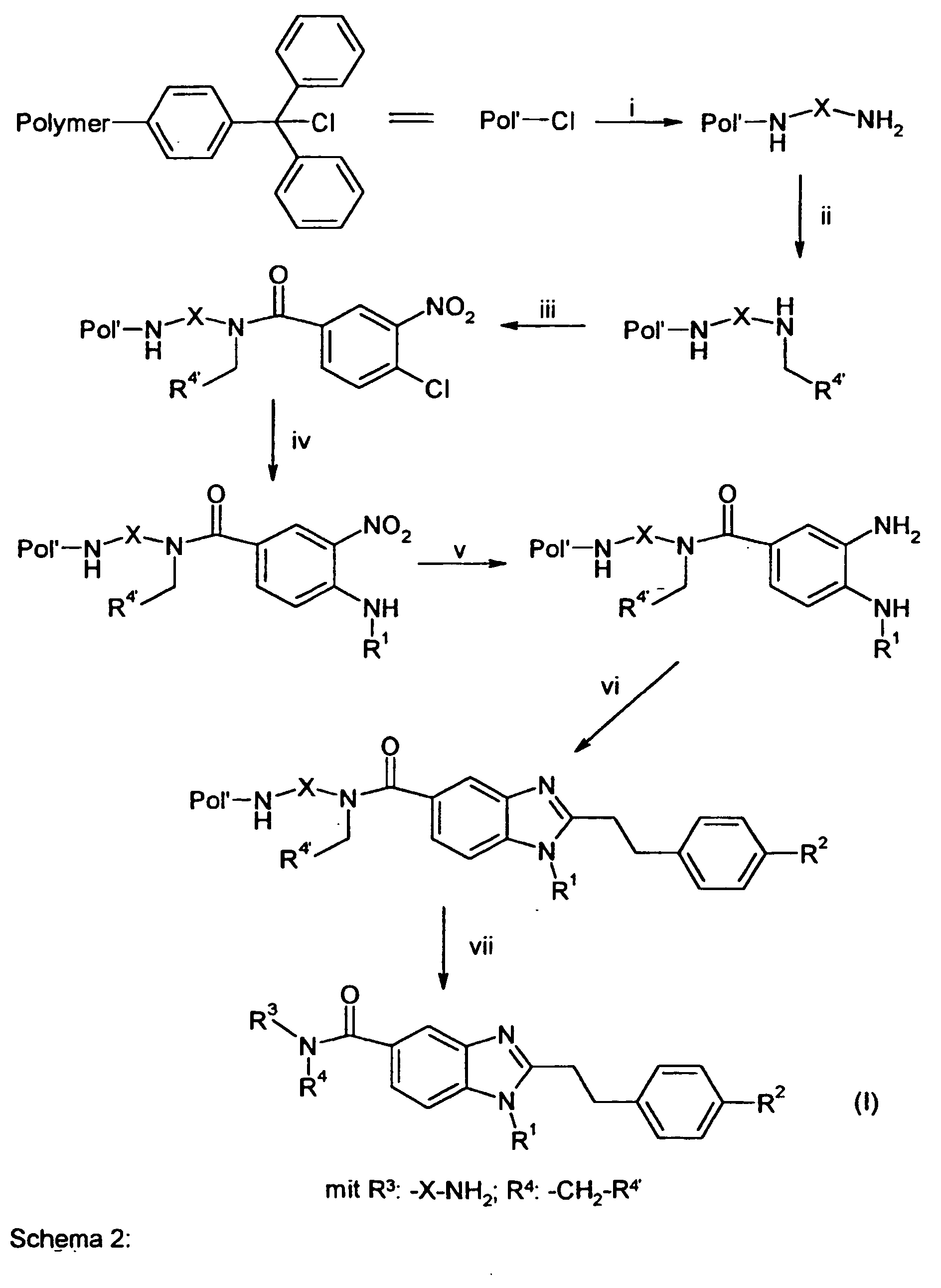

In einem ersten Syntheseschritt (Schema 2, Stufe i) erfolgt die Umsetzung eines Harz gebundenen Diamins. Als Harze kommen üblicherweise in Betracht Trityl-, 2-Chlortrityl-, 4-Methoxytrityl-Harze mit Polymermatrices von quervernetztem Polystyrol oder Tentagel oder auch Synphase™ crowns. Zur Durchführung des Syntheseschritts gemäß Schema 2 (Stufe i) wird erfindungsgemäß wie folgt vorgangen. 2-4 Äquivalente des Diamins, basierend auf der Harzbeladung, werden in einem organischen Lösemittel ausgewählt aus der Gruppe Dichlormethan Tetrahydrofuran, 1,2-Dichlorethan oder Dimetyhlformamid gelöst. Die Lösung wird zu dem Tritylharz gegeben und für 2-16 h bei Raumtemperatur gerührt. Anschließend wird das Harz abfiltriert und mehrmals mit trockenem Pyridin oder auch einem Gemisch aus Dichlormethan / Diisopropylamin und Dichlormethan oder trockenem Diethylether gewaschen. Das Harz wird bis zur Gewichtskonstanz im Vakuum getrocknet.
In einem zweiten Verfahrensschritt (Schema 2, Stufe ii) wird das an das Harz gekoppelte Diamin im Sinne einer reduktiven Aminierung mit den Aldehyden R⁴-CHO, wobei R⁴ die vorstehend definierten Bedeutungen aufweisen kann, umgesetzt. Hierzu wird erfindungsgemäß wie folgt vorgegangen. 2-10 Äquivalente des Aldehyds R⁴-CHO, gelöst in Tetramethylorthoformiat / Dichlormethan oder Dichlormethan oder Dichlormethan / Dimethylformamid oder Dimethylformamid oder 1,2-Dichlormethan, werden zu Diamin beladenem Harz, suspendiert in Tetramethylorthoformiat oder Dichlormethan, Dimethylformamid, 1,2-Dichlormethan, 1-Methyl-2-pyrrolidon (jeweils mit Tetramethylorthoformiat Zusatz) gegeben und 2-12 h bei 0-30°C geschüttelt. Anschließend wird das Harz gewaschen und mit einer Lösung von Natriumtriacetoxyborhydrid oder Natriumcyanborhydrid in Dichlormethan oder DMF oder 1,2-Dichlormethan bei 0-30°C versetzt und 2-24 h geschüttelt. Alternativ kann der Aldehyd und das Reduktionsmittel direkt zum Harz zugegeben werden.
Im dritten Syntheseschritt erfolgt die Umsetzung des an das Harz gekuppelten Amins mit 4-Chlor-3-nitrobenzoylchlorid im Sinne einer Acylierungsreaktion (Schema 2, Stufe iii). Hierzu wird erfindungsgemäß wie folgt vorgegangen. Das Harz wird in Dichlormethan oder Dimethylformamid oder 1,2-Dichlormethan oder 1-Methyl-2-pyrrolidon oder Tetrahydrofuran mit Basenzusatz wie Triethylamin oder Diisopropylethylamin oder Pyridin suspendiert und bei Raumtemperatur mit einer Lösung von 4-Chlor-3-nitro-benzoylchlorid in Dichlormethan oder 1,2-Dichlorethan oder 1-Methyl-2-pyrrolidon oder Dimethylformamid oder Tetrahydrofuran versetzt und 1 - 12 h bei Raumtemperatur geschüttelt. Anschließend wird abfiltriert und mit verschiedenen Lösungsmitteln gewaschen. Alternativ zu der in Schema 2 exemplarisch dargestellten Vorgehensweise ist an Stelle des 4-Chlor-3-nitro-benzoylchlorids auch beispielsweise 4-Fluor-3-nitro-benzoylchlorid einsetzbar. Nachfolgend erfolgt eine Nucleophile Substitution am an das Harz gekoppelten Benzoesäureamid durch die primären Amine R¹-NH₂. (Schema 2, Stufe iv). Hierzu wird erfindungsgemäß wie folgt vorgegangen. Das Harz, in Diisopropylethylamin-Lösung oder in 1-Methyl-2-pyrrolidon oder Dimethylformamid (20% v/v) suspendiert, wird mit einer Lösung eines Amins R¹-NH₂ in 1-Methyl-2-pyrrolidon oder Dimethylforrmamid versetzt und 2-24 h in einem Temperaturbereich von 50-110°C erhitzt. Nach Abkühlen auf Raumtemperatur wird das Harz abfiltriert und mit verschiedenen Lösungsmitteln gewaschen.
Die Reduktion der Nitrogruppe führt gemäß Stufe v (Schema 2) zu den an das Harz gekoppelten Diaminobenzoesäureamiden. Hierzu wird erfindungsgemäß wie folgt vorgegangen. Das Harz wird in Dimethylformamid oder 1-Methyl-2-pyrrolidon suspendiert, mit 5-50 Äquivalenten 1.0 M SnCl₂ -Lösung in Dimethylformamid oder in 1-Methyl-2-pyrrolidon versetzt und 12-48 h bei Raumtemperatur geschüttelt. Anschließend wird das Harz abfiltriert und intensiv mit verschiedenen Lösungsmitteln gewaschen. Alternativ zur Reduktion mit vorstehend genannten SnCl₂-Lösungen kann die Umsetzung auch beispielsweise mit Natriumborhydrid/Cu(acac)₂ (cat.) oder Na₂S₂O₄ in protischen organischen Lösungsmitteln wie Alkoholen, vorzugsweise in Ethanol, durchgeführt werden. Durch Umsetzung mit den Aldehyden R²-C₆H₄-CH₂C-H₂-CHO nach Stufe vi (Schema 2) werden im Sinne einer oxidativen Cyclisierung die Harz-gekuppelten Benzimidazolheterocyclen erhalten. Hierzu wird erfindungsgemäß wie folgt vorgegangen. Das Harz wird in Tetrahydrofuran oder Dioxan oder 1-Methyl-2-pyrrolidon suspendiert, mit einer Lösung eines Aldehyds R²-C₆H₄-CH₂CH₂-CHO in THF oder Dioxan oder 1-Methyl-2-pyrrolidon versetzt und 12-48 h bei Raumtemperatur unter Luftsauerstoff geschüttelt. Anschließend wird das Harz abfiltriert und gewaschen.
Nach Abspaltung vom Harz sind so die erfindungsgemäßen Verbindungen der allgemeinen Formel (I) zugänglich (Schema 2, Stufe vii). Zur Abspaltung wird erfindungsgemäß wie folgt vorgegangen. Das Harz wird mit Trifluoressigsäure (10 - 70% v/v) in Dichlormethan, 1 h bei Raumtemperatur geschüttelt und abgesaugt. Anschließend wird das zurückbleibende Harz nochmals mit Trifluoressigsäure (10-70% v/v) in Dichlormethan versetzt, abgesaugt und die vereinigten Filtrate im Vakuüm eingeengt. Nach Behandlung des Harzrückstandes mit einem Gemisch aus Dichlormethan / Methanol wird nach 1 h Schütteln bei Raumtemperatur abfiltriert. Die Filtrate und die erhaltenen Rückstande werden vereinigt und im Vakuum zur Trockene eingeengt.

Im Folgenden werden exemplarische Vorgehensweisen zur Herstellung der erfindungsgemäßen Verbindungen detaillierter beschrieben. Die nachfolgenden Beispiele dienen ausschließlich der detaillierteren Erläuterung, ohne den Gegenstand der Erfindung zu beschränken.

### Beispiel 1: 2-[2-(4-Amidinophenyl)ethyl]-1-methyl-benzimidazol-5-yl-carbonsäure-N-(pyridin-3-yl-methyl)-N-methyl-amid-hydrochlorid

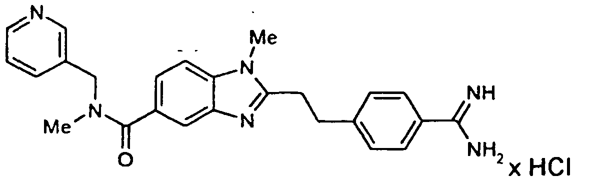

a) 4-Methylamino-3-nitrobenzoesäure:
   20 g (100 mmol) 4-Chlor-3-nitro-benzoesäure werden in 80 mL 40 %iger wäßriger. Methylaminlösung aufgenommen, 15 h bei Raumtemperatur und 1.5 h bei 40 - 50 °C gerührt. Nach dem Abkühlen wird mit Essigsäure angesäuert. Die sich bildenden Kristalle werden abfiltriert, mit kaltem Wasser gewaschen und getrocknet. Ausbeute: 18.2 g (93 %); Schmp.: > 220 °C
b) 4-Methylamino-3-nitro-benzoesäure-methylester:
   9.8 g (50 mmol) 4-Methylamino-3-nitro-benzosäure werden in 50 mL DMF mit K₂CO₃ (14 g) versetzt. Zu dieser Suspension werden innerhalb von 10 min. unter Rühren 5 mL Dimethylsulfat zugetropft. Die Temperatur steigt dabei auf ca. 35 °C an. Es wird 15 Minuten gerührt und anschließend für 0.5 h auf 60 °C erhitzt. Nach dem Abkühlen wird mit Wasser verdünnt, der ausgefallene Feststoff abfiltriert, mit Wasser gewaschen und getrocknet. Ausbeute: 9.8 g (93 %); Schmp.: 138-140 °C;
c) 3-Amino-4-methylamino-benzoesäure-methylester:
   71 g 4-Methylamino-3-nitro-benzoesäure-methylester (338 mmol) werden in 1,4 L Methanol und 67 mL konzentrierter wässriger Salzsäure in Gegenwart von 15 g Pd/C (5%) bei 2-5 bar unter Raumtemperatur hydriert. Nach Abfiltrieren des Katalysators und Abdestillieren des Lösemittels im Vakuum wird der Rückstand in 200 mL Wasser aufgenommen, mit Ethylacetat überschichtet und mit 50-%iger wässriger Kaliumcarbonatlösung alkalisch gestellt. Das Produkt wird in die organische Phase extrahiert, diese wird nochmals mit Wasser gewaschen und abschließend über Natriumsulfat getrocknet. Nach Abdestillieren des größten Teils des Lösemittels im Vakuum wird mit Diethylether versetzt und abgekühlt. Die entstehenden Kristalle werden abfiltriert. Ausbeute: 54 g (81 %); Schmp.: 215-220 °C (Zersetzung);
d) 2-[2-(4-Cyanophenyl)-ethyl]-1-methyl-benzimidazol-5-yl-carbonsäure-methylester: 7.5 g 3-Amino-4-methylamino-benzoesäure-methylester (42 mmol) und 7.3 g *p*-Cyano-phenylpropionsäure (42 mmol) werden in 50 mL Phosphoroxychlorid aufgenommen und 2 h unter Rückfluß erhitzt. Nach dem Abkühlen wird das überschüssige Phosphoroxychlorid mit Eiswasser zersetzt. Es wird mit Ethylacetat überschichtet und mit Kaliumcarbonat unter Rühren alkalisch gestellt. Die organische Phase wird abgetrennt, mit Wasser gewaschen und getrocknet. Nach Abdestillieren des größten Teils des Lösemittels im Vakuum wird abgekühlt. Die ausfallenden Kristalle werden abfiltriert und mit kaltem Ethylacetat oder Diethylether gewaschen. Ausbeute: 8.5 g (63 %); Schmp.: 148-150 °C;
   Masse: ber.: [319], gef.: [M+H]⁺ 320, [M+Na]⁺ 342, [2M+H]⁺ 639;
   ¹H-NMR (250MHz, DMSO-d6): δ [ppm] = 8.18 (7H, m, aryl-H); 3.86 (3H, s, OCH₃); 3.75 (3H, s, aryl-N-CH₃); 3.26 (4H, s, aryl-CH₂-CH₂-).
e) 2-[2-(4-Cyanophenyl)-ethyl]-1-methyl-benzimidazol-5-yl-carbonsäure: 5.0 g 2-[2-(4-Cyanophenyl)-ethyl]-1-methyl-benzimidazol-5-yl-carbonsäuremethylester (15.7 mmol) werden in 50 mL Methanol aufgenommen, mit 20 mL wässriger Natriumhydroxidlösung (1N) versetzt und 0.5h unter Rückfluß gekocht. Es wird mit 20 mL wässriger Salzsäure (1N) versetzt und mit Wasser verdünnt. Die ausfallenden Kristalle werden abfiltriert , mit Wasser, Aceton und Ether gewaschen. Das erhaltene Rohprodukt wird aus Dimethylformamid umkristallisiert.
   Ausbeute: 4.5 g (94 %); Schmp.: > 220 °C ;
f) 2-[2-(4-Cyanophenyl)-ethyl]-1-methyl-benzimidazol-5-yl-carbonsäure-N-(pyridin-3-yl-methyl)-N-methyl-amid:
   1.2 g 2-[2-(4-Cyanophenyl)-ethyl]-1-methyl-benzimidazol-5-yl-carbonsäure (4 mmol), 3-(Methylaminomethyl)-pyridin (0.49 g, 4 mmol) und 0,7 mL N-Methylmorpholin werden in 20 mL Dimethylformamid aufgenommen. Anschließend werden 1.6 g O-(Benzotriazol-1-yl)-N,N,N',N'-tetramethyluroniumtetrafluoroborat (= TBTU; 5 mmol) zugesetzt und 16 h bei RT gerührt. Nach dem Verdünnen mit 75 mL Ethylacetat wird mit gesättigter, wässriger Natriumhydrogencarbonatlösung und mit Wasser gewaschen und über Natriumsulfat getrocknet. Nach Abdestillieren des größten Teils des Lösemittels wird der ausfallende Niederschlag abfiltriert und mit Ether gewaschen. Ausbeute: 1.2 g (73 %); Schmp.: 150-153 °C;
g) 2-[2-(4-Amidinophenyl)-ethyl]-1-methyl-benzimidazol-5-yl-carbonsäure-N-(pyridin-3-yl-methyl)-N-methyl-amid-hydrochlorid:
   1.1 g 2-[2-(4-Cyanophenyl)-ethyl]-1-methyl-benzimidazol-5-yl-carbonsäure-[N-(pyridin-3-yl-methyl)-N-methyl-amid (2.7 mmol) werden in 25 mL einer gekühlten und bei 0 °C gesättigten, ethanolischen HCl-Lösung aufgenommen. Es wird bis zur vollständigen Auflösung des Eduktes gerührt und die Temperatur ca. 12h bei 0-5 °C gehalten. Das Ethanol wird bei maximal 40 °C abdestilliert und der Rückstand in 30 mL einer bei 0 °C gesättigten, ethanolischen Ammoniak-Lösung aufgenommen. Es wird 1 h bei Raumtemperatur und 2h bei 40-50 °C gerührt, mit weiteren 10 mL vorstehend gennater Ammoniak-Lösung versetzt, 1 h unter Rückfluß gekocht und 12h bei Raumtemperatur stehen gelassen. Die ausgefallenen anorganischen Salze werden abfiltriert, das Filtrat auf die Hälfte eingeengt und mit 50 mL Aceton verdünnt. Die ausgefallenen Kristalle werden abfiltriert und mit Aceton gewaschen. Ausbeute: 1,0 g (80%); Schmp.: > 220 °C
   Masse: ber.: [426], gef.: [M+H]⁺ 427, [M+2H]²⁺ 214;
   ¹H-NMR (250MHz, DMSO-d6): δ [ppm] = 8.64-7.26 (11H, m, aryl-/pyridyl-H); 4.79 (2H, s, N-CH₂-); 3.72 (3H, s, aryl-N-CH₃); 3.30 (4H, s, aryl-CH₂-CH₂-); 3.02 (3H, s, CO-N-CH₃).

### Beispiel 2: 2-[2-(4-Amidinophenyl)ethyl]-1-methyl-benzimidazol-5-yl-carbonsäure-N-[2-(N,N-dibenzylamino)ethyl]-N-cyclohexyl-amid-dihydrochlorid

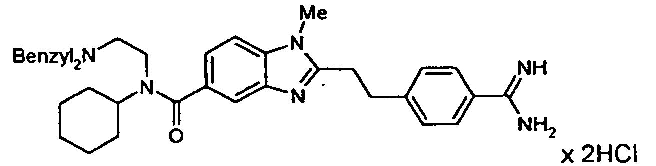

a) 2-[2-(4-Cyanophenyl)-ethyl]-1-methyl-benzimidazol-5-yl-carbonsäurechlorid: 7.0g (23 mmol) 2-[2-(4-Cyanophenyl)-ethyl]-1-methyl-benzimidazol-5-yl-carbonsäure (erhältlich gemäß Beispiel 1, Stufe e), 2 Tropfen Diemthylformamid und 70 mL Thionylchlorid werden 3h unter Rückfluß gekocht. Das überschüssige Thionylchlorid wird abdestilliert und der verbleibende feste Rückstand in Acetonitril/Diethylether aufgenommen und filtriert. Der abfiltrierte Feststoff wird mit Diethylether gewaschen. Ausbeute: 7.8 g (94 %);
b) 2-[2-(4-Cyanophenyl)-ethyl]-1-methyl-benzimidazol-5-yl-carbonsäure-N-[2-(N',N'-dibenzylamino)-ethyl]-N-cyclohexyl-amid:
   3.2 g N',N'-Dibenzyl-N-cyclohexyl-ethylendiamin (10 mmol) und 3 mL N-Methylmorpholin werden in 75 mL Ethylacetat unter Rühren bei RT portionsweise mit 4.0 g (11 mmol) 2-[2-(4-Cyanophenyl)-ethyl]-1-methyl-benzimidazol-5-yl-carbonsäurechlorid versetzt. Es wird 0.5h bei 40-50 °C gerührt, nach dem Abkühlen auf Wasser gegossen und mit Ethylacetat extrahiert. Die organische Phase wird mit verdünnter wässriger Natriumhydroxid-Lösung und mit Wasser gewaschen, über Natriumsulfat getrocknet und der größte Teil des Lösemittels im Vakuum abdestilliert. Es wird mit Diethylether verdünnt und abgekühlt. Die ausgefallenen Kristalle werden abfiltriert und mit Diethylether gewaschen.
   Ausbeute: 5.7 g (94 %); Schmp.: 124-126 °C;
c) 2-[2-(4-Amidinophenyl)-ethyl]-1-methyl-benzimidazol-5-yl-carbonsäure-N-[2-(N',N'-dibenzylamino)-ethyl]-N-cyclohexyl-amid-dihydrochlorid:
   Die Umsetzung erfolgte ausgehend von 9.4 mmol 2-[2-(4-Cyanophenyl)-ethyl]-1-methyl-benzimidazol-5-yl-carbonsäure-N-[2-(N',N'-dibenzylamino)-ethyl]-N-cyclohexyl-amid in Analogie zu Beispiel 1, Stufe g. Das Produkt wird erhalten durch Kristallisation aus Aceton mit etwas Wasser. Ausbeute: 67 %;
   Schmp.: 140-148 °C (Dihydrochlorid, enthält Kristallwasser);
   Masse: ber.: [626], gef.: [M+H]⁺ 627, [M+2H]²⁺ 314;
   ¹H-NMR (250MHz, DMSO-d6): δ [ppm] = 9.35, 9.10 (4H, 2s, C(=NH₂⁺)NH₂); 7.86-7.01 (17H, m, aryl-H); 3.66, 2.60 (4H, 2m, N-CH₂-CH₂-N); 3.75 (3H, s, aryl-N-CH₃); 3.62 (1H, m, N-cyclohexyl-H); 3.26 (4H, s, aryl-CH₂-CH₂-); 1.71-054 (10H, m, cyclohexyl).

### Beispiel 3: 2-[2-(4-Amidinophenyl)-ethyl]-1-methyl-benzimidazol-5-yl-carbonsäureN-[2-(N,N-dimethylamino)-ethyl]-N-methyl-amid-dihydrochlorid

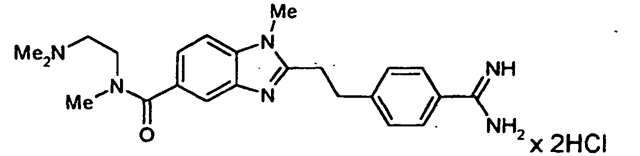

a) 2-[2-(4-Cyanophenyl)-ethyl]-1-methyl-benzimidazol-5-yl-carbonsäure-N-[2-(N,N-dimethylamino)-ethyl]-N-methyl-amid:
   Die Synthese erfolgt in Analogie zu Stufe f (Beispiel 1) durch Umsetzung von 5 mmol 2-[2-(4-Cyanophenyl)-ethyl]-1-methyl-benzimidazol-5-yl-carbonsäure mit N,N,N'-Trimethylethylendiamin. Das Produkt wird aus Ethylacetat/Diethylether kristallisiert. Ausbeute: 62 %; Schmp.: 130-132 °C;
b) 2-[2-(4-Amidinophenyl)-ethyl]-1-methyl-benzimidazol-5-yl-carbonsäure- N-[2-(N,N-dimethylamino)-ethyl]-N-methyl-amid-dihydrochlorid: Die Umsetzung erfolgte ausgehend von 6 mmol 2-[2-(4-Cyanophenyl)-ethyl]-1-methyl-benzimidazol-5-yl-carbonsäure-N-[2-(N,N-dimethylamino)-ethyl]-N-methyl-amid in Analogie zu Beispiel 1, Stufe g. Das Produkt wird erhalten durch Kristallisation aus Aceton und wird mit kaltem Ethanol/Diethylether gewaschen. Ausbeute: 71 %; Schmp.: > 220 °C (Dihydrochlorid);
   Masse: ber.: [406], gef.: [M+H]⁺ 407, [2M+H]⁺ 814;
   ¹H-NMR (250MHz, DMSO-d6): δ [ppm] = 10.70 (1H, s, H⁺); 9.38, 9.21 (4H, 2s, -C(=NH₂⁺)NH₂); 7.89-7.25 (7H, m, aryl-H); 3.80, 3.33 (4H, 2m, N-CH₂-CH₂-); 3.75 (3H, s, aryl-N-CH3); 3.26 (4H, m, aryl-CH₂-CH₂-); 2.99 (3H, s, CO-N-CH₃); 2.80 (6H, s, N-(CH₃)₂).

### Beispiel 4: 2-[2-(4-Amidinophenyl)-ethyl]-1-methyl-benzimidazol-5-yl-carbonsäureN-(4-amidino-benzyl)-N-methyl-amid-trihydrochlorid

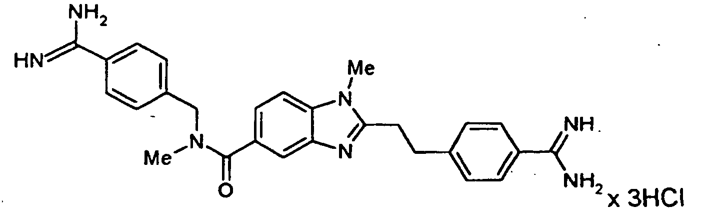

a) 2-[2-(4-Cyanophenyl)-ethyl]-1-methyl-benzimidazol-5-yl-carbonsäure-N-(4-cyanobenzyl)-N-methyl-amid:
   Die Synthese erfolgt in Analogie zu Stufe f (Beispiel 1) durch Umsetzung von 10 mmol 2-[2-(4-Cyanophenyl)-ethyl]-1-methyl-benzimidazol-5-yl-carbonsäure mit N-(4-Cyanobenzyl)-N-methylamin. Ausbeute: 81%; Schmp.: 138-140 °C;
b) 2-[2-(4-Amidinophenyl)-ethyl]-1-methyl-benzimidazol-5-yl-carbonsäure- N-(4-amidino-benzyl)-N-methyl-amid-trihydrochlorid:
   Die Umsetzung erfolgte ausgehend von 7 mmol 2-[2-(4-Cyanophenyl)-ethyl]-1-methyl-benzimidazol-5-yl-carbonsäure-N-(4-cyanobenzyl)-N-methyl-amid in Analogie zu Beispiel 1, Stufe g. Das Produkt wird erhalten durch Kristallisation aus Aceton und wird mit kaltem Ethanol/Diethylether gewaschen.
   Ausbeute: 68 %; Schmp.: > 220 °C (Trihydrochlorid);
   Masse: ber.: [467], gef.: [M+H]⁺ 468;
   ¹H-NMR (250MHz, DMSO-d6): δ [ppm] = 10.2 (1H, breit, H⁺); 9.52, 9.47, 9.36, 9.31 (8H, 4s, 2 -C(=NH₂⁺)NH₂); 8.00-7.20 (11H, m, aryl-H); 4.77 (2H, s, N-CH₂-); 3.78 (3H, s, aryl-N-CH₃); 3.26 (4H, s, aryl-CH₂-CH₂-); 2.95 (3H, s, CO-N-CH₃).

### Beispiel 5: 2-[2-(4-Amidinophenyl)-ethyl]-1-methyl-benzimidazol-5-yl-carbonsäureN-(4-amidino-benzyl)-N-ethoxycarbonylmethyl-amid-dihydrochlorid

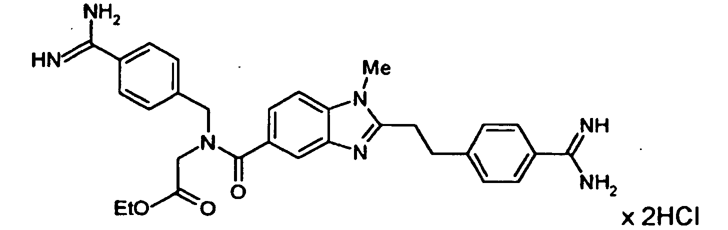

a) 2-[2-(4-Cyanophenyl)-ethyl]-1-methyl-benzimidazol-5-yl-carbonsäure-N-(4-cyanobenzyl)-N-ethoxycarbonylmethyl-amid:
   Die Synthese erfolgt in Analogie zu Stufe f (Beispiel 1) durch Umsetzung von 9 mmol 2-[2-(4-Cyanophenyl)-ethyl]-1-methyl-benzimidazol-5-yl-carbonsäure mit N-(4-cyanobenzyl)-N-ethoxycarbonylmethyl-amin. Die Reinigung des Rohrodukts erfolgt durch Chromatographie an Kieselgel (Dichlormethan:Ethanol = 25:1). Ausbeute: 62%; gelbes Öl;
b) 2-[2-(4-Amidinophenyl)-ethyl]-1-methyl-benzimidazol-5-yl-carbonsäure- N-(4-amidino-benzyl)-N-ethoxycarbonylmethyl-amid-dihydrochlorid:
   Die Umsetzung erfolgte ausgehend von 4.5 mmol 2-[2-(4-Cyanophenyl)-ethyl]-1-methyl-benzimidazol-5-yl-carbonsäure-N-(4-cyanobenzyl)-N-ethoxycarbonylmethyl-amid in Analogie zu Beispiel 1, Stufe g. Das Produkt wird erhalten durch Chromatographie an Kieselgel (Dichlormethan:Ethanol = 4:1).
   Ausbeute: 89 %; fester Schaum;
   Masse: ber.: [539], gef.: [M+H]⁺ 540;
   ¹H-NMR (250MHz, DMSO-d6): δ [ppm] = 9.63, 9.54, 9.44, 9.38 (8H, s, 2 C(=NH₂⁺)NH₂); 8.13-7.30 (11H, m, aryl-H); 4.83 (2H, s, N-CH₂-aryl); 4.19 (2H, s, N-CH₂-C=O); 3.81 (3H, s, aryl-N-CH₃); 3.89 (2H, q, J=7.2 Hz, -O-CH₂-); 3.30 (4H, s, aryl-CH₂-CH₂-); 1.07 (3H, t, J=7.2 Hz, -O-CH₂-CH₃).

### Beispiel 6: 2-[2-(4-Amidinophenyl)-ethyl]-1-methyl-benzimidazol-5-yl-carbonsäureN-(4-dimethylaminobenzyl)-N-methyl-amid-hydrochlorid

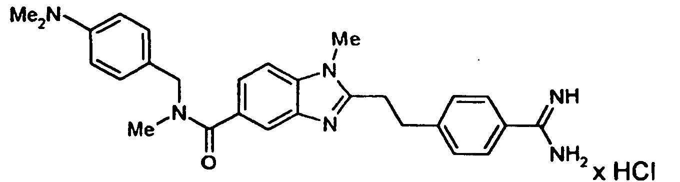

a) 2-[2-(4-Cyanophenyl)-ethyl]-1-methyl-benzimidazol-5-yl-carbonsäure-N-(4-dimethylaminobenzyl)-N-methyl-amid:
   Die Synthese erfolgt in Analogie zu Stufe f (Beispiel 1) durch Umsetzung von 2.3 mmol 2-[2-(4-Cyanophenyl)-ethyl]-1-methyl-benzimidazol-5-yl-carbonsäure mit N-(4-dimethylaminobenzyl)-N-methyl-amin. Die Reinigung des Rohrodukts erfolgt durch Chromatographie an Kieselgel (Dichlormethan:Ethanol = 98:2). Ausbeute: 77%;
b) 2-[2-(4-Amidinophenyl)-ethyl]-1-methyl-benzimidazol-5-yl-carbonsäure- N-(4-dimethylaminobenzyl)-N-methyl-amid-hydrochlorid:
   Die Umsetzung erfolgte ausgehend von 2-[2-(4-Cyanophenyl)-ethyl]-1-methylbenzimidazol-5-yl-carbonsäure-N-(4-dimethylaminobenzyl)-N-methyl-amid in Analogie zu Beispiel 1, Stufe g. Das Produkt wird erhalten durch Chromatographie an Kieselgel (Dichlormethan:Methanol = 9:1). Ausbeute: 62%; fester Schaum; Masse: ber.: [468], gef.: [M+H]⁺ 469;
   ¹H-NMR (250MHz, DMSO-d6): δ [ppm] = 9.33, 9.12 (4H, 2s, C(=NH₂⁺)NH₂); 7.89-6.66 (11H, m, aryl-H); 4.49 (2H, s, N-CH₂-); 3.75 (3H, s, aryl-N-CH₃); 3.27 (4H, s, aryl-CH₂-CH₂-); 2.88 (6H, s, N-(CH₃)₂); 2.85 (3H, s, CO-N-CH₃);

### Beispiel 7: 2-[2-(4-Amidinophenyl)-ethyl]-1-methylrbenzimidazol-5-yl-carbonsäureN-(2-amidinoethyl)-N-methyl-amid-dihydrochlorid

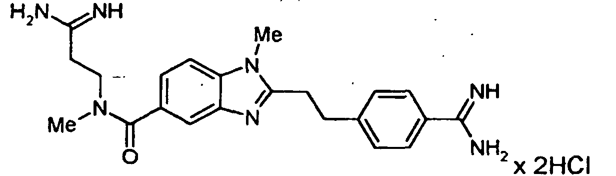

a) 2-[2-(4-Cyanophenyl)-ethyl]-1-methyl-benzimidazol-5-yl-carbonsäure-N-(2-cyanoethyl)-N-methyl-amid:
   Die Synthese erfolgt in Analogie zu Stufe f (Beispiel 1) durch Umsetzung von 4 mmol 2-[2-(4-Cyanophenyl)-ethyl]-1-methyl-benzimidazol-5-yl-carbonsäure mit N-(2-cyanoethyl)-N-methyl-amin. Ausbeute: 61%; Schmp.: 150-152°C;
b) 2-[2-(4-Amidinophenyl)-ethyl]-1-methyl-benzimidazol-5-yl-carbonsäure- N-(2-amidinoethyl)-N-methyl-amid-dihydrochlorid:
   Die Umsetzung erfolgte ausgehend von 2 mmol 2-[2-(4-Cyanophenyl)-ethyl]-1-methyl-benzimidazol-5-yl-carbonsäure-N-(2-cyanoethyl)-N-methyl-amid in Analogie zu Beispiel 1, Stufe g. Das Produkt wird erhalten durch Chromatographie an Kieselgel (Dichlormethan:Methanol = 7:3). Ausbeute: 67%; fester Schaum;
   Masse: ber.: [405], gef.: [M+H]⁺ 406;
   ¹H-NMR (250MHz, CD₃OD): δ [ppm] = 7.70 (7H, m, aryl-H); 3.88, 2.81 (4H, m, N-CH₂-CH₂-); 3.71 (3H, s, aryl-N-CH₃); 3.29 (4H, s, aryl-CH₂-CH₂-); 3.08 (3H, s, CO-N-CH₃).

### Beispiel 8: 2-[2-(4-Amidinophenyl)-ethyl]-1-methyl-benzimidazol-5-yl-carbonsäureN-(2-amidinoethyl)-N-(chinolin-3-yl-methyl)-amid-dihydrochlorid

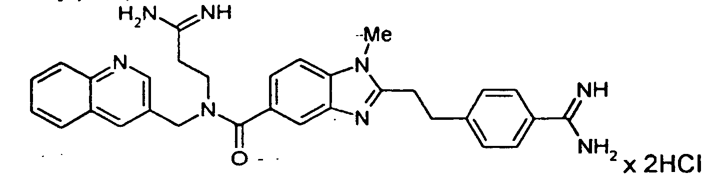

a) 3-(Chinolin-3-yl-methyl-amino)-propionitril:
   1.3 mL Acrylnitril in 4 mL Ethanol werden zu einer gut gerührten bei maximal 30 °C gehaltenen Lösung von 3-Aminomethyl-chinolin (3.0 g, 19 mmol) in 10 mL Ethanol über einen Zeitraum von ca. 0.5 h zugetropft. Die Mischung wird 16 h bei Raumtemperatur gehalten, 1 h unter Rückfluß gekocht und das Lösemittel im Vakuum abdestilliert. Der Rückstand wird über Kieselgel (Dichlormethan:Methanol 50:1) chromatographiert. Ausbeute: 3.0 g (75%); gelbes Öl;
b) 2-[2-(4-Cyanophenyl)-ethyl]-1-methyl-benzimidazol-5-yl-carbonsäure-N-(2-cyanoethyl)-N-(chinolin-3-yl-methyl)-amid:
   Die Synthese erfolgt in Analogie zu Stufe f (Beispiel 1) durch Umsetzung von 5 mmol 2-[2-(4-Cyanophenyl)-ethyl]-1-methyl-benzimidazol-5-yl-carbonsäure mit 3-(Chinolin-3-yl-methyl-amino)-propionitril. Das erhaltene Rohprodukt wird ohne weitere Reinigung direkt weiter umgesetzt.
c) 2-[2-(4-Amidinophenyl)-ethyl]-1-methyl-benzimidazol-5-yl-carbonsäure- N-(2-amidinoethyl)-N-(chinolin-3-yl-methyl)-amid-dihydrochlorid:
   Die Umsetzung erfolgte ausgehend von 5 mmol 2-[2-(4-Cyanophenyl)-ethyl]-1-methyl-benzimidazol-5-yl-carbonsäure-N-(2-cyanoethyl)-N-(chinolin-3-yl-methyl)-amid in Analogie zu Beispiel 1, Stufe g. Das Produkt wird erhalten durch Chromatographie an Kieselgel (Dichlormethan:Methanol = 3:1) oder durch Kristallisation aus Methanol/Aceton. Ausbeute: 46%; Schmp.: >220°C;
   Masse: ber.: [532.65], gef.: [M+H]⁺ 533, [M+2H]²⁺ 267;
   ¹H-NMR (250MHz, DMSO-d6): δ [ppm] = 7.75-7.40 (13H, m, aryl-H); 5.00 (2H, s, N-CH₂-Ph); 4.00, 2.94 (4H, 2m, N-CH₂-CH₂-N); 3,86 (3H, s, aryl-N-CH₃); 3.41 (4H, s, aryl-CH₂-CH₂-).

### Beispiel 9: 2-[2-(4-Amidinophenyl)-ethyl]-1-methyl-benzimidazol-5-yl-carbonsäureN-(2-amidinoethyl)-N-benzyl-amid-dihydrochlorid

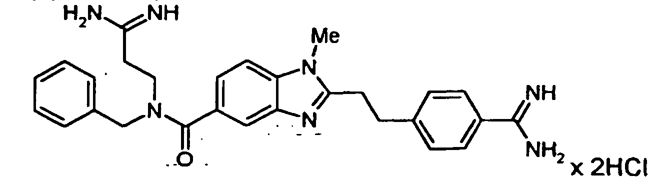

a) 2-[2-(4-Cyanophenyl)-ethyl]-1-methyl-benzimidazol-5-yl-carbonsäure-N-(2-cyanoethyl)-N-benzyl-amid:
   Die Synthese erfolgt ausgehend von 4.4 mmol 2-[2-(4-Cyanophenyl)-ethyl]-1-methylbenzimidazol-5-yl-carbonsäure durch Umsetzung mit 3-(Benzylamino)-propionitril in Analogie zu Beispiel 1, Stufe f. Das Produkt wird durch Kristallisation aus Essigsäureethylester/Diethylether gereinigt.
   Ausbeute: 89 %.
   Schmp.: 140-148 °C
b) 2-[2-(4-Amidinophenyl)-ethyl]-1-methyl-benzimidazol-5-yl-carbonsäure- N-(2-amidinoethyl)-N-benzyl-amid-dihydrochlorid:
   Ausgehend von 2-[2-(4-Cyanophenyl)-ethyl]-1-methyl-benzimidazol-5-yl-carbonsäure-N-(2-cyanoethyl)-N-benzyl-amid; Ausbeute: 56%; Schmp.: > 220 °C; Masse: ber.: [481,60] gef.: [M+H]⁺ 482, [M+2H]²⁺ 242;
   ¹H-NMR (250MHz, DMSO-d6): δ [ppm] = 9.38, 9.26, 9.20, 8.81 (8H, 4s, 2 C(=NH₂⁺)NH₂); 7.83-7.07 (12H, m, aryl-H); 4.63 (2H, s, N-CH₂-Ph); 3.67 (3H, s, aryl-N-CH₃); 3.68, 2.76 (4H, 2m, N-CH₂-CH₂-N); 3.24 (4H, s, aryl-CH₂-CH₂).

### Beispiel 10: 2-[2-(4-Amidinophenyl)-ethyl]-1-methyl-benzimidazol-5-yl-carbonsäureN-(2-amidinoethyl)-N-(3-pyridylmethyl)-amid-dihydrochlorid

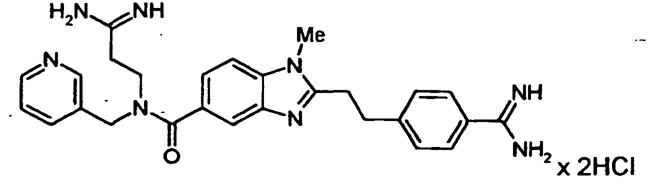

a) 2-[2-(4-Cyanophenyl)-ethyl]-1-methyl-benzimidazol-5-yl-carbonsäure-N-(2-cyanoethyl)-N-(3-pyridylmethyl)-amid:
   Die Synthese erfolgt in Analogie zu Stufe f (Beispiel 1) durch Umsetzung von 4.4 mmol 2-[2-(4-Cyanophenyl)-ethyl]-1-methyl-benzimidazol-5-yl-carbonsäure mit 3-(3-Pyridyl-methyl-amino)-propionitril. Das erhaltene Rohprodukt wird ohne weitere Reinigung direkt weiter umgesetzt.
b) 2-[2-(4-Amidinophenyl)-ethyl]-1-methyl-benzimidazol-5-yl-carbonsäure- N-(2-amidinoethyl)-N-(3-pyridylmethyl)-amid-dihydrochlorid:
   Ausgehend von 2-[2-(4-Cyanophenyl)-ethyl]-1-methyl-benzimidazol-5-yl-carbonsäure-N-(2-cyanoethyl)-N-(3-pyridylmethyl)-amid gelingt die Synthese in Analogie zu Beispiel 1 (Stufe g). Die Reinigung des Produkts erfolgt durch Chromatographie über Kieselgel (Dichlormethan:Methanol 7:3) und/oder Kristallisation aus Methanol/Aceton mit Wasser.
   Ausbeute: 25%; Schmp.: 225-228°C;
   Masse: ber.: [482] gef.: [M+H]⁺ 483, [M+2H]²⁺ 242;
   ¹H-NMR (250MHz, DMSO-d6): δ [ppm] = 9.41, 9.27, 9.19, 8.77 (8H, 4s, 2 C(=NH₂⁺)NH₂); 8.62-7.29 (11H, m, aryl-/pyridyl-H); 4.71 (2H, s, N-CH₂-Ph); 3.78 (3H, s, aryl-N-CH₃); 3.72, 2.78 (4H, 2m, N-CH₂-CH₂); 3.29 (4H, s, aryl-CH₂-CH₂-).

### Beispiel 11: 2-[2-(4-Amidinophenyl)-ethyl]-1-methyl-benzimidazol-5-yl-carbonsäureN-(2-amidinoethyl)-N-iso-butyl-amid-dihydrochlorid

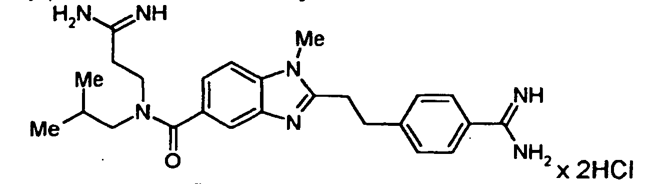

a) 3-(*i*-Butylamino)-propionitril
   Die Darstellung erfolgt ausgehend von 40 mmol iso-Butylamin in Analogie zu Stufe a (Beispiel 8). Das erhaltene Produkt wird ohne weitere Reinigung direkt in die nächste Stufe eingesetzt. Ausbeute: 99%;
b) 2-[2-(4-Cyanophenyl)-ethyl]-1-methyl-benzimidazol-5-yl-carbonsäure-N-(2-cyanoethyl)-N-iso-butyl-amid:
   Die Synthese erfolgt ausgehend von 2-[2-(4-Cyanophenyl)-ethyl]-1-methylbenzimidazol-5-yl-carbonsäurechlorid durch Umsetzung mit 3-(i-Butylamino)-propionitril in Analogie zu Beispiel 2, Stufe b. Das Produkt wird ohne Reinigung direkt weiter umgesetzt.
c) 2-[2-(4-Amidinophenyl)-ethyl]-1-methyl-benzimidazol-5-yl-carbonsäure- N-(2-amidinoethyl)-N-iso-butyl-amid-dihydrochlorid:
   Ausgehend von 2-[2-(4-Cyanophenyl)-ethyl]-1-methyl-benzimidazol-5-yl-carbonsäure-N-(2-cyanoethyl)-N-iso-butyl-amid gelingt die Synthese in Analogie zur Vorschrift unter Beispiel 1 (Stufe g). Ausbeute: 43%; Schmp.: 204-210°C;
   Masse: ber.: [447] gef.: [M+H]⁺ 448;
   ¹H-NMR (250MHz, DMSO-d6): δ [ppm] = 9.56, 9.42, 9.36, 8.96 (8H, 4s, 2 C(=NH₂⁺)NH₂); 8.32-7.27 (7H, m, aryl-H); 3.88, 2.79 (4H, 2m, N-CH₂-CH₂); 3.35 (2H, m, N-CH₂-CH); 3.32 (4H, s, aryl-CH₂-CH₂-); 1.89 (1H, m, N-CH₂-CH); 0.68 (6H, m, CH(CH₃)₂).

### Beispiel 12: 2-[2-(4-Amidinophenyl)-ethyl]-1-methyl-benzimidazol-5-yl-carbonsäureN-(3-phenylpropyl)-amid-hydrochlorid

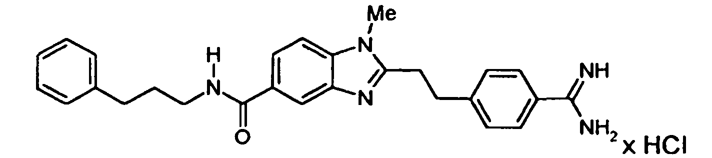

a) 2-[2-(4-Cyanophenyl)-ethyl]-1-methyl-benzimidazol-5-yl-carbonsäure-N-(3-phenylpropyl)-amid:
   Die Synthese erfolgt ausgehend von 6.2 mmol 2-[2-(4-Cyanophenyl)-ethyl]-1-methylbenzimidazol-5-yl-carbonsäure durch Umsetzung mit 3-Phenylpropylamin in Analogie zu Beispiel 1, Stufe f. Das Produkt wird ohne Reinigung direkt weiter umgesetzt. Ausbeute: 89%;
b) 2-[2-(4-Amidinophenyl)-ethyl]-1-methyl-benzimidazol-5-yl-carbonsäure-N -(3-phenylpropyl)-amid-hydrochlorid:
   Ausgehend von 5.4 mmol 2-[2-(4-Cyanophenyl)-ethyl]-1-methyl-benzimidazol-5-yl-carbonsäure-N-(3-phenylpropyl)-amid gelingt die Synthese in Analogie zur Vorschrift unter Beispiel 1 (Stufe g). Das Produkt wird durch Chromatographie über Kieselgel (Dichlormethan:Methanol 9:1) gereinigt. Ausbeute: 56%; amorpher Feststoff;
   Masse: ber. [439,56], gef.: [M+H]⁺ 4400;
   ¹H-NMR (250MHz, DMSO-d6): δ [ppm] = 9.53, 9.33 (4H, 2s, C(=NH₂⁺)NH₂); 8.65 (1H, t, J=5.2 Hz, NHCO); 8.32-7.22 (12H, m, aryl-H); 3.82 (3H, s, aryl-N-CH₃); 3.41, 2.70, 1.88 (6H, 3m, N-CH₂-CH₂-CH₂-); 3.33 (4H, s, aryl-CH₂-CH₂-).

### Beispiel 13: 2-[2-(4-Amidinophenyl)-ethyl]-1-methyl-benzimidazol-5-yl-carbonsäureN-(3,3-diphenylpropyl)-amid-hydrochlorid

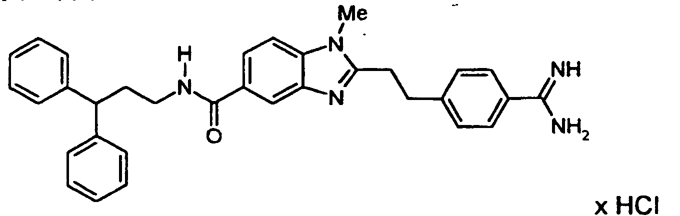

a) 2-[2-(4-Cyanophenyl)-ethyl]-1-methyl-benzimidazol-5-yl-carbonsäure-N-(3,3-diphenylpropyl)-amid:
   Die Synthese erfolgt ausgehend von 6.2 mmol 2-[2-(4-Cyanophenyl)-ethyl]-1-methylbenzimidazol-5-yl-carbonsäure durch Umsetzung mit 3,3-Diphenylpropylamin in Analogie zu Beispiel 1, Stufe f. Das Produkt wird ohne Reinigung direkt weiter umgesetzt. Ausbeute: 81 %;
b) 2-[2-(4-Amidinophenyl)-ethyl]-1-methyl-benzimidazol-5-yl-carbonsäure- N-(3,3-diphenylpropyl)-amid-hydrochlorid:
   Ausgehend von 5 mmol 2-[2-(4-Cyanophenyl)-ethyl]-1-methyl-benzimidazol-5-yl-carbonsäure-N-(3,3-diphenylpropyl)-amid gelingt die Synthese in Analogie zur Vorschrift unter Beispiel 1 (Stufe g). Das Produkt wird durch Chromatographie über Kieselgel (Dichlormethan:Methanol 95:5) gereinigt.
   Ausbeute: 43%; Schmp.: 185°C;
   Masse: ber. [515], gef.: [M+H]⁺ 516
   ¹H-NMR (250MHz, DMSO-d6): δ [ppm] = 8.63 (1H, t, J=6.2 Hz, NHCO); 8.32-7.25 (17H, m, aryl-H); 7.79 (4h, breit, c(=NH₂⁺)NH₂); 4.15 (1H, t, J=6.8 Hz, -CHPh₂); 3.81 (3H, s, aryl-N-CH₃); 3.32 (4H, s, aryl-CH₂-CH₂-); 3.26, 2.36 (4H, 2m, N-CH₂-CH₂-).

### Beispiel 14: 2-[2-(4-Amidinophenyl)-ethyl]-1-methyl-benzimidazol-5-yl-carbonsäure-(N'-phenyl-piperazid)-hydrochlorid

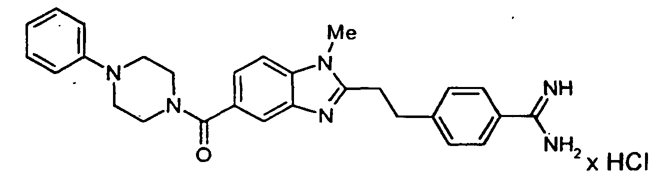

a) 2-[2-(4-Cyanophenyl)-ethyl]-1-methyl-benzimidazol-5-yl-carbonsäure-(N'-phenyl-piperazid):
   Die Synthese erfolgt ausgehend von 6.2 mmol 2-[2-(4-Cyanophenyl)-ethyl]-1-methylbenzimidazol-5-yl-carbonsäure durch Umsetzung mit N-Phenylpiperazin in Analogie zu Beispiel 1, Stufe f. Das Produkt wird ohne weitere Reinigung direkt weiter umgesetzt. Ausbeute: 86%;
b) 2-[2-(4-Amidinophenyl)-ethyl]-1-methyl-benzimidazol-5-yl-carbonsäure- (N'-phenyl-piperazid)- hydrochlorid:
   Ausgehend von 5.4 mmol 2-[2-(4-Cyanophenyl)-ethyl]-1-methyl-benzimidazol-5-yl-carbonsäure-(N'-phenyl-piperazid) gelingt die Synthese in Analogie zur Vorschrift unter Beispiel 1 (Stufe g). Das Produkt wird durch Chromatographie über Kieselgel (Dichlormethan:Methanol 95:5) gereinigt. Ausbeute: 47%; amorpher Feststoff; Masse: ber.: [466,59], gef.: [M+H]⁺ 467;
   ¹H-NMR (250MHz, DMSO-d6): δ [ppm] = 9.41, 9.22 (4H, 2s, C(=NH₂⁺)NH₂); 7.89-6.79 (12H, m, aryl-H); 3.80 (3H, s, aryl-N-CH₃); 3.69, 3.19 (8H, 2m, piperazinyl); 3.29 (4H, s, aryl-CH₂-CH₂-).

### Beispiel 15: 2-[2-(4-Amidinophenyl)-ethyl]-1-methyl-benzimidazol-5-yl-carbonsäure-[N'-(2-methyl-phenyl)-diazepid]-hydrochlorid

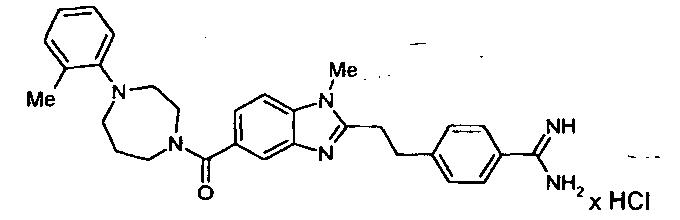

a) 2-[2-(4-Cyanophenyl)-ethyl]-1-methyl-benzimidazol-5-yl-carbonsäure-[N'-(2-methyl-phenyl)-diazepid]:
   Die Synthese erfolgt ausgehend von 6.2 mmol 2-[2-(4-Cyanophenyl)-ethyl]-1-methylbenzimidazol-5-yl-carbonsäure durch Umsetzung mit N-(2-Methylphenyl)-diazepin in Analogie zu Beispiel 1, Stufe f. Das Produkt wird ohne Reinigung direkt weiter umgesetzt.
   ¹H-NMR (250MHz, CDCl₃): δ [ppm] = 7.87-6.90 (11H, m, aryl-H); 3.96, 3.65, 3.11, 2.31, 1.94 (10H, 5m, diazacycloheptyl); 3.62 (3H, s, N-CH₃); 3.34, 3.27 (4H, 2m, aryl-CH₂-CH₂); 2.81 (3H, s, aryl-N-CH₃).
b) 2-[2-(4-Amidinophenyl)-ethyl]-1-methyl-benzimidazol-5-yl-carbonsäure- [N'-(2-methyl-phenyl)-diazepid]-hydrochlorid:
   Ausgehend von 2.5 mmol 2-[2-(4-Cyanophenyl)-ethyl]-1-methyl-benzimidazol-5-yl-carbonsäure-[N'-(2-methyl-phenyl)-diazepid] gelingt die Synthese in Analogie zur Vorschrift unter Beispiel 1 (Stufe g). Das Produkt wird durch Chromatographie über Kieselgel (Dichlormethan:Methanol 95:5) gereinigt.
   Ausbeute: 48%; amorpher Feststoff;
   Masse: ber.: [494], gef.: [M+H]⁺ 495;
   ¹H-NMR (250MHz, DMSO-d6): δ [ppm] = 9.42, 9.21 (4H, 2s, C(=NH₂⁺)NH₂); 7.92-6.87 (11H, m, aryl-H); 3.84-1.65 (10H, 5m, diazacycloheptan); 3.78 (3H, s, aryl-N-CH₃); 3.30 (4H, s, aryl-CH₂-CH₂-); 2.21 (3H, s, aryl-CH₃).

### Beispiel 16: 2-[2-(4-Amidinophenyl)-ethyl]-1-methyl-benzimidazol-5-yl-carbonsäure-N-(4-amidinobenzyl)-N-(3-phenylpropyl)-amid-dihydrochlorid

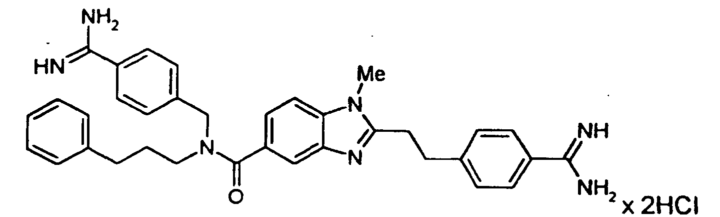

a) (4-Cyanobenzyl)-(3-phenyl-propyl)-amin:
   3-Phenylpropylamin (2.5 g, 18.5 mmol), 4-Cyanobenzaldehyd (2.2 g, 16.8 mmol) und 0.22 mL Essigsäure in 130 mL Dichlormethan werden unter Rühren mit 11.0g Na[BH(OAc)₃] (51.9 mmol) versetzt. Es wird 1 h bei RT gerührt, das Dichlormethan abdestilliert, in Ethylacetat aufgenommen, mit Wasser versetzt und mit verdünnter Salzsäure sauer gestellt. Nachdem keine Gasentwicklung mehr sichtbar ist, wird mit 4 N wässriger Natriumhydroxidlösung alkalisch gestellt, das Amin in die organische Phase extrahiert und die organische Phase mit Wasser gewaschen und getrocknet. Das Lösemittel wird im Vakuum abdestilliert und das verbleibende Rohprodukt (nach
   ¹H-NMR: 079381, 85 %ig) wird über Kieselgel (Dichlormethan:Petrolether 9:1 bis Dichlormethan) chromatographiert.
   Ausbeute: 3.6 g (78%).
b) 2-[2-(4-Cyanophenyl)-ethyl]-1-methyl-benzimidazol-5-yl-carbonsäure-N-(4-cyanobenzyl)-N-(3-phenylpropyl)-amid:
   Die Synthese erfolgt ausgehend von 15.2 mmol 2-[2-(4-Cyanophenyl)-ethyl]-1-methyl-benzimidazol-5-yl-carbonsäure durch Umsetzung mit (4-Cyanobenzyl)-(3-phenyl-propyl)-amin in Analogie zu Beispiel 1, Stufe f. Das Produkt wird ohne Reinigung direkt weiter umgesetzt. Ausbeute: >90%;
c) 2-[2-(4-Amidinophenyl)-ethyl]-1-methyl-benzimidazol-5-yl-carbonsäure- N-(4-amidinobenzyl)-N-(3-phenylpropyl)-amid-dihydrochlorid:
   Ausgehend von 7.4 mmol 2-[2-(4-Cyanophenyl)-ethyl]-1-methyl-benzimidazol-5-yl-carbonsäure-N-(4-cyanobenzyl)-N-(3-phenylpropyl)-amid gelingt die Synthese in Analogie zur Vorschrift unter Beispiel 1 (Stufe g). Das Produkt wird durch Chromatographie über Kieselgel (Dichlormethan:Methanol 95:5 - 9:1) gereinigt. Ausbeute: 18%; amorpher Feststoff;
   Masse: ber.: [571] gef.: [M+H]⁺ 572;
   ¹H-NMR (250MHz, CD₃OD): δ [ppm] = 7.88-6.70 (16H, m, aryl-H); 3.75 (5H, s, N-CH₂-, aryl-N-CH₃); 3.34 (4H, s, aryl-CH₂-CH₂-); 3.50-1.68 (6H, m, N-CH₂-CH₂-CH₂).

### Beispiel 17: 2-[2-(4-Amidinophenyl)-ethyl]-1-methyl-benzimidazol-5-yl-carbonsäureN-(4-amidinobenzyl)-N-(3,3-diphenylpropyl)-amid-dihydrochlorid

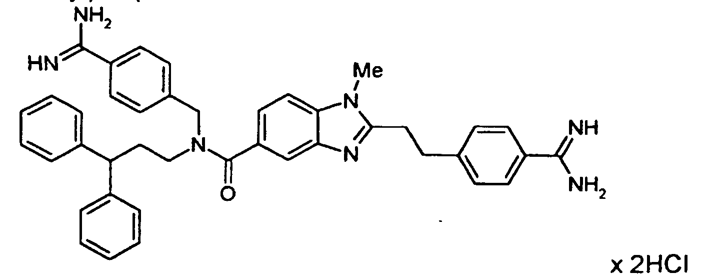

a) (4-Cyanobenzyl)-(3-phenyl-propyl)-amin:
   Die Umsetzung erfolgt in analogie zur unter Beispiel 16 (Stufe a) beschriebenen Vorgehensweise ausgehend von 18.5 mmol 3,3-Diphenylpropylamin.Ausbeute: 65%;
b) 2-[2-(4-Cyanophenyl)-ethyl]-1-methyl-benzimidazol-5-yl-carbonsäure-N-(4-cyanobenzyl)-N-(3,3-diphenylpropyl)-amid:
   Die Synthese erfolgt ausgehend von 12.1 mmol 2-[2-(4-Cyanophenyl)-ethyl]-1-methyl-benzimidazol-5-yl-carbonsäure durch Umsetzung mit (4-Cyanobenzyl)-(3,3-diphenyl-propyl)-amin in Analogie zu Beispiel 1, Stufe f. Das Produkt wird ohne weitere Reinigung direkt weiter umgesetzt. Ausbeute: >90%;
c) 2-[2-(4-Amidinophenyl)-ethyl]-1-methyl-benzimidazol-5-yl-carbonsäure- N-(4-amidinobenzyl)-N-(3,3-diphenylpropyl)-amid-dihydrochlorid:
   Ausgehend von 5.4 mmol 2-[2-(4-Cyanophenyl)-ethyl]-1-methyl-benzimidazol-5-yl-carbonsäure-N-(4-cyanobenzyl)-N-(3,3-diphenylpropyl)-amid gelingt die Synthese in Analogie zur Vorschrift unter Beispiel 1 (Stufe g). Das Produkt wird durch Chromatographie über Kieselgel (Dichlormethan:Methanol 85:15) gereinigt. Ausbeute: 26%; amorpher Feststoff;
   Masse: ber.: [647], gef.: [M+H]⁺ 648; [M+2H]²⁺ 325;
   ¹H-NMR (250MHz, CD₃OD): δ [ppm] = 7.85-6.81 (21H, m, aryl-H); 3.80 (3H, s, aryl-N-CH₃); 4.16-2.13 (11H, m, aryl-CH₂-CH₂-, N-CH₂, N-CH₂-CH₂-CH).

### Beispiel 18: 2-[2-(4-Amidinophenyl)-ethyl]-1-methyl-benzimidazol-5-yl-carbonsäureN-methyl-N-(3-pyridyl)-amid-hydrochlorid

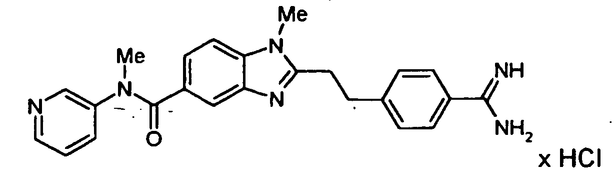

a) 2-[2-(4-Cyanophenyl)-ethyl]-1-methyl-benzimidazol-5-yl-carbonsäure-N-methyl-N-(3-pyridyl)-amid:
   Die Synthese erfolgt ausgehend von 10 mmol 2-[2-(4-Cyanophenyl)-ethyl]-1-methylbenzimidazol-5-yl-carbonsäurechlorid durch Umsetzung mit N-methyl-N-(3-pyridyl)-amin in Analogie zu Beispiel 2, Stufe b. Das Produkt wird ohne weitere Reinigung direkt weiter umgesetzt.
b) 2-[2-(4-Amidinophenyl)-ethyl]-1-methyl-benzimidazol-5-yl-carbonsäure- N-methyl-N-(3-pyridyl)-amid- hydrochlorid:
   Ausgehend von 5 mmol 2-[2-(4-Cyanophenyl)-ethyl]-1-methyl-benzimidazol-5-yl-carbonsäure- N-methyl-N-(3-pyridyl)-amid gelingt die Synthese in Analogie zur Vorschrift unter Beispiel 1 (Stufe g). Die Reinigung des Produkts erfolgt durch Chromatographie über Kieselgel (Dichlormethan:Methanol 4:1).
   Ausbeute: 22%; Schmp.: >220°C;
   Masse: ber.: [412], gef.: [M+H]⁺ 413, [2M+H]⁺ 825;
   ¹H-NMR (250MHz, DMSO-d6): δ [ppm] = 9.30 (4H, s, C(=NH₂⁺)NH₂); 8.40-7.10 (11H, m, aryl-/pyridyl-H); 3.69 (3H, s, aryl-N-CH₃); 3.42 (3H, s, CO-N-CH₃); 3.21 (4H, s, aryl-CH₂-CH₂-).

### Beispiel 19: 2-[2-(4-Amidinophenyl)-ethyl]-1-methyl-benzimidazol-5-yl-carbonsäureN-[2-N',N'-dibenzylamino)ethyl]-N-phenyl-amid- dihydrochlorid

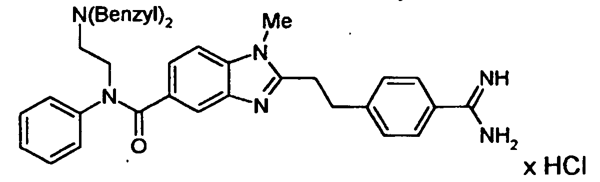

a) N,N-Dibenzyl-N'-phenyl-ethylendiamin wird aus N,N-Dibenzylethanolamin-Hydrochlorid durch Umsetzung mit SOCl₂ in Chloroform und anschließende nukleophile Substitution mit Anilin erhalten.
b) 2-[2-(4-Cyanophenyl)-ethyl]-1-methyl-benzimidazol-5-yl-carbonsäure-N-[2-N',N'-dibenzylamino)ethyl]-N-phenyl-amid:
   Die Synthese erfolgt ausgehend von 10.4 mmol 2-[2-(4-Cyanophenyl)-ethyl]-1-methyl-benzimidazol-5-yl-carbonsäurechlorid durch Umsetzung mit N,N-Dibenzyl-N'-Phenylethylendiamin in Analogie zu Beispiel 2, Stufe b. Das Produkt wird durch Kristallisation aus Diethylether erhalten. Ausbeute: 92%;
c) 2-[2-(4-Amidinophenyl)-ethyl]-1-methyl-benzimidazol-5-yl-carbonsäure- N-[2-N',N'-dibenzylamino)ethyl]-N-phenyl-amid- dihydrochlorid:
   Ausgehend von 8.3 mmol 2-[2-(4-Cyanophenyl)-ethyl]-1-methyl-benzimidazol-5-yl-carbonsäure-N-[2-N',N'-dibenzylamino)ethyl]-N-phenyl-amid gelingt die Synthese in Analogie zur Vorschrift unter Beispiel 1 (Stufe g). Die Reinigung des Produkts erfolgt durch Chromatographie über Kieselgel (Dichlormethan:Methanol 95:5).
   Ausbeute: 70%; amorpher Feststoff;
   Masse: ber.: [620], gef.: [M+H]⁺ 621, [M+2H]²⁺ 311;
   ¹H-NMR (250MHz, DMSO-d6): δ [ppm] = 9.34, 9.13 (4H, 2s, C(=NH₂⁺)NH₂); 7.84-6.90 (22H, m, aryl-H); 4.05, 2.66 (4H, 2t, J=6.2 Hz, N-CH₂-CH₂-N); 3.67 (3H, s, aryl-N-CH₃); 3.57 (4H, s, N-(CH₂-Ph)₂); 3.20 (4H, s, aryl-CH₂-CH₂-).

### Beispiel 20: 2-[2-(4-Amidinophenyl)-ethyl]-1-methyl-benzimidazol-5-yl-carbonsäureN-(2-amidinoethyl)-N-(2-naphthyl)-amid- dihydrochlorid

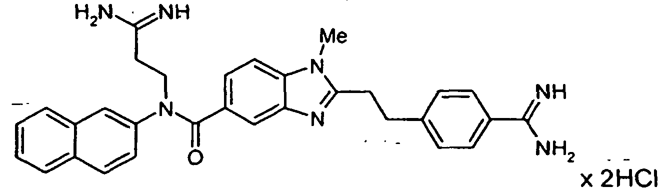

a) 3-(β-Naphthyl-amino)propionitril:
   Die Umsetzung erfolgt ausgehend von 24 mmol 2-Amino-naphthalin in Analogie zu Beispiel 22 (Stufe a). Die Reinigung des N-Cyanoethyl-N-formylnaphthylamins erfolgt durch Kristallisation aus Ethanol (Ausbeute: 68%; Schmp.: 82-84 °C). Das Endprodukt wird aus Ethanol umkristallisiert (Ausbeute: 68%).
   Schmp.: 96-98 °C;
b) 2-[2-(4-Cyanophenyl)-ethyl]-1-methyl-benzimidazol-5-yl-carbonsäure-N-(2-cyanoethyl)-N-(2-naphthyl)-amid:
   Die Synthese erfolgt ausgehend von 3.8 mmol 2-[2-(4-Cyanophenyl)-ethyl]-1-methylbenzimidazol-5-yl-carbonsäurechlorid durch Umsetzung mit 3-(β-Naphthyl-amino)propionitril in Analogie zu Beispiel 2, Stufe b. Das Produkt wird durch Kristallisation aus Ethylacetat zugänglich. Ausbeute: 50%; Schmp.: 196-198°C;
   ¹H-NMR (250MHz, DMSO-d6): δ [ppm] = 7.85-7.12 (14H, m, aryl-H); 4.19, 2.89 (4H, 2m, N-CH₂-CH₂); 3.55 (3H, s, aryl-N-CH₃); 3.08 (4H, s, aryl-CH₂-CH₂-).
c) 2-[2-(4-Amidinophenyl)-ethyl]-1-methyl-benzimidazol-5-yl-carbonsäure- N-(2-amidinoethyl)-N-(2-naphthyl)-amid- dihydrochlorid:
   Ausgehend von 1.9 mmol 2-[2-(4-Cyanophenyl)-ethyl]-1-methyl-benzimidazol-5-yl-carbonsäure-N-(2-cyanoethyl)-N-(2-naphthyl)-amid gelingt die Synthese der Titelverbindung in Analogie zu Beispiel 1 (Stufe g).
   Ausbeute: 55%; amorpher Feststoff;
   Masse: ber.: [517], gef.: [M+H]⁺ 518;
   ¹H-NMR (250MHz, CD₃OD): δ [ppm] = 7.80-7.12 (14H, m, aryl-H); 4.39, 2.89 (4H, 2m, N-CH₂-CH₂); 3.55 (3H, s, aryl-N-CH₃); 3.15 (4H, s, aryl-CH₂-CH₂-).

### Beispiel 21: 2-[2-(4-Amidinophenyl)-ethyl]-1-methyl-benzimidazol-5-yl-carbonsäureN-(2-amidinoethyl)-N-phenyl-amid-dihydrochlorid

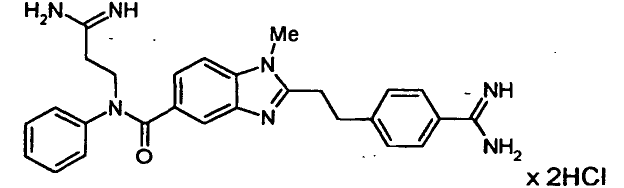

a) 2-[2-(4-Cyanophenyl)-ethyl]-1-methyl-benzimidazol-5-yl-carbonsäure-N-(2-cyanoethyl)-N-phenyl-amid:
   Die Synthese erfolgt ausgehend von 6.6 mmol 2-[2-(4-Cyanophenyl)-ethyl]-1-methylbenzimidazol-5-yl-carbonsäurechlorid durch Umsetzung mit 3-Phenylamino-propionitril in Analogie zu Beispiel 2, Stufe b. Das Produkt wird durch Kristallisation aus Ethylacetat zugänglich. Ausbeute: 70%; Schmp.: 188-190°C;
b) 2-[2-(4-Amidinophenyl)-ethyl]-1-methyl-benzimidazol-5-yl-carbonsäure- N-(2-amidinoethyl)-N-phenyl-amid- dihydrochlorid:
   Ausgehend von 4.1 mmol 2-[2-(4-Cyanophenyl)-ethyl]-1-methyl-benzimidazol-5-yl-carbonsäure-N-(2-cyanoethyl)-N-phenyl-amid gelingt die Umsetzung in Analogie zu Beispiel 1, Stufe g. Ausbeute: 62%; amorpher Feststoff;
   Masse: ber.: [467], gef.: [M+H]⁺ 468;
   ¹H-NMR (250MHz, DMSO-d6): δ [ppm] = 9.48, 9.30, 8,97, 8.60 (8H, 4s, 2 C(=NH₂⁺)NH₂); 8.00.7.13 (12H, m, aryl-H); 2.82 (4H, 2t, J=6.2 Hz, N-CH₂-CH₂-); 3.71 (3H, s, aryl-N-CH₃); 3.22 (4H, s, aryl-CH₂-CH₂-).

### Beispiel 22: 2-[2-(4-Amidinophenyl)-ethyl]-1-methyl-benzimidazol-5-yl-carbonsäureN-(2-amidinoethyl)-N-(3-ethyl-phenyl)-amid- dihydrochlorid

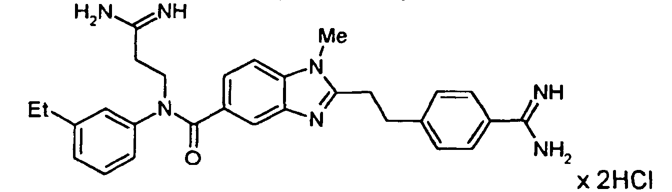

a) 3-(3-Ethylphenyl-amino)-propionitril: 6.1 g (50 mmol) 3-Ethylanilin werden in 5 mL Ameisensäure für 5 h auf 100-120 °C erhitzt und nach dem Abkühlen mit 100 mL Ethylacetat versetzt. Anschließend wird mit verdünnter wässriger Salzsäure und mit verdünnter wässriger Natriumhydroxidlösung gewaschen. Die organische Phase wird über Magnesiumsulfat getrocknet und das Lösemittel im Vakuum abdestilliert.
   Der verbleibende Rückstand (6.7 g N-Formyl-3-ethylanilin) wird in 4.6 mL Acrylnitril aufgenommen, mit pulverisiertem Natriumhydroxid (0.2 g) versetzt, 6 h bei 80-90 °C gerührt und 16 h bei Raumtemperatur gehalten. Es wird mit 100 mL Ethylacetat verdünnt und mit Wasser gewaschen. Anschließend wird über Magnesiumsulfat getrocknet und das Lösemittel im Vakuum abdestilliert. Der Rückstand (8.7 g N-Formyl-3-(3-ethylphenyl-amino)-propionitril) wird in 22.5 mL Acetonitril aufgenommen, mit 22.5 mL wässriger Salzsäure (5N) versetzt und 5 h bei 80-90 °C gerührt. Es wird mit 100 mL Ethylacetat verdünnt, auf Wasser gegossen und mit Natriumhydroxid alkalisch gestellt. Die organische Phase wird mit Wasser gewaschen, über Magnesiumsulfat getrocknet und das Lösemittel wird im Vakuum abdestilliert. Das reine Produkt wird durch Chromatographie über Kieselgel (Hexan:Ethylacetat 9:1) gewonnen. Ausbeute: 6,0 g (69%)
b) 2-[2-(4-Cyanophenyl)-ethyl]-1-methyl-benzimidazol-5-yl-carbonsäure-N-(2-cyanoethyl)-N-(3-ethyl-phenyl)-amid:
   Die Synthese erfolgt ausgehend von 4.5 mmol 2-[2-(4-Cyanophenyl)-ethyl]-1-methylbenzimidazol-5-yl-carbonsäurechlorid durch Umsetzung mit 3-(3-Ethylphenyl-amino)-propionitril in Analogie zu Beispiel 2, Stufe b. Das Produkt wird durch Kristallisation aus Ethylacetat zugänglich. Schmp.: 122-124°C;
c) 2-[2-(4-Amidinophenyl)-ethyl]-1-methyl-benzimidazol-5-yl-carbonsäure- N-(2-amidinoethyl)-N-(3-ethyl-phenyl)-amid- dihydrochlorid:
   Ausgehend von 4.5 mmol 2-[2-(4-Cyanophenyl)-ethyl]-1-methyl-benzimidazol-5-yl-carbonsäure-N-(2-cyanoethyl)-N-(3-ethyl-phenyl)-amid gelingt die Umsetzung in Analogie zu Beispiel 1, Stufe g.
   Ausbeute: 43%; amorpher Feststoff;
   Masse: ber.: [495] gef.: [M+H]⁺ 496, [M+2H]²⁺ 249;
   ¹H-NMR (250MHz, DMSO-d6): δ [ppm] = 9.47, 9.30, 9.27, 8.87 (8H, 4s, 2 C(=NH₂⁺)NH₂); 7.94-6.96 (11H, m, aryl-H); 4.22, 2.82 (4H, 2m, N-CH₂-CH₂); 3.69 (3H, s, aryl-N-CH₃); 3.21 (4H, s, aryl-CH₂-CH₂-); 2.51 (2H, q, J=7.6 Hz, -Ph-CH₂-CH₃); 1.01 (3H, t, J=7.6 Hz, -Ph-CH₂-CH₃).

### Beispiel 23: 2-[2-(4-Amidinophenyl)-ethyl]-1-methyl-benzimidazol-5-yl-carbonsäureN-(2-amidinoethyl)-N-(3-phenoxy-phenyl)-amid- dihydrochlorid

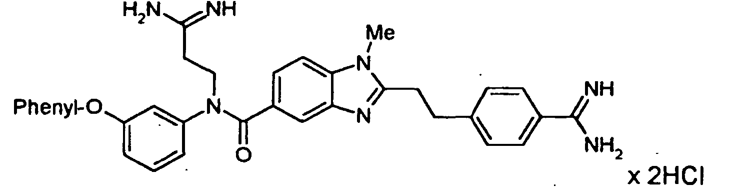

a) 3-(3-Phenoxyphenyl-amino)-propionitril:
   Die Umsetzung gelingt analog zur Synthese nach Beispiel 22, Stufe a ausgehend von 3-Phenoxyanilin. Die Reinigung des Produkts erfolgt durch Kristallisation des Hydrochlorides aus Ethylacetat mit etherischer Salzsäurelösung.
   Ausbeute: 72; Schmp.: 141-146 °C (Hydrochlorid)
b) 2-[2-(4-Cyanophenyl)-ethyl]-1-methyl-benzimidazol-5-yl-carbonsäure-N-(2-cyanoethyl)-N-(3-phenoxy-phenyl)-amid:
   Die Synthese erfolgt ausgehend von 4.5 mmol 2-[2-(4-Cyanophenyl)-ethyl]-1-methylbenzimidazol-5-yl-carbonsäurechlorid durch Umsetzung mit 43-(3-Phenoxyphenyl-amino)-propionitril in Analogie zu Beispiel 2, Stufe b. Das Produkt wird durch Kristallisation aus Ethylacetat/Diethylether zugänglich. Schmp.: 125-127°C;
   ¹H-NMR (250MHz, CDCl₃): δ [ppm] = 7.70-6.53 (16H, m, aryl-H); 4.16, 2.82 (4H, 2t, J=7.5 Hz, N-CH₂-CH₂); 3.55 (3H, s, aryl-N-CH₃); 3.28, 3.15 (4H, 2m, aryl-CH₂-CH₂).
c) 2-[2-(4-Amidinophenyl)-ethyl]-1-methyl-benzimidazol-5-yl-carbonsäure- N-(2-amidinoethyl)-N-(3-phenoxy-phenyl)-amid-dihydrochlorid:
   Ausgehend von 4.5 mmol 2-[2-(4-Cyanophenyl)-ethyl]-1-methyl-benzimidazol-5-yl-carbonsäure-N-(2-cyanoethyl)-N-(3-phenoxy-phenyl)-amid gelingt die Umsetzung in Analogie zu Beispiel 1, Stufe g. Ausbeute: 23%, amorpher Feststoff;
   Masse: ber.: [559] gef.: [M+H]⁺ 560;
   ¹H-NMR (250MHz, CD₃OD): δ [ppm] = 7.75-6.40 (16H, m, aryl-H); 4.30, 2.76 (4H, 2m, N-CH₂-CH₂); 3.73 (3H, s, aryl-N-CH₃); 3.30 (4H, s, aryl-CH₂-CH₂-).

### Beispiel 24: 2-[2-(4-Amidinophenyl)-ethyl]-1-methyl-benzimidazol-5-yl-carbonsäureN-(2-amidinoethyl)-N-(2-phenylethyl)-amid- dihydrochlorid

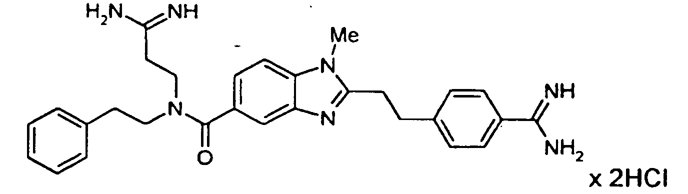

a) 3-(Phenethylamino)-propionitril
   Die Darstellung erfolgt in Analogie zu Beispiel 8 (Stufe a) ausgehend von 19 mmol Phenethylamin. Ausbeute: 79%;
b) 2-[2-(4-Cyanophenyl)-ethyl]-1-methyl-benzimidazol-5-yl-carbonsäure-N-(2-cyanoethyl)-N-(2-phenylethyl)-amid:
   Die Synthese erfolgt ausgehend von 4.5 mmol 2-[2-(4-Cyanophenyl)-ethyl]-1-methylbenzimidazol-5-yl-carbonsäurechlorid durch Umsetzung mit 3-(Phenethylamino)-propionitril in Analogie zu Beispiel 2, Stufe b. Das Produkt wird durch Kristallisation aus Ethylacetat/Diethylether zugänglich. Schmp.: 132-133°C;
c) 2-[2-(4-Amidinophenyl)-ethyl]-1-methyl-benzimidazol-5-yl-carbonsäure- N-(2-amidinoethyl)-N-(2-phenylethyl)-amid-dihydrochlorid
   Ausgehend von 3.8 mmol 2-[2-(4-Cyanophenyl)-ethyl]-1-methyl-benzimidazol-5-yl-carbonsäure-N-(2-cyanoethyl)-N-(2-phenylethyl)-amid gelingt die Umsetzung in Analogie zu Beispiel 1, Stufe g. Die Reinigung erfolgt durch Chromatographie über Kieselgel (Dichlormethan:Methanol 4:1 - 7:3) und/oder Kristallisation Methanol/Aceton mit Wasser. Ausbeute: 52%; Schmp.: 185-190°C;
   Masse: ber.: [495] gef.: [M+H]⁺ 496;
   ¹H-NMR (250MHz, DMSO-d6): δ [ppm] = 9.38, 9.26, 9.19, 8.77 (8H, 4s, 2 C(=NH₂⁺)NH₂); 7.84-6.83 (12H, m, aryl-H); 3.84, 2.78 (4H, 2m, N-CH₂-CH₂); 3.74 (3H, s, aryl-N-CH₃); 3.46, 2.78 (4H, 2m, N-CH₂-CH₂-Ph); 3.26 (4H, s, aryl-CH₂-CH₂-).

### Beispiel 25: 2-[2-(4-Amidinophenyl)-ethyl]-1-methyl-benzimidazol-5-yl-carbonsäureN-(2-amidinoethyl)-N-cyclooctyl-amid-dihydrochlorid

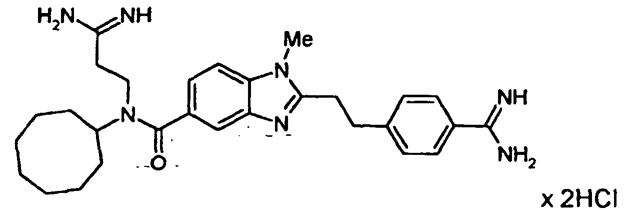

a) 2-[2-(4-Cyanophenyl)-ethyl]-1-methyl-benzimidazol-5-yl-carbonsäure-N-(2-cyanoethyl)-N-cyclooctyl-amid:
   Die Synthese erfolgt ausgehend von 4.5 mmol 2-[2-(4-Cyanophenyl)-ethyl]-1-methylbenzimidazol-5-yl-carbonsäurechlorid durch Umsetzung mit 3-(Cyclooctylamino)-propionitril in Analogie zu Beispiel 2, Stufe b. Das Produkt wird durch Kristallisation aus Ethylacetat zugänglich. Schmp.: 181-183°C;
b) 2-[2-(4-Amidinophenyl)-ethyl]-1-methyl-benzimidazol-5-yl-carbonsäure- N-(2-amidinoethyl)-N-cyclooctyl-amid-dihydrochlorid:
   Ausgehend von 3.8 mmol 2-[2-(4-Cyanophenyl)-ethyl]-1-methyl-benzimidazol-5-yl-carbonsäure-N-(2-cyanoethyl)-N-cyclooctyl-amid gelingt die Umsetzung in Analogie zu Beispiel 1, Stufe g. Die Reinigung erfolgt durch Chromatographie über Kieselgel (Dichlormethan:Methanol 4:1) und/oder Kristallisation Methanol/Aceton mit Wasser. Ausbeute: 47%, Schmp.: 200-210°C;
   Masse: ber.: [501] gef.: [M+H]⁺ 502;
   ¹H-NMR (250MHz, DMSO-d6): δ [ppm] = 9.33, 9.18, 9.13, 8.77 (8H, 4s, 2 C(=NH₂⁺)NH₂); 7.82-7.01 (8H, m, aryl-H); 3.88 (1H, m, N-CH-Cyclooctyl); 3.74 (3H, s, aryl-N-CH₃); 3.58, 2.79 (4H, 2m, N-CH₂-CH₂); 3.24 (4H, s, aryl-CH₂-CH₂-); 1.95-0.74 (14H, m, cyclooctyl).

### Beispiel 26: 2-[2-(4-Amidinophenyl)-ethyl]-1-methyl-benzimidazol-5-yl-carbonsäureN-(2-amidinoethyl)-N-(4-methyl-phenyl)-amid-dihydrochlorid

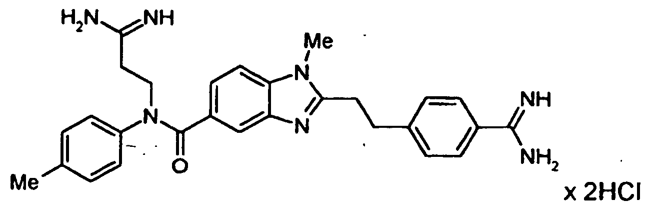

a) 3-(*p*-Toluyl-amino)-propionitril:
   Die Umsetzung gelingt analog zur Synthese nach Beispiel 22, Stufe a ausgehend von 4-Methylanilin. Die Reinigung des Produkts erfolgt durch Chromatographie über Kieselgel (Hexan: Ethylacetat 9:1) und/oder Kristallisation aus Hexan.
   Ausbeute: 69%;
b) 2-[2-(4-Cyanophenyl)-ethyl]-1-methyl-benzimidazol-5-yl-carbonsäure-N-(2-cyanoethyl)-N-(4-methyl-phenyl)-amid:
   Die Synthese erfolgt ausgehend von 4.5 mmol 2-[2-(4-Cyanophenyl)-ethyl]-1-methylbenzimidazol-5-yl-carbonsäurechlorid durch Umsetzung von 3-(*p*-Toluyl-amino)-propionitril in Analogie zu Beispiel 2, Stufe b. Das Produkt wird durch Kristallisation aus Ethylacetat/Diethylether zugänglich. Schmp.: 149-152°C;
c) 2-[2-(4-Amidinophenyl)-ethyl]-1-methyl-benzimidazol-5-yl-carbonsäure- N-(2-amidinoethyl)-N-(4-methyl-phenyl)-amid-dihydrochlorid:
   Ausgehend von 2-[2-(4-Cyanophenyl)-ethyl]-1-methyl-benzimidazol-5-yl-carbonsäure-N-(2-cyanoethyl)-N-(4-methyl-phenyl)-amid gelingt die Umsetzung in Analogie zu Beispiel 1, Stufe g. Ausbeute: 44 %; Schmp.: 222-225°C;
   Masse: ber.: [481] gef.: [M+H]⁺ 482;
   ¹H-NMR (250MHz, DMSO-d6): δ [ppm] = 9.41, 9.25, 9.18, 8.80 (8H, 4s, 2 C(=NH₂⁺)NH₂); 7.89-7.01 (11H, m, aryl-H); 4.15, 2.78 (4H, 2m, N-CH₂-CH₂); 3.67 (3H, s, aryl-N-CH₃); 3.18 (4H, s, aryl-CH₂-CH₂-); 2.20 (3H, s, aryl-CH₃).

### Beispiel 27: 2-[2-(4-Amidinophenyl)-ethyl]-1-methyl-benzimidazol-5-yl-carbonsäureN-(2-amidinoethyl)-N-iso-propyl-amid-dihydrochlorid

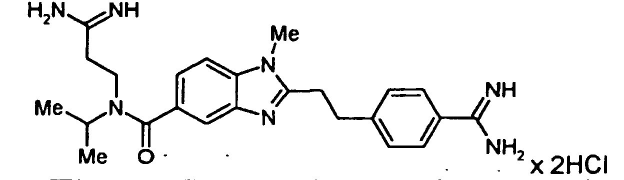

a) 3-(*i*-Propylamino)-propionitril:
   Die Synthese gelingt in Analogie zu Beispiel 8 (Stufe a) ausgehend von 40 mmol iso-Propylamin. Das erhaltene Produkt wird ohne weitergehende Reinigung direkt weiter umgesetzt. Ausbeute: 89%;
b) 2-[2-(4-Cyanophenyl)-ethyl]-1-methyl-benzimidazol-5-yl-carbonsäure-N-(2-cyanoethyl)-N-iso-propyl-amid:
   Die Synthese erfolgt ausgehend von 3.1 mmol 2-[2-(4-Cyanophenyl)-ethyl]-1-methylbenzimidazol-5-yl-carbonsäurechlorid durch Umsetzung mit 3-(i-Propylamino)-propionitril in Analogie zu Beispiel 2, Stufe b. Das Produkt wird durch Kristallisation aus Ethylacetat/Diethylether zugänglich und direkt in die nächste Stufe eingesetzt.
c) 2-[2-(4-Amidinophenyl)-ethyl]-1-methyl-benzimidazol-5-yl-carbonsäure-N-(2-amidinoethyl)-N-iso-propyl-amid-dihydrochlorid:
   Ausgehend von 3.1 mmol 2-[2-(4-Cyanophenyl)-ethyl]-1-methyl-benzimidazol-5-yl-carbonsäure-N-(2-cyanoethyl)-N-iso-propyl-amid gelingt die Umsetzung in Analogie zu Beispiel 1, Stufe g. Das Produkt wird durch Chromatographie über Kieselgel (Dichlormethan:Methanol 4:1 - 7:3) und/oder Kristallisation aus Methanol/Aceton mit Wasser gereinigt. Ausbeute: 59%; Schmp.: 195-200°C;
   Masse: ber.: [433] gef.: [M+H]⁺ 434;
   ¹H-NMR (250MHz, DMSO-d6): δ [ppm] = 9.93, 9.22, 9.20, 8.80 (8H, 4s, 2 C(=NH₂⁺)NH₂); 7.89-7.19 (7H, m, aryl-H); 3.39 (1H, m, N-CH-Me₂); 3.65, 2.74 (4H, 2m, N-CH₂-CH₂); 3.78 (3H, s, aryl-N-CH₃); 3.29 (4H, s, aryl-CH₂-CH₂-); 1.12 (6H, d, J=6.7 Hz, CH(CH₃)₂).

### Beispiel 28: 2-{2-[2-(4-Amidinophenyl)-ethyl]-1-methyl-benzimidazol-5-yl-carbonylamino}-3-(4-amidinophenyl)-propionsäure-ethyl-ester

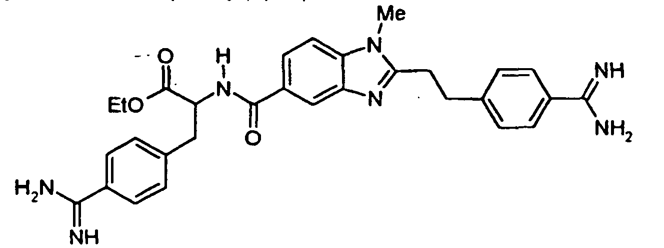

a) 2-{2-[2-(4-Cyanophenyl)-ethyl]-1-methyl-benzimidazol-5-yl-carbonylamino}-3-(4-cyanophenyl)-propionsäureethylester:
   Die Synthese erfolgt ausgehend von 5 mmol 2-[2-(4-Cyanophenyl)-ethyl]-1-methylbenzimidazol-5-yl-carbonsäure durch Umsetzung mit 2-Amino-3-(4-cyanophenyl)-propionsäureethylester in Analogie zu Beispiel 1, Stufe f. Das Produkt wird über Kieselgel chromatographiert (Dichlormethan:Methanol 50:1). Ausbeute: 92%;
   ¹H-NMR (250MHz, DMSO-d6): δ [ppm] = 8.26-7.37 (11 H, m, aryl-H); 6.87 (1 H, m, NHCO); 5.23 (1H, m, NH-CH-); 4.32 (2H, q, J=7.5 Hz, -O-CH₂); 3.86 (3H, s, OCH₃); 3.78 (3H, s, aryl-N-CH₃); 3.56-3.19 (6H, m, aryl-CH₂, aryl-CH₂-CH₂-); 1.31 (3H, t, J=7.5 Hz, -O-CH₂-CH₃).
b) 2-{2-[2-(4-Amidinophenyl)-ethyl]-1-methyl-benzimidazol-5-yl-carbonylamino}-3-(4-amidinophenyl)-propionsäureethylester:
   Ausgehend von 5 mmol 2-{2-[2-(4-Cyanophenyl)-ethyl]-1-methyl-benzimidazol-5-yl-carbonylamino}-3-(4-cyanophenyl)-propionsäureethylester gelingt die Synthese in Analogie zur Vorschrift unter Beispiel 1 (Stufe g). Das Produkt wird durch Kristallisation aus Ethanol/Ethylacetat gereinigt. Ausbeute: 70%;
   ¹H-NMR (250MHz, DMSO-d6): δ [ppm] = 9.27, 9.24, 9.06, 9.03 (8H, 4s, 2 C(=NH₂⁺)NH₂); 8.81 (1H, m, NHCO); 8.10-6.90 (11H, m, aryl-H); 4.71 (1H, m, NH-CH-); 4.08 (2H, q, J=7.5 Hz, -O-CH₂); 3.72 (3H, s, aryl-N-CH₃); 3.50-3.18 (2H, m, aryl-CH₂); 3.24 (4H, s, aryl-CH₂-CH₂-); 1.16 (3H, t, J=7.5 Hz, -O-CH₂-CH₃).

### Beispiel 29: 2-{2-[2-(4-Amidinophenyl)-ethyl]-1-methyl-benzimidazol-5-yl-carbonyl-(methylamino)}-3-(4-amidinophenyl)-propionsäure-ethyl-ester -formiat

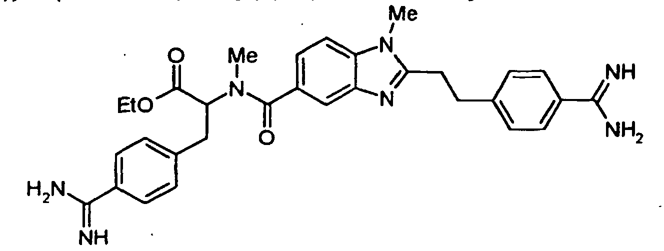

a) 2-{2-[2-(4-Cyanophenyl)-ethyl]-1-methyl-benzimidazol-5-yl-carbonyl-(methylamino)}-3-(4-cyanophenyl)-propionsäure-ethyl-ester:
   Die Synthese erfolgt ausgehend von 7 mmol 2-[2-(4-Cyanophenyl)-ethyl]-1-methylbenzimidazol-5-yl-carbonsäure durch Umsetzung mit 2-Methylamino-3-(4-cyanophenyl)-propionsäureethylester in Analogie zu Beispiel 1, Stufe f. Das Produkt wird über Kieselgel chromatographiert (Dichlormethan:Methanol 20:1). Ausbeute: 61%;
   ¹H-NMR (250MHz, CDCl₃): δ [ppm] = 7.83-7.23 (11H, m, aryl-H); 5.47 (1H, m, N-CH); 4.38 (2H, m, -O-CH₂-); 3.69 (3H, s, aryl-N-CH₃); 3.38, 3.22 (4H, 2m, aryl-CH₂-CH₂); 3.12 (2H, m, aryl-CH₂-CH-); 2.91 (3H, s, CO-N-CH₃); 1.35 (3H, t, -O-CH₂-CH₃).
b) 2-{2-[2-(4-Amidinophenyl)-ethyl]-1-methyl-benzimidazol-5-yl-carbonyl-(methylamino)}-3-(4-amidinophenyl)-propionsäure-ethyl-ester:
   Ausgehend von 2.8 mmol 2-{2-[2-(4-Cyanophenyl)-ethyl]-1-methyl-benzimidazol-5-yl-carbonyl-(methylamino)}-3-(4-cyanophenyl)-propionsäureethylester gelingt die Synthese in Analogie zur Vorschrift unter Beispiel 1 (Stufe g). Das Produkt wird über Kieselgel chromatographiert (Acetonitril:Dichlormethan:Ameisensäure:Wasser 75:20:7.5:5). Ausbeute: 36%; Schmp.:230°C;
   Masse: ber.: [539]/[553], gef.: [M+H]⁺ 540, [M+H]⁺ 554;
   ¹H-NMR (250MHz, DMSO-d6): δ [ppm] = 8.17 (1H, s, HCOOH); 8.03-6.94 (11H, m, aryl-H); 5.37 (1H, m, N-CH-); 4.35 (2H, m, -O-CH₂-); 3.90 (3H, s, aryl-N-CH₃); 3.52, 3.39 (4H, 2m, aryl-CH₂-CH₂-); 2.87 (3H, s, CO-N-CH₃); 3.48 (2H, m, aryl-CH₂-CH-); 1.35 (3H, m, -O-CH₂-CH₃). .

### Beispiel 30: 2-{2-[2-(4-Amidinophenyl)-ethyl]-1-methyl-benzimidazol-5-yl-carbonylamino}-3-(4-amidinophenyl)-propionsäure -diformiat

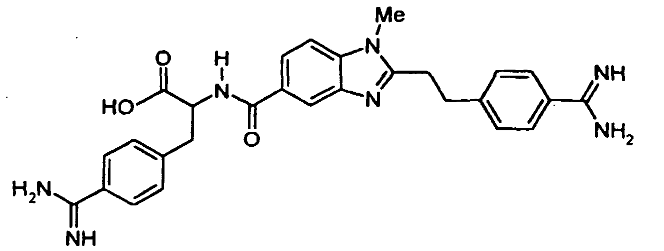

Nach Verseifung von 2-{2-[2-(4-Amidinophenyl)-ethyl]-1-methyl-benzimidazol-5-yl-carbonylamino}-2-(4-amidinobenzyl)-propionsäureethylester (Beispiel 28; 1.55 g, 2.5 mmol) mit überschüssiger wässriger Natriumhydroxidlösung (1N) in Methanol im Verhältnis 1:2 bei Raumtemperatur über 16 h wird mit einer der zugesetzten Natriumhydroxid-Menge entstprechenden Menge wässriger Salzsäurelösung (1N) versetzt und nach dem Abdestillieren des Lösemittels im Vakuum über Kieselgel chromatographiert (Acetonitril:Dichlormethan:Ameisensäure:Wasser 75:20:7.5:5). Ausbeute: 0.55 g (36%); Schmp: 165°C;
Masse: ber.: [511], gef.: [M+H]⁺ 512;
¹H-NMR (250MHz, CD₃OD): δ [ppm] = 8.07 (2H, s, HCOOH ); 7.95-7.32 (11H, m, aryl-H); 4.90 (1H, m, NH-CH); 3.69 (3H, s, aryl-N-CH₃); 3.55-3.12 (6H, m, aryl-CH₂-CH₂-, aryl-CH₂).

### Beispiel 31: 2-{2-[2-(4-Amidinophenyl)-ethyl]-1-methyl-benzimidazol-5-yl-carbonyl-methylamino}-3-(4-amidinophenyl)-propionsäure -formiat

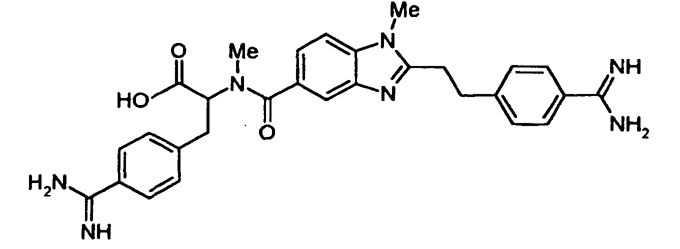

Die Umsetzung gelingt in Analogie zu der unter Beispiel 30 beschriebenen Vorgehensweise ausgehend von 2-{2-[2-(4-Amidinophenyl)-ethyl]-1-methylbenzimidazol-5-yl-carbonyl-(methylamino)}-3-(4-amidinophenyl)-propionsäure-ethylester (Beispiel 29, 1.7 mmol). Das Produkt wird durch Kristallisation aus Dichlormethan/Methanol erhalten. Ausbeute: >90%; Schmp.: 255°C;
Masse: ber.: [525], gef.: [M+H]⁺ 526;
¹H-NMR (250MHz, DMSO-d6): δ [ppm] = 10.20 (1H, s, COOH); 8.61 (1H, s, HCOOH); 8.09-6.77 (17H, m, 2 C(=NH)NH₂, aryl-H); 5.37 (1H, m, N-CH-); 3.78 (3H, s, aryl-N-CH₃); 3.33 (4H, s, aryl-CH2-CH₂-); 2.98 (3H, s, CO-N-CH₃).

### Beispiel 32: 2-{2-[2-(4-Amidinophenyl)-ethyl]-1-methyl-benzimidazol-5-yl-carbonsäure-[N-(4-amidinobenzyl)-N-carboxymethyl-amid]-diacetat

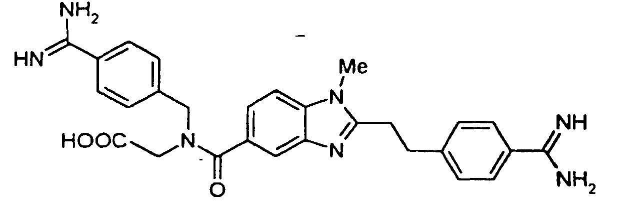

Die Umsetzung gelingt in Analogie zu der unter Beispiel 30 beschriebenen Vorgehensweise ausgehend vom entsprechenden Ethylester (4.4 mmol).
Ausbeute. 48%;
Masse: ber. [511], gef.: [M+H]⁺ 512, [M+2H]²⁺ 256;
¹H-NMR (250MHz, DMSO-d6/CD₃OD): δ [ppm] = 8.17-7.44 (11H, m, aryl-H); 4.65 (2H, s, N-CH₂-); 4.09 (2H, s, N-CH₂-C=O); 3.82 (3H, s, aryl-N-CH₃); 3.57, 3.32 (4H, 2m, aryl-CH₂-CH₂-); 1.86 (6H, s, CH₃-COO).

### Beispiel 33: 2-{2-[4-(Amino-hydroximino-methyl) phenyl]-ethyl}-1-methylbenzimidazol-5-yl-carbonsäure-N-[2-(N',N'-dimethylamino)-ethyl]-N-methyl-amid

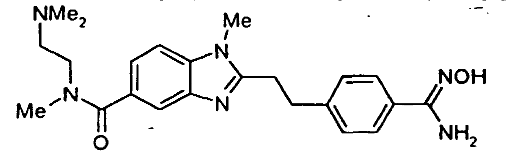

1.0 g (2.8 mmol) 2-[2-(4-Cyanophenyl)-ethyl]-1-methyl-benzimidazol-5-yl-carbonsäure-[(N',N'-dimethylaminoethyl)-N-methyl-amid] (erhältlich gemäß Beispiel 3, Stufe a), NH₂OH*HCl (0,83g) und Na₂CO₃ (0,65 g) in 50 mL Methanol werden 3 h unter Rückfluß erhitzt. Es werden nochmals NH₂OH*HCl (0,4g) und Na₂CO₃ (0,3 g) hinzugefügt. Nach 1 h Erhitzen unter Rückfluß wird das MeOH abdestilliert, der Rückstand mit Wasser aufgeschlämmt, filtriert, mit Wasser gewaschen und getrocknet. Ausbeute: 59 %.
¹H-NMR (250MHz, DMSO-d6): δ [ppm] = 9.58 (1H, s, OH); 7.56-7.18 (7H, m, aryl-H); 5.77 (2H, s, NH₂); 3.72 (3H, s, aryl-N-CH₃); 3.18 (6H, m, aryl-CH₂-CH₂-; N-CH₂-); 2.98 (3H, s, CO-N-CH₃); 2.43 (2H, m, Me₂-N-CH₂-); 2.16, 2.00 (6H, 2s, N-(CH₃)₂);

### Beispiel 34: 2-{2-[2-(4-(Amidinophenyl)-ethyl]-1-methyl-benzimidazol-5-yl-carbonylamino}-6-acetylamino-hexansäure-formiat

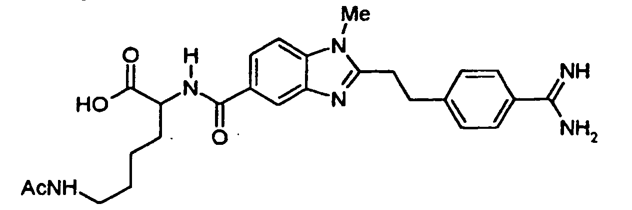

a) 2-{2-[2-(4-Cyanophenyl)-ethyl]-1-methyl-benzimidazol-5-yl-carbonylamino}-6-benzyloxycarbonyl-amino-hexansäurebenzylester:
   Die Synthese erfolgt ausgehend von 5 mmol 2-[2-(4-Cyanophenyl)-ethyl]-1-methylbenzimidazol-5-yl-carbonsäure durch Umsetzung mit 2-Amino-6-benzyloxycarbonyl-amino-hexansäurebenzylester in Analogie zu Beispiel 1, Stufe f. Das Produkt wird über Kieselgel chromatographiert (Dichlormethan:Methanol 50:1).
   Ausbeute: 66%;
b) 2-{2-[2-(4-(Amino-hydroximino-methyl)-phenyl)-ethyl]-1-methyl-benzimidazol-5-yl-carbonylamino}-6-benzyloxycarbonylamino-hexansäurebenzylester:
   Ausgehend von 3 mmol 2-{2-[2-(4-Cyanophenyl)-ethyl]-1-methyl-benzimidazol-5-yl-carbonylamino}-6-benzyloxycarbonyl-amino-hexansäurebenzylester gelingt die Synthese in Analogie zur Vorschrift unter Beispiel 33. Als Base wird Kaliumtert.-butylat verwendet. Das Produkt wird über Kieselgel chromatographiert (Dichlormethan:Methanol 20:1). Ausbeute: 75%;
c) 2-{2-[2-(4-(Amidinophenyl)-ethyl]-1-methyl-benzimidazol-5-yl-carbonylamino}-6-acetylamino-hexansäure-formiat:
   1.4 g (2 mmol) 2-{2-[2-(4-(Amino-hydroximino-methyl)-phenyl)-ethyl]-1-methylbenzimidazol-5-yl-carbonylamino}-6-benzyloxycarbonylamino-hexansäurebenzylester werden in 10mL Essigsäure und 2 mL Acetanhydrid aufgenommen und 16 h bei Raumtemperatur gehalten. Das Lösemittel wird im Vakuum abdestilliert und der verbleibende Rückstand in 40 mL Methanol in Gegenwart von 5 %igem Pd/C (0.6 g) bei Normaldruck hydriert. Der Katalysator wird abfiltriert, das Filtrat eingeengt und der Rückstand über Kieselgel (Acetonitril:Dichlormethan:Ameisensäure:Wasser 75:20:7.5:5) chromatographiert. Ausbeute: 0.4 g (35%); Schmp: 158 °C;
   Masse: ber.: [492], gef.: [M+H]⁺ 493;
   ¹H-NMR (250MHz, CD₃OD): δ [ppm] = 8.27 (1H, s, HCOOH); 8.01-7.55 (7H, m, aryl-H); 4.66 (1H, m, N-CH-); 3.78 (3H, s, aryl-N-CH₃); 3.42-3.26 (4H, s, aryl-CH₂-CH₂-); 3.23 (2H, m, -N-CH₂-); 2.02-1.58 (6H, m, -N-CH₂-CH₂-CH₂-CH₂-); 1.92 (3H, s, NH-CO-CH₃).

### Beispiel 35: 2-{2-[2-(4-(Amidinophenyl)-ethyl]-1-methyl-benzimidazol-5-yl-carbonylamino}-2-(4-acetylaminomethyl-phenyl)-propionsäure

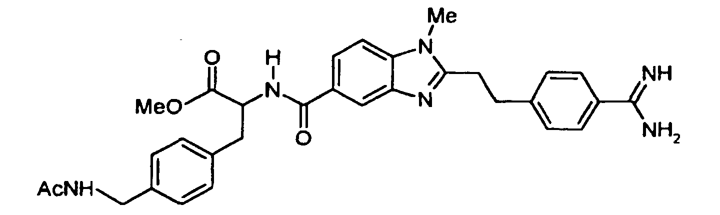

a) 2-{2-[2-(4-(Cyanophenyl)-ethyl]-1-methyl-benzimidazol-5-yl-carbonylamino}-3-[4-benzyloxycarbonylaminomethyl-phenyl]-propionsäure-methyl-ester:
   Die Synthese erfolgt ausgehend von 1.2 mmol 2-[2-(4-Cyanophenyl)-ethyl]-1-methylbenzimidazol-5-yl-carbonsäure durch Umsetzung mit 2-Amino-3-[4-benzyloxy-carbonylaminomethyl-phenyl]-propionsäure-methyl-ester in Analogie zu Beispiel 1, Stufe f. Das Produkt wird über Kieselgel chromatographiert (Dichlormethan:Methanol 50:1). Ausbeute: 90%;
b) 2-{2-[2-(4-(Amino-hydroximino-methyl)-phenyl)-ethyl]-1-methyl-benzimidazol-5-yl-carbonylamino}-3-[4-benzyloxycarbonylaminomethyl-phenyl]-propionsäure-methylester:
   Ausgehend von 1 mmol 2-{2-[2-(4-(Cyanophenyl)-ethyl]-1-methyl-benzimidazol-5-yl-carbonylamino}-3-[4-benzyloxycarbonylaminomethyl-phenyl]-propionsäure-methylester gelingt die Synthese in Analogie zur Vorschrift unter Beispiel 33. Als Base wird Kaliumtert.-butylat verwendet. Das Produkt wird über Kieselgel chromatographiert (Dichlormethan:Methanol 20:1). Ausbeute: 74%;
   ¹H-NMR (250MHz, DMSO-d6): δ [ppm] = 9.57 (1H, s, OH); 8.79 (1H, d, J=7.5 Hz, NH-CH-); 7.79 (1H, t, J=6.5 Hz, NH-CH₂-); 8.18-7.13 (16H, m, aryl-H); 5.76 (2H, s, NH₂); 5.04 (2H, s, O-CH₂-); 4.65 (1H, m, NH-CH-); 4.17 (1H, d, J=6.5 Hz, NH-CH₂-); 3.71 (3H, s, -OCH₃); 3.66 (3H, s, aryl-N-CH₃); 3.39 (4H, m, aryl-CH₂-CH₂-); 3.18 (2H, m, aryl-CH₂-).
c) 2-{2-[2-(4-(Amidinophenyl)-ethyl]-1-methyl-benzimidazol-5-yl-carbonylamino}-2-(4-acetylaminomethyl-phenyl)-propionsäure:
   Ausgehend von 0.7 mmol 2-{2-[2-(4-(Amino-hydroximino-methyl)-phenyl)-ethyl]-1-methyl-benzimidazol-5-yl-carbonylamino}-3-[4-benzyloxycarbonylaminomethyl-phenyl]-propionsäure-methyl-ester gelingt die Synthese in Analogie zur Vorschrift unter Beispiel 34 (letzte Stufe). Masse: ber.: [492], gef.: [M+H]⁺ 493;
   ¹H-NMR (250MHz, DMSO-d6): δ [ppm] = 10.1 (3H, breit, C(=NH)NH₂); 8.89 (1H, d, J=7.6 Hz, NH-CH); 8.40 (1H, t, J=5.8 Hz, NH-CH₂); 8.23-7.14 (11H, m, aryl-H); 4.69 (1H, m, CH-CH₂-); 4.24 (2H, d, J=5.8 Hz, NH-CH₂); 3.78 (3H, s, OCH₃); 3.69 (3H, s, aryl-N-CH₃); 3.29 (4H, s, aryl-CH₂-CH₂-); 3.15 (2H, m, CH-CH₂-); 1.86 (3H, s, COCH₃).

### Beispiel 36: 2-[2-(4-Amidinophenyl)-ethyl]-1-methyl-benzimidazol-5-yl-carbonsäure-[N-(2-(N'-benzylamino)-ethyl)-N-cyclohexyl-amid]-dihydrochlorid:

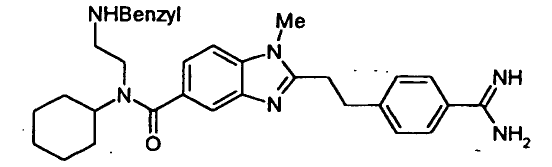

4.0 g (5.7 mmol) 2-[2-(4-Amidinophenyl)-ethyl]-1-methyl-benzimidazol-5-yl-carbonsäure-N-(2-(N',N'-dibenzylamino)-ethyl)-N-cyclohexyl-amid (Beispiel 2) werden in 100 mL Methanol in Gegenwart von 5 %igem Pd/C bei Normaldruck und 40-60 °C hydriert. Der Katalysator wird abfiltriert, das Filtrat eingeengt und der Rückstand über Kieselgel (Dichlormethan/Methanol 9:1 - 7:3) chromatographiert. Ausbeute: 0.6 g (17%).
Masse: ber.: [536], gef.: [M+H]⁺ 537, [M+2H]²⁺ 269;
¹H-NMR (250MHz, DMSO-d6): δ [ppm] = 9.34, 9.80 (4H, 2s, C(=NH₂⁺)NH₂); 7.84-7.10 (12H, m, aryl-H); 4.14, 3.09 (4H, 2m, N-CH₂-CH₂-N); 3.76 (3H, s, aryl-N-CH₃); 3.70 (2H, s, N-CH₂-Ph); 3.47 (1H, m, N-cyclohexyl-H); 3.26 (4H, s, aryl-CH₂-CH₂-); 1.92-0.54 (10H, m, cyclohexyl).
Aus dem gleichen Ansatz wurde ferner erhalten:

### Beispiel 37: 2-[2-(4-Amidinophenyl)-ethyl]-1-methyl-benzimidazol-5-yl-carbonsäure-[N-(2-amino-ethyl)-N-cyclohexyl-amid]-dihydrochlorid;

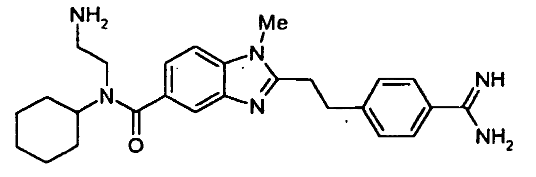

Ausbeute: 0,4 g (13%);
Masse: ber.: [446], gef.: [M+H]⁺ 447, [M+2H]²⁺ 224;
¹H-NMR (250MHz, DMSO-d6): δ [ppm] = 8.56 (4H, breit, C(=NH₂⁺)NH₂); 7.66-6.98 (7H, m, aryl-H); 3.57 (3H, s, aryl-N-CH₃); 3.45, 2.78 (4H, 2m, N-CH₂-CH₂-N); 3.35 (1H, m, N-cyclohexyl-H); 3.07 (4H, s, aryl-CH₂-CH₂-); 1.49 (2H, s, NH₂); 1.65-0.55 (10H, m, cyclohexyl).

### Beispiel 38: 2-[2-(4-Amidinophenyl)-ethyl]-1-methyl-benzimidazol-5-yl-carbonsäureN-(2-phenylamino-ethyl)-amid-dihydrochlorid

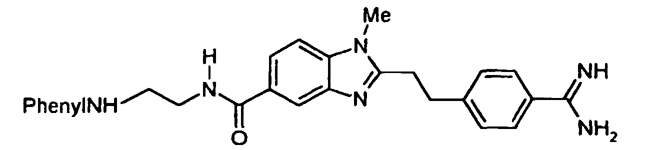

Bei einer analogen Durchführung zu Beispiel 36 wurde ausgehend von 2-[2-(4-Amidinophenyl)-ethyl]-1-methyl-benzimidazol-5-yl-carbonsäure- [N-(2-(N',N'-dibenzylamino)-ethyl)-N-phenyl-amid] (Beispiel 19) Beispiel 38 isoliert.
Ausbeute: 7%;
Masse: ber. [440], gef.: [M+H]⁺ 441, [M+2H]²⁺ 221;
¹H-NMR (250MHz, DMSO-d6): δ [ppm] = 8.20-6.57 (12H, m, aryl-H); 3.78 (3H, s, aryl-N-CH₃); 3.51, 3.26 (4H, 2m, N-CH₂-CH₂-N); 3.32 (4H, s, aryl-CH₂-CH₂-).

### Beispiel 39: 2-[2-(4-Aminomethyl-phenyl)-ethyl]-1-methyl-benzimidazol-5-yl-carbonsäure- N-(2 -phenylamino-ethyl)-amid- trihydrochlorid

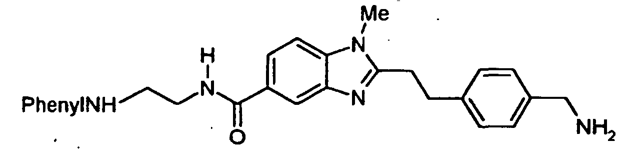

a) 2-[2-(4-Cyanophenyl)-ethyl]-1-methyl-benzimidazol-5-yl-carbonsäure-N-(2 - phenylamino-ethyl)-amid:
   Die Synthese erfolgt ausgehend von 8.2 mmol 2-[2-(4-Cyanophenyl)-ethyl]-1-methylbenzimidazol-5-yl-carbonsäure durch Umsetzung mit N-Phenylethylendiamin in Analogie zu Beispiel 1, Stufe f. Das Produkt wird durch Kristallisation aus Ethylacetat/Diethylether erhalten und ggebenenfalls aus Acetonitril umkristallisiert.. Ausbeute: 86%; Schmp.: 161-163°C;
   ¹H-NMR (250MHz, CDCl₃): δ [ppm] = 8.24-6-67 (12H, m, aryl-H); 4.21 (1H, breit, NHCO); 3.79, 3.36 (4H, 2m, N-CH₂-CH₂-N); 3.66 (3H, s, aryl-N-CH₃); 3.66, 3.21 (4H, 2m, aryl-CH₂-CH₂-); 1.89 (1H, breit, Ph-NH).
b) 2-[2-(4-Aminomethyl-phenyl)-ethyl]-1-methyl-benzimidazol-5-yl-carbonsäure- [N-(2 -phenylamino-ethyl)-amid]:
   2.0 g (4.7 mmol) 2-[2-(4-Cyanophenyl)-ethyl]-1-methyl-benzimidazol-5-yl-carbonsäure-N-(2 -phenylamino-ethyl)-amid werden in 100 mL Methanol in Gegenwart von ca. 2.5 g Methanol-feuchtem Raney-Nickel bei Raumtemperatur unter Normaldruck hydriert. Der Katalysator wird abfiltriert, mit Methanol gewaschen und das Lösemittel im Vakuum abdestilliert. Der Rückstand wird über Kieselgel (Dichlormethan:Methanol 85:1) chromatographiert, das Trihydrochlorid aus Ethanol/Aceton mit konz. Salzsäure kristallisiert.
   Ausbeute: 1.5 g (59%); Schmp: > 220°C;
   Masse: ber.: [427], gef.: [M+H]⁺ 428;
   ¹H-NMR (250MHz, DMSO-d6): δ [ppm] = 8.51 (1H, t, J=5.5 Hz, NHCO); 8.15-6.44 (12H, m, aryl-H); 5.71 (1H, t, J=5.5 Hz, -NH-Ph); 3.68 (3H, s, aryl-N-CH₃); 3.65 (2H, s, -N-CH₂-Ph); 3.44, 3.12 (4H, 2m, N-CH₂-CH₂-N); 3.22, 3.16 (4H, 2m, aryl-CH₂-CH₂-); 3.35 (2H, breit, NH₂).

### Beispiel 40: 2-[2-(4-Aminomethyl-phenyl)-ethyl]-1-methyl-benzimidazol-5-yl-carbonsäure-N-(3,3 -diphenylpropyl)-amid-dihydrochlorid:

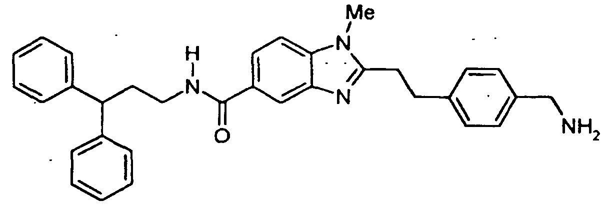

Ausgehend von 2.8 mmol 2-[2-(4-Cyanophenyl)-ethyl]-1-methyl-benzimidazol-5-yl-carbonsäure-N-(3,3-diphenylpropyl)-amid (Beispiel 13, Stufe b) gelingt die Synthese in Anlehnung an die Vorschrift gemäß Beispiel 39, Stufe b. Die Umsetzung wird in Gegenwart von 8 g Ammoniak bei 5 bar und 60°C durchgeführt. Der Rückstand wird über Kieselgel (Dichlormethan:Methanol 95:5 - 9:1) chromatographiert.
Ausbeute: 23%; Schmp: 140°C;
-Masse: ber.: [502], gef.: [M+H]⁺ 503;
¹H-NMR (250MHz, DMSO-d6): δ [ppm] = 8.99 (1H, t, J=5.4 Hz, NHCO); 8.67 (3H, breit NH₃⁺); 8.50-7.29 (17H, m, aryl-H); 4.29, 4.15, 2.52 (5H, 3m, N-CH₂-CH₂-CH); 4.06 (2H, s, -CH₂-NH₂); 3.68 (3H, s, aryl-N-CH₃); 3.63, 3.39 (4H, 2m, aryl-CH₂-CH₂-).

### Beispiel 41: 2-[2-(4-Aminomethyl-phenyl)-ethyl]-1-methyl-benzimidazol-5-yl-carbonsäure-[N'-(2-methyl-phenyl)-diazepid]- hydrochlorid

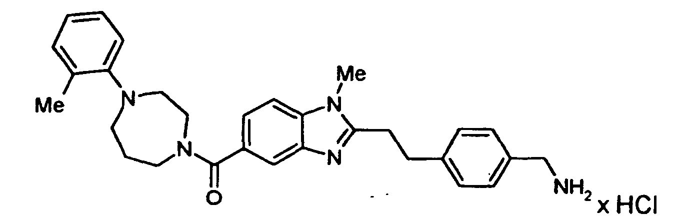

Ausgehend von 2.5 mmol 2-[2-(4-Cyanophenyl)-ethyl]-1-methyl-benzimidazol-5-yl-carbonsäure-[N'-(2-methyl-phenyl)-diazepid] (Beispiel 15, Stufe b) gelingt die Synthese in Anlehnung an die Vorschrift gemäß Beispiel 39, Stufe b. Die Umsetzung wird in Gegenwart von 8 g Ammoniak bei 5 bar und 60°C durchgeführt. Der Rückstand wird über Kieselgel (Dichlormethan:Methanol 9:1) chromatographiert.
Ausbeute: 48%;
Masse: ber.: [481], gef.: [M+H]⁺ 482;
¹H-NMR (250MHz, CD₃OD): δ [ppm] = 8.13-7.24 (11H, m, aryl-H); 4.10 (2H, s, -CH₂-NH₂); 3.95 (3H, s, aryl-N-CH₃); 2.63 (3H, s, aryl-CH₃); 4.06-2.49 (10H, m, diazacycloheptanyl).

### Beispiel 42: 2-[2-(4-Aminomethyl-phenyl)-ethyl]-1-methyl-benzimidazol-5-yl-carbonsäure-N-(4-aminomethyl-benzyl)-N-(3-phenylpropyl)-amid-dihydrochlorid:

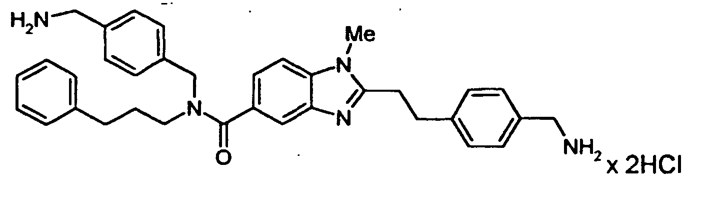

Ausgehend von 7.8 mmol 2-[2-(4-Cyanophenyl)-ethyl]-1-methyl-benzimidazol-5-yl-carbonsäure-N-(4-cyanobenzyl)-N-(3-phenylpropyl)-amid (Beispiel 12, Stufe b) gelingt die Synthese in Anlehnung an die Vorschrift gemäß Beispiel 39, Stufe b. Die Umsetzung wird in Gegenwart von 8 g Ammoniak bei 5 bar und 60°C durchgeführt. Der Rückstand wird über Kieselgel (Dichlormethan:Methanol 95:5 - 85:15) chromatographiert.
Ausbeute: 42%;
Masse: ber.: [545], gef.: [M+H]⁺ 546;
¹H-NMR (250MHz, DMSO-d6): δ [ppm] = 8.76 (6H, s, 2 -NH₃⁺); 8.15-6.93 (16H, m, aryl-H); 4.68 (2H, s, N-CH₂-Ph); 4.08, 4.02 (4H, 2s, CH₂-NH₂); 3.96 (3H, s, aryl-N-CH₃); 3.34 (4H, s, aryl-CH₂-CH₂-); 3.16-1.64 (6H, m, N-CH₂-CH₂-CH₂-).

### Beispiel 43: 2-[2-(4-Aminomethyl-phenyl)-ethyl]-1-methyl-benzimidazol-5-yl-carbonsäure-N-(4-aminomethylbenzyl)-N-(3,3 -diphenylpropyl)-amid-dihydrochlorid:

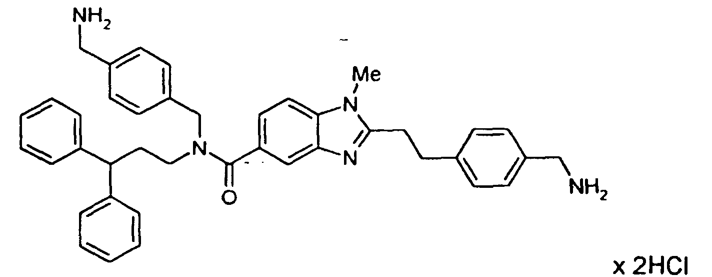

Ausgehend von 8.3 mmol 2-[2-(4-Cyanophenyl)-ethyl]-1-methyl-benzimidazol-5-yl-carbonsäure-N-(4-cyanobenzyl)-N-(3,3-diphenylpropyl)-amid (Beispiel 17, Stufe b) gelingt die Synthese in Anlehnung an die Vorschrift gemäß Beispiel 39, Stufe b. Die Umsetzung wird in Gegenwart von 10 g Ammoniak bei 5 bar und 60°C durchgeführt. Der Rückstand wird über Kieselgel (Dichlormethan:Methanol 9:1 - 4:1) chromatographiert. Ausbeute: 36%; Schmp: 140°C;
Masse: ber.: [621], gef.: [M+H]⁺ 622, [M+2H]²⁺ 312;
¹H-NMR (250MHz, DMSO-d6): δ [ppm] = 8.51 (6H, breit, -NH₃⁺); 7.74-7.08 (21H, m, aryl-H); 4.66 (2H, s, N-CH₂-); 4.02, 4.00 (4H, 2s, CH₂-NH₂); 3.84 (3H, s, aryl-N-CH₃); 3.84, 3.41, 2.24 (5H, 3m, N-CH₂-CH₂-CH).

### Beispiel 44: 2-{2-[4-(Amino-hydroximino-methyl) phenyl]-ethyl}-1-methylbenzimidazol-5-yl-carbonsäure-[N'-(2-methylphenyl)-piperazid]

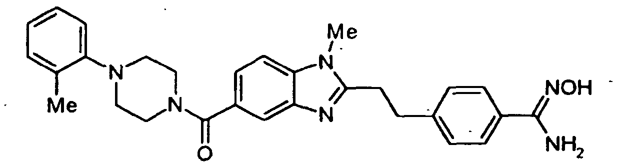

a) 2-[2-(4-Cyanophenyl)-ethyl]-1-methyl-benzimidazol-5-yl-carbonsäure-[N'-(2-methyl-phenyl)-piperazid]:
   Die Synthese erfolgt ausgehend von 7.5 mmol 2-[2-(4-Cyanophenyl)-ethyl]-1-methylbenzimidazol-5-yl-carbonsäurechlorid durch Umsetzung mit N-(2-Methylphenyl)piperazin in Analogie zu Beispiel 1, Stufe f. Das Produkt wird über Kieselgel chromatographiert (Dichlormethan:Methanol 50:1). Ausbeute: 58%;
b) 2-{2-[4-(Amino-hydroximino-methyl) phenyl]-ethyl}-1-methyl-benzimidazol-5-yl-carbonsäure-[N'-(2-methylphenyl)-piperazid]:
   Ausgehend von 5.4 mmol 2-[2-(4-Cyanophenyl)-ethyl]-1-methyl-benzimidazol-5-yl-carbonsäure-[N'-(2-methyl-phenyl)-piperazid] gelingt die Synthese in Analogie zur Vorschrift unter Beispiel 33. Als Base wird Kaliumtert.-butylat verwendet. Das Produkt wird über Kieselgel chromatographiert (Dichlormethan:Methanol 20:1). Masse: ber.: [496], gef.: [M+H]⁺ 497;
   ¹H-NMR (250MHz, DMSO-d6): δ [ppm] = 9.59 (1H, s, OH); 7.66-6.90 (11H, m, aryl-H); 5.76 (2H, s, -NH₂); 3.71 (3H, s, N-CH₃); 3.67 (4H, m -CH₂-CH₂-Ph); 3.17, 2.84 (8H, 2m, piperazinyl); 2.26 (3H, s, aryl-N-CH₃).

### Beispiel 45: 2-{2-[4-(Amino-hydroximino-methyl) phenyl)-ethyl}-1-methylbenzimidazol-5-yl-carbonsäure-[N'-(3-methylphenyl)-piperazid]

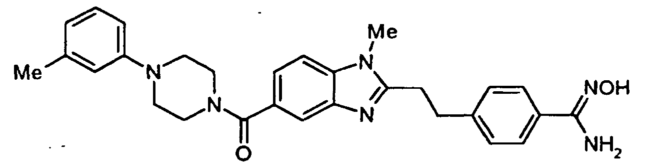

a) 2-[2-(4-Cyanophenyl)-ethyl]-1-methyl-benzimidazol-5-yl-carbonsäure-[N'-(3-methyl-phenyl)-piperazid]:
   Die Synthese erfolgt ausgehend von 7.5 mmol 2-[2-(4-Cyanophenyl)-ethyl]-1-methylbenzimidazol-5-yl-carbonsäurechlorid durch Umsetzung mit N-(3-Methylphenyl)piperazin in Analogie zu Beispiel 1, Stufe f. Das Produkt wird über Kieselgel chromatographiert (Dichlormethan: Methanol 50:1). Ausbeute: 88%;
b) 2-{2-[4-(Amino-hydroximino-methyl) phenyl]-ethyl}-1-methyl-benzimidazol-5-yl-carbonsäure-[N'-(3-methylphenyl)-piperazid]:
   Ausgehend von 6.4 mmol 2-[2-(4-Cyanophenyl)-ethyl]-1-methyl-benzimidazol-5-yl-carbonsäure-[N'-(3-methyl-phenyl)-piperazid] gelingt die Synthese in Analogie zur Vorschrift unter Beispiel 33. Als Base wird Kaliumtert.-butylat verwendet. Das Produkt wird über Kieselgel chromatographiert (Dichlormethan:Methanol 20:1). Masse: : ber.: [496], gef.: [M+H]⁺ 497;
   ¹H-NMR (250MHz, DMSO-d6): δ [ppm] = 9.56 (1H, s, OH); 7.66-6.90 (11H, m, aryl-H); 5.76 (2H, s, NH₂); 3.72 (3H, s, N-CH₃); 3.64 (4H, m, -CH₂-CH₂-Ph); 3.17 (8H, m, piperazinyl); 2.25 (3H, s, aryl-N-CH₃).

### Beispiel 46: 2-{2-[4-(Amino-hydroximino-methyl) phenyl]-ethyl}-1-methylbenzimidazol-5-yl-carbonsäure-(N'-benzyl-piperazid)

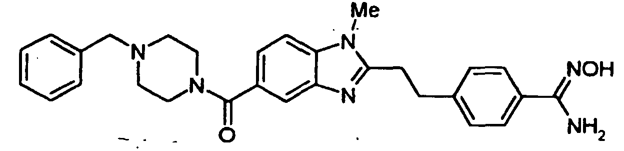

a) 2-[2-(4-Cyanophenyl)-ethyl]-1-methyl-benzimidazol-5-yl-carbonsäure-(N'-benzyl-piperazid):
   Die Synthese erfolgt ausgehend von 7.5 mmol 2-[2-(4-Cyanophenyl)-ethyl]-1-methylbenzimidazol-5-yl-carbonsäurechlorid durch Umsetzung mit N-Benzylpiperazin in Analogie zu Beispiel 1, Stufe f. Das Produkt wird über Kieselgel chromatographiert (Dichlormethan:Methanol 50: 1). Ausbeute: 91 %;
b) 2-{2-[4-(Amino-hydroximino-methyl) phenyl]-ethyl}-1-methyl-benzimidazol-5-yl-carbonsäure-(N'-benzyl-piperazid):
   Ausgehend von 6.6 mmol 2-[2-(4-Cyanophenyl)-ethyl]-1-methyl-benzimidazol-5-yl-carbonsäure-(N'-benzyl-piperazid) gelingt die Synthese in Analogie zur Vorschrift unter Beispiel 33. Als Base wird Kaliumtert.-butylat verwendet. Das Produkt wird über Kieselgel chromatographiert (Dichlormethan:Methanol 20:1).
   Masse: ber.: [496], gef.: [M+H]⁺ 497, [M+2H]²⁺ 249;
   ¹H-NMR (250MHz, DMSO-d6): δ [ppm] = 9.56 (1H, s, OH); 7.62-7.16 (12H, m, aryl-H); 5.76 (2H, s, NH₂); 3.70 (3H, s, N-CH₃); 3.50 (2H, s, N-CH₂-Ph); 3.39 (4H, m, -CH₂-CH₂-Ph); 3.16, 2.39 (8H, 2m, piperazinyl).

In Analogie zu den vorstehend beschriebenen Verfahren wurden ferner die folgenden Verbindungen erhalten:

### Beispiel 47: 2-[2-(4-Amidinophenyl)-ethyl]-1-methyl-benzimidazol-5-yl-carbonsäure-[N'-(3-methylphenyl)-piperazid]

### Beispiel 48: 2-[2-(4-Amidinophenyl)-ethyl]-1-methyl-benzimidazol-5-yl-carbonsäure-[N'-(3-ethoxyphenyl)-piperazid]

### Beispiel 49: 2-[2-(4-Amidinophenyl)-ethyl]-1-methyl-benzimidazol-5-yl-carbonsäure-[N'-(3-isopropyloxyphenyl)-piperazid]

### Beispiel 50: 2-[2-(4-Amidinophenyl)-ethyl]-1-methyl-benzimidazol-5-yl-carbonsäure-[N'-(cyclopentyl)-piperazid]

### Beispiel 51: 2-[2-(4-Amidinophenyl)-ethyl]-1-methyl-benzimidazol-5-yl-carbonsäure-[N'-(2-methylphenyl)-piperazid]

### Beispiel 52: 2-[2-(4-Amidinophenyl)-ethyl]-1-methyl-benzimidazol-5-yl-carbonsäure-[N'-(2-pyridyl)-piperazid]

### Beispiel 53: 2-[2-(4-Amidinophenyl)-ethyl]-1-methyl-benzimidazol-5-yl-carbonsäure-[N'-(2-methoxyphenyl)-piperazid]

### Beispiel 54: 2-[2-(4-Amidinophenyl)-ethyl]-1-methyl-benzimidazol-5-yl-carbonsäure-[N'-(2-ethoxyphenyl)-piperazid]

### Beispiel 55: 2-[2-(4-Amidinophenyl)-ethyl]-1-methyl-benzimidazol-5-yl-carbonsäure-[N'-benzyl-piperazid]

### Beispiel 56: 2-{2-[4-(Amino-methoxycarbonylimino-methyl)phenyl]-ethyl}-1-methylbenzimidazol-5-yl-carbonsäure-N-phenyl-N-[3-amino-3-methoxycarbonylimino-propyl]-amid

### Beispiel 57: 2-{2-[4-(Amino-hydroximino-methyl) phenyl]-ethyl}-1-methyl-benzimidazol-5-yl-carbonsäure-[N'-(3-hydroxyphenyl)-piperazid]

### Beispiel 58: 2-{2-[4-(Amino-methoxycarbonylimino-methyl)phenyl]-ethyl}-1-methylbenzimidazol-5-yl-carbonsäure-N-(2-phenylethyl)-N-[3-amino-3-methoxycarbonylimino-propyl]-amid

### Beispiel 59: 2-{2-[4-(Amino-hydroximino-methyl) phenyl]-ethyl}-1-methyl-benzimidazol-5-yl-carbonsäure-[N'-(2-pyridyl)-piperazid]

### Beispiel 60: 2-{2-[4-(Amino-hydroximino-methyl) phenyl]-ethyl}-1-methylbenzimidazol-5-yl-carbonsäure-[N'-(cyclohexyl)-piperazid]

### Beispiel 61: 2-{2-[4-(Amino-hydroximino-methyl) phenyl]-ethyl}-1-methylbenzimidazol-5-yl-carbonsäure-[N'-(cyclopentyl)-piperazid]

### Beispiel 62: 2-{2-[4-(Amino-hydroximino-methyl) phenyl]-ethyl}-1-methylbenzimidazol-5-yl-carbonsäure-[N'-(2-ethoxy-phenyl)-piperazid]

### Beispiel 63: 2-{2-[4-(Amino-hydroximino-methyl) phenyl]-ethyl}-1-methylbenzimidazol-5-yl-carbonsäure-[N'-(3-methoxy-phenyl)-piperazid]

### Beispiel 64: 2-{2-[4-(Amino-hydroximino-methyl) phenyl]-ethyl}-1-methylbenzimidazol-5-yl-carbonsäure-[N'-(3-isopropyloxy-phenyl)-piperazid]

### Beispiel 65: 2-{2-[4-(Amino-hydroximino-methyl) phenyl]-ethyl}-1-methylbenzimidazol-5-yl-carbonsäure-[N'-(3-ethoxy-phenyl)-piperazid]

### Beispiel 66: 2-{2-[4-(Amino-hydroximino-methyl)phenyl]-ethyl}-1-methylbenzimidazol-5-yl-carbonsäure-N-phenyl-N-[3-amino-3-hydroxyimino-propyl]-amid

### Beispiel 67: 2-{2-[4-(Amino-hydroximino-methyl)phenyl]-ethyl}-1-methylbenzimidazol-5-yl-carbonsäure-N-(2-phenylethyl)-N-[3-amino-3-hydroxyimino-propyl]-amid

### Beispiel 68: 2-{2-[4-(Amino-hydroximino-methyl) phenyl]-ethyl}-1-methylbenzimidazol-5-yl-carbonsäure-[N'-(cyclohexyl)-piperazid]

### Allgemeine Arbeitsvorschrift zur Synthese der Verbindungen der allgemeinen Formel (I) an der festen Phase:

### a) Reduktive Aminierung:

Eine Suspension aus 0.06 mmol des an das Tritylharz gekoppelten Amins und 300 µl einer 1:1 (v/v) Lösung aus Tetramethylorthoformiat / Dichlormethan wird mit 500 µl einer 0.12 M Lösung der Aldehyde R⁴-CHO in Tetramethylorthoformiat /Dichlormethan (1:1 v/v) versetzt und 2 h bei Raumtemperatur geschüttelt. Anschließend wird das Harz abfiltriert und je 2 mal mit 1,5 ml Dichlormethan, Dimethylformamid, Dichlormethan gewaschen.
Das Harz wird in 300 µl Dichlormethan suspendiert, mit 1200 µl einer 0.167 M Lösung von Natriumtriacetoxyborhydrid in Dichlormethan versetzt und 12 h bei Raumtemperatur geschüttelt. Das Harz wird abfiltriert und je 2 mal mit je 1.5 ml Wasser, Dimethylformamid / Wasser 7:3 (v/v), Dimethylformamid / Wasser /Essigsäure 90:10:5 (v/vlv), Dimethylformamid / Wasser 9:1 (v/v), Dimethylformamid, Dichlormethan gewaschen.

### b) Acylierung mit 4-Chlor-3-nitro-benzoylchlorid:

Das Harz, in 500µl Diisopropylethylamin-Lösung (20% in Dichlormethan) suspendiert, wird mit 1000 µl einer 0.2 M Lösung von 4-Chlor-3-nitro-benzoylchlorid in Dichlormethan versetzt und 12 h bei Raumtemperatur geschüttelt. Anschließend wird abfiltriert und 3 mal mit je 1.5 ml Dichlormethan und 1-Methyl-2-pyrrolidon gewaschen.

### c) Nucleophile Substitution:

Das Harz, in 500 µl Diisopropylethylamin-Lösung in 1-Methyl-2-pyrrolidon (20% v/v) suspendiert, wird mit 1000 µl einer 0.1 M Lösung eines Amins R¹-NH₂ in 1-Methyl-2-pyrrolidon versetzt und 12 h bei 85°C erhitzt. Nach Abkühlen auf Raumtemperatur wird das Harz abfiltriert und 3 mal mit je 1.5 ml 1-Methyl-2-pyrrolidon und Dimethylformamid gewaschen.

### d) Reduktion der Nitrogruppe:

Das Harz wird in 500 µl Dimethylformamid suspendiert, mit 1000 µl 1.0 M SnCl₂ - Lösung in Dimethylformamid versetzt und 48 h bei Raumtemperatur geschüttelt. Anschließend wird das Harz abfiltriert und 2 mal mit je 1.5 ml Dimethylformamid, Dioxan, Methanol/NH₄OH 98:2 (v/v), wässriges Methanol (80%), Methanol und Tetrahydrofuran gewaschen.

### e) Oxidative Cyclisierung zum Benzimidazol:

Das Harz wird in 500µl Tetrahydrofuran suspendiert, mit 1000µl 0.1M Lösung eines Aldehyds R²-C₆H₄-CH₂CH₂-CHO Lösung in THF versetzt und 48 h bei Raumtemperatur unter Luftsauerstoff geschüttelt. Anschließend wird das Harz abfiltriert und 5 mal mit je 1.5 ml Tetrahydrofuran, Dichlormethan / Methanol 95/5 und Dichlormethan gewaschen.

### f) Abpaltung des Produktes vom Harz:

Das Harz wird mit 1000 µl Trifluoressigsäure 10% v/v in Dichlormethan, 1 h bei Raumtemperatur geschüttelt und abgesaugt. Anschließend wird das zurückbleibende Harz nochmals mit 500µl Trifluoressigsäure 10% v/v in Dichlormethan versetzt abgesaugt und die vereinigten Filtrate im Vakuum eingeengt. Nach Behandlung des Harzrückstandes mit 1000 µl Dichlormethan /Methanol 95:5 wird nach 1 h Schütteln bei Raumtemperatur abfiltriert.
Die Filtrate und die erhaltenen Rückstande werden vereinigt und im Vakuum zur Trockene eingeengt.

Die nachfolgenden Tabellen fassen weitere erfindungsgemäß synthetisierte Verbindungen der allgemeinen Formel (I) zusammen. Diese sind sowohl in Analogie zu den vorstehend beschriebenen Beispielen 1- 68 als auch gemäß der vorstehend beschriebenen Festphasensynthese erhältlich.

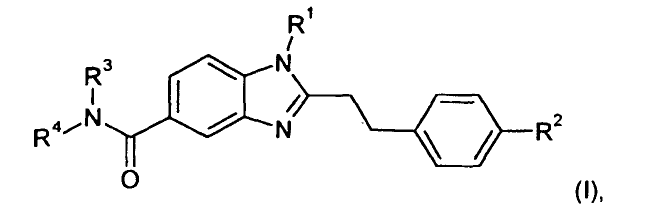

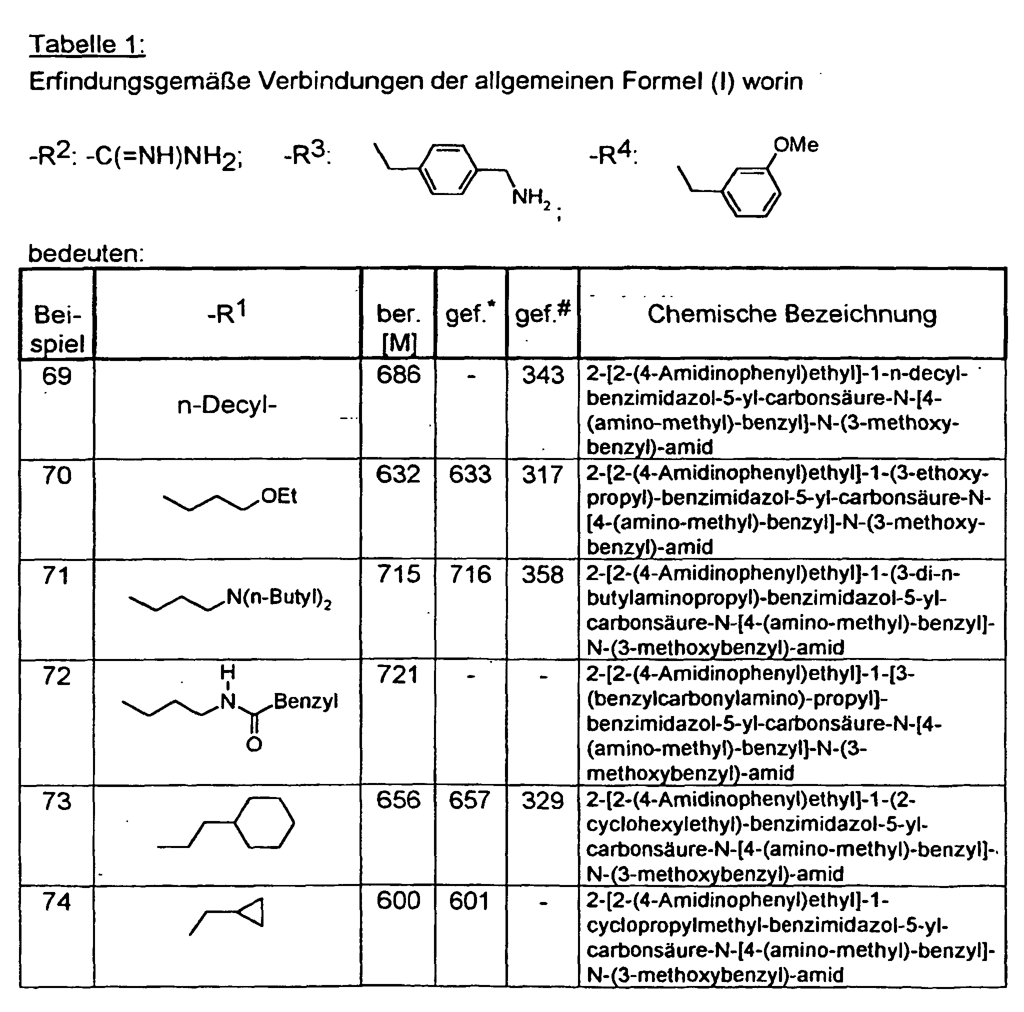

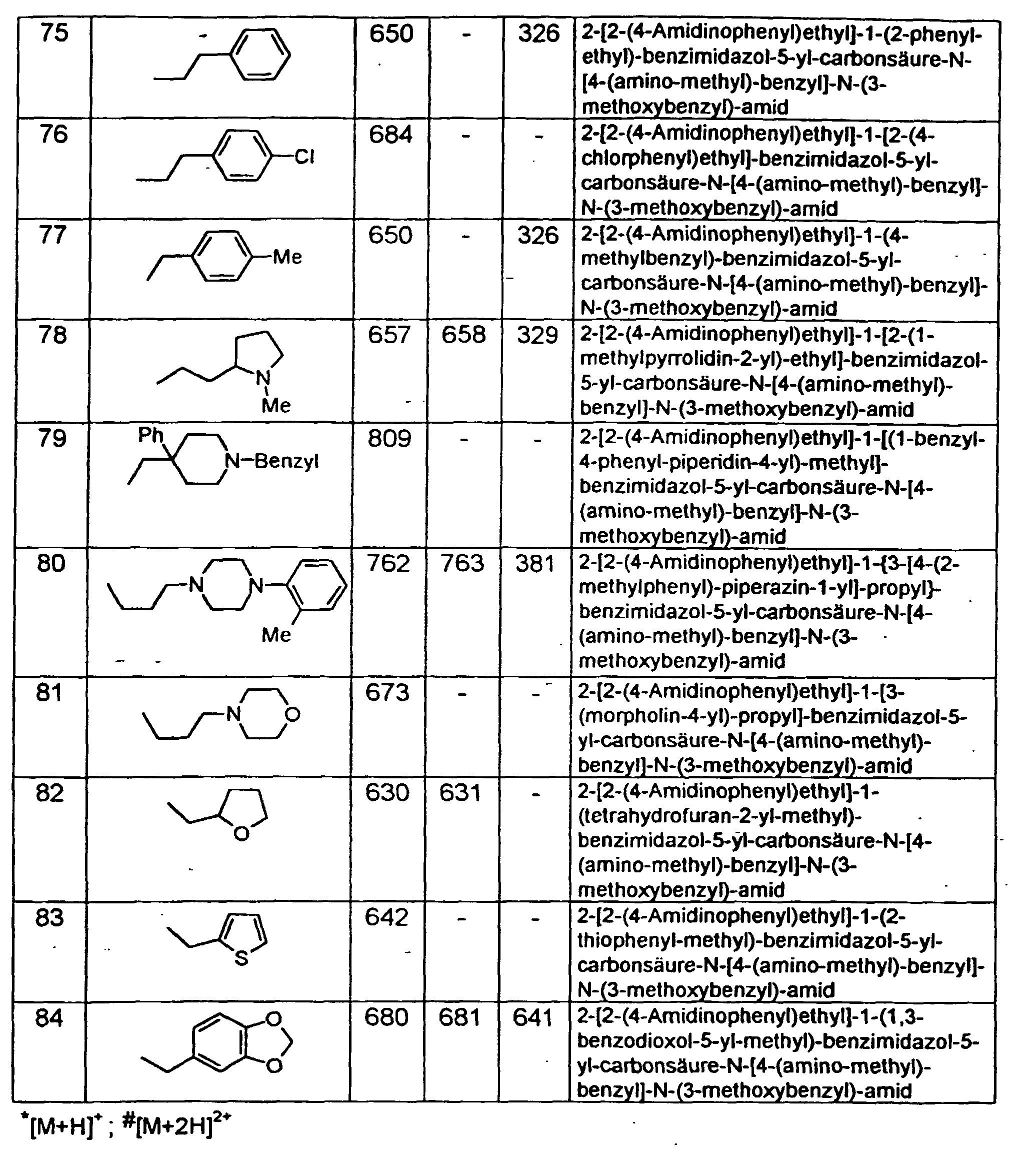

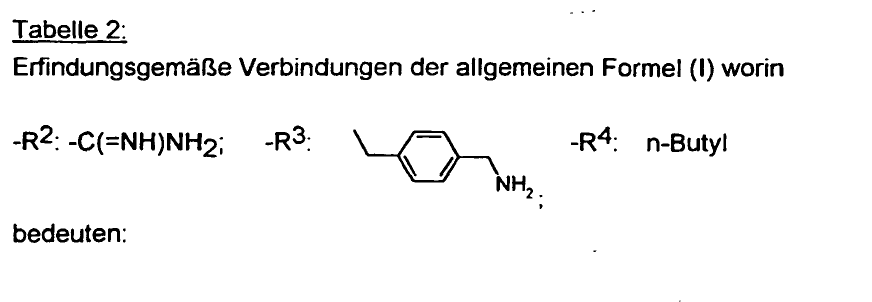

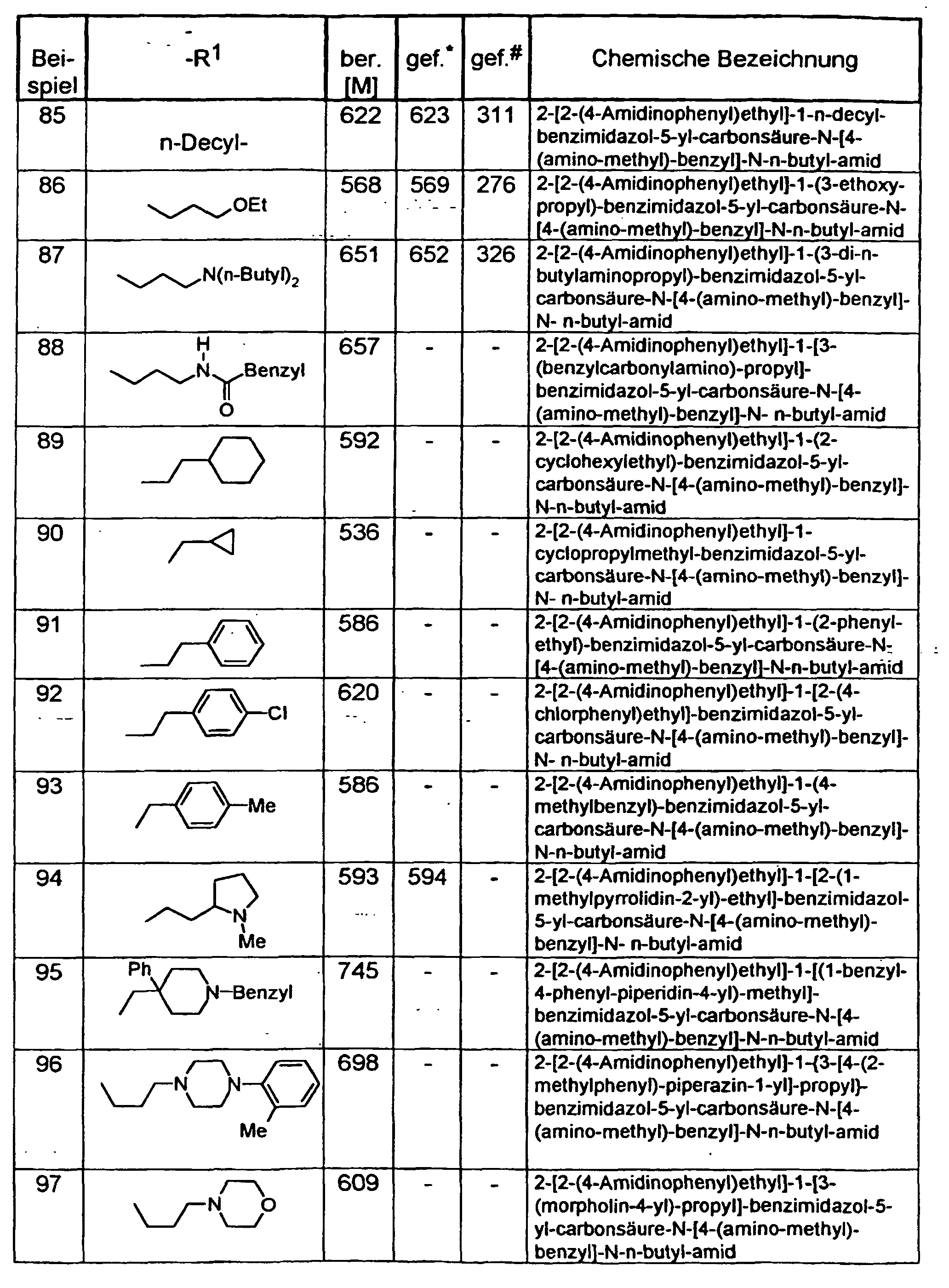

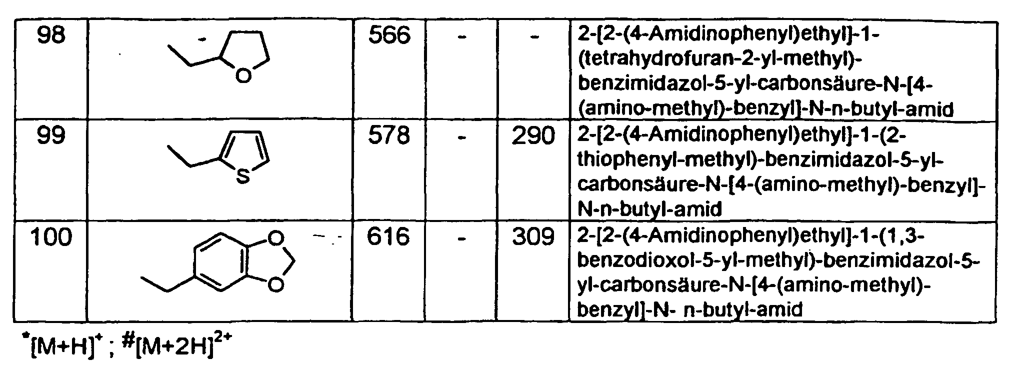

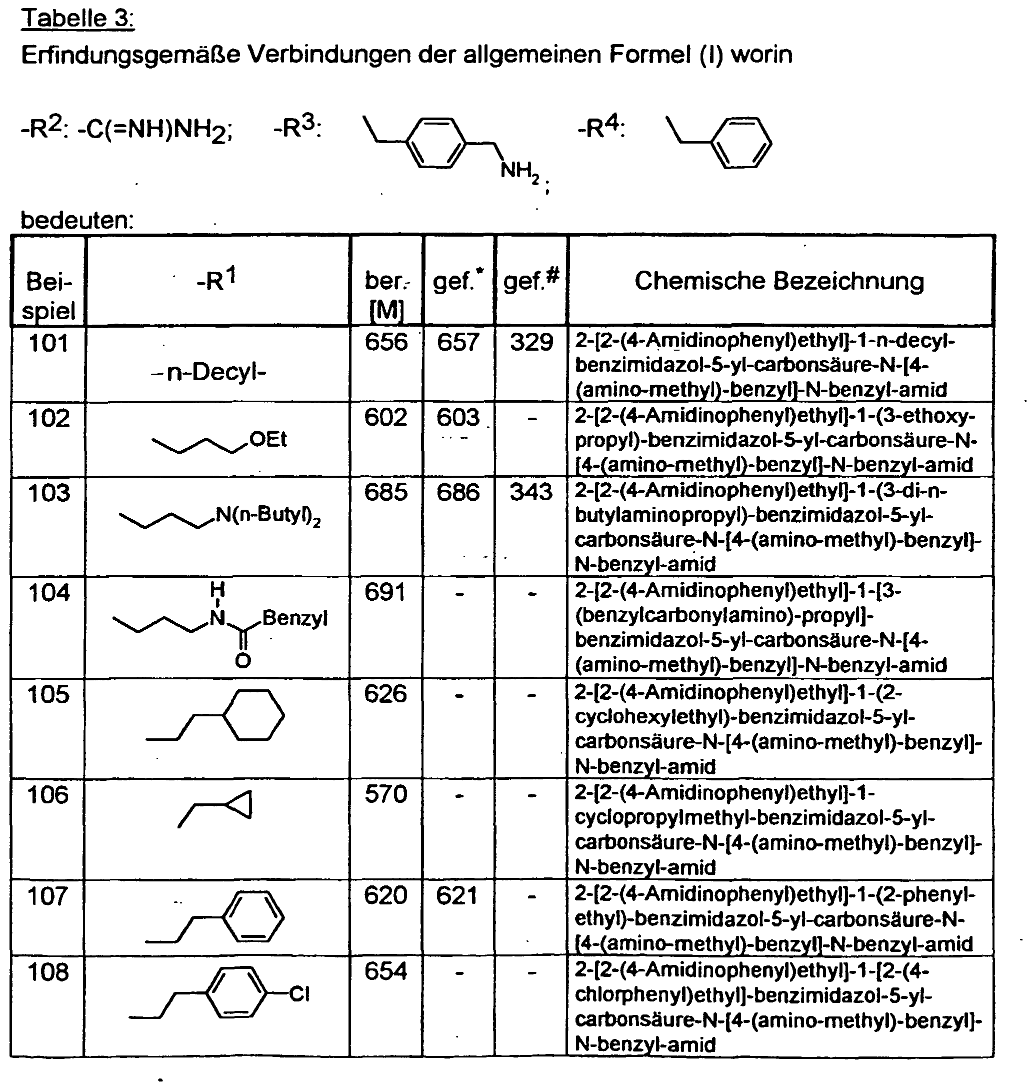

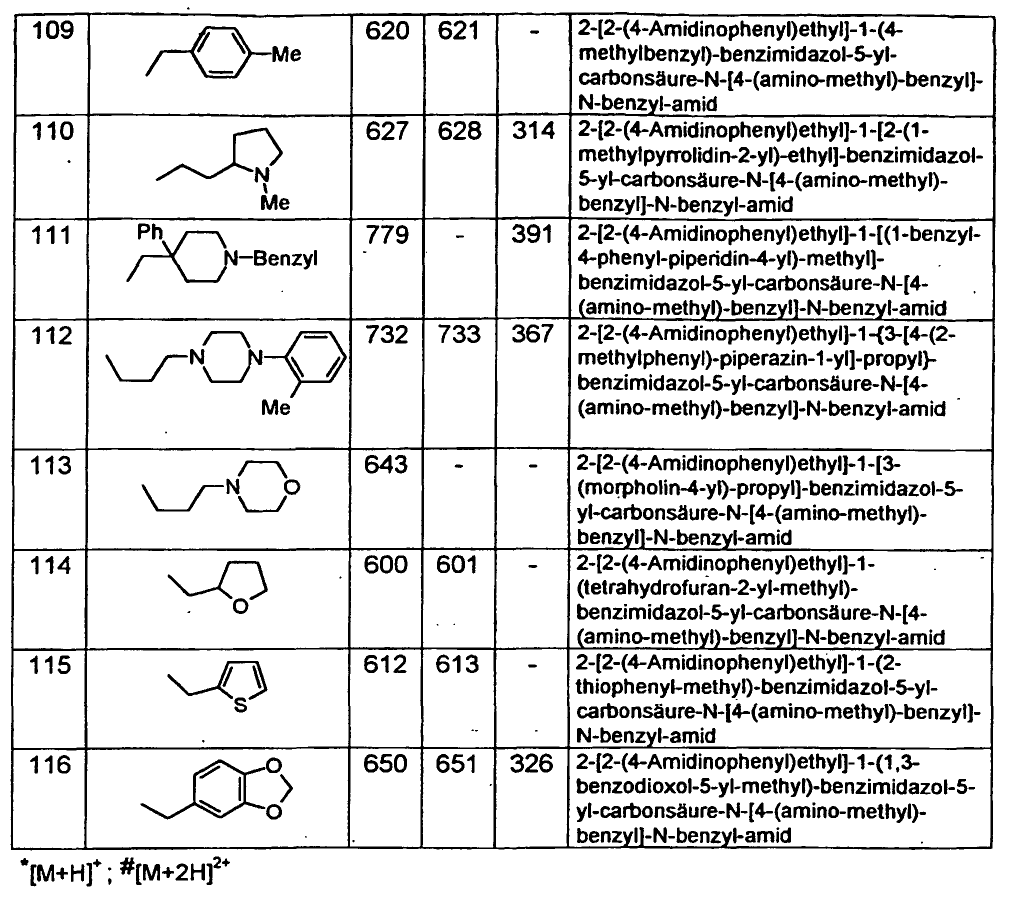

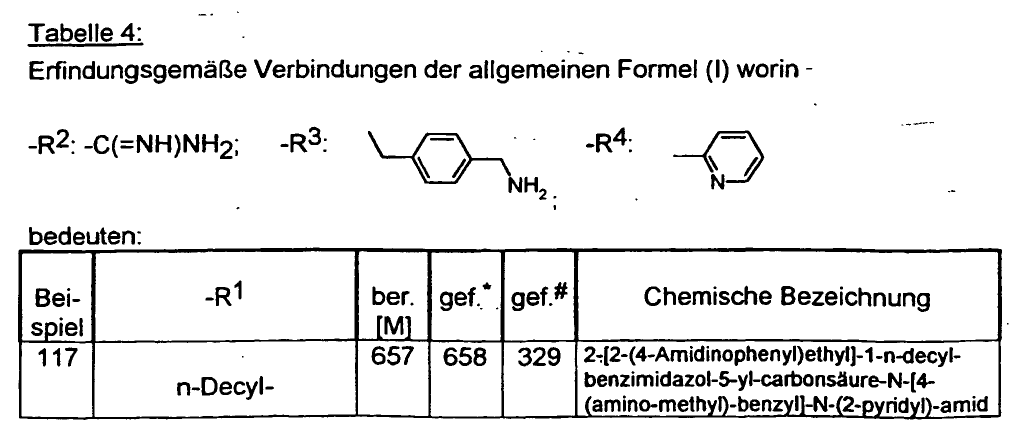

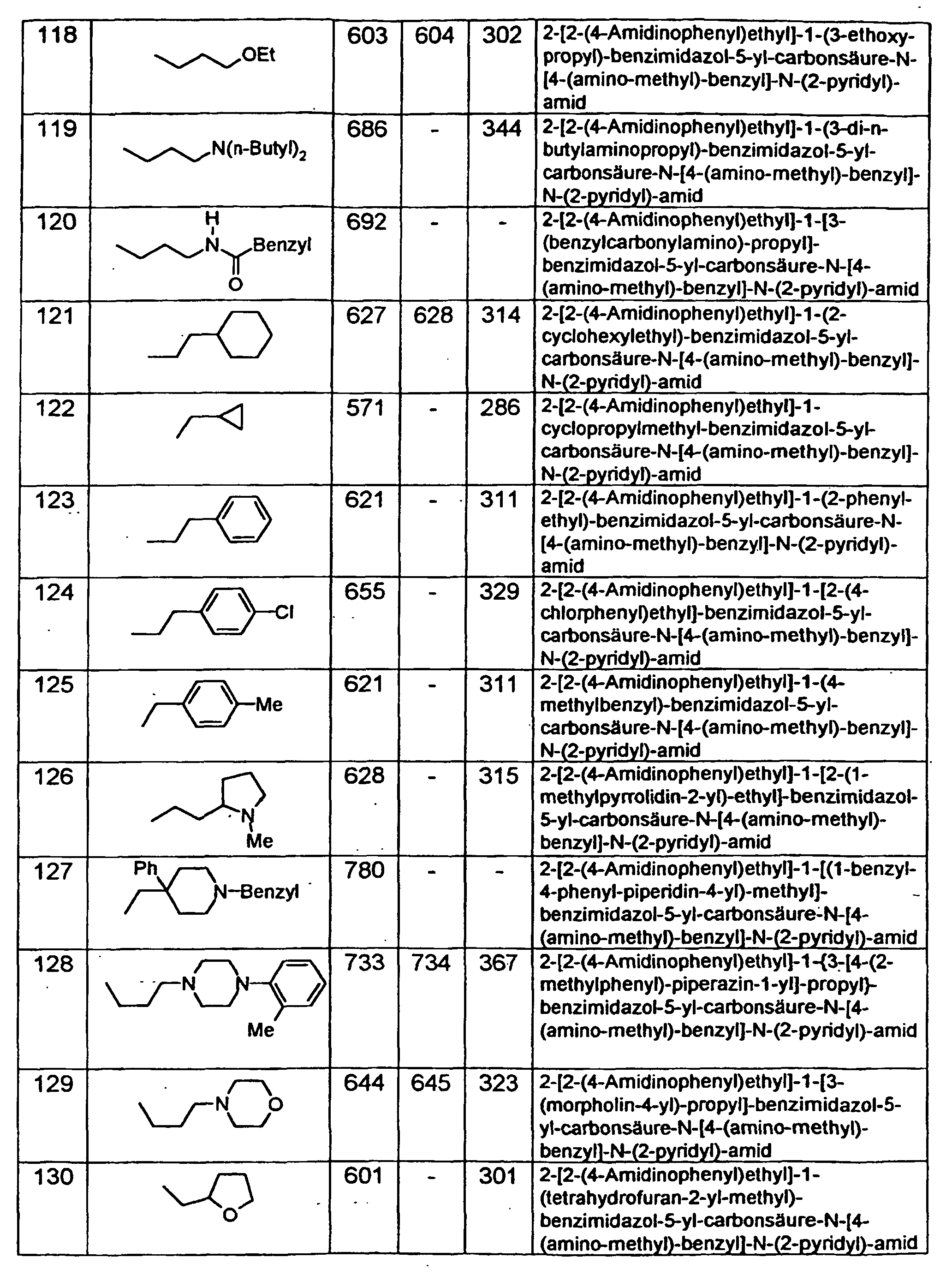

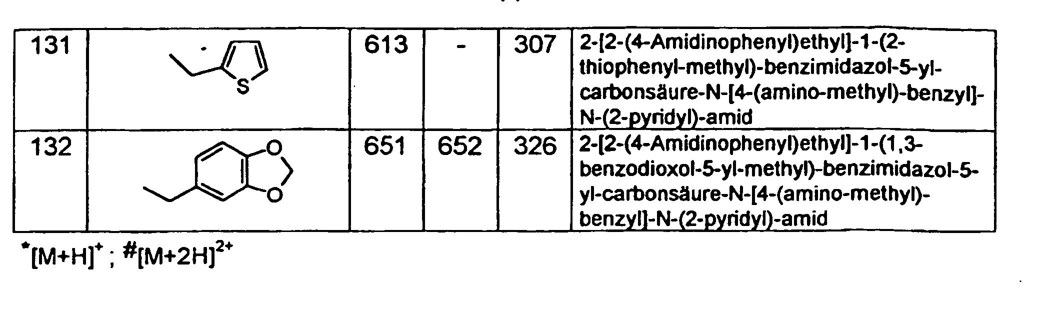

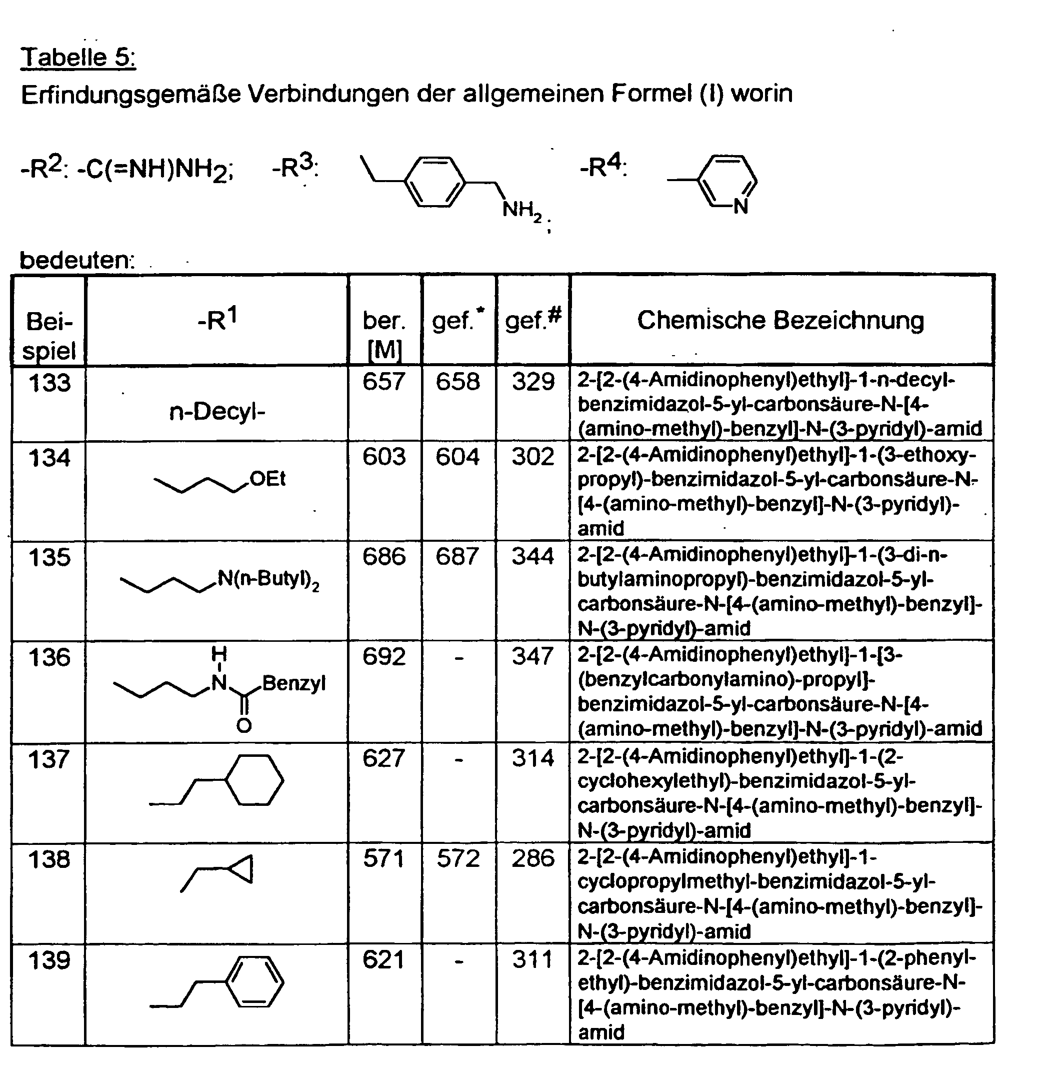

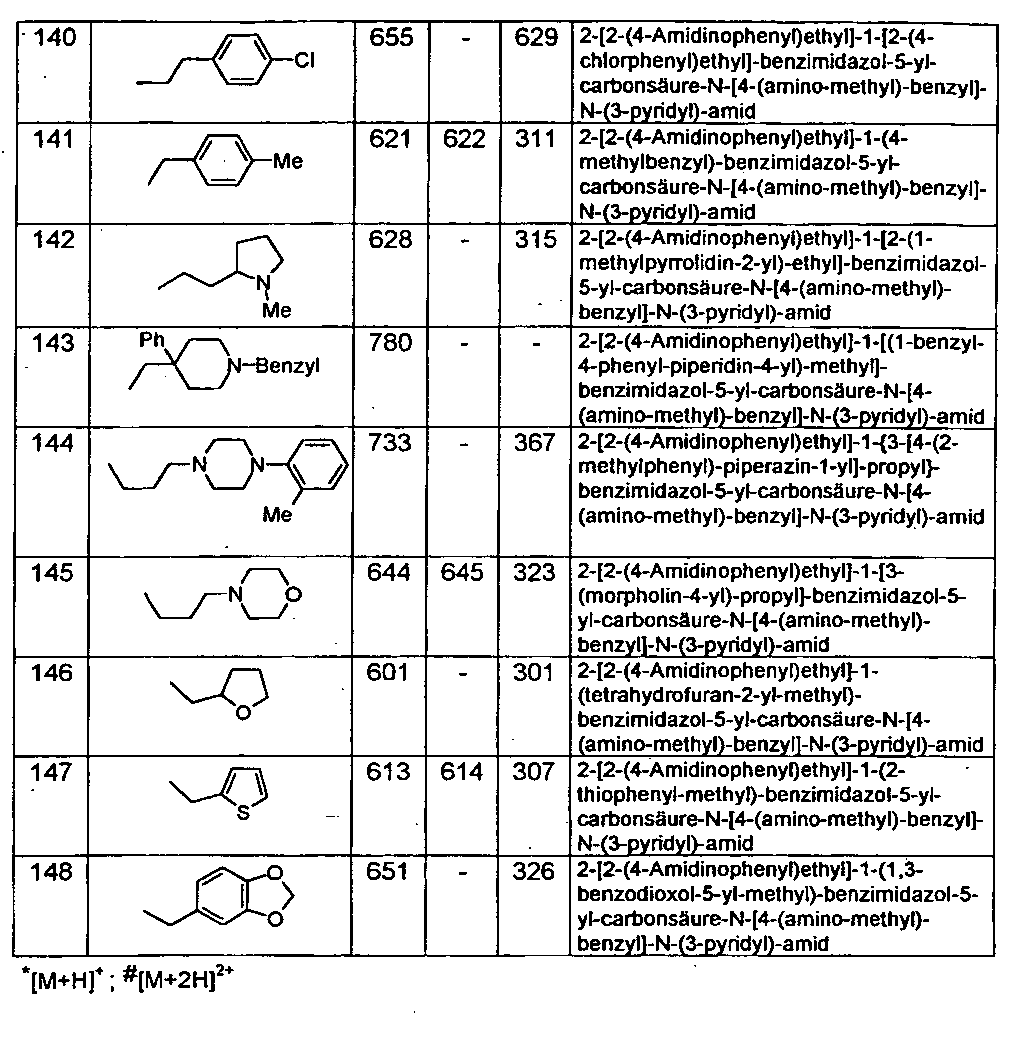

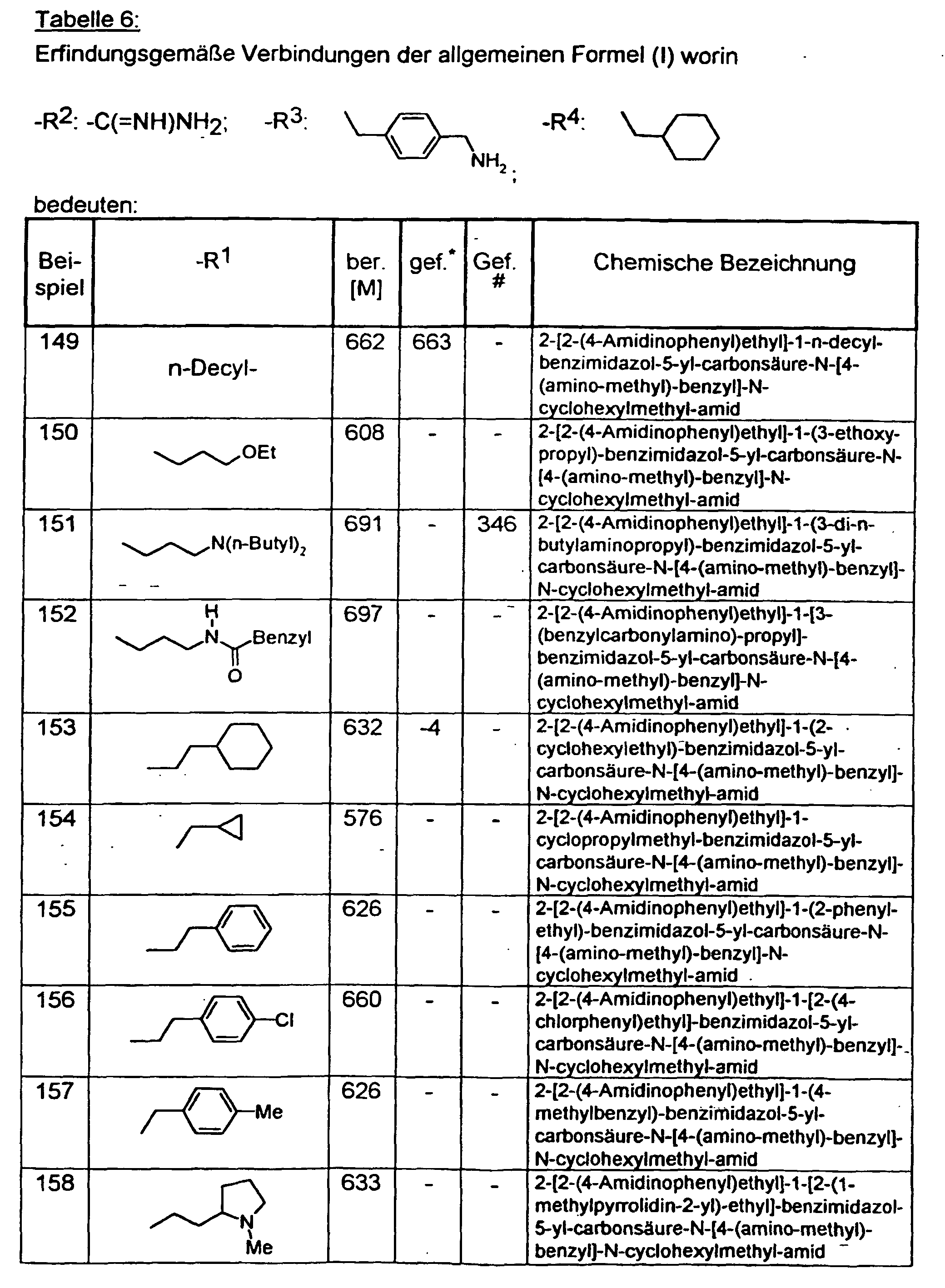

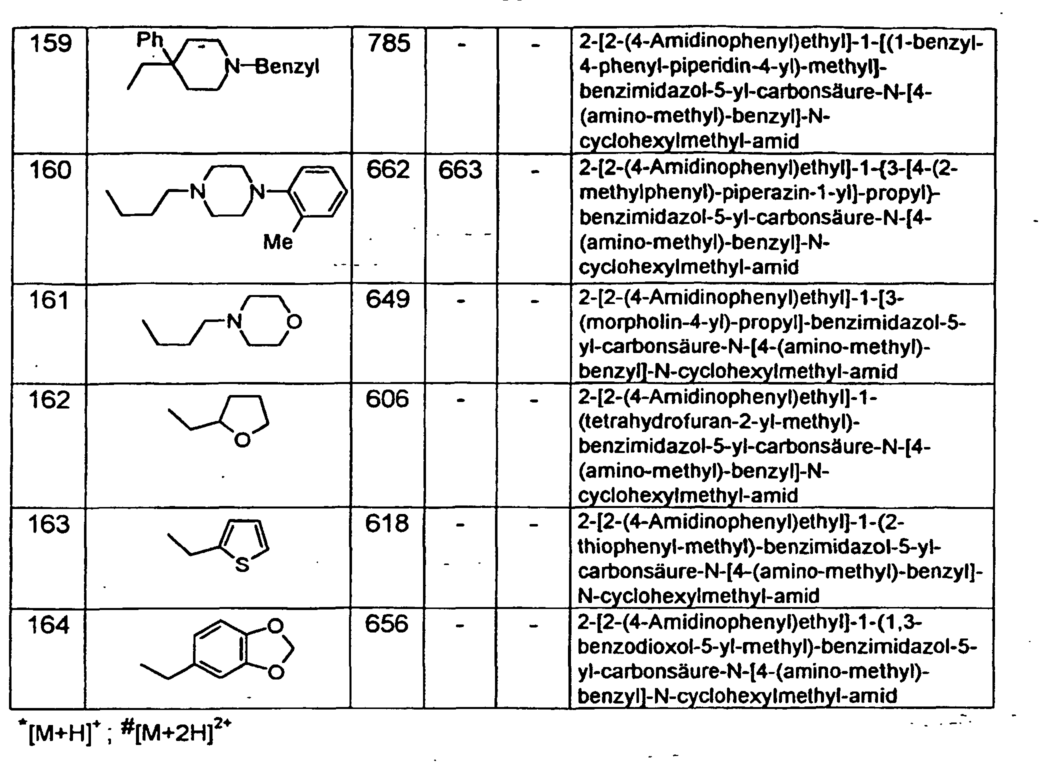

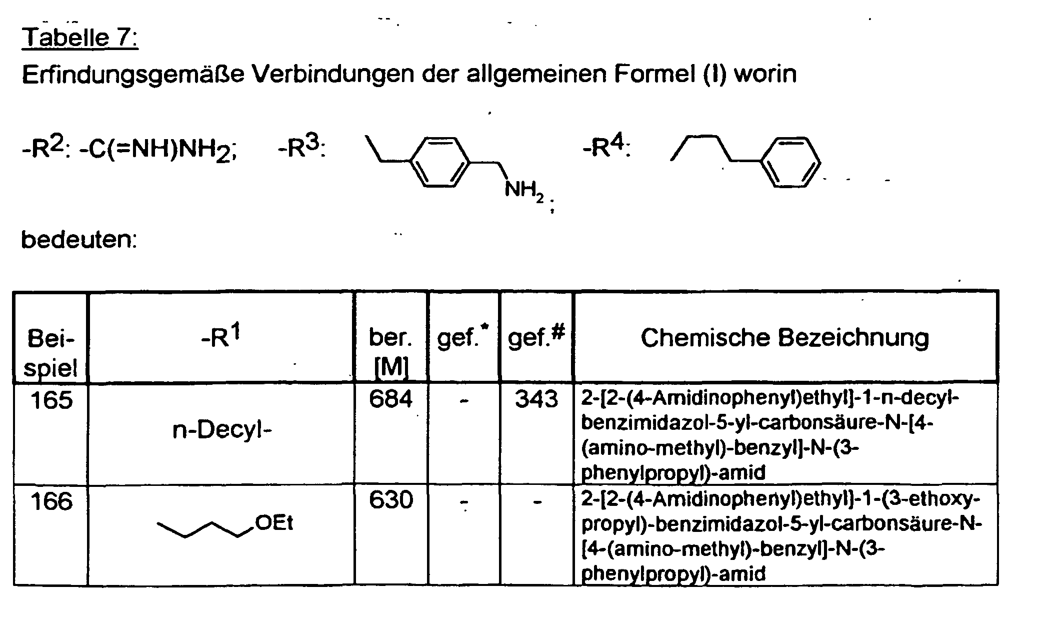

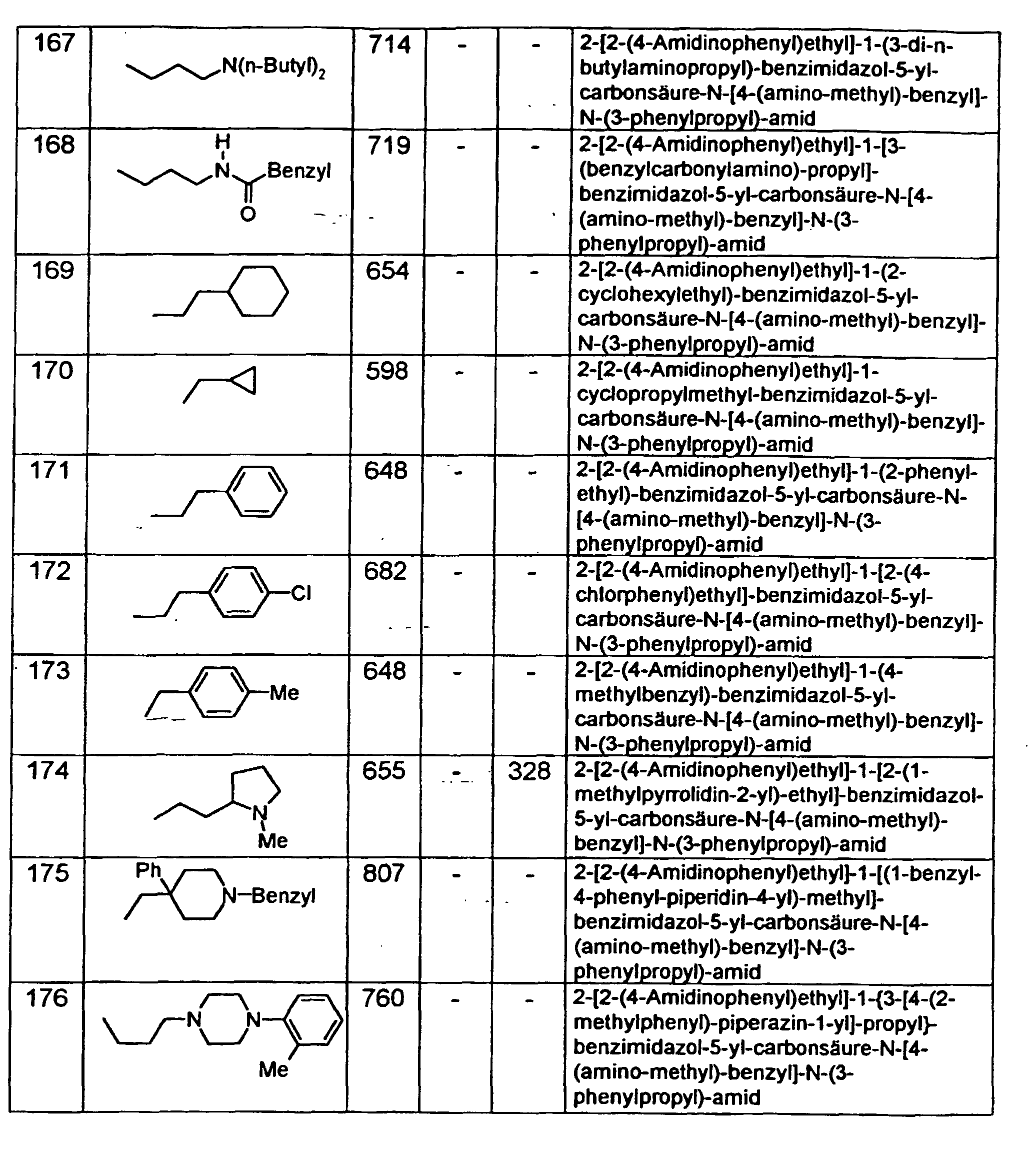

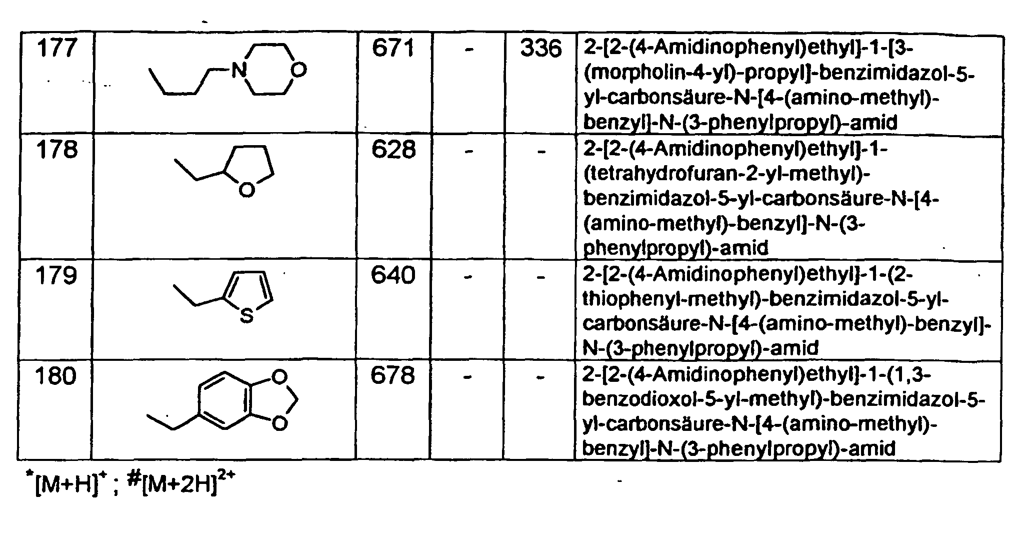

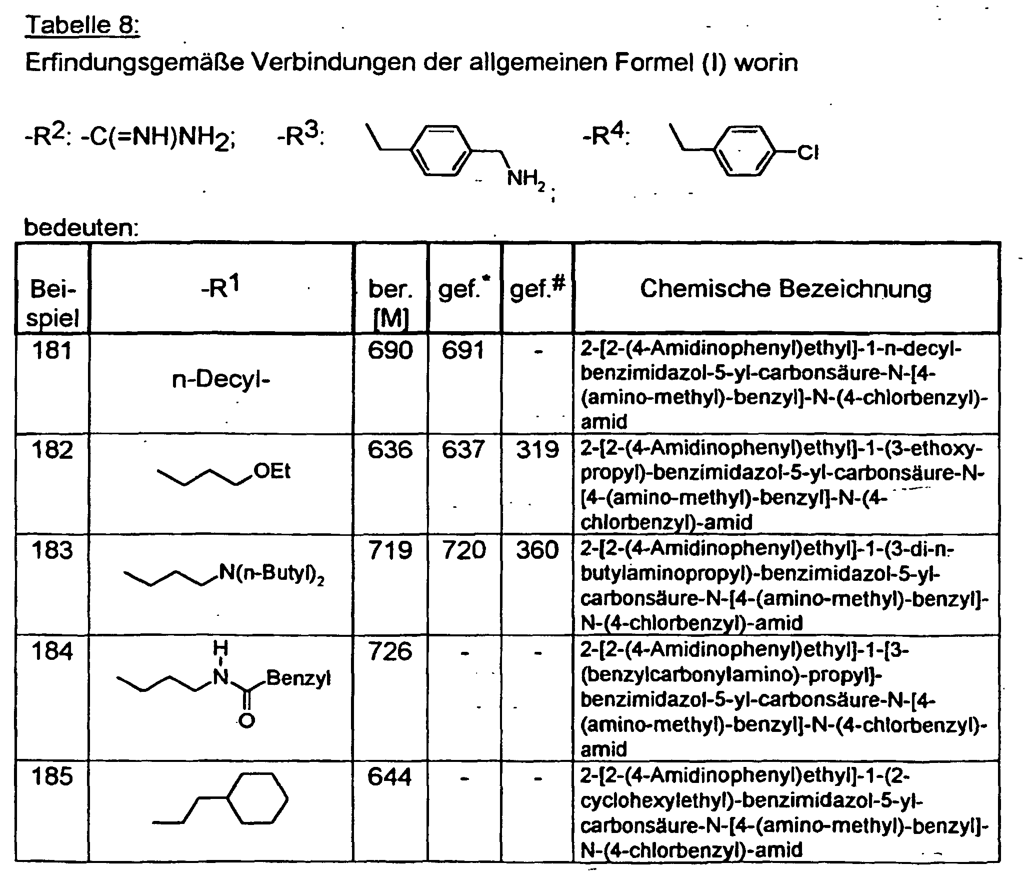

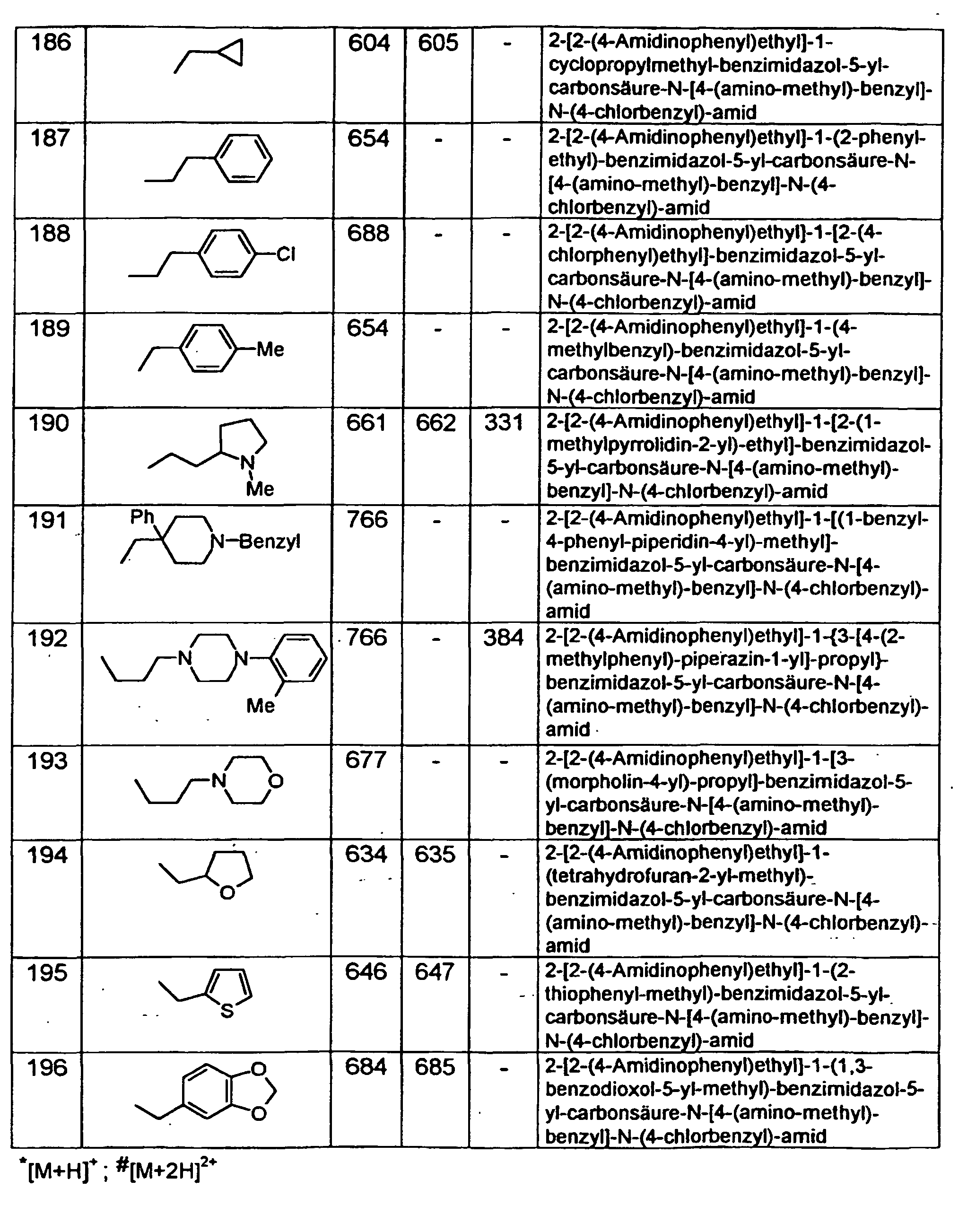

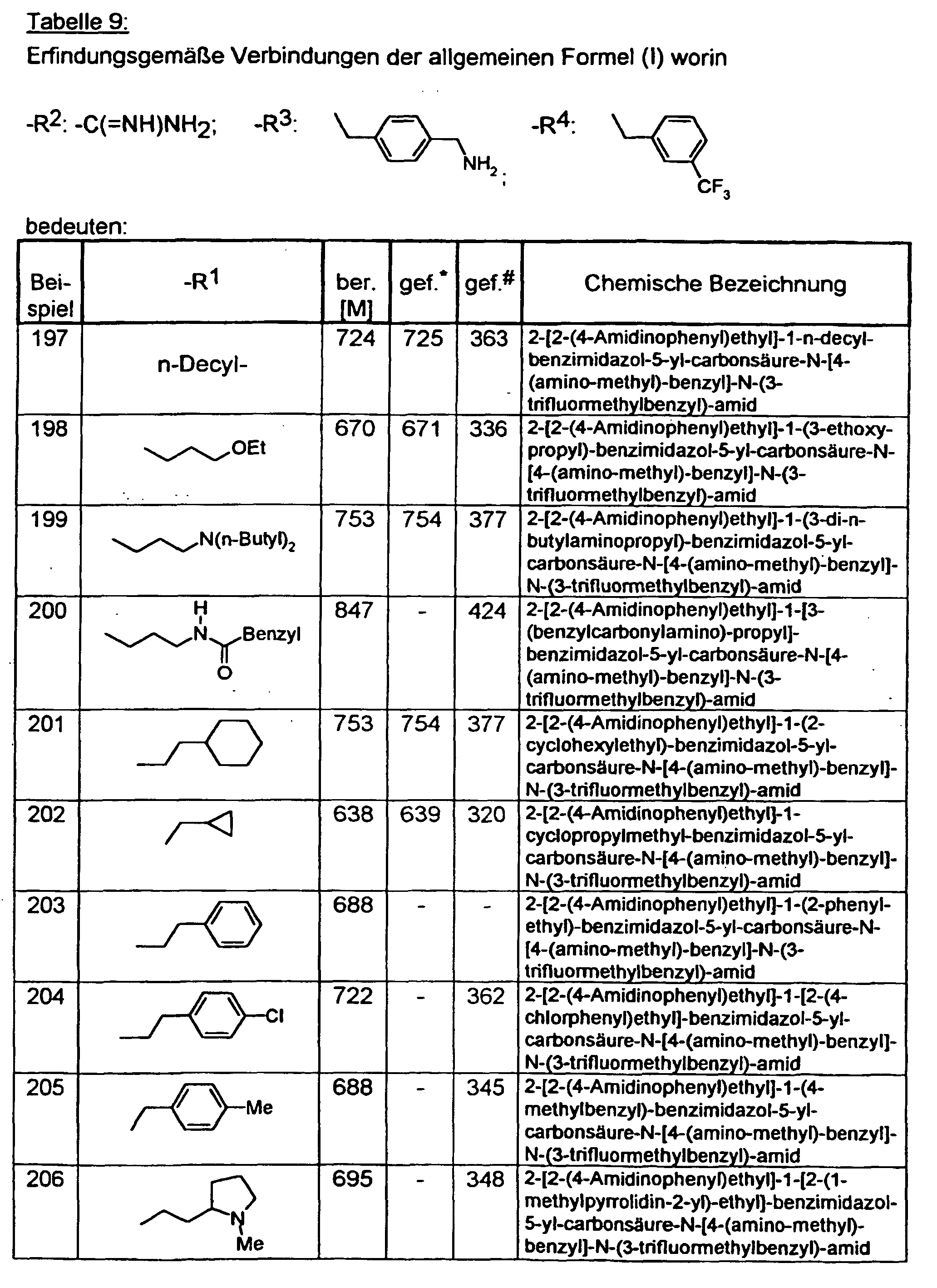

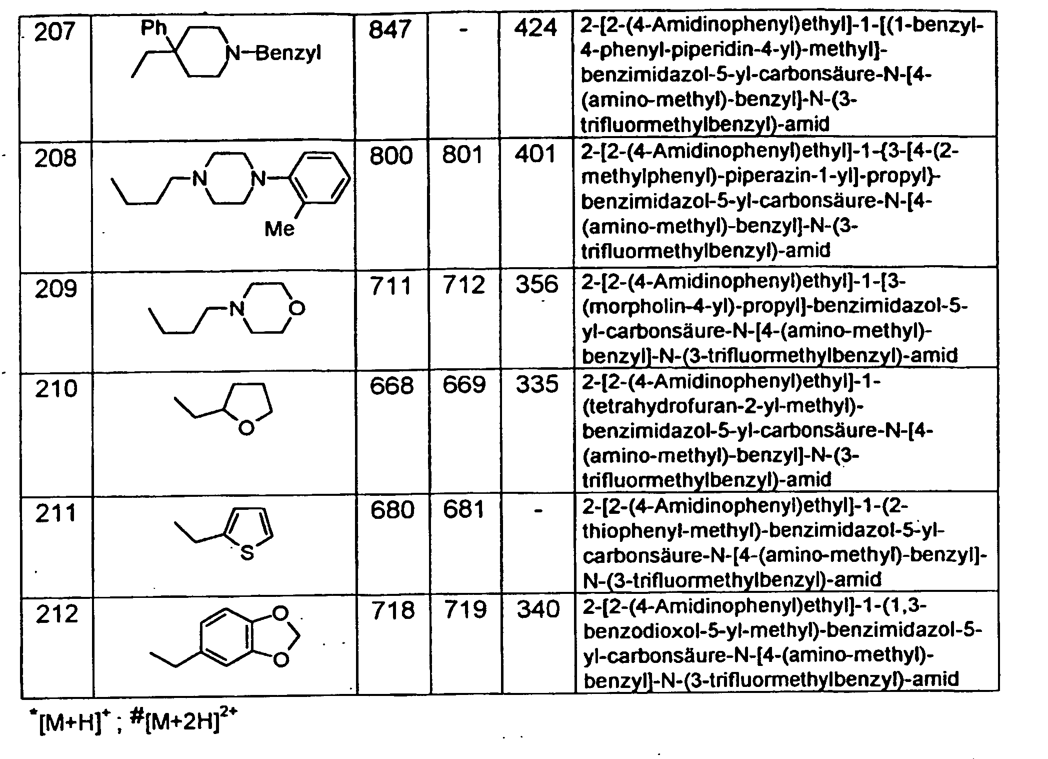

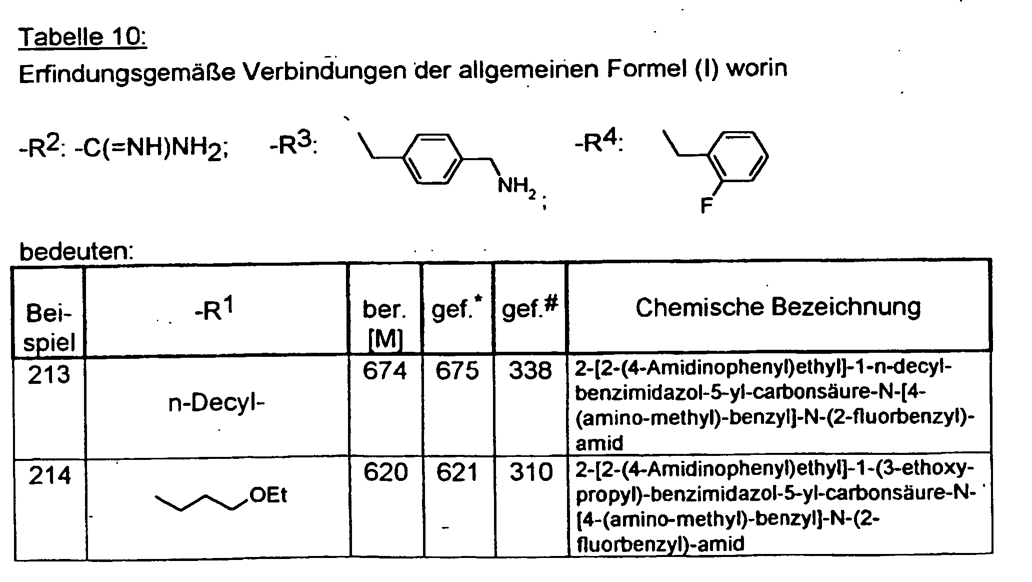

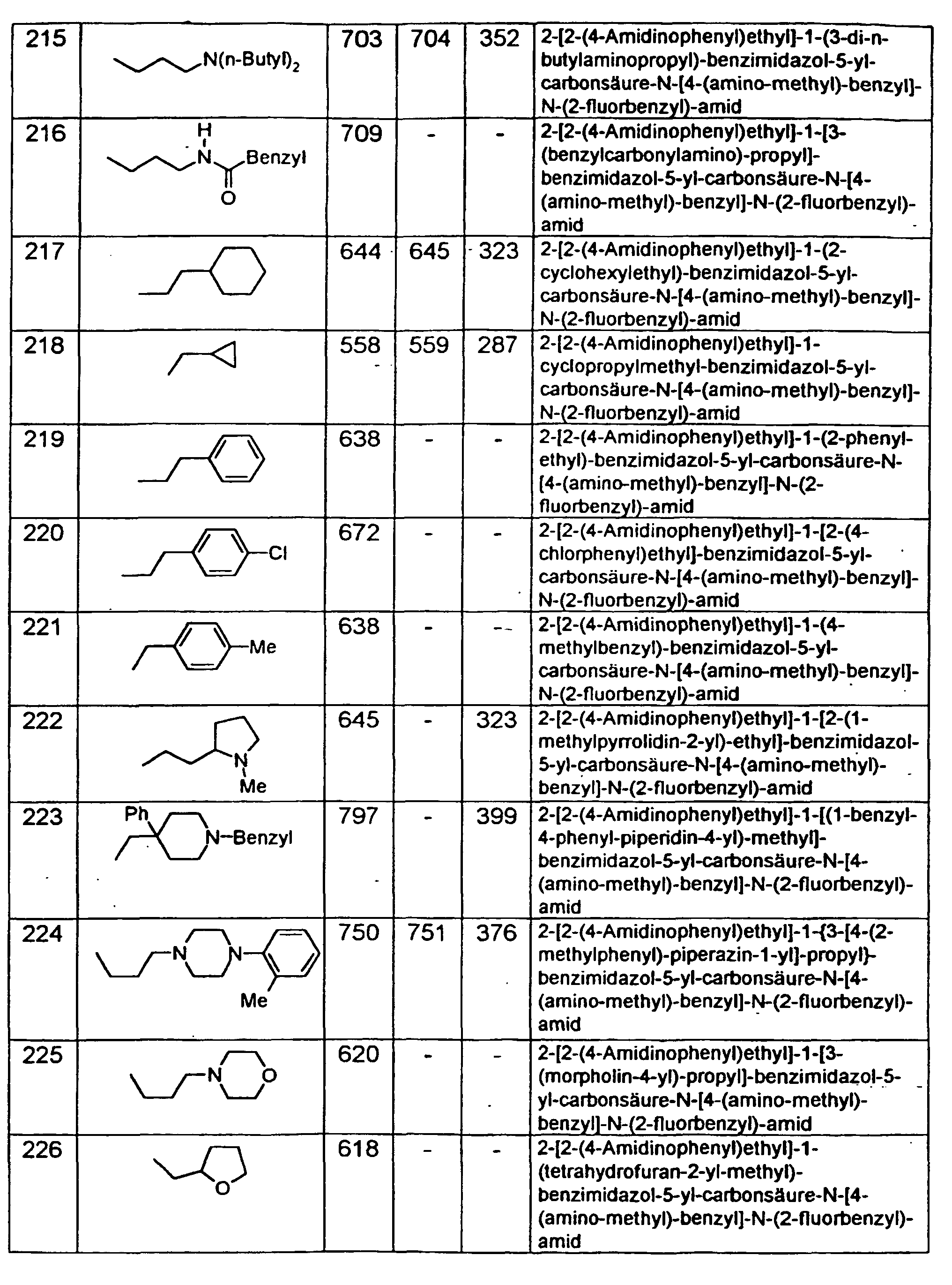

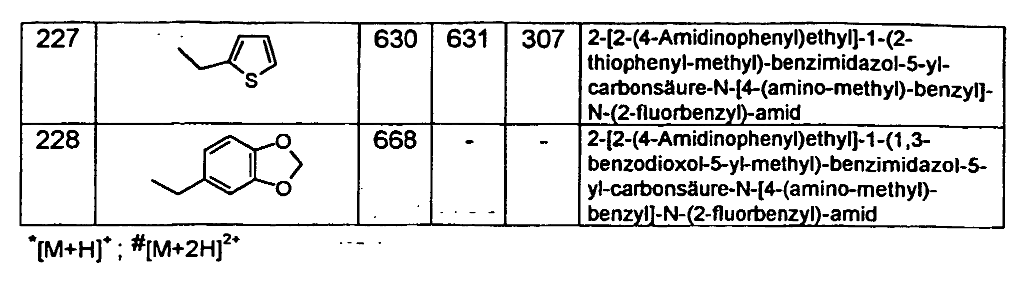

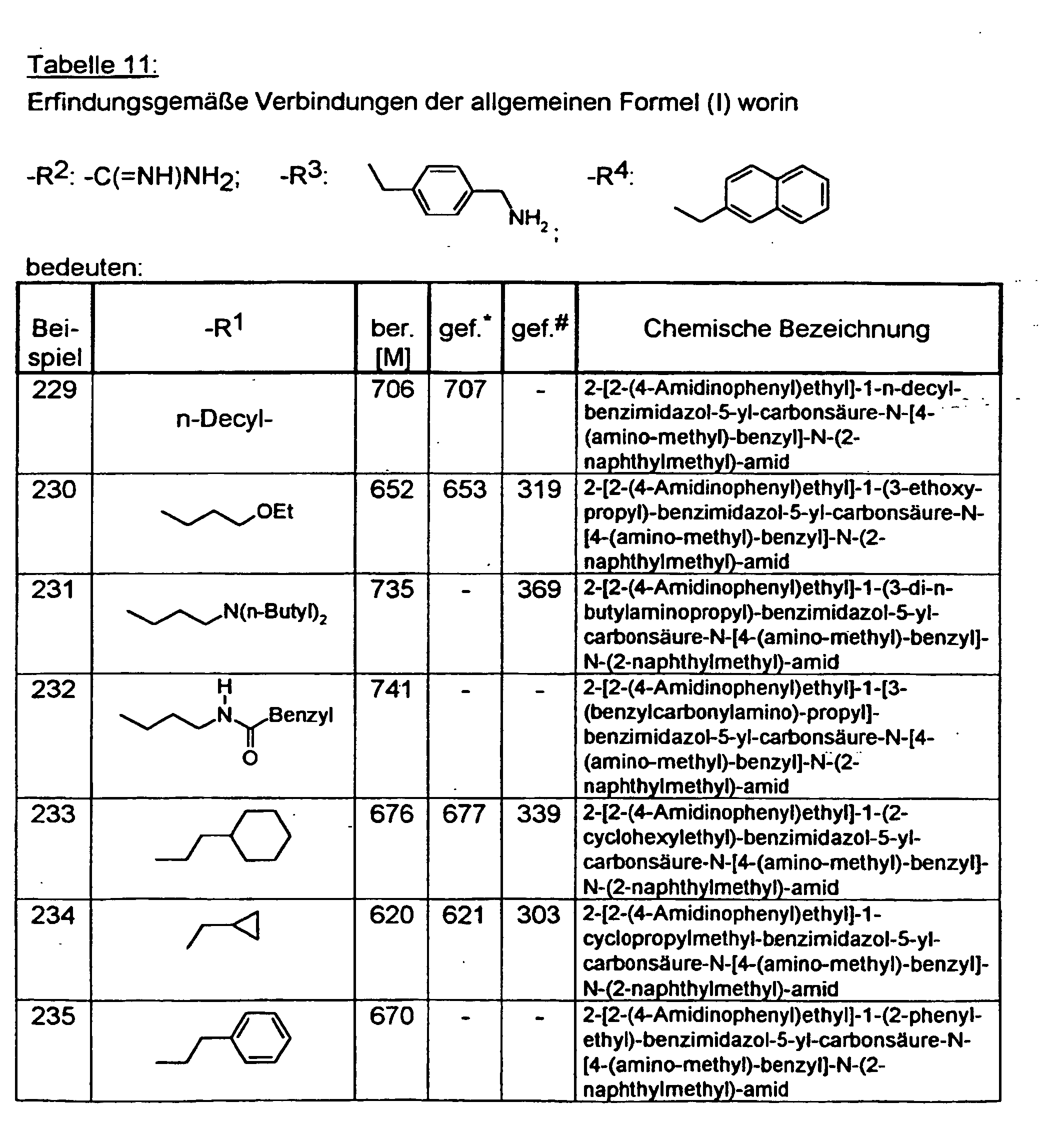

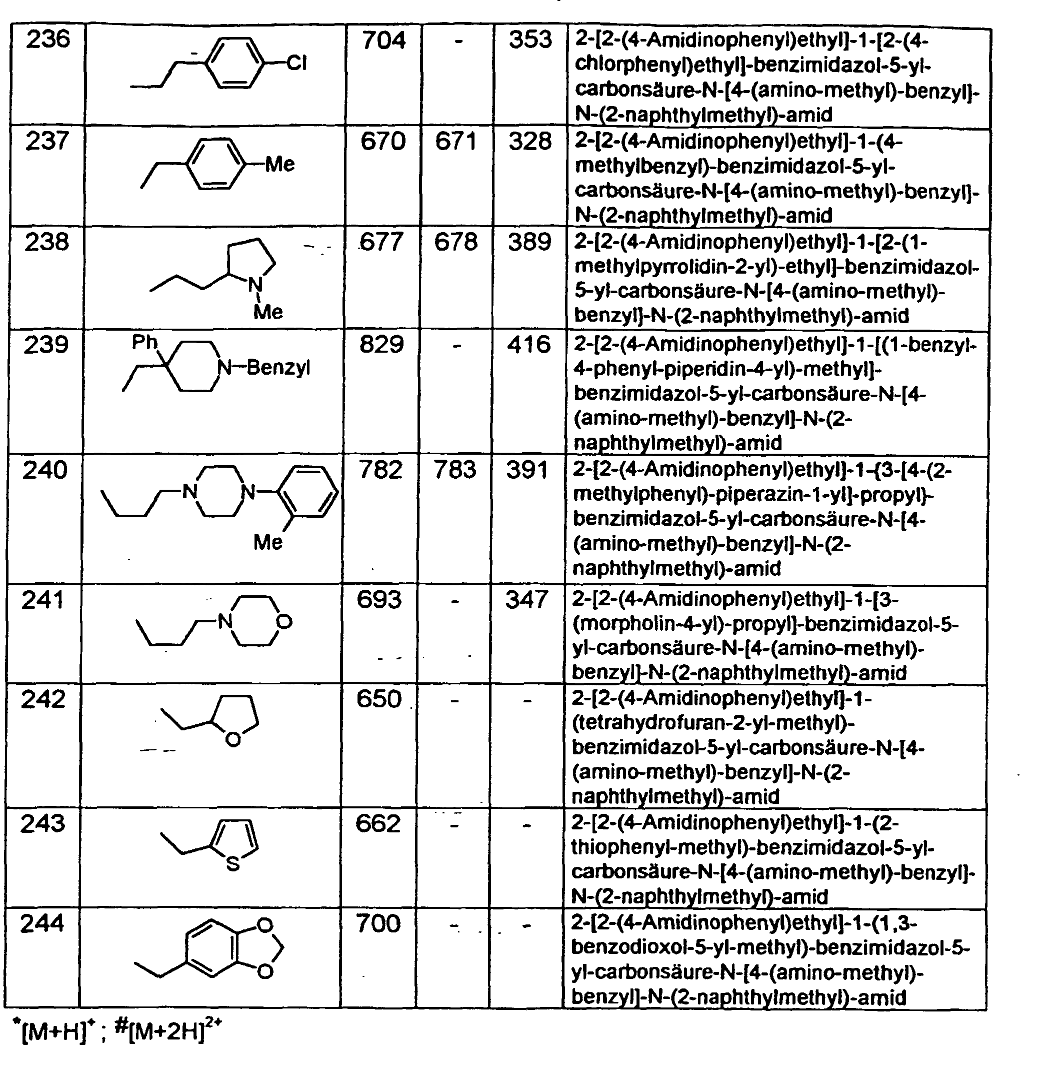

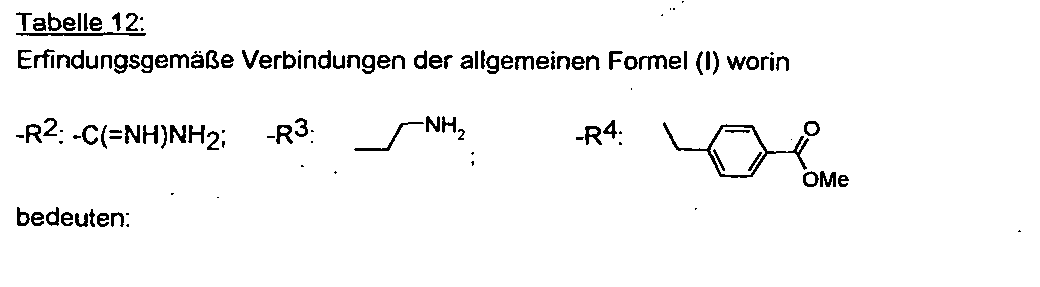

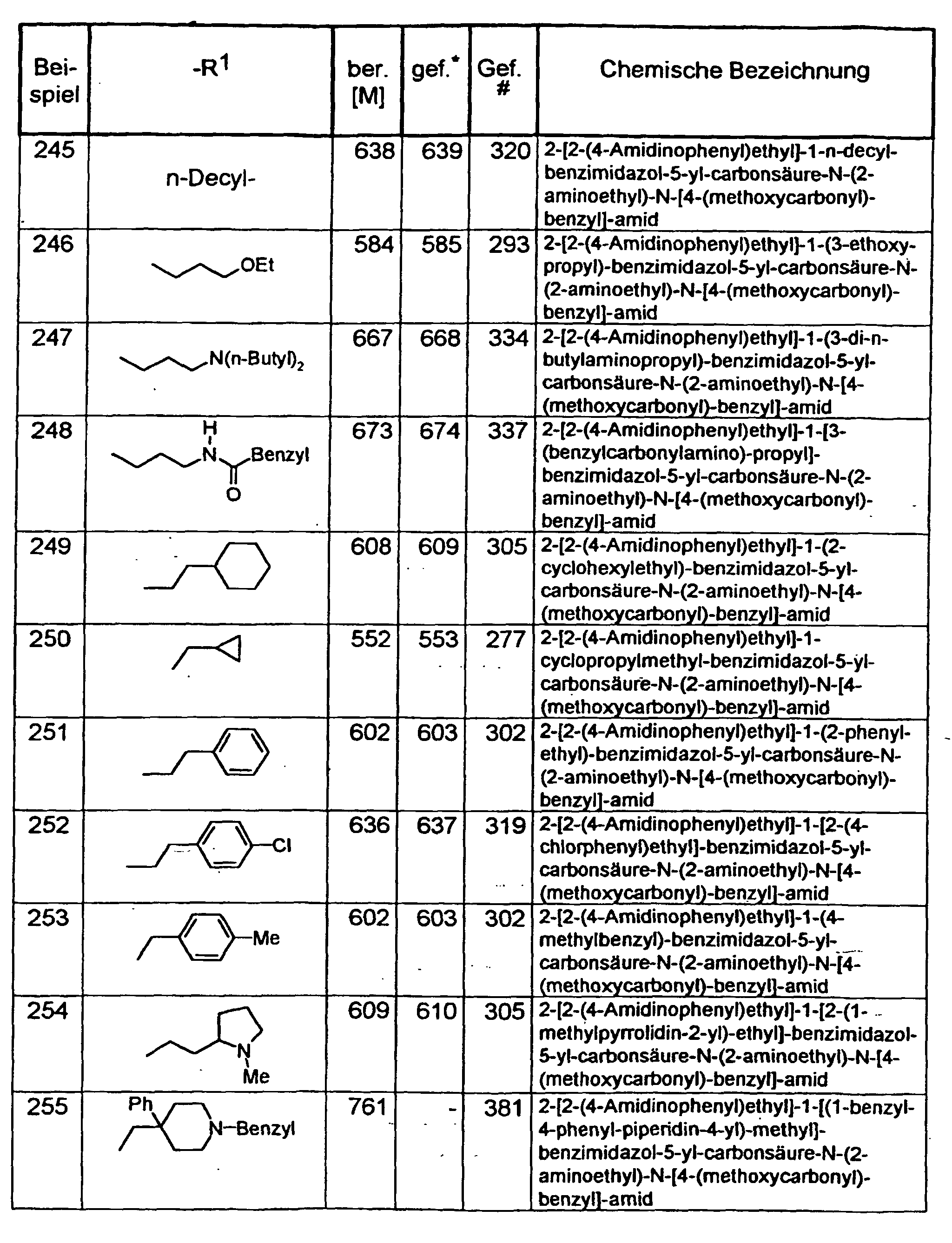

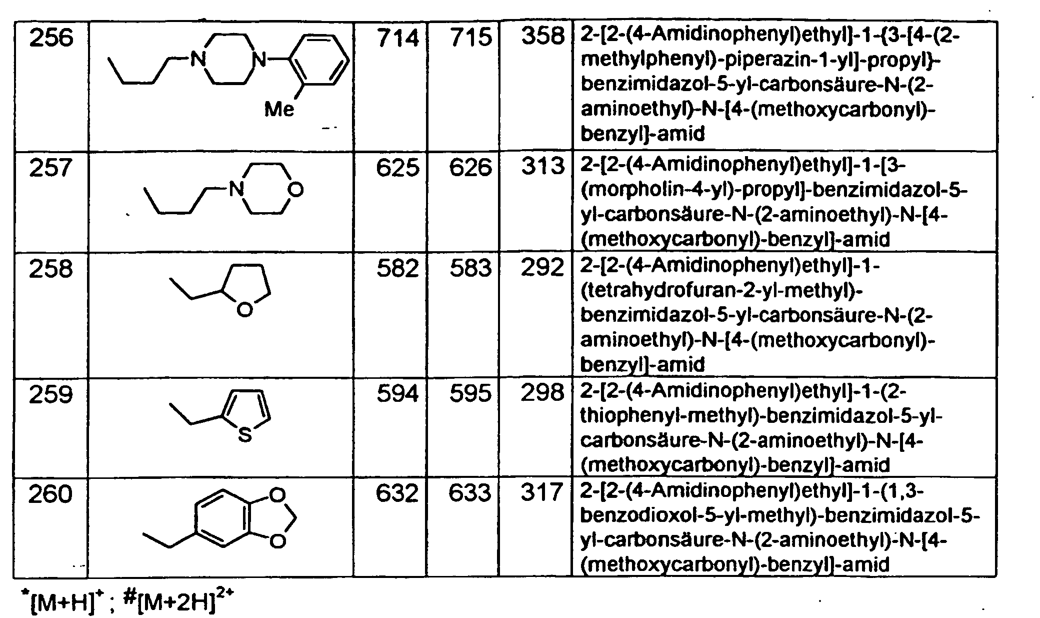

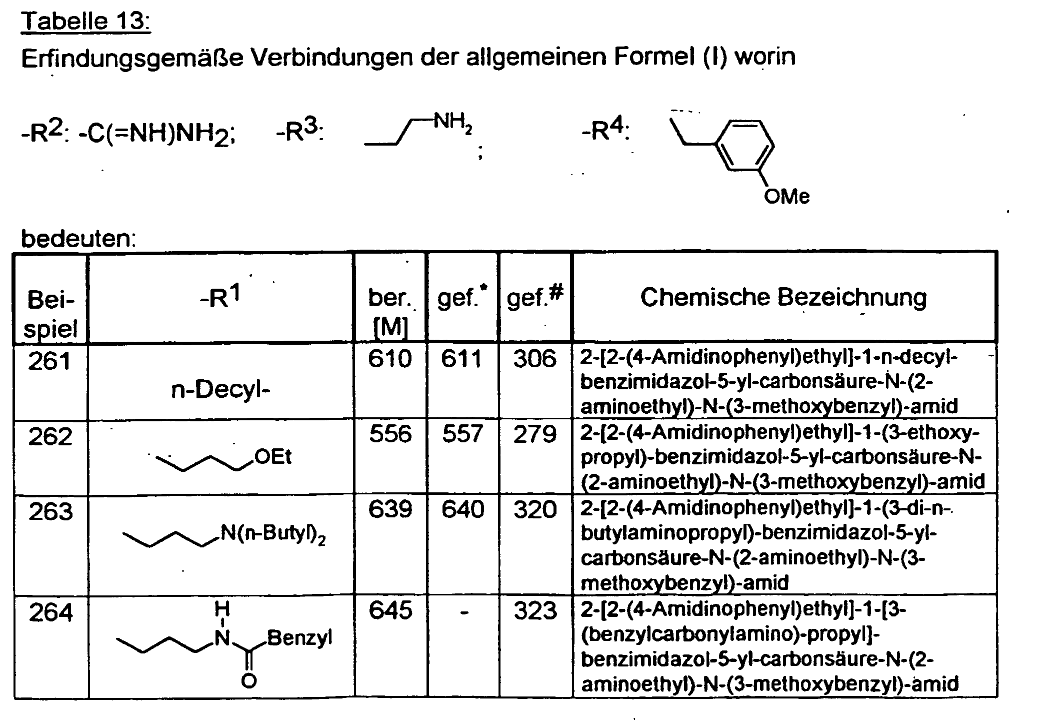

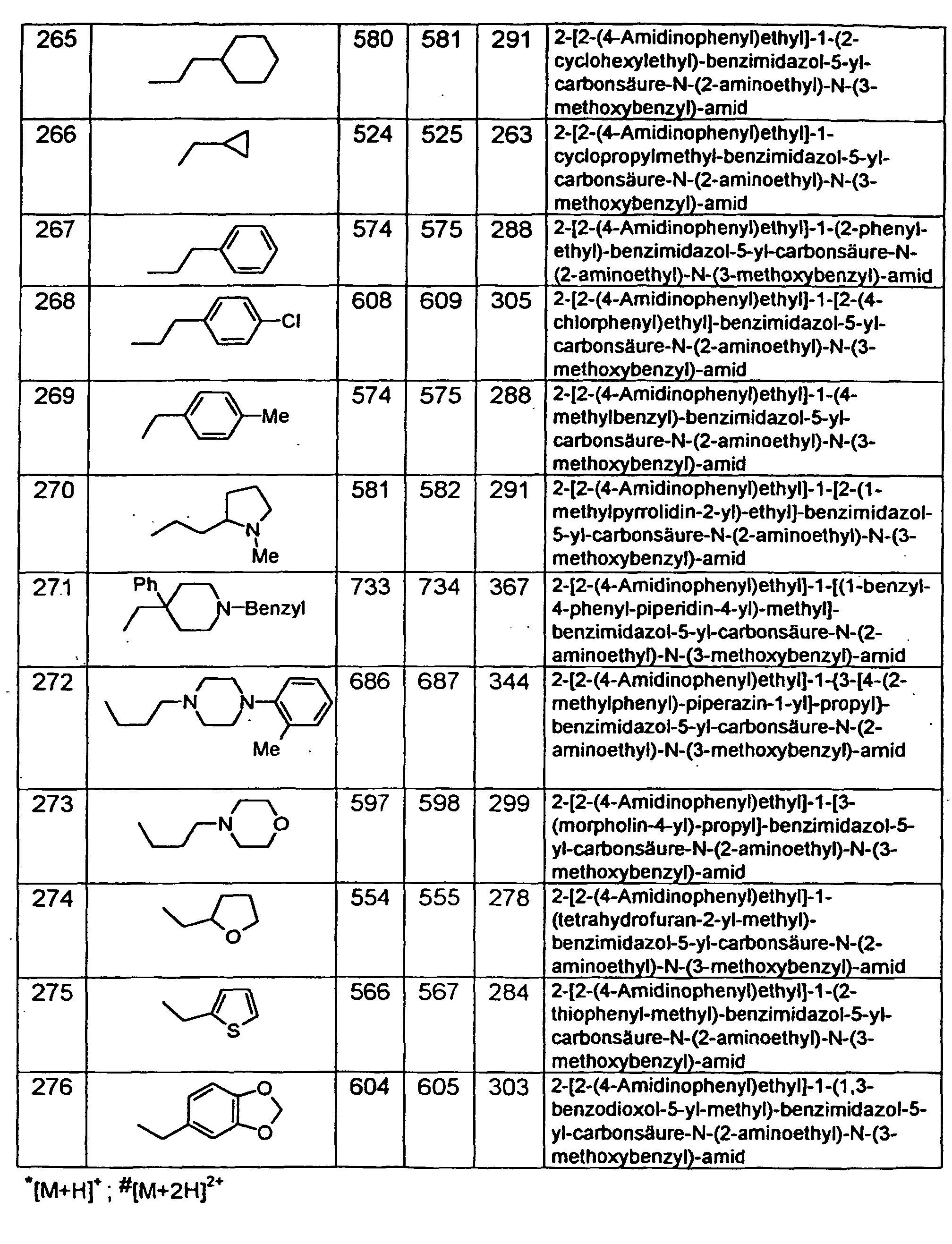

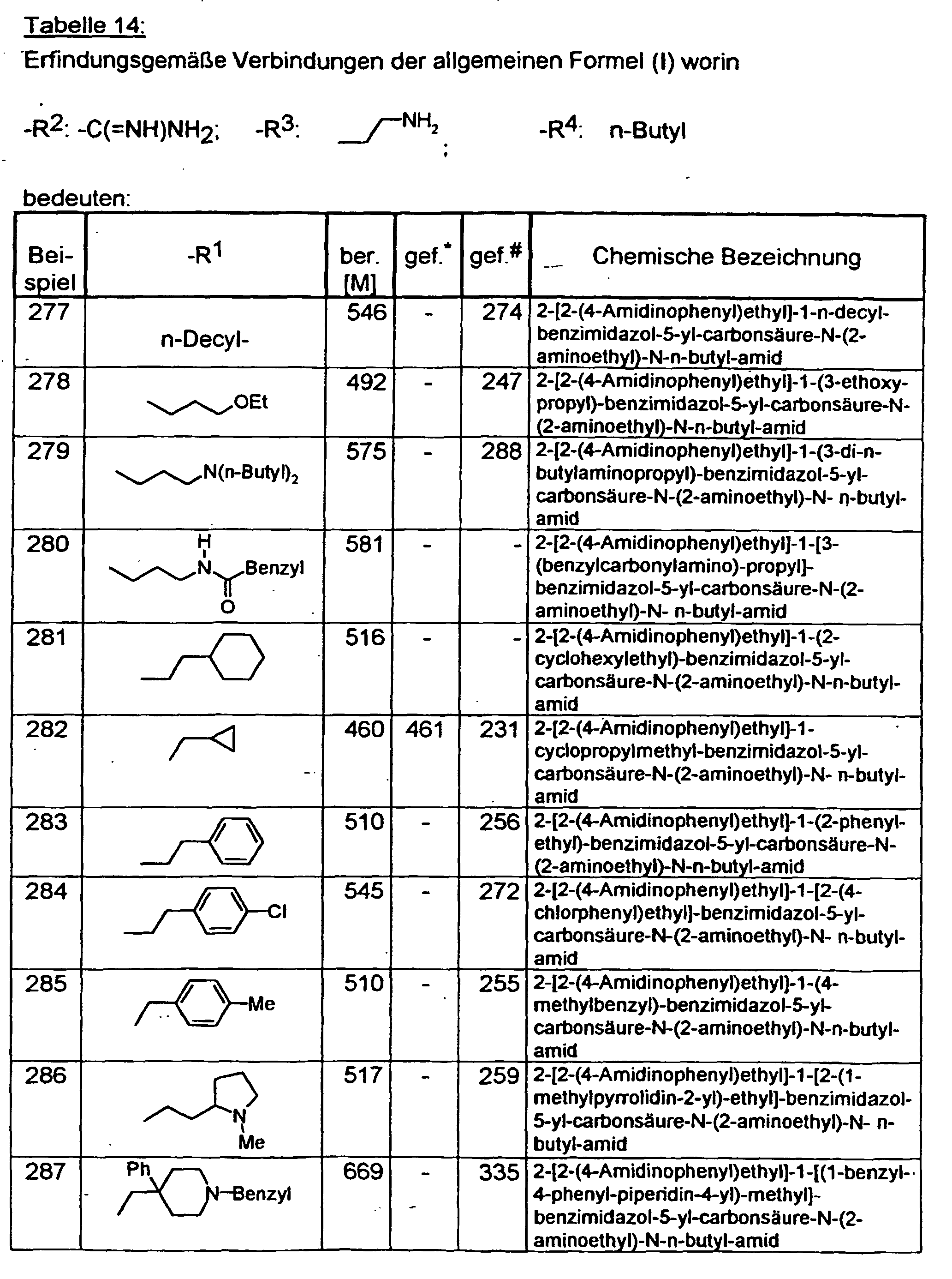

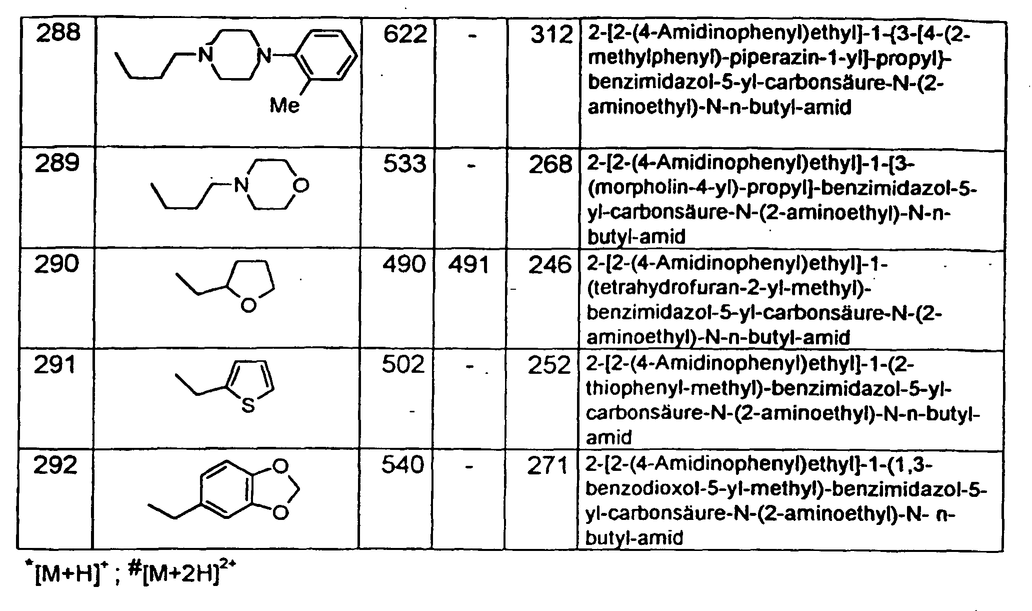

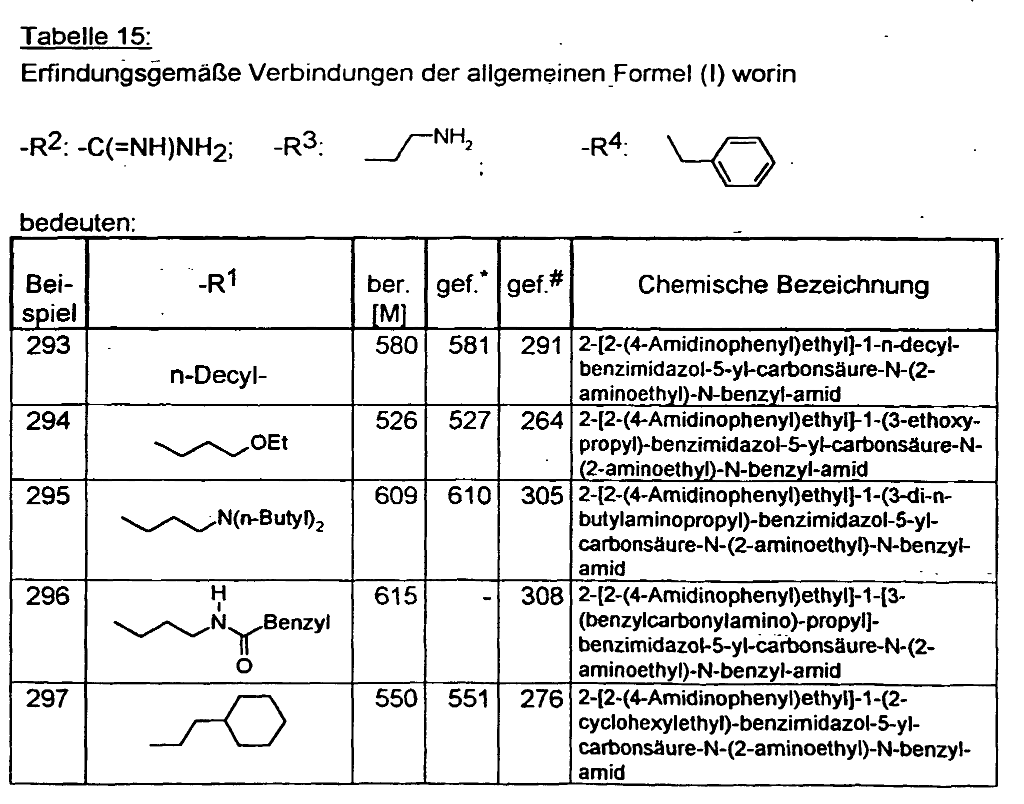

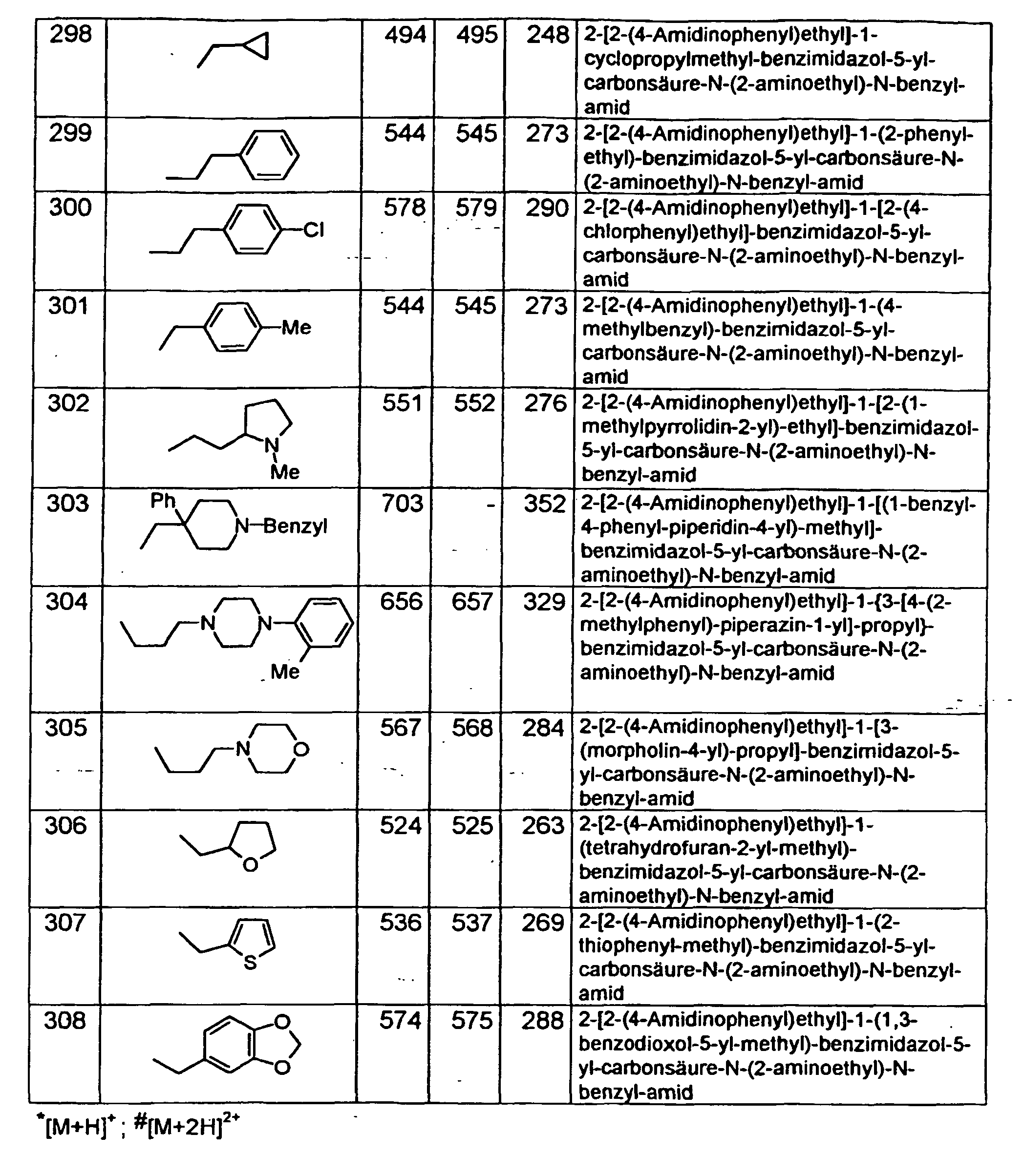

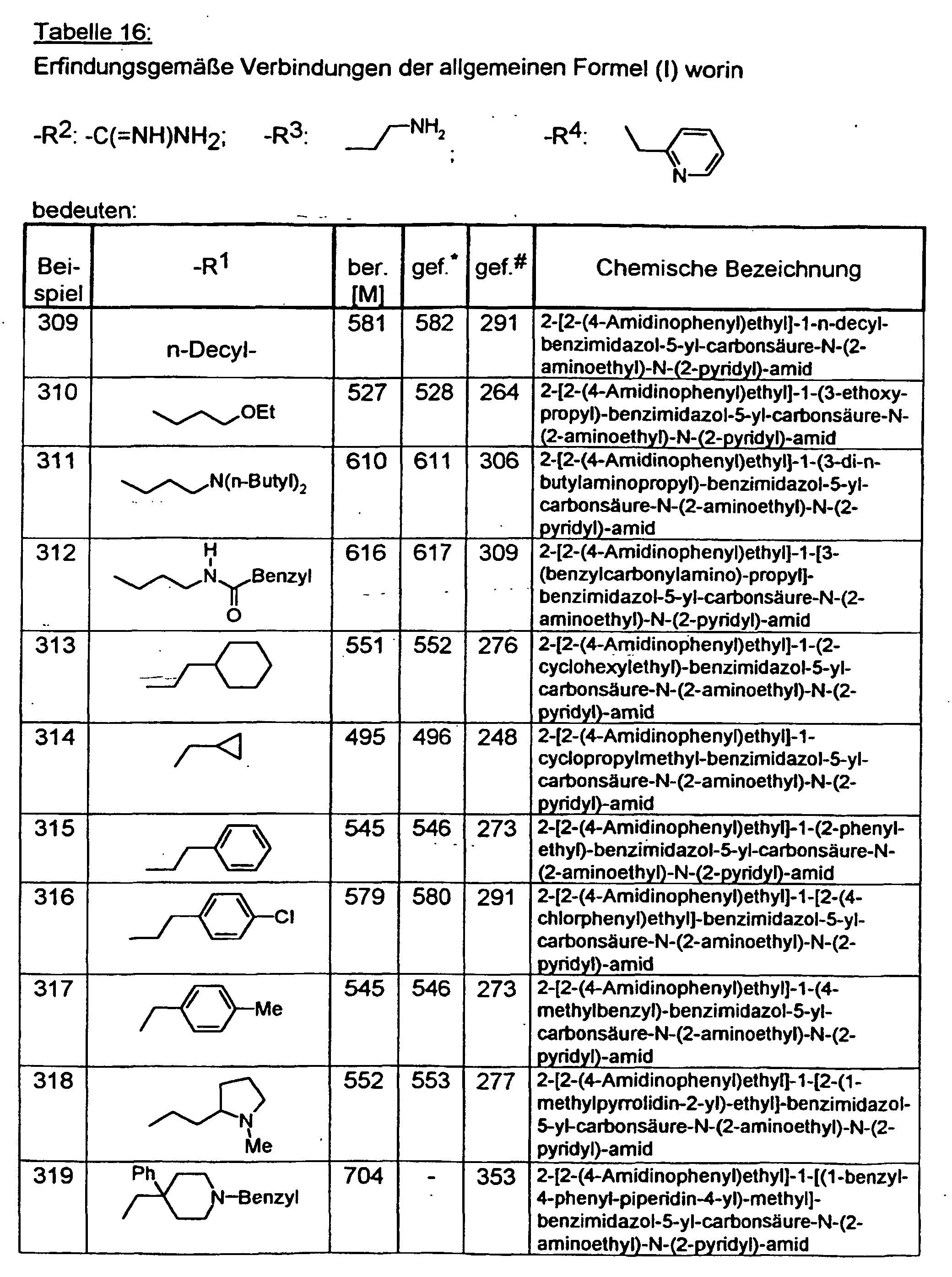

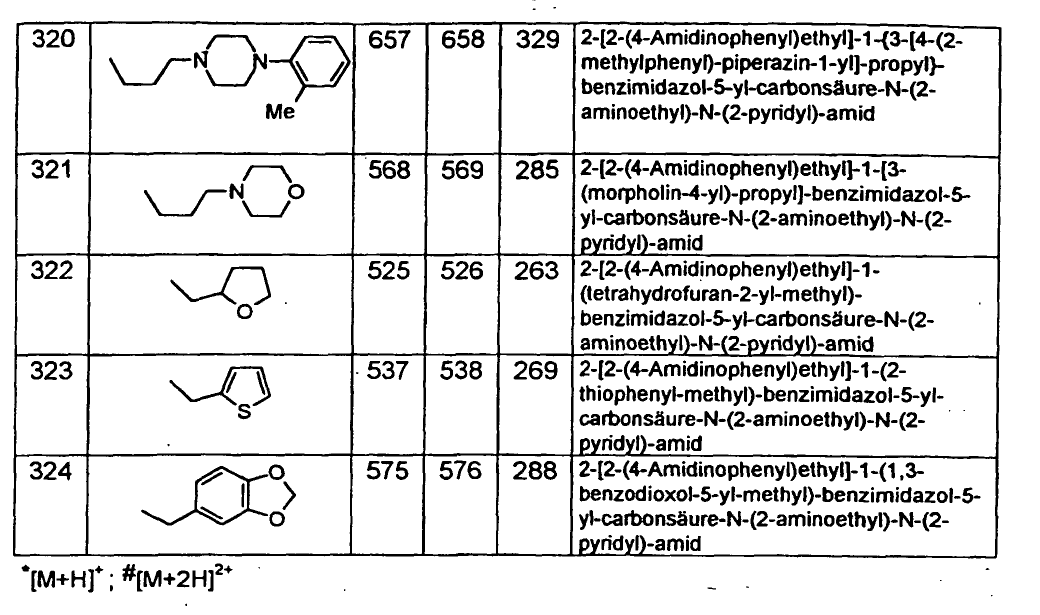

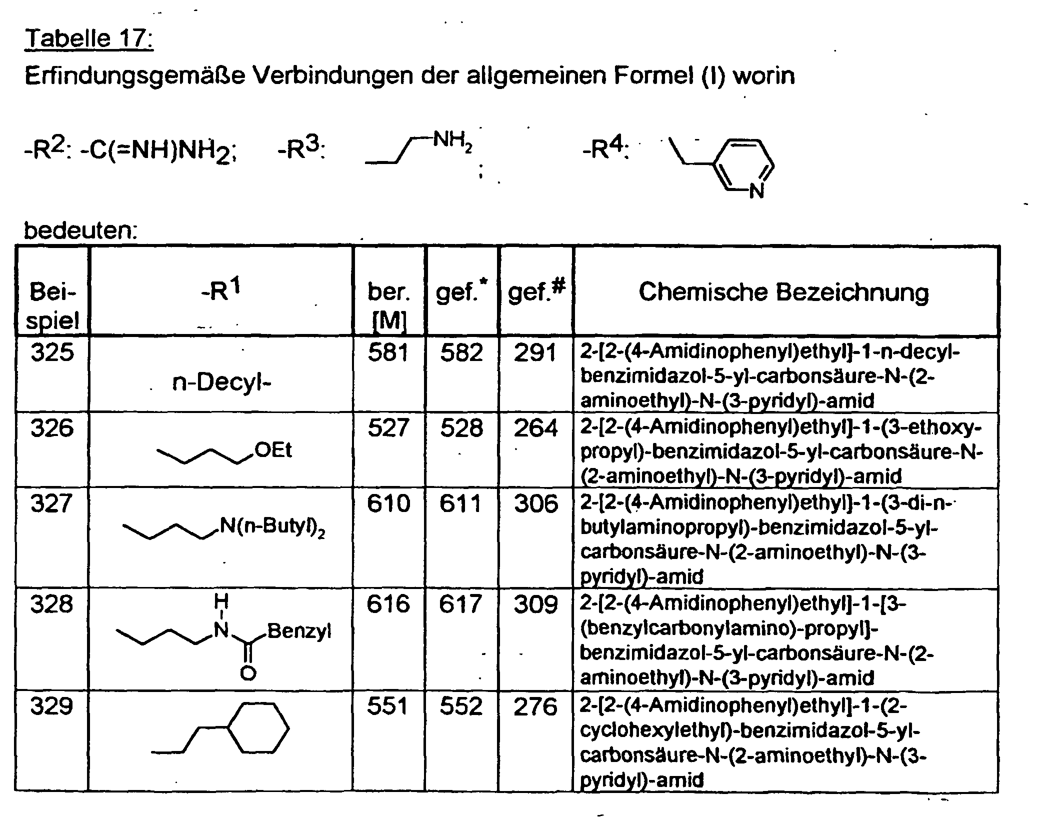

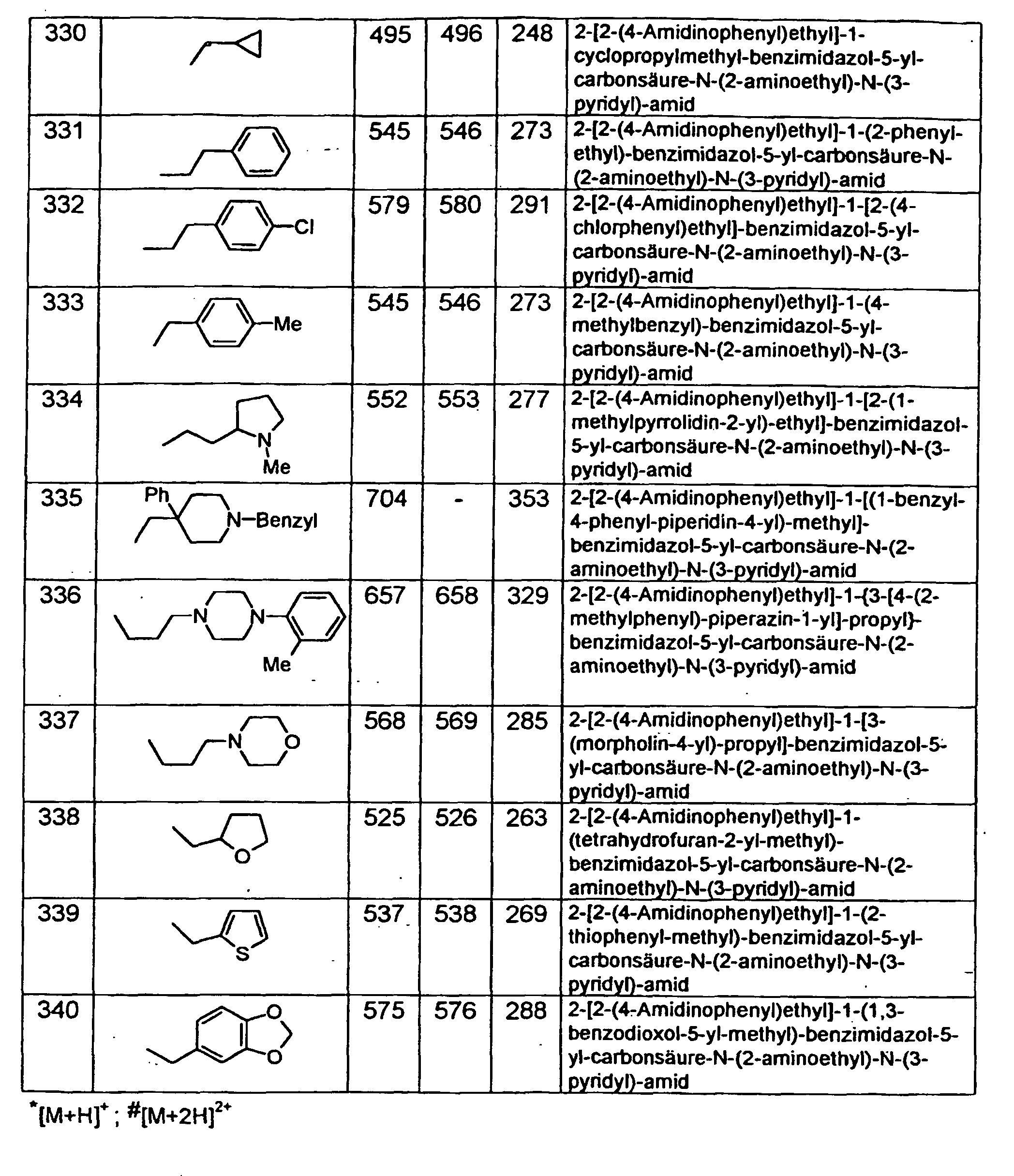

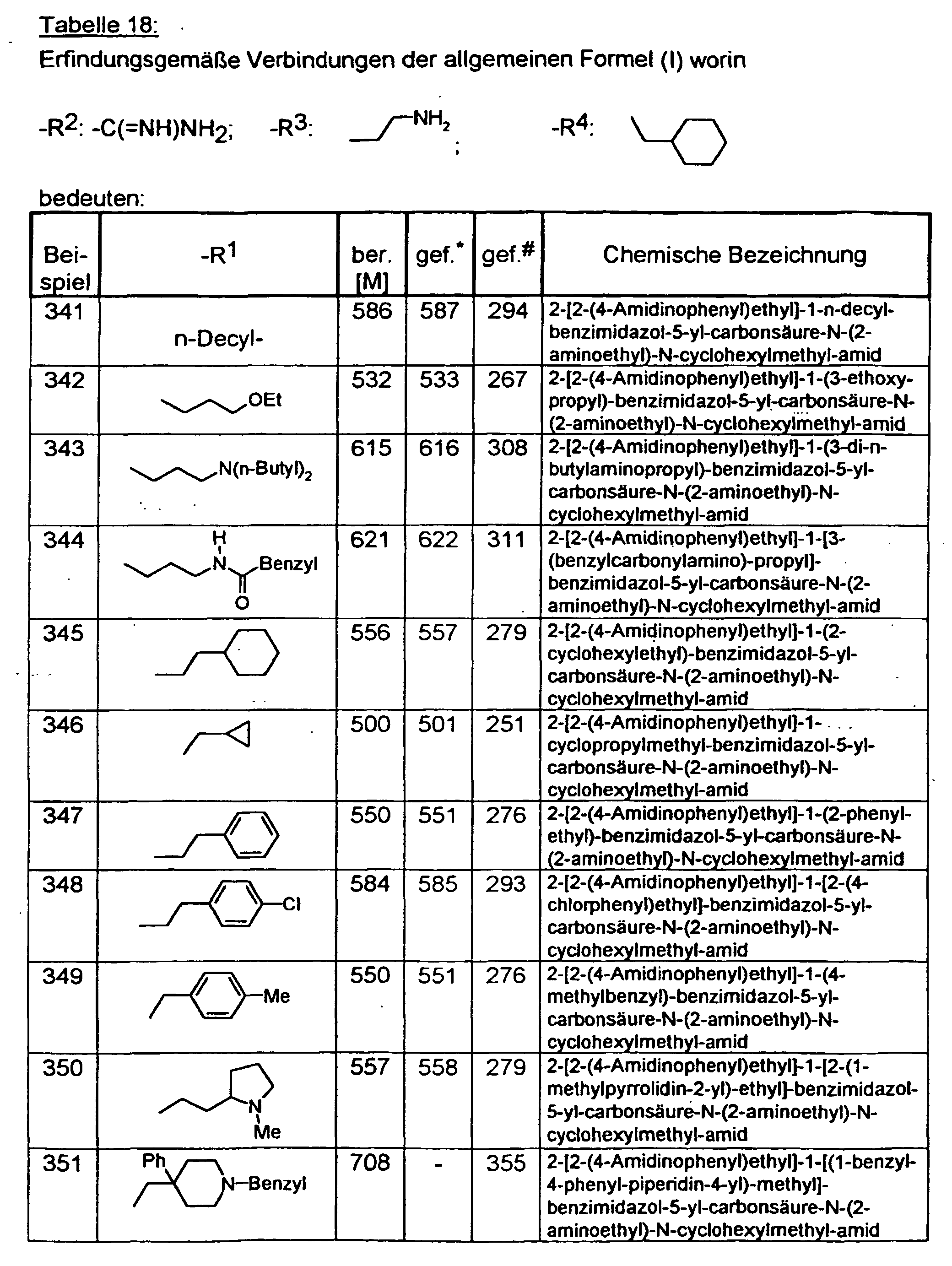

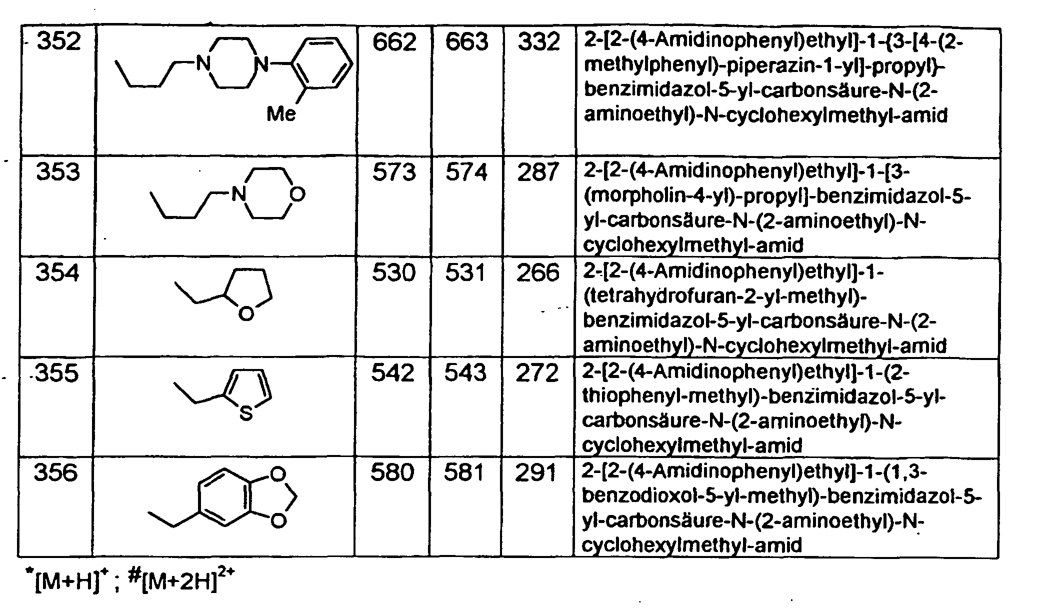

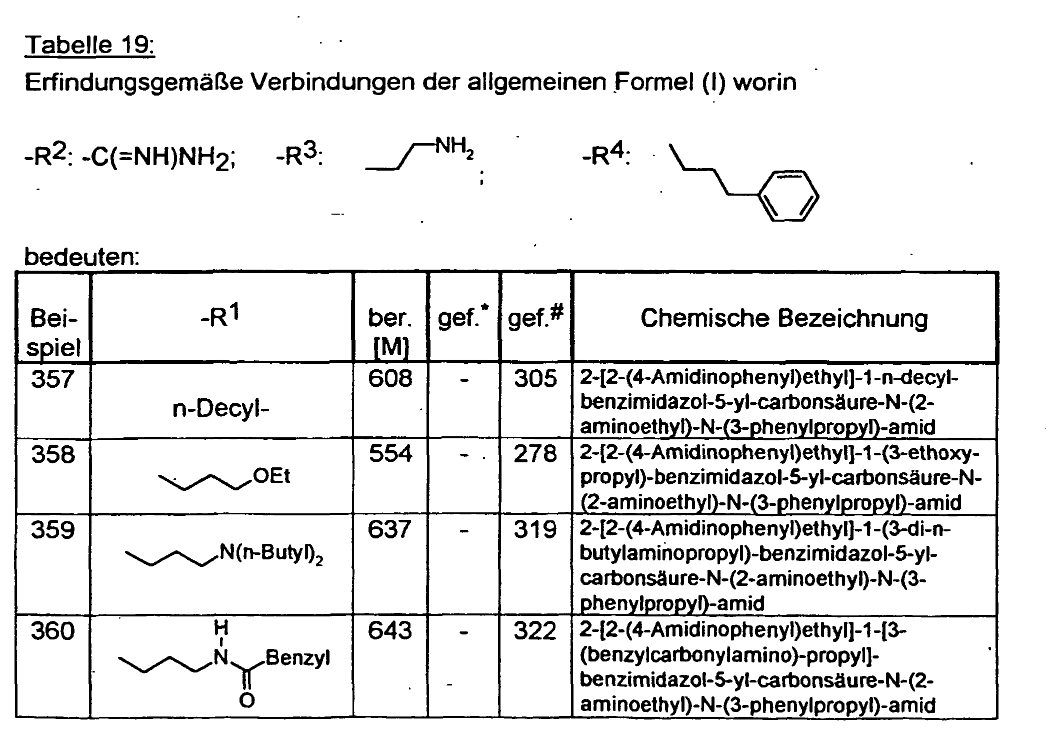

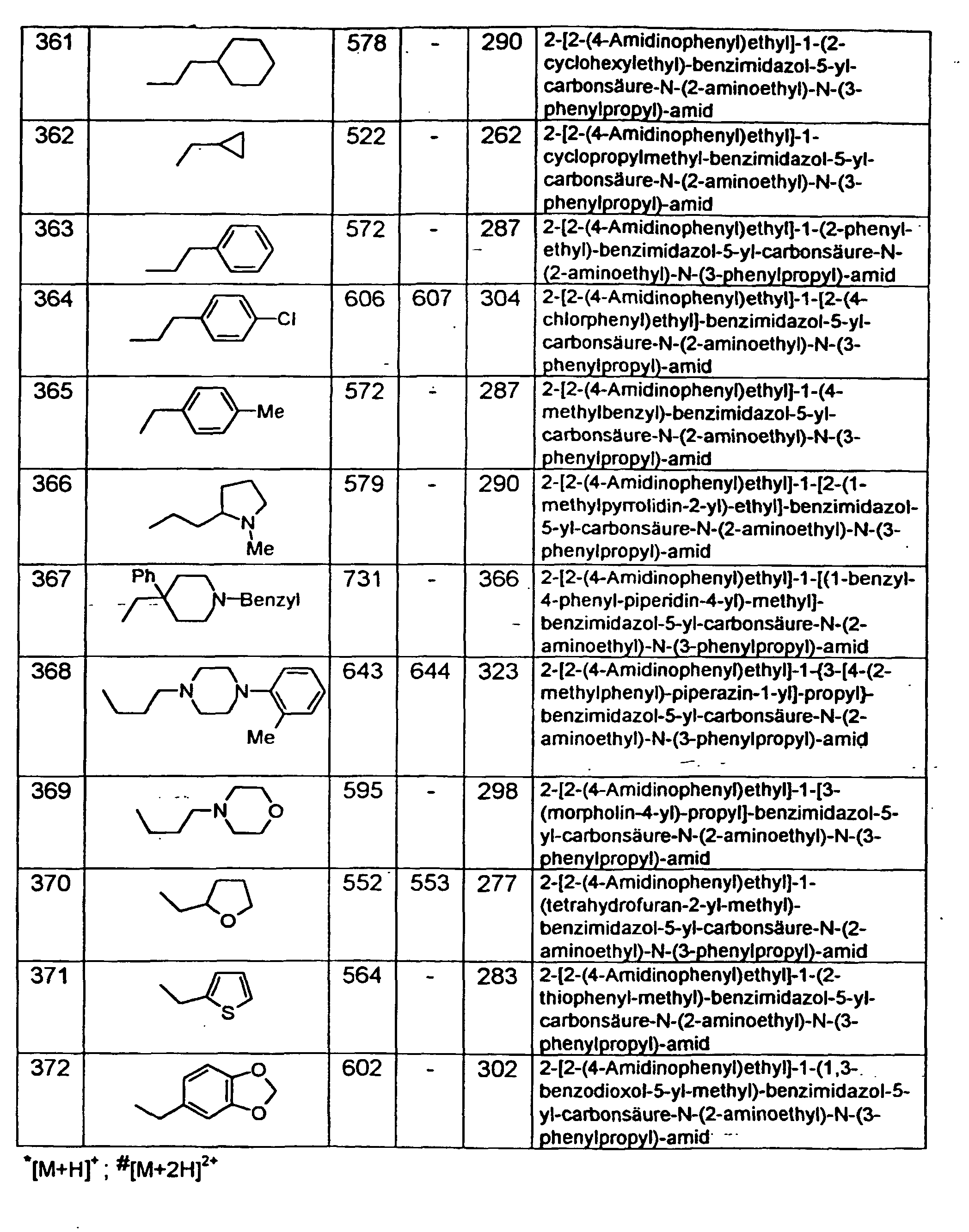

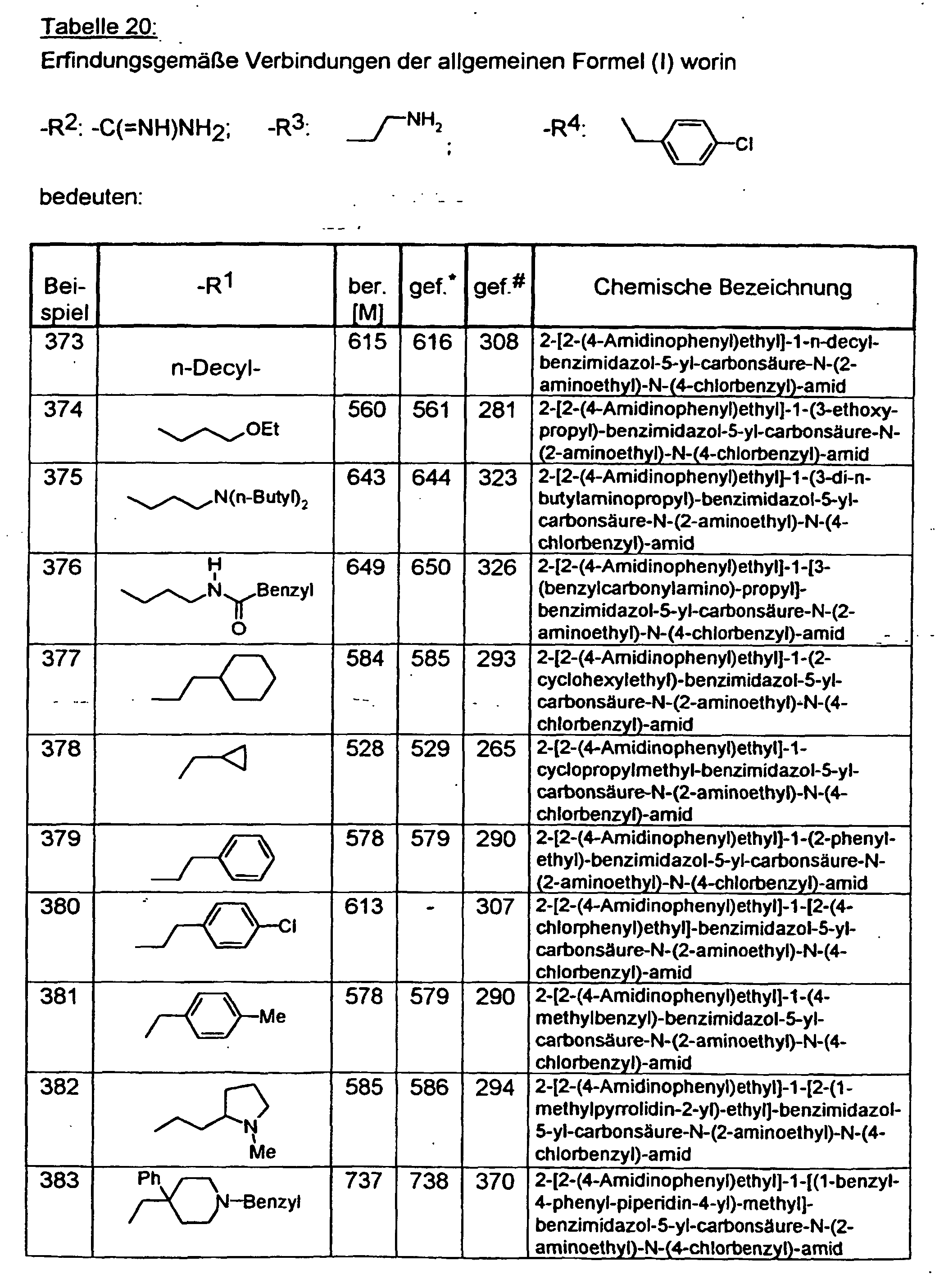

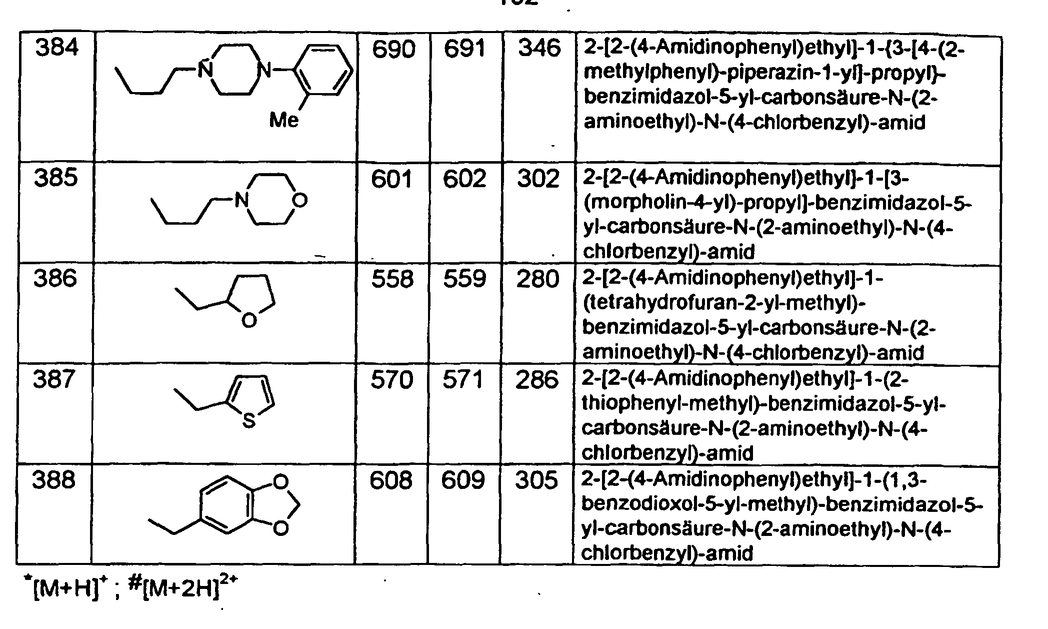

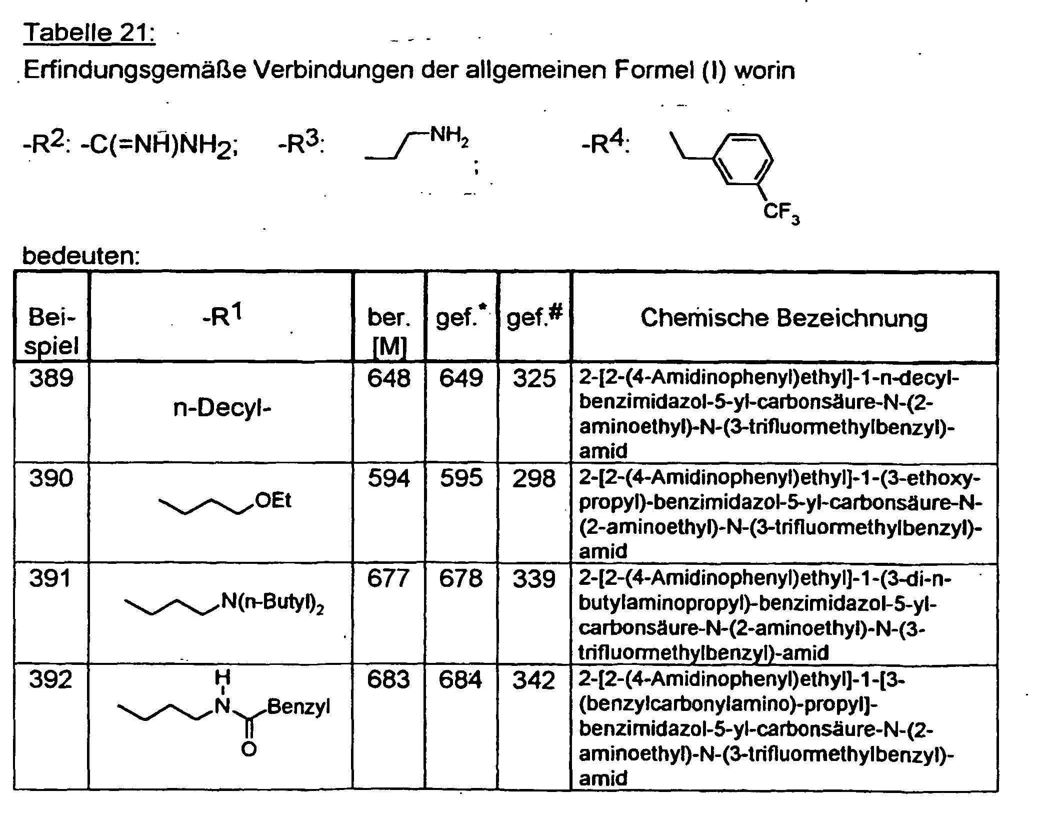

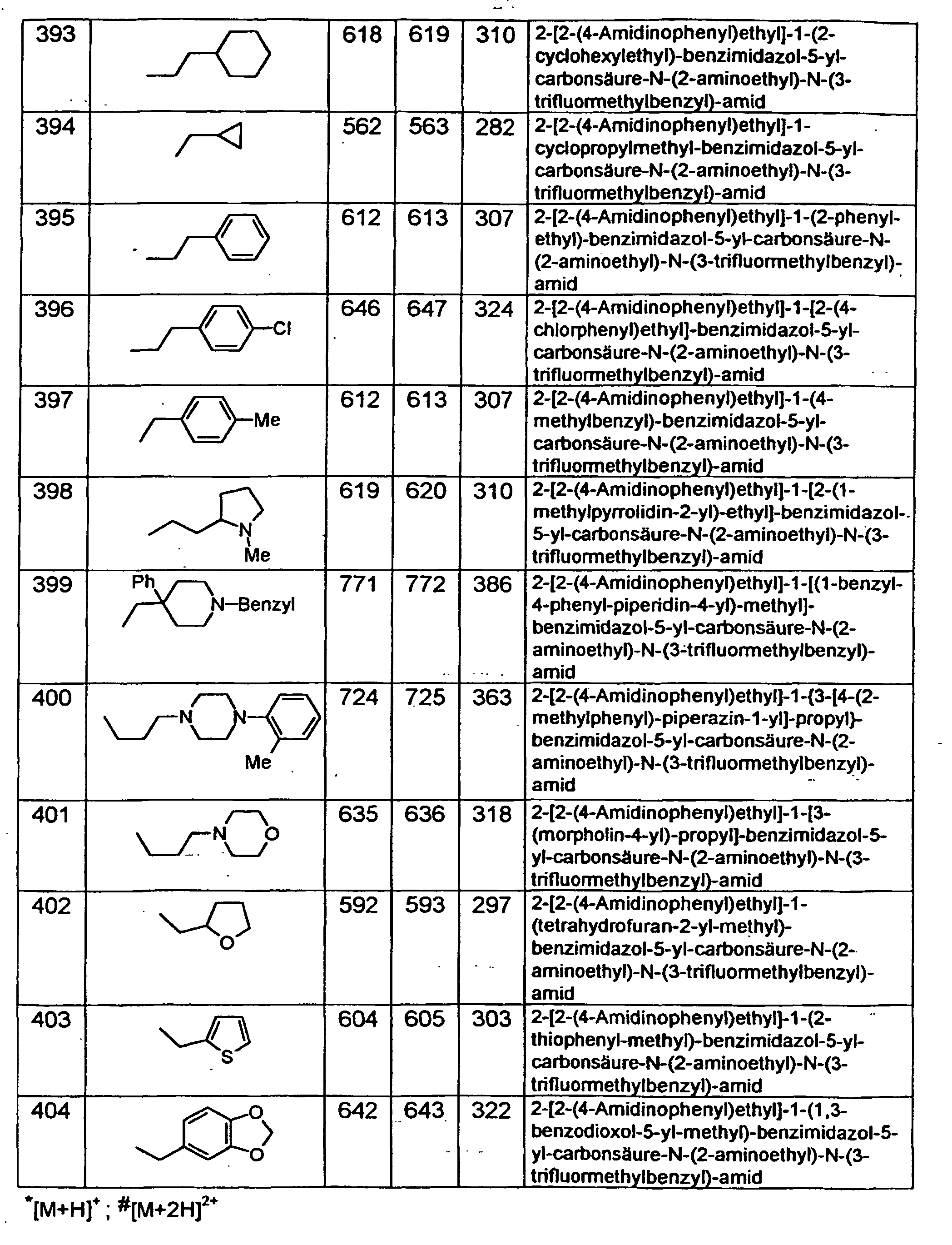

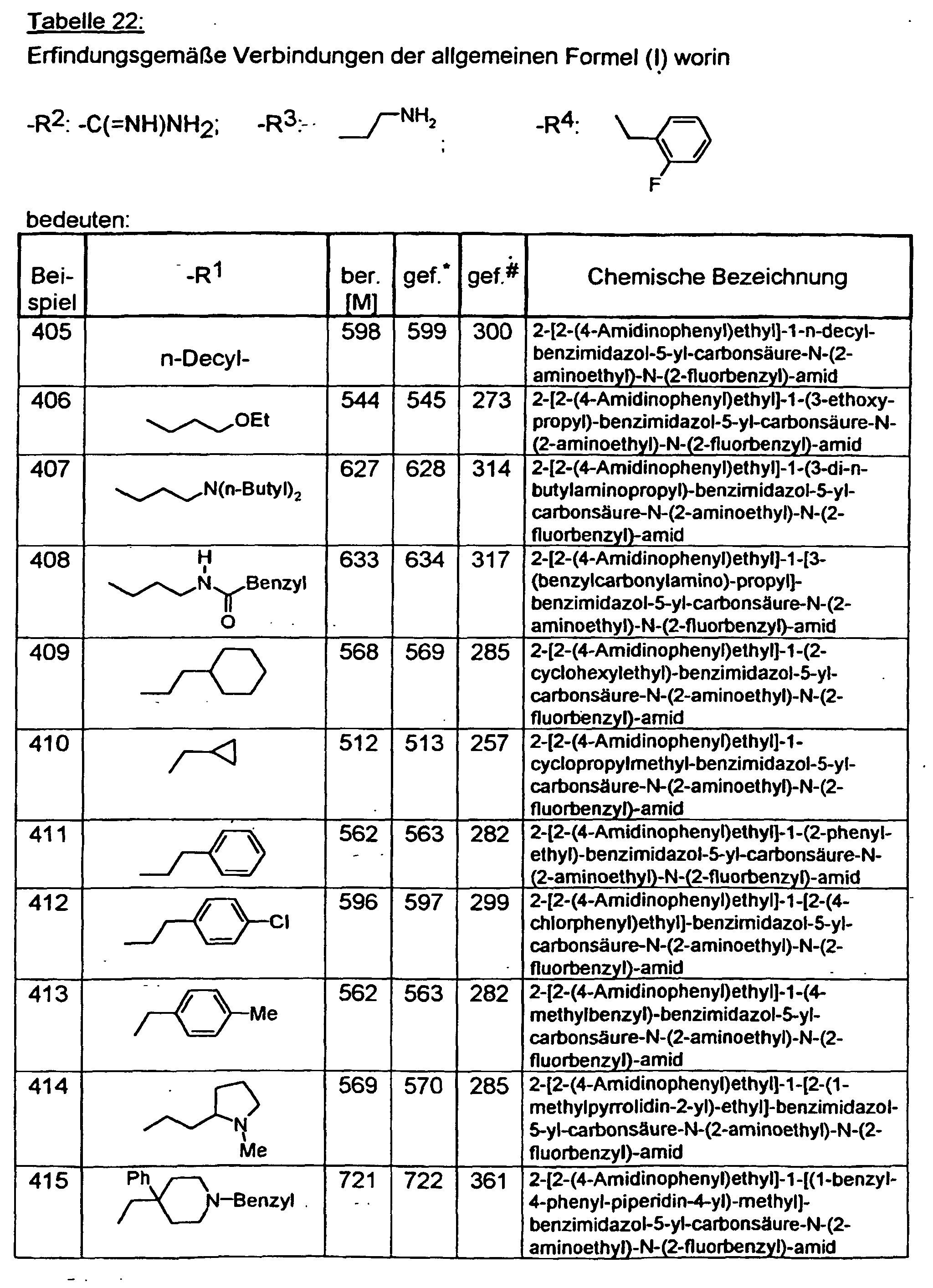

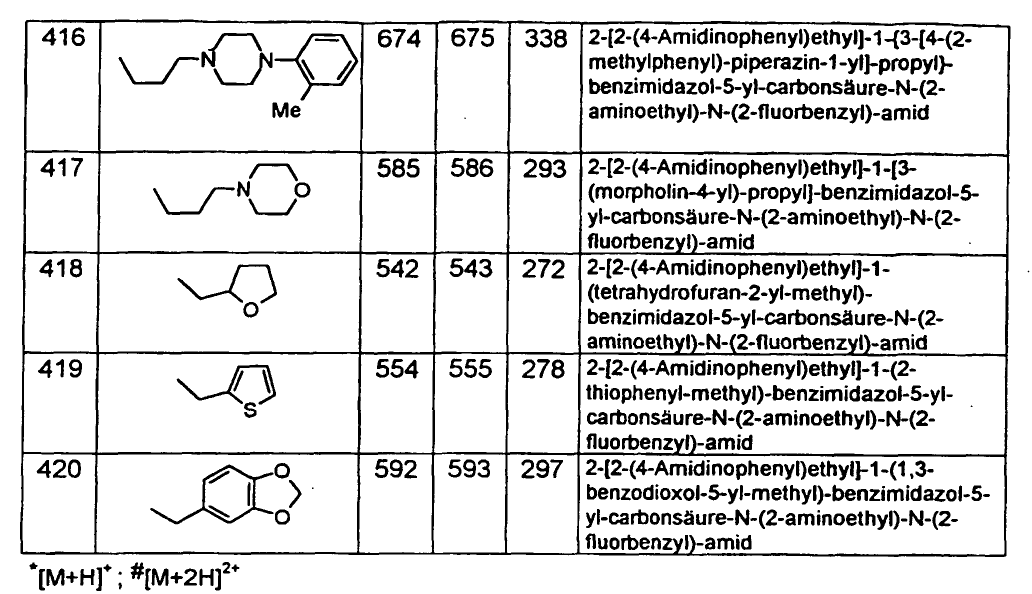

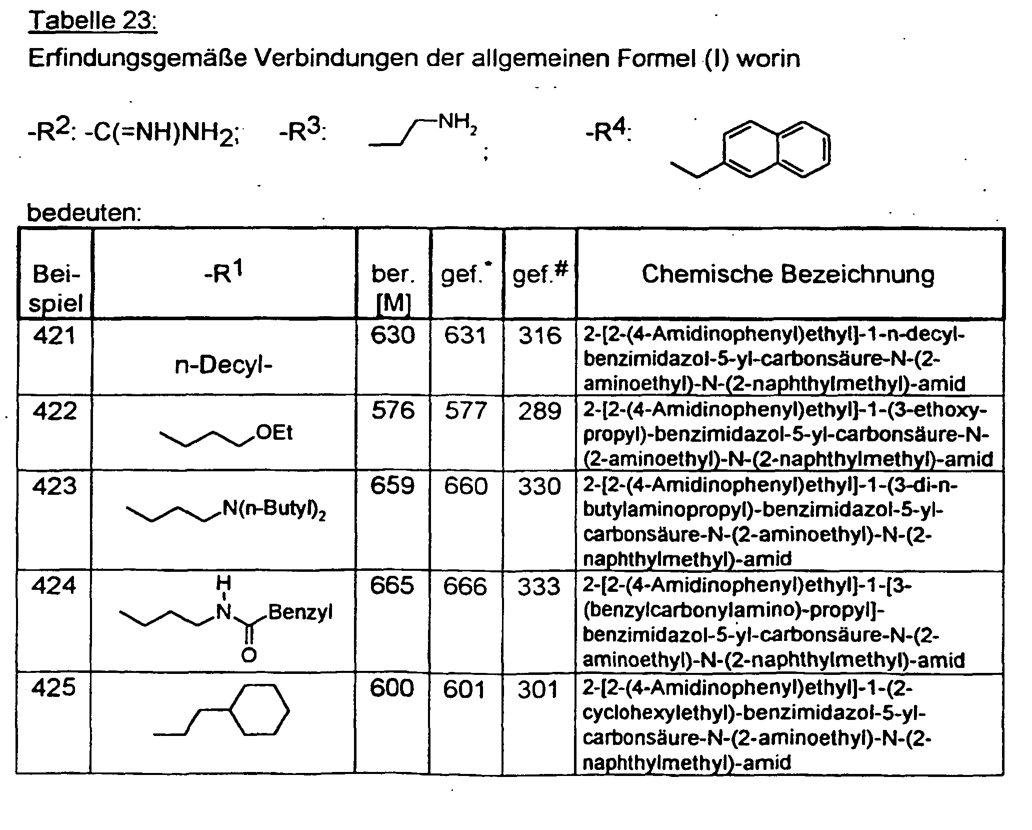

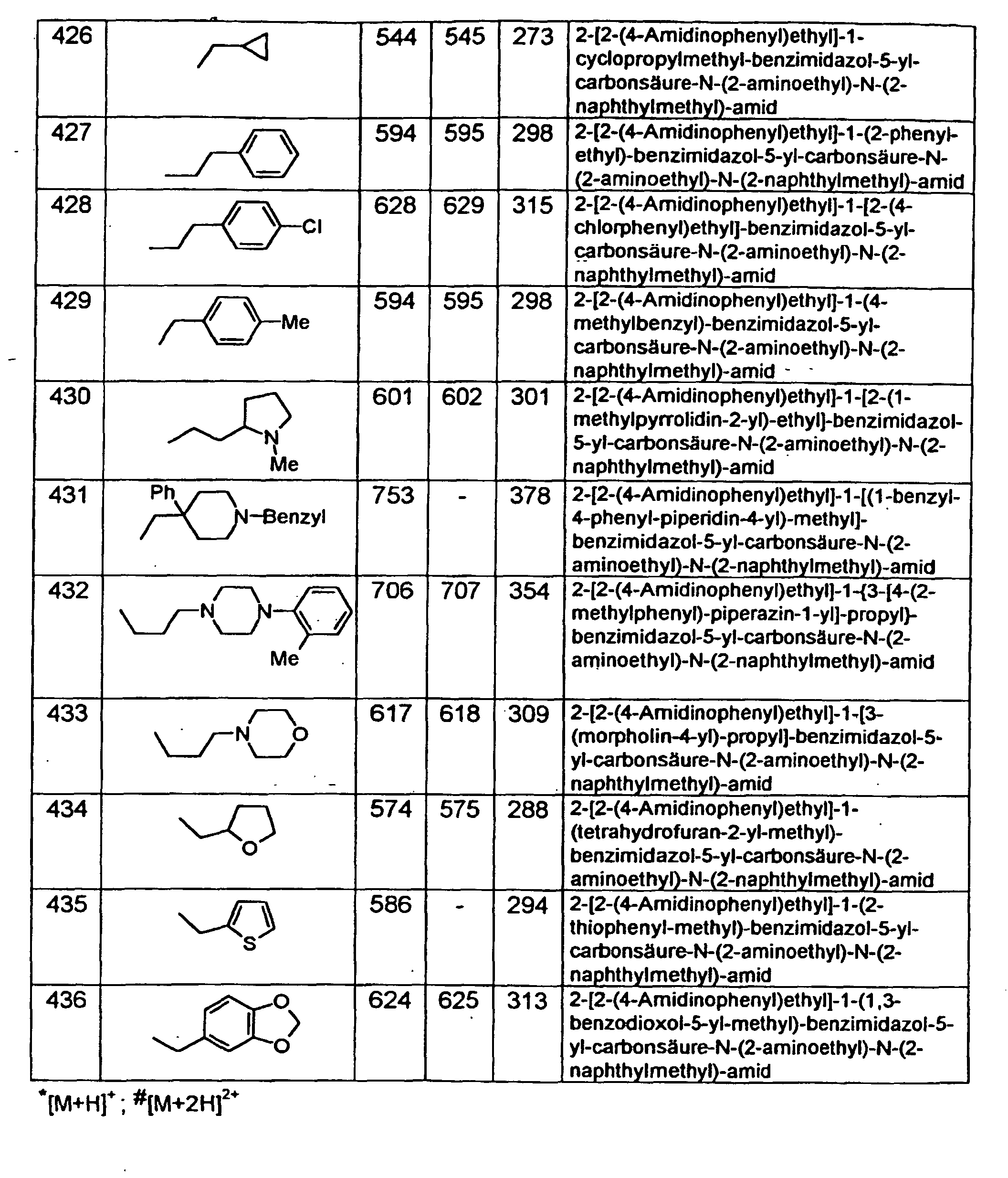

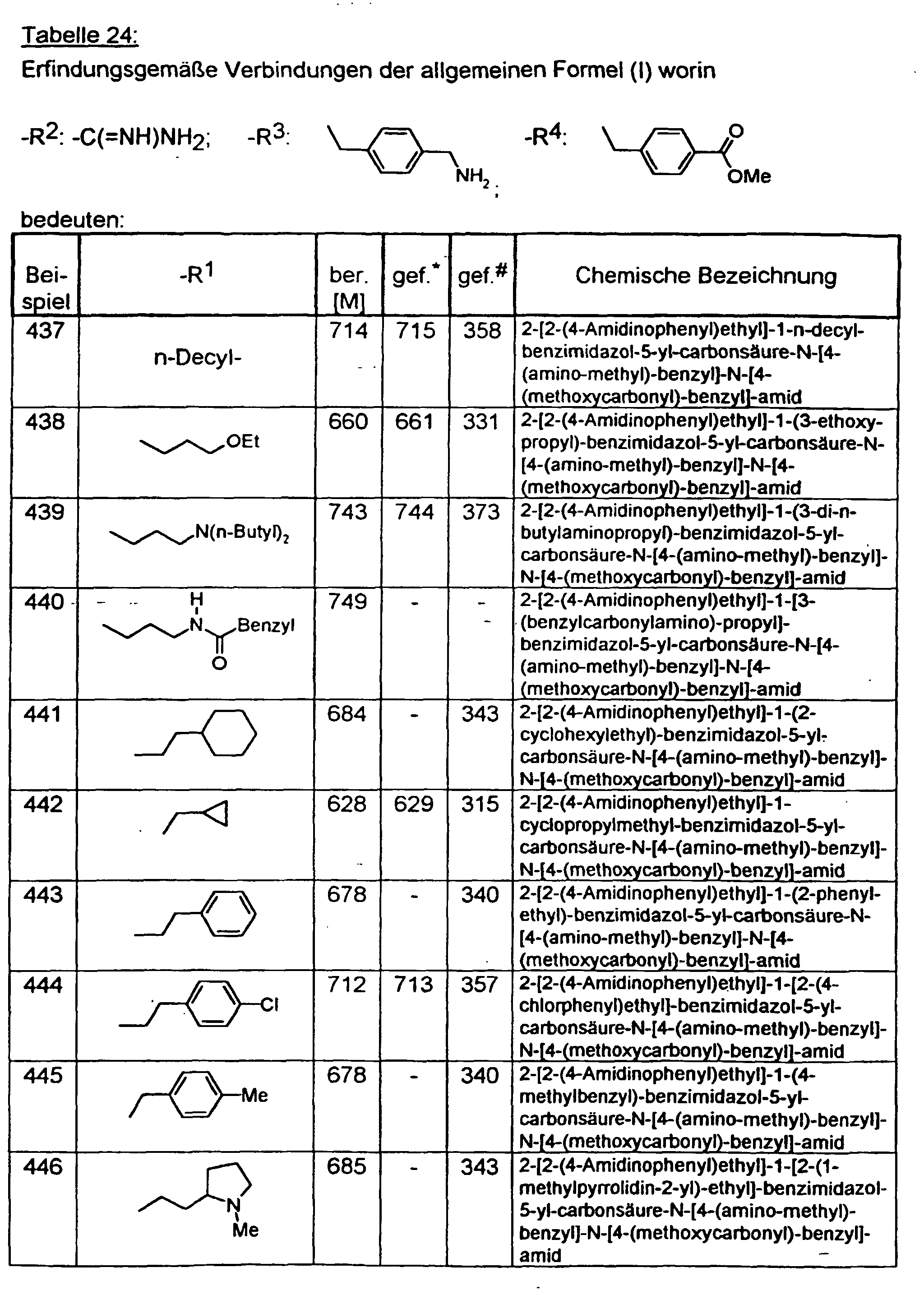

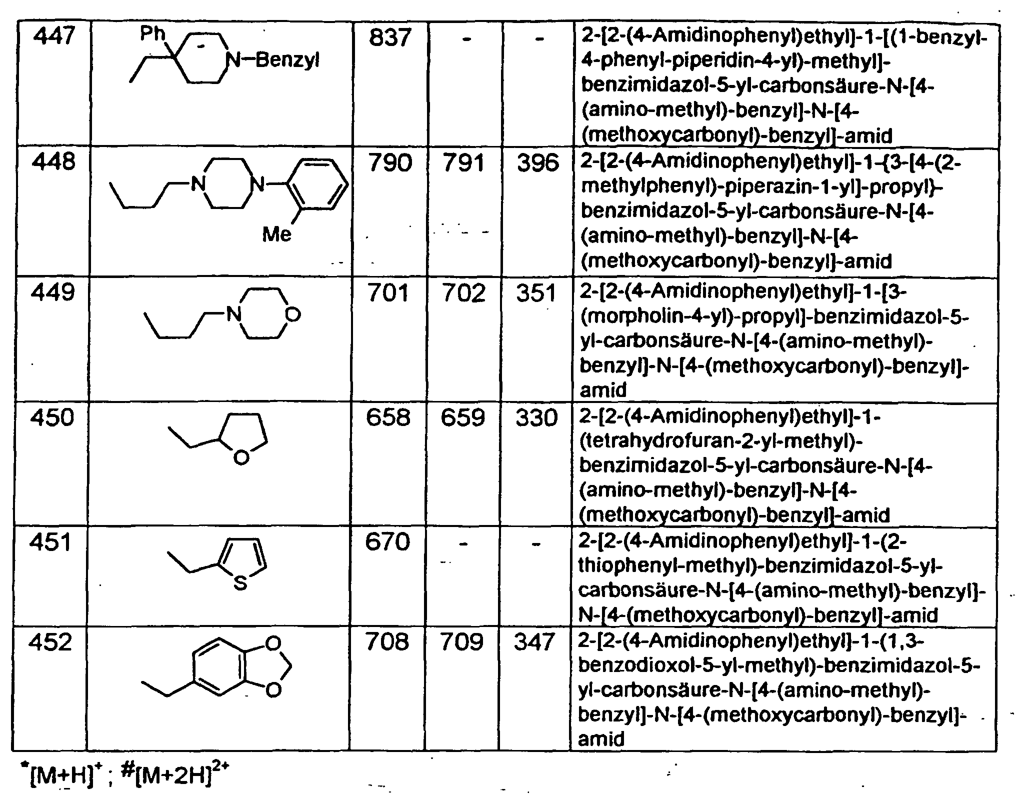

Die vorstehend aufgeführten massenspektroskopischen Daten wurden über MS-ESI (Electrospray Ionisation) bestimmt.

Die erfindungsgemäßen Verbindungen zeichnen sich durch ihre Tryptase-inhibierende Wirksamkeit aus. Besagte Fähigkeit, die Tryptase zu inhibieren, wurde gemäß der nachfolgenden Testbeschreibung untersucht.
Die Bestimmung wird in Tris HCl Puffer (100 mM), der zusätzlich Calcium (5 mM) und Heparin (100 mg/ml) enthält, bei pH 7.4 durchgeführt. Als Standard wird rh beta Tryptase eingesetzt, die beispielsweise von Promega käuflich zu erwerben ist. Als Substrat dient N-p-Tosyl-Gly-Pro-Lys-para-nitroanilin in einer Konzentration von 0.6 mM. Das Substrat wird durch Tryptase verdaut wobei p-Nitroanilin entsteht, das bei 405 nm gemessen werden kann. Üblicherweise wird eine Inkubationszeit von 5 Minuten und eine Inkubationstemperatur von 37°C gewählt. Als Enzymaktivität werden 0.91 U/ml eingesetzt. Die Bestimmung erfolgt in einem Autoanalyser (Cobas Bio) der Firma Hofmann LaRoche. Die potentiellen Hemmsubstanzen werden in Konzentrationen von 10 µM im Screening eingesetzt, wobei die Hemmung der Tryptase in Prozent angegeben wird. Bei über 70 % Hemmung wird die IC₅₀ bestimmt (Konzentration bei der 50% der Enzymaktivität gehemmt ist). Nach 5-minütiger Vorinkubation der potentiellen Hemmsubstanzen, wird das Substrat zum Starten der Reaktion zugegeben, wobei die Bildung von p-Nitroanilin nach 5 Minuten, nach Testung der Linearität, als Maß für die Enzymaktivität genommen wird.

Die nach Durchführung des vorstehend beschriebenen Tests erhaltenen Daten (% Inhibition) sind für die erfindungsgemäßen Verbindungen in Tabelle 25 zusammengefaßt. Die sich für die erfindungsgemäßen Verbindungen ergebenden IC50-Werte sind Tabelle 26 zu entnehmen.

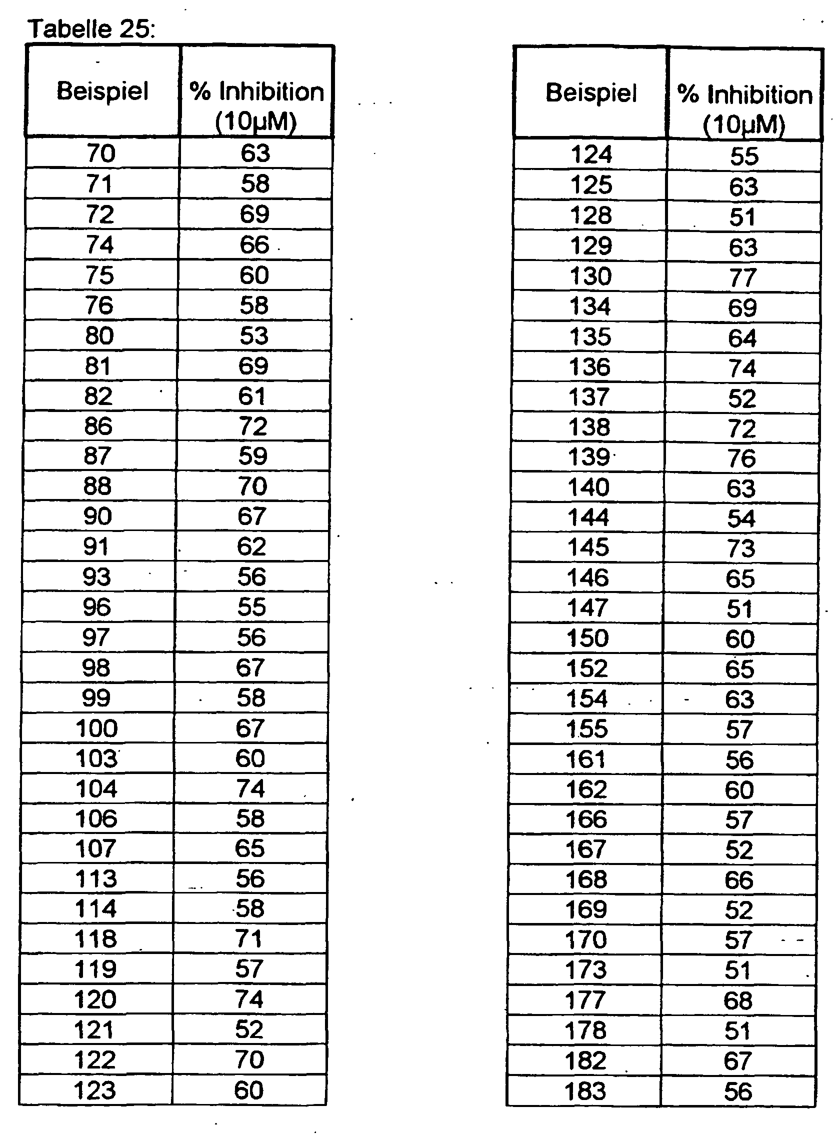

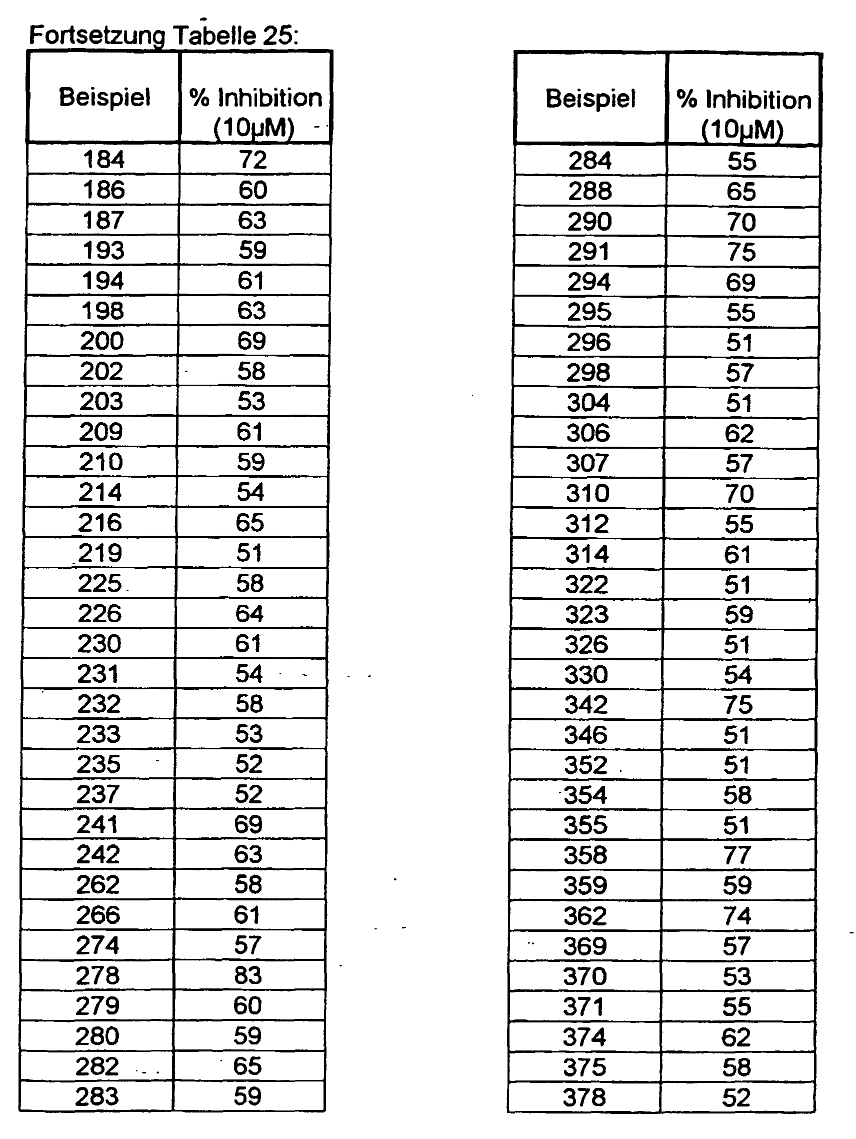

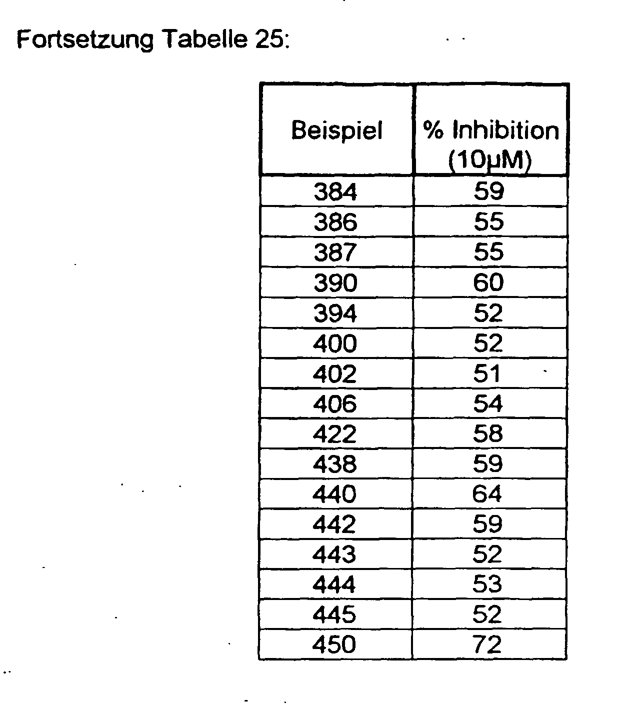

Tabelle 26 faßt-die für erfindungsgemäße Verbindungen erhalten IC50 Bindungswerte zusammen. Diese wurden wie vorstehend ausgeführt ermittelt.

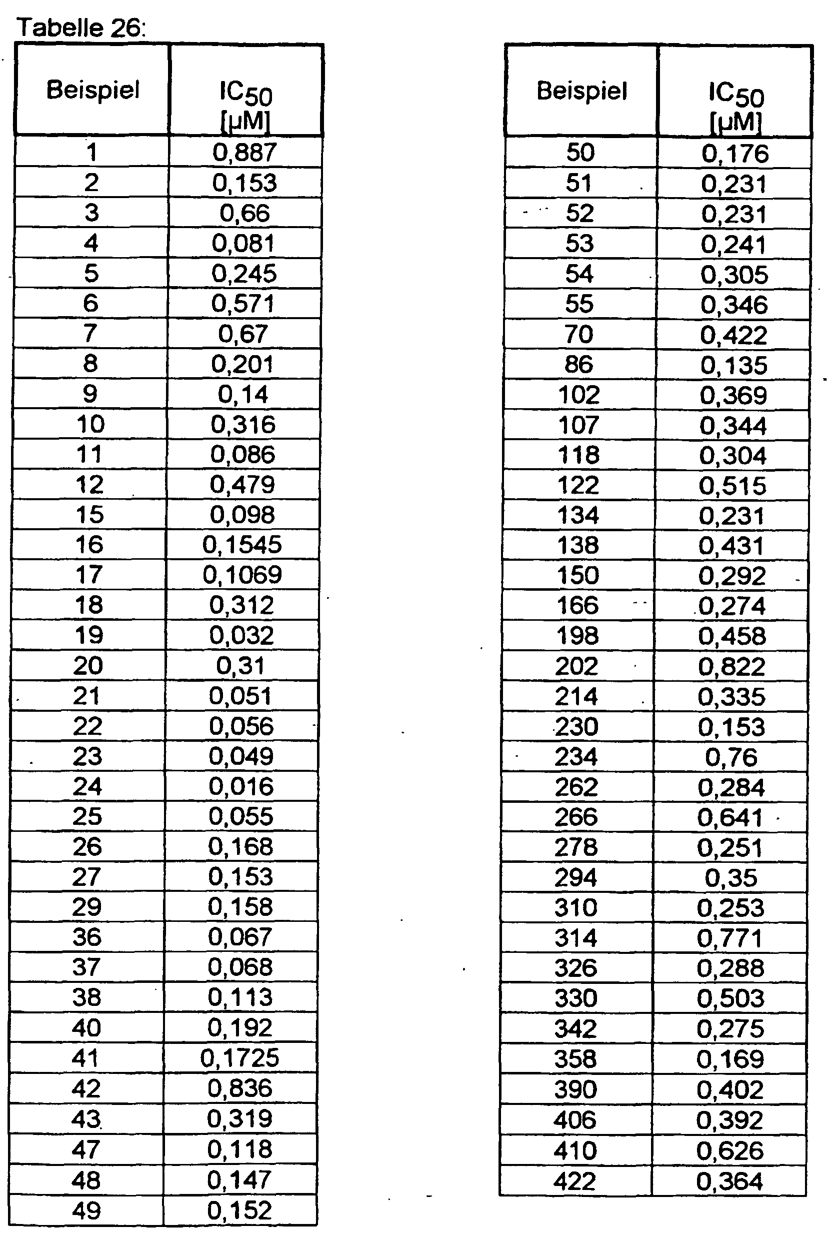

Die erfindungsgemäßen Tryptase-Inhibitoren können oral, transdermal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Maisstärke | 190 mg |
| | Milchzucker | 55 mg |
| | Mikrokristalline Cellulose | 35 mg |
| | Polyvinylpyrrolidon - | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée |
|---|---|---|
| | Wirkstoff | 5 mg |
| | Maisstärke | 41,5 mg |
| | Milchzucker | 30 mg |
| | Polyvinylpyrrolidon | 3 mg |
| | Magnesiumstearat | 0,5 mg |
| | | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragees werden mit Wachs poliert.

| D) | Kapseln | pro Kapsel |
|---|---|---|
| | Wirkstoff | 50 mg |
| | Maisstärke | 268,5 mg |
| | Magnesiumstearat | 1,5 mg |
| | | 320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Ampullenlösung | |
|---|---|---|
| | Wirkstoff - | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) | Suppositorien | |
|---|---|---|
| | Wirkstoff | 50 mg |
| | Adeps solidus | 1650 mg |
| | | 1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

## Patentansprüche

1. Verwendung von Verbindungen der allgemeinen Formel (I) 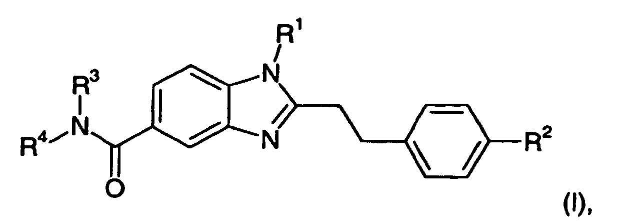 zur Herstellung eines Arzneimittels zur Vorbeugung und/oder Behandlung von Krankheiten, in denen Tryptase-Inhibitoren einen therapeutischen Nutzen entfalten können, worin
R¹ C₁-C₁₀-Alkyl, welches gegebenenfalls ein-, zwei- oder dreifach durch eine oder mehrere der Gruppen C₁-C₄-Alkoxy, Phenoxy, Hydroxyphenoxy, C₁-C₄-Alkoxy-phenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiert sein kann, oder
Phenyl-C₁-C₄-alkyl, wobei der Phenylring gegebenenfalls ein-, zwei-, oder dreifach durch einen oder mehrere der Reste C₁-C₄-Alkyl, CF₃, Fluor, Chlor, Brom, COOH oder COO-C₁-C₄-Alkyl substituiert sein kann, oder
ein über eine Einfachbindung oder über eine C₁-C₄-Alkylenbrücke verknüpfter 5 oder 6 gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, gegebenenfalls durch C₁-C₄-Alkyl substituiertes Phenyl oder gegebenenfalls durch C₁-C₄-Alkyl substituiertes Benzyl substituiert sein kann oder an den über zwei benachbarte Kohlenstoffatome gegebenenfalls ein Benzolring ankondensiert sein kann;
R² -C(=NH)NH₂ oder -CH₂-NH₂;
R³ und R⁴ gleich oder verschieden,
Wasserstoff, C₁-C₆-Alkyl, welches ein- oder zweifach durch eine oder mehrere der Gruppen COOH, COO-C₁-C₄-Alkyl, C₃-C₆-Cycloalkyl, -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert sein kann, oder
Phenyl-C₁-C₄-alkyl, wobei die C₁-C₄-Alkylenbrücke gegebenfalls durch Phenyl, COOH oder COO-C₁-C₄-Alkyl substituiert sein kann und wobei der Phenylring gegebenenfalls ein-, zwei-, oder dreifach, direkt oder über eine C₁-C₄-Alkylenbrücke durch einen oder mehrere der Reste C₁-C₄-Alkyl, C₁-C₄-Alkoxy, CF₃, Fluor, Chlor, Brom, COOH, COO-C₁-C₄-Alkyl, -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert sein kann, oder
ein direkt oder über eine C₁-C₄-Alkylenbrücke verknüpfter 5-, 6- oder 7-gliedriger, gesättigter oder ungesättigter Heterocyclus, der ein, zwei, drei oder vier Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, Phenyl oder Benzyl substituiert sein kann oder an den über zwei benachbarte Kohlenstoffatome gegebenenfalls ein Benzolring ankondensiert sein kann, oder
C₃-C₈-Cycloalkyl, Naphthyl oder Phenyl, welches gegebenenfalls ein-, zwei-, oder dreifach durch einen oder mehrere der Reste C₁-C₄-Alkyl, C₁-C₄-Alkoxy, Phenyloxy, Benzyloxy, CF₃, Fluor, Chlor, Brom, COOH oder COO-C₁-C₄-Alkyl substituiert sein kann, oder
oder
R³ und R⁴ bilden gemeinsam mit dem Stickstoffatom einen 5-, 6- oder 7-gliedrigen, gesättigten oder ungesättigten Heterocyclus, der ein oder zwei weitere Heteroatome ausgewählt aus der Gruppe Sauerstoff, Stickstoff oder Schwefel enthalten kann und der gegebenenfalls durch einen oder mehrere der Reste C₁-C₄-Alkyl, C₅-C₆-Cycloalkyl, Benzyl, das gegebenenfalls durch C₁-C₄-Alkyl substituiert sein kann, Pyridyl oder Phenyl, das gegebenenfalls durch C₁-C₄-Alkyl-, C₁-C₄-Alkoxy- oder Hydroxy substituiert ist, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

2. Aminocarbonyl-substituierte Benzimidazolderivate der allgemeinen Formel (I) 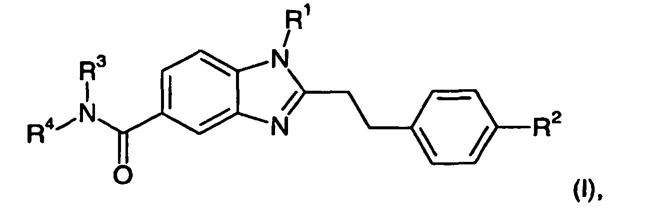 worin
R¹ C₁-C₁₀-Alkyl, welches gegebenenfalls ein-, zwei- oder dreifach durch eine oder mehrere der Gruppen C₁-C₄-Alkoxy, Phenoxy-, C₁-C₄-Alkoxy-phenoxy, Hydroxyphenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiert sein kann, oder
Phenyl-C₁-C₄-alkyl, wobei der Phenylring gegebenenfalls ein-, zwei-, oder dreifach durch einen oder mehrere der Reste C₁-C₄-Alkyl, CF₃, Fluor, Chlor, Brom, COOH oder COO-C₁-C₄-Alkyl substituiert sein kann, oder
ein über eine Einfachbindung oder über eine C₁-C₄-Alkylenbrücke verknüpfter 5 oder 6 gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, gegebenenfalls durch C₁-C₄-Alkyl substituiertes Phenyl oder gegebenenfalls durch C₁-C₄-Alkyl substituiertes Benzyl substituiert sein kann oder an den über zwei benachbarte Kohlenstoffatome gegebenenfalls ein Benzolring ankondensiert sein kann;
R² -C(=NH)NH₂ oder -CH₂-NH₂;
R³ C₁-C₆-Alkyl, welches ein- oder zweifach durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Benzyl, wobei der Phenylring ein- oder zweifach, direkt oder über eine C₁-C₄-Alkylenbrücke durch eine oder mehrere der Gruppen -NH₂,
-NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Phenyl-C₂-C₄-alkyl, wobei die C₂-C₄-Alkylenbrücke gegebenfalls durch Phenyl, COOH oder COO-C₁-C₄-Alkyl substituiert sein kann und
wobei der Phenylring gegebenenfalls ein- oder zweifach, direkt oder über eine C₁-C₄-Alkylenbrücke durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert sein kann, oder
ein über eine C₁-C₄-Alkylenbrücke verknüpfter 5 oder 6 gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein-, zwei- oder dreifach durch einen oder mehrere der Reste C₁-C₄-Alkyl, Phenyl oder Benzyl substituiert sein kann;
R⁴ Wasserstoff, C₁-C₆-Alkyl, welches ein- oder zweifach durch eine oder mehrere der Gruppen COOH, COO-C₁-C₄-Alkyl oder C₃-C₆-Cycloalkyl substituiert sein kann, oder
Phenyl-C₁-C₄-alkyl, wobei die C₁-C₄-Alkylenbrücke gegebenfalls durch Phenyl substituiert sein kann und wobei der Phenylring gegebenenfalls ein-, zwei-, oder dreifach durch einen oder mehrere der Reste C₁-C₄-Alkyl, C₁-C₄-Alkoxy, CF₃, Fluor, Chlor, Brom, COOH oder COO-C₁-C₄-Alkyl substituiert sein kann, oder
C₃-C₈-Cycloalkyl, Naphthyl oder Phenyl, welches gegebenenfalls ein-, zwei-, oder dreifach durch einen oder mehrere der Reste C₁-C₄-Alkyl, C₁-C₄-Alkoxy, Phenyloxy, Benzyloxy, CF₃, Fluor, Chlor, Brom, COOH oder COO-C₁-C₄-Alkyl substituiert sein kann, oder
ein über eine C₁-C₄-Alkylenbrücke verknüpfter 5-,6- oder 7-gliedriger, gesättigter oder ungesättigter Heterocyclus, der ein Heteroatom ausgewählt aus der Gruppe Sauerstoff, Stickstoff oder Schwefel enthält und der gegebenenfalls ein-, zwei- oder dreifach durch einen oder mehrere der Reste C₁-C₄-Alkyl,
Phenyl oder Benzyl substituiert sein kann oder an den über zwei benachbarte Kohlenstoffatome gegebenenfalls ein Benzolring ankondensiert sein kann, oder
R³ und R⁴ bilden gemeinsam mit dem Stickstoffatom einen 5-, 6- oder 7-gliedrigen, gesättigten oder ungesättigten Heterocyclus, der ein oder zwei weitere Heteroatome ausgewählt aus der Gruppe Sauerstoff, Stickstoff oder Schwefel enthält und der gegebenenfalls durch einen oder mehrere der Reste C₁-C₄-Alkyl, Benzyl, das gegebenenfalls durch C₁-C₄-Alkyl-substituiert ist, C₅-C₆-Cycloalkyl, Pyridyl oder Phenyl, das gegebenenfalls einen Rest ausgewählt aus der Gruppe C₁-C₄-Alkyl, C₁-C₄-Alkoxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

3. Verbindungen der allgemeinen Formel (I) gemäß Anspruch 2, worin
R¹ unsubstituiertes C₁-C₁₀-Alkyl, oder
ein- oder zweifach durch C₁-C₄-Alkoxy, Phenoxy-, C₁-C₄-Alkoxy-phenoxy, Hydroxyphenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiertes C₁-C₄-Alkyl, oder
Phenyl-C₁-C₃-alkyl, wobei der Phenylring gegebenenfalls ein- oder zweifach durch C₁-C₄-Alkyl, CF₃, Fluor, Chlor, Brom, COOH oder COO-C₁-C₄-Alkyl substituiert sein kann, oder
ein über eine C₁-C₃-Alkylenbrücke verknüpfter 5-, 6- oder 7- gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls ein- oder zweifach durch einen oder mehrere der Reste Methyl, Ethyl, Propyl, Phenyl, Methylphenyl- oder Benzyl substituiert sein kann oder an den über zwei benachbarte Kohlenstoffatome gegebenenfalls ein Benzolring ankondensiert sein kann;
R² -C(=NH)NH₂ oder -CH₂-NH₂;
R3 C₁-C₆-Alkyl, welches ein- oder zweifach durch eine oder mehrere der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Benzyl, wobei der Phenylring direkt oder über eine C₁-C₄-Alkylenbrücke durch eine der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -NH-CO-(C₁-C₄-Alkyl), -C(=NH)NH₂ oder -NH-C(=NH)NH₂ substituiert ist, oder
Phenyl-C₂-C₄-alkyl, wobei die C₂-C₄-Alkylenbrücke gegebenfalls durch Phenyl, COOH oder COO-C₁-C₄-Alkyl substituiert sein kann und
wobei der Phenylring direkt oder über eine C₁-C₄-Alkylenbrücke durch eine der Gruppen -NH₂, -NH(C₁-C₄-Alkyl), -N(C₁-C₄-Alkyl)₂, -NHPhenyl, -N(Phenyl)₂, -NHBenzyl, -N(Benzyl)₂, -C(=NH)NH₂ oder -NH-C(=NH)NH₂ substituiert sein kann, oder
ein über eine C₁-C₄-Alkylenbrücke verknüpfter 5-, 6- oder 7-gliedriger, gesättigter oder ungesättigter Heterocyclus, der ein oder zwei Heteroatome ausgewählt aus der Gruppe Sauerstoff oder Stickstoff enthalten kann und der gegebenenfalls ein- oder zweifach durch einen oder mehrere der Reste Methyl, Ethyl, Propyl, Phenyl oder Benzyl substituiert sein kann;
R⁴ Wasserstoff, C₁-C₄-Alkyl, welches durch eine der Gruppen COOH, COO-C₁-C₄-Alkyl oder C₃-C₆-Cycloalkyl substituiert sein kann, oder
Phenyl-C₁-C₄-alkyl, wobei die C₁-C₄-Alkylenbrücke gegebenfalls durch Phenyl substituiert sein kann und wobei der Phenylring gegebenenfalls ein- oder zweifach durch einen oder mehrere der Reste C₁-C₄-Alkyl, C₁-C₄-Alkoxy, CF₃, Fluor, Chlor, Brom, COOH oder COO-C₁-C₄-Alkyl substituiert sein kann, oder
C₃-C₈-Cycloalkyl, Naphthyl oder Phenyl, welches gegebenenfalls ein- oder zweifach durch einen oder mehrere der Reste C₁-C₄-Alkyl, C₁-C₄-Alkoxy, Phenyloxy, Benzyloxy, CF₃, Fluor, Chlor, Brom, COOH oder COO-C₁-C₄-Alkyl substituiert sein kann, oder
ein über eine C₁-C₄-Alkylenbrücke verknüpfter 5-, 6- oder 7-gliedriger, gesättigter oder ungesättigter Heterocyclus, der ein Heteroatom ausgewählt aus der Gruppe Sauerstoff, Stickstoff oder Schwefel enthält und der gegebenenfalls ein- oder zweifach durch einen oder mehrere der Reste Methyl, Ethyl, Propyl, Phenyl oder Benzyl substituiert sein kann oder an den über zwei benachbarte Kohlenstoffatome gegebenenfalls ein Benzolring ankondensiert sein kann, oder
R³ und R⁴ bilden gemeinsam mit dem Stickstoffatom einen 6- oder 7-gliedrigen, gesättigten oder ungesättigten Heterocyclus, der ein oder zwei weitere Heteroatome ausgewählt aus der Gruppe Sauerstoff oder Stickstoff enthält und der gegebenenfalls durch einen oder mehrere der Reste Methyl, Ethyl, Propyl, Benzyl, Cyclopentyl, Cyclohexyl, Pyridyl oder Phenyl, das gegebenenfalls einen Rest ausgewählt aus der Gruppe Methyl, Methoxy, Ethoxy, Propyloxy oder Hydroxy trägt, substituiert sein kann, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

4. Verbindungen der allgemeinen Formel (I) gemäß Anspruch 2 oder 3, worin
R¹ unsubstituiertes C₁-C₁₀-Alkyl, oder durch C₁-C₄-Alkoxy, Phenoxy, C₁-C₄-Alkoxy-phenoxy, Hydroxyphenoxy, C₃-C₆-Cycloalkyl, -NH₂, -NH(C₁-C₄-Alkyl), -N(C₁-C₄-Alkyl)₂, -NH-CO-(C₁-C₄-Alkyl), -CO-NH₂, -CO-NH-(C₁-C₄-Alkyl) oder -NH-CO-Benzyl substituiertes C₁-C₄-Alkyl, oder
Phenyl-C₁-C₃-alkyl, wobei der Phenylring gegebenenfalls ein- oder zweifach durch C₁-C₄-Alkyl, CF₃, Fluor, Chlor, Brom, COOH oder COO-C₁-C₄-Alkyl substituiert sein kann, oder
ein über eine C₁-C₃-Alkylenbrücke verknüpfter, gegebenenfalls ein- oder zweifach durch einen oder mehrere der Reste Methyl, Ethyl, Propyl, Phenyl, Methylphenyl- oder Benzyl substituierter Heterocyclus, ausgewählt aus der Gruppe Pyrrol, Pyrrolin, Pyrrolidin, Pyridin, Piperidin, Pyrimidin, Piperazin, Morpholin, Thiomorpholin, Imidazol, Imidazolin, Imidazolidin, Pyrazol, Pyrazolin,
Pyrazolidin, Triazol, Furan, Tetrahydrofuran, α-Pyran, γ-Pyran, Dioxolan, Tetrahydropyran, Dioxan, Thiophen, Dihydrothiophen, Thiolan, Dithiolan, Oxazol, Isoxazol, Thiazol, Isothiazol, Oxadiazol, Benzodioxol, Benzimidazol, Benzthiophen, Benzfuran oder Indol;
R² -C(=NH)NH₂ oder -CH₂-NH₂;
R³ C₁-C₃-Alkyl, das durch -NH₂, -NH(C₁-C₃-Alkyl), -N(C₁-C₃-Alkyl)₂, -NHPhenyl, -N(Phenyl)₂, -NHBenzyl, -N(Benzyl)₂, -NH-CO-(C₁-C₃-Alkyl), -C(=NH)NH₂ oder -NH-C(=NH)NH₂ substituiert ist, oder
Benzyl, wobei der Phenylring direkt oder über eine Methylenbrücke durch eine der Gruppen -NH₂, -NH(C₁-C₃-Alkyl). -N(C₁-C₄-Alkyl)₂, -NHPhenyl, -N(Phenyl)₂, -NHBenzyl, -N(Benzyl)₂, -NH-CO-(C₁-C₃-Alkyl), -C(=NH)NH₂ oder -NH-C(=NH)NH₂ substituiert ist, oder
Phenyl-C₂-C₃-alkyl, wobei die C₂-C₃-Alkylenbrücke gegebenfalls durch Phenyl, COOH oder COO-C₁-C₃-Alkyl substituiert sein kann und
wobei der Phenylring direkt oder über eine Methylenbrücke durch eine der Gruppen -NH₂, -NH(C₁-C₃-Alkyl), -N(C₁-C₃-Alkyl)₂, -NHPhenyl, -N(Phenyl)₂, -NHBenzyl, -N(Benzyl)₂, -C(=NH)NH₂ oder -NH-C(=NH)NH₂ substituiert sein kann, oder
ein über eine C₁-C₃-Alkylenbrücke verknüpfter, gegebenenfalls ein- oder zweifach durch einen oder mehrere der Reste Methyl, Ethyl, Propyl, Phenyl oder Benzyl substituierter Heterocyclus, ausgewählt aus der Gruppe Pyrrol, Pyrrolin, Pyrrolidin, Pyridin, Piperidin, Pyrimidin, Piperazin, Morpholin, Diazepan, Imidazol, Imidazolin, Imidazolidin, Pyrazol, Pyrazolin, Pyrazolidin, Furan, Tetrahydrofuran, α-Pyran, γ-Pyran, Dioxolan, Tetrahydropyran, Dioxan, Oxazol oder Isoxazol;
R⁴ Wasserstoff, C₁-C₄-Alkyl, welches durch eine der Gruppen COOH, COO-C₁-C₃-Alkyl oder C₃-C₆-Cycloalkyl substituiert sein kann, oder
Phenyl-C₁-C₃-alkyl, wobei die C₁-C₃-Alkylenbrücke gegebenfalls durch Phenyl substituiert sein kann und wobei der Phenylring gegebenenfalls durch C₁-C₃-Alkyl, C₁-C₃-Alkoxy, CF₃, Fluor, Chlor, Brom, COOH oder
COO-C₁-C₃-Alkyl substituiert sein kann, oder
C₃-C₈-Cycloalkyl, Naphthyl oder Phenyl, welches gegebenenfalls ein- oder zweifach durch einen oder mehrere der Reste C₁-C₃-Alkyl, C₁-C₃-Alkoxy, Phenyloxy, Benzyloxy, CF₃, Fluor, Chlor, Brom, COOH oder COO-C₁-C₃-Alkyl substituiert sein kann, oder
ein über eine C₁-C₃-Alkylenbrücke verknüpfter, gegebenenfalls ein- oder zweifach durch einen oder mehrere der Reste Methyl, Ethyl, Propyl, Phenyl, Methylphenyl- oder Benzyl substituierter Heterocyclus, ausgewählt aus der Gruppe Pyrrol, Pyrrolin, Pyrrolidin, Pyridin, Piperidin, Pyrimidin, Piperazin, Morpholin, Thiomorpholin, Imidazol, Imidazolin, Imidazolidin, Pyrazol, Pyrazolin, Pyrazolidin, Triazol, Furan, Tetrahydrofuran, α-Pyran, γ-Pyran, Dioxolan, Tetrahydropyran, Dioxan, Thiophen, Dihydrothiophen, Thiolan, Dithiolan, Oxazol, Isoxazol, Thiazol, Isothiazol, Oxadiazol, Benzodioxol, Benzimidazol, Benzthiophen, Benzofuran oder Indol;
oder
R³ und R⁴ bilden gemeinsam mit dem Stickstoffatom einen 6- oder 7-gliedrigen, gesättigten Heterocyclus, der ein oder zwei weitere Stickstoff-Heteroatome enthält und der gegebenenfalls durch einen oder mehrere der Reste Methyl, Ethyl, Propyl, Benzyl, Cyclopentyl, Cyclohexyl, Pyridyl oder Phenyl, das gegebenenfalls einen Rest ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

5. Verbindungen der allgemeinen Formel (I) gemäß einem der Ansprüche 2-4, worin
R¹ Methyl, Ethyl, Propyl, Butyl, Pentyl, Hexyl, Heptyl, Octyl, Nonyl oder Decyl, oder
ein Methyl-, Ethyl- oder Propyl-Rest, der durch Methoxy, Ethoxy, Cyclopropyl, Cyclopentyl, Cyclohexyl, Phenyl, Methoxyphenoxy, -NH₂, -NH(C₁-C₄-Alkyl), -N(C₁-C₄-Alkyl)₂, -NH-CO-Methyl, -CO-NH₂, -CO-NH-Methyl oder -NH-CO-Benzyl substituiert ist, oder
Benzyl, das ein- oder zweifach durch Methyl, Ethyl, Propyl, CF₃, Fluor, Chlor, Brom, COOH, COOMe oder COOEt substituiert ist, oder
Phenylethyl, daß ein- oder zweifach durch Methyl, Ethyl, Propyl, CF₃, Fluor, Chlor, Brom, COOH, COOMe oder COOEt substituiert ist, oder
ein über eine Methylen-, Ethylen oder Propylenbrücke verknüpfter, gegebenenfalls ein- oder zweifach durch einen oder mehrere der Reste Methyl, Ethyl, Propyl, Phenyl, Methylphenyl- oder Benzyl substituierter Heterocyclus, ausgewählt aus der Gruppe Pyrrol, Pyrrolidin, Pyridin, Piperidin, Piperazin, Morpholin, Furan, Tetrahydrofuran, Thiophen, Benzodioxol oder Benzimidazol;
R2 -C(=NH)NH₂ oder -CH₂-NH₂;
R3 ein Methyl-, Ethyl- oder Propyl-Rest, der durch -NH₂, -NH(C₁-C₃-Alkyl), -N(C₁-C₃-Alkyl)₂, -NHPhenyl, -N(Phenyl)₂, -NHBenzyl, -N(Benzyl)₂, -NH-CO-(C₁-C₃-Alkyl), -NH-CO-Benzyl (?) oder -C(=NH)NH₂ substituiert ist, oder
Benzyl, das direkt oder über eine Methylenbrücke durch eine der Gruppen -NH₂, -NH(C₁-C₃-Alkyl), -N(C₁-C₄-Alkyl)₂, -NHPhenyl, -N(Phenyl)₂, -NHBenzyl, -N(Benzyl)₂, -NH-CO-(C₁-C₃-Alkyl) oder -C(=NH)NH₂ substituiert ist, oder
Phenyl-C₂-C₃-alkyl, wobei die C₂-C₃-Alkylenbrücke gegebenfalls durch Phenyl, COOH oder COO-C₁-C₃-Alkyl substituiert sein kann und wobei der Phenylring direkt oder über eine Methylenbrücke durch eine der Gruppen -NH₂, -NH(C₁-C₃-Alkyl), -N(C₁-C₃-Alkyl)₂, -NHPhenyl, -N(Phenyl)₂, -NHBenzyl, -N(Benzyl)₂ oder -C(=NH)NH₂ substituiert sein kann, oder
ein über eine Methylen-, Ethylen oder Propylenbrücke verknüpfter, gegebenenfalls ein- oder zweifach durch Methyl, Ethyl, Propyl, Phenyl oder Benzyl substituierter Heterocyclus, ausgewählt aus der Gruppe Pyrrol, Pyrrolidin, Pyridin, Piperidin, Piperazin, Morpholin, Diazepan, Furan, Tetrahydrofuran, Thiophen, Benzodioxol oder Benzimidazol;
R⁴ Wasserstoff oder ein Methyl-, Ethyl-, Propyl- oder Butyl-Rest, der durch eine der Gruppen COOH, COOMe, COOEt, Cyclopropyl, Cyclopentyl oder Cyclohexyl substituiert sein kann, oder
Benzyl, das gegebenfalls durch Methyl, Ethyl, Propyl, Methoxy, Ethoxy, CF₃, Fluor, Chlor, Brom, COOH, COOMe oder COOEt substituiert sein kann, oder Phenylethyl, Phenylpropyl, Diphenylpropyl;
Cyclopropyl, Cyclopentyl, Cyclohexyl, Cyclooctyl, Naphthyl oder Phenyl, das gegebenenfalls durch Methyl, Ethyl, Propyl, Methoxy, Ethoxy, Phenyloxy, Benzyloxy, CF₃, Fluor, Chlor, Brom, COOH, COOMe oder COOEt substituiert sein kann, oder
ein über eine Methylen-, Ethylen oder Propylenbrücke verknüpfter, gegebenenfalls ein- oder zweifach durch Methyl, Ethyl, Propyl, Phenyl oder Benzyl substituierter Heterocyclus, ausgewählt aus der Gruppe Pyrrol, Pyrrolidin, Pyridin, Piperidin, Piperazin, Morpholin, Furan, Tetrahydrofuran, Thiophen, Chinolin, Isochinolin, Benzodioxol oder Benzimidazol; oder
R³ und R⁴ bilden gemeinsam mit dem Stickstoffatom einen Piperazin- oder Diazepan-Ring, der gegebenenfalls durch einen der Reste Methyl, Ethyl, Propyl, Cyclopentyl, Cyclohexyl, Pyridyl, Benzyl oder Phenyl, das gegebenenfalls einen Rest ausgewählt aus der Gruppe Methyl, Methoxy Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

6. Verbindungen der allgemeinen Formel (I) gemäß einem der Ansprüche 2-5, worin
R¹ Methyl, Ethyl, Propyl, Pentyl oder n-Decyl,
oder
ein Methyl-, Ethyl- oder Propyl-Rest, der durch Methoxy, Ethoxy, Cyclopropyl, Cyclopentyl, Cyclohexyl, Phenyl oder Methoxyphenoxy substituiert ist, oder Benzyl, das ein- oder zweifach durch Methyl, CF₃, COOH, COOMe oder COOEt substituiert ist, oder
ein über eine Methylenbrücke verknüpftes Tetrahydrofuran;
R2 -C(=NH)NH₂ oder -CH₂-NH₂;
R³ ein Ethyl- oder Propyl-Rest, der durch -NH₂, -NHMe, -NMe₂, -NHEt, -NEt₂, -NHPhenyl, -N(Phenyl)₂, -NHBenzyl, -N(Benzyl)₂ oder -C(=NH)NH₂ substituiert ist, oder
Benzyl, das durch eine der Gruppen -NH₂, -CH₂-NH₂, -NMe₂, -NHMe, -NEt₂, -NHEt, -NH-CO-Me, -CH₂-NH-CO-Me oder -C(=NH)NH₂ substituiert ist, oder
Phenylethyl, wobei die Ethylenbrücke gegebenfalls durch COOH, COOMe oder COOEt substituiert sein kann und wobei der Phenylring durch eine der Gruppen -NH₂, -CH₂-NH₂, -NMe₂, -NHMe, -NEt₂, -NHEt, -NH-CO-Me, -CH₂-NH-CO-Me oder -C(=NH)NH₂ substituiert ist, oder
Phenylpropyl, Diphenylpropyl oder Pyridylmethyl;
R⁴ Wasserstoff oder ein Methyl-, Ethyl-, Propyl- oder Butyl-Rest, der durch eine der Gruppen COOH, COOMe, COOEt, Cyclopropyl, Cyclopentyl oder Cyclohexyl substituiert sein kann, oder
Benzyl, das gegebenfalls durch Methyl, Ethyl, Propyl, Methoxy, Ethoxy, CF₃, Fluor, Chlor, Brom, COOH, COOMe oder COOEt substituiert sein kann, oder Phenyl-ethyl, Phenylpropyl, Diphenylpropyl, oder
Cyclopentyl, Cyclohexyl, Cyclooctyl, Naphthyl oder Phenyl, das gegebenenfalls durch Methyl, Ethyl, Methoxy, Ethoxy, Phenyloxy oder CF₃ substituiert sein kann, oder
ein über eine Methylenbrücke verknüpftes Pyridin oder Chinolin,
oder
R³ und R⁴ bilden gemeinsam mit dem Stickstoffatom einen Piperazin- oder Diazepan-Ring, der gegebenenfalls durch einen der Reste Cyclopentyl, Cyclohexyl, Pyridyl, Benzyl oder Phenyl, das gegebenenfalls einen der Reste ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

7. Verbindungen der allgemeinen Formel (I) gemäß einem der Ansprüche 2-6, worin
R¹ Methyl, Ethyl, Propyl, Pentyl, Phenylethyl, Phenylpropyl, Cyclopropylmethyl, Tetrahydrofuranylmethyl oder Benzyl, das ein- oder zweifach durch CF₃, COOH, COOMe oder COOEt substituiert ist;
R2 -C(=NH)NH₂ oder -CH₂-NH₂;
R3 ein Ethyl- oder Propyl-Rest, der durch -NH₂, -NHMe, -NMe₂, -NHEt, -NEt₂, -NHPhenyl, -N(Phenyl)₂, -NHBenzyl, -N(Benzyl)₂ oder -C(=NH)NH₂ substituiert ist, oder
Benzyl, das durch eine der Gruppen -NH₂, -CH₂-NH₂, -NMe₂, -NHMe, -NEt₂, -NHEt, -NH-CO-Me, -CH₂-NH-CO-Me oder -C(=NH)NH₂ substituiert ist, oder
Phenylethyl, wobei die Ethylenbrücke durch COOH, COOMe oder COOEt substituiert ist und wobei der Phenylring eine der Gruppen -NH₂, -CH₂-NH₂, -NMe₂, -NHMe, -NEt₂, -NHEt, -NH-CO-Me, -CH₂-NH-CO-Me oder -C(=NH)NH₂ trägt, oder
Phenylpropyl, Diphenylpropyl oder Pyridylmethyl;
R⁴ Wasserstoff oder ein Methyl-, Ethyl-, Propyl- oder Butyl-Rest, der durch eine der Gruppen COOH, COOMe, COOEt oder Cyclohexyl substituiert sein kann, oder Phenyl, das gegebenenfalls durch Methyl, Ethyl, Methoxy, Ethoxy, Phenyloxy oder CF₃ substituiert sein kann, oder
Benzyl, Phenylethyl, Phenylpropyl, Diphenylpropyl, Cyclohexyl, Cyclooctyl oder Naphthyl, oder
ein über eine Methylenbrücke verknüpftes Pyridin oder Chinolin,
oder
R³ und R⁴ bilden gemeinsam mit dem Stickstoffatom einen Piperazin- oder Diazepan-Ring, der gegebenenfalls durch einen der Reste Cyclopentyl, Cyclohexyl, Pyridyl, Benzyl oder Phenyl, das gegebenenfalls einen Rest ausgewählt aus der Gruppe Methyl, Methoxy, Ethoxy, Propyloxy oder Hydroxy trägt, substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

8. Verbindungen der allgemeinen Formel (I) gemäß einem der Ansprüche 2-7, worin
R¹ Methyl;
R² -C(=NH)NH₂ oder -CH₂-NH₂;
R³ Ethyl, das durch -NH₂, -NMe₂, -NHPhenyl, -NHBenzyl, -N(Benzyl)₂, Pyrrolidin, Piperidin, Diazepan oder -C(=NH)NH₂ substituiert ist, Benzyl, das durch eine der Gruppen -CH₂-NH₂, -NMe₂ oder -C(=NH)NH₂ substituiert ist, Phenylethyl, wobei die Ethylenbrücke durch COOH, COOMe oder COOEt substituiert ist und wobei der Phenylring eine der Gruppen -CH₂-NH-CO-Me oder -C(=NH)NH₂ trägt, Diphenylpropyl oder Pyridylmethyl;
R⁴ Wasserstoff oder ein Methyl- oder Ethyl-Rest, der gegebenfalls durch eine der Gruppen COOH oder COOEt substituiert sein kann, Propyl, Butyl oder Phenyl, das gegebenenfalls durch Methyl, Ethyl, Methoxy, Ethoxy, Phenyloxy oder CF₃ substituiert sein kann, Benzyl, Phenylethyl, Phenylpropyl, Diphenylpropyl, Cyclohexyl, Cyclooctyl, Naphthyl, Pyridylmethyl oder Chinolinylmethyl
oder
R³ und R⁴ bilden gemeinsam mit dem Stickstoffatom einen Piperazin- oder Diazepan-Ring, der durch einen der Reste Benzyl, Cyclopentyl, Cyclohexyl oder Phenyl, das gegebenenfalls einen Rest ausgewählt aus der Gruppe Methyl, Ethoxy, Propyloxy oder Hydroxy trägt, substituiert ist, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

9. Verbindungen der allgemeinen Formel (I) gemäß einem der Ansprüche 2-8, worin
R¹ Methyl;
R² -C(=NH)NH₂;
R³ Ethyl, das durch -NH₂, -NHPhenyl, -NHBenzyl, -N(Benzyl)₂, Pyrrolidin, Piperidin, Diazepan oder -C(=NH)NH₂ substituiert ist, Benzyl, das durch -C(=NH)NH₂ substituiert ist oder Diphenylpropyl;
R4 Wasserstoff, Methyl, Propyl, Butyl, Benzyl oder Phenyl, das gegebenenfalls durch Ethyl oder Phenyloxy substituiert sein kann, Phenylethyl, Cyclohexyl oder Cyclooctyl, oder
R³ und R⁴ bilden gemeinsam mit dem Stickstoffatom einen Piperazin-Ring, der durch einen Rest ausgewählt aus der Gruppe Cyclopentyl, Cyclohexyl, Phenyl, Methylphenyl, Ethoxyphenyl oder Propoxyphenyl substituiert ist, oder einen Diazepan-Ring, der durch Methylphenyl substituiert ist,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

10. Verbindungen der allgemeinen Formel (I) gemäß einem der Ansprüche 2-9, worin
R¹ Methyl;
R² -C(=NH)NH₂;
R³ Ethyl, das durch -NH₂, -NHBenzyl, -N(Benzyl)₂, Pyrrolidin, Piperidin, Diazepan oder -C(=NH)NH₂ substituiert ist, Benzyl, das durch -C(=NH)NH₂ substituiert ist oder Diphenylpropyl;
R⁴ Wasserstoff, Methyl, Butyl oder Phenyl, das gegebenenfalls durch Ethyl oder Phenyloxy substituiert sein kann, Phenylethyl, Cyclohexyl oder Cyclooctyl, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

11. Prodrugs der allgemeinen Formel (II) 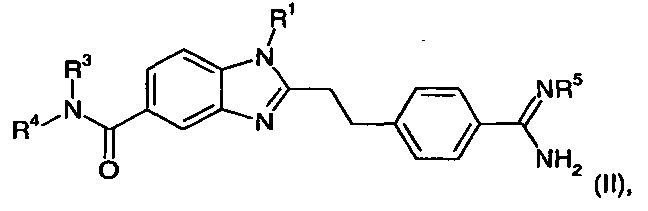 worin
R¹ und R⁴ die in den Ansprüchen 2-10 genannten Bedeutungen aufweisen können;
R³ die in den Ansprüchen 2-10 genannten Bedeutungen aufweisen kann oder C₁-C₄-Alkyl bedeutet, welches durch einen Rest ausgewählt aus der Gruppe -C(=NOH)NH₂, -C(=NCOO-C₁-C₄-alkyl)NH₂ oder -C(=NCOO-C₁-C₄-alkyl-Phenyl)NH₂ substituiert ist;
R5 Hydroxy, -COO-C₁-C₈-Alkyl oder -COO-C₁-C₄-Alkyl-Phenyl,
wobei in der vorstehend genannten Gruppe der Phenylring jeweils durch C₁-C₄-Alkyl oder C₁-C₄-Alkoxy substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

12. Prodrugs der allgemeinen Formel (II) gemäß Anspruch 11, worin
R¹ und R⁴ die in den Ansprüchen 2-10 genannten Bedeutungen aufweisen können;
R³ die in den Ansprüchen 2-10 genannten Bedeutungen aufweisen kann oder C₁-C₄-Alkyl bedeutet, welches durch einen Rest ausgewählt aus der Gruppe -C(=NOH)NH₂, -C(=NCOOMethyl)NH₂, -C(=NCOOEthyl)NH₂, -C(=NCOOPropylyl)NH₂ oder -C(=NCOO-Benzyl)NH₂ substituiert ist;
R⁵ Hydroxy, Methoxycarbonyl, Ethoxycarbonyl, Propyloxycarbonyl, Butyloxycarbonyl, Pentyloxycarbonyl, Hexyloxycarbonyl, Benzyloxycarbonyl, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

13. Zwischenprodukte der allgemeinen Formel (III) 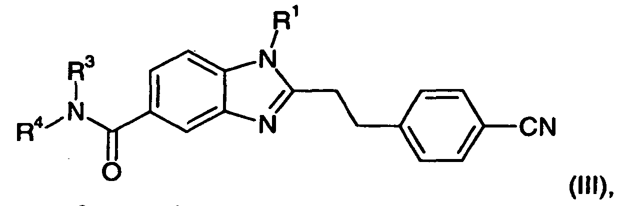 in der die Reste R¹, R³ und R⁴ die in den Ansprüchen 2-12 genannten Bedeutungen aufweisen können.

14. Verwendung von Verbindungen der allgemeinen Formel (I) gemäß einem der Ansprüche 2-10 zur Herstellung eines Arzneimittels zur Vorbeugung und/oder Behandlung von Krankheiten, in denen Tryptase-Inhibitoren einen therapeutischen Nutzen entfalten können.

15. Verwendung von Prodrugs der allgemeinen Formel (II) gemäß einem der Ansprüche 11 oder 12 zur Herstellung eines Arzneimittels zur Vorbeugung und/oder Behandlung von Krankheiten, in denen Tryptase-Inhibitoren einen therapeutischen Nutzen entfalten können.

16. Pharmazeutische Zusammensetzung **gekennzeichnet durch** einen Gehalt einer oder mehrer Verbindungen gemäß einem der Ansprüche 2-12.

17. Verfahren zur Herstellung von Verbindungen der allgemeinen Formel (I) 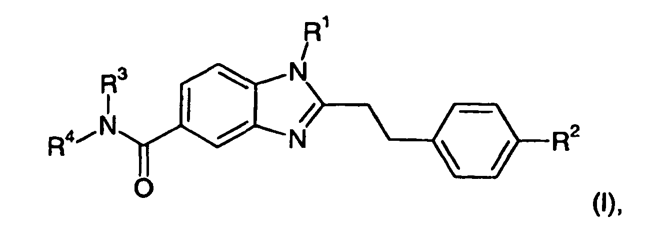 worin die Reste R¹, R², R³ und R⁴ die in den Ansprüchen 2-10 genannten Bedeutungen haben können, **dadurch gekennzeichnet, daß** eine Verbindung der allgemeinen Formel (III) 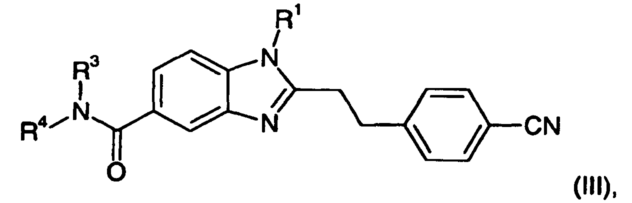 in der die Reste R¹, R³ und R⁴ die in den Ansprüchen 2-10 genannten Bedeutungen haben können, direkt in die Verbindungen der allgemeinen Formel (I) überführt wird.

18. Verfahren zur Herstellung von Verbindungen der allgemeinen Formel (I) 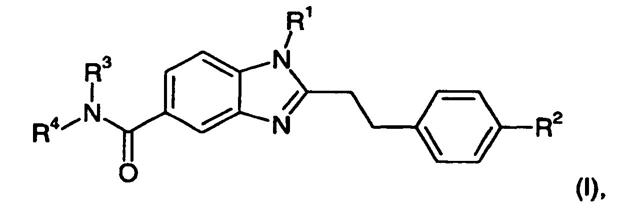 worin die Reste R¹, R², R³ und R⁴ die in den Ansprüchen 2-10 genannten Bedeutungen haben können, **dadurch gekennzeichnet, daß** eine Verbindung der allgemeinen Formel (II) 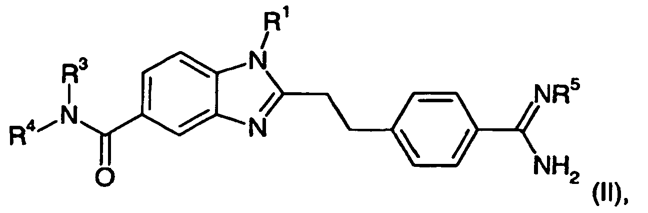 worin die Reste R¹ und R⁴ die in den Ansprüchen 2-10 genannten Bedeutungen aufweisen können und die Reste R³ und R⁵ die in den Ansprüchen 11 und 12 genannten Bedeutungen aufweisen können, in die Verbindungen der allgemeinen Formel (I) überführt wird.

19. Verfahren zur Herstellung von Verbindungen der allgemeinen Formel (II) 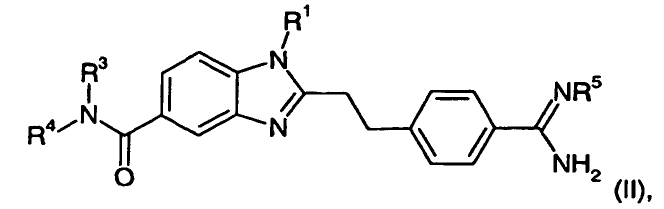 worin die Reste R¹ und R⁴ die in den Ansprüchen 2-10 genannten Bedeutungen aufweisen können und die Reste R³ und R⁵ die in den Ansprüchen 11 und 12 genannten Bedeutungen aufweisen können, **dadurch gekennzeichnet, daß** eine Verbindung der allgemeinen Formel (III) 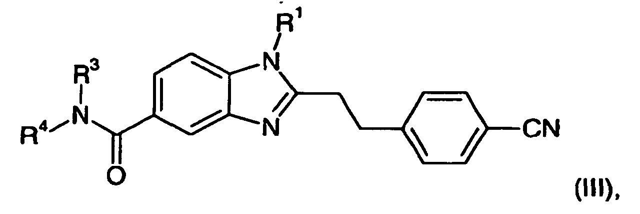 in der die Reste R¹, R³ und R⁴ die in den Ansprüchen 2-10 genannten Bedeutungen haben können, in eine Verbindung der allgemeinen Formel (II) überführt wird.

20. Verfahren zur Herstellung von Verbindungen der allgemeinen Formel (I) 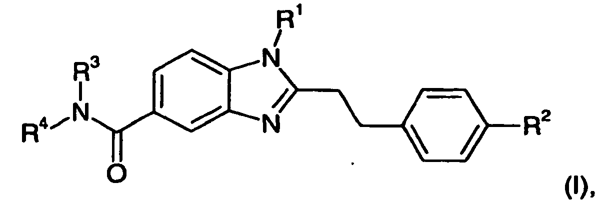 worin die Reste R¹, R², R³ und R⁴ die in den Ansprüchen 2-10 genannten Bedeutungen haben können, **dadurch gekennzeichnet, daß** die Synthese durch Umsetzung an einem polymeren Träger (Harz) erfolgt und die folgenden Syntheseschritte umfaßt:
- Kupplung eines Diamins an den polymeren Träger,
- Reduktive Aminierung unter Umsetzung mit einem Aldehyd R⁴-CHO,
- Acylierung mit Fluor- oder Chlornitrobenzoylchlorid,
- Nucleophile Substitution mit einem Amin R¹-NH₂,
- Reduktion,
- Oxidative Cyclisierung und
- Abspaltung der Verbindungen der allgemeinen Formel (I) vom polymeren Träger.

## Claims

1. Use of compounds of general formula (I) 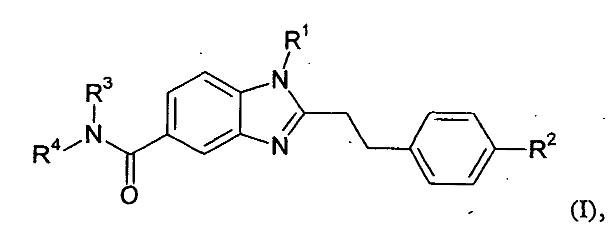 for the preparation of a pharmaceutical composition for the prevention and/or treatment of diseases in which tryptase inhibitors may have a therapeutic value, wherein
R¹ denotes C₁-C₁₀-alkyl, which may optionally be mono-, di- or tri-substituted by one or more of the groups C₁-C₄-alkoxy, phenoxy, hydroxyphenoxy, C₁-C₄-alkoxy-phenoxy, C₃-C₆-cycloalkyl, -NH₂, -NH(C₁-C₄-alkyl), -N(C₁-C₄-alkyl)₂, -NH-CO-(C₁-C₄-alkyl), -CO-NH₂, -CO-NH-(C₁-C₄-alkyl) or -NH-CO-benzyl, or
phenyl-C ₁-C₄-alkyl, wherein the phenyl ring may optionally be mono-, di- or tri-substituted by one or more of the groups C₁-C₄-alkyl, CF₃, fluorine, chlorine, bromine, COOH or COO-C₁-C₄-alkyl, or
a 5 or 6 membered, saturated or unsaturated heterocyclic group linked via a single bond or via a C₁-C₄-alkylene bridge, which may contain one, two or three heteroatoms selected from the group comprising oxygen, nitrogen or sulphur and which may optionally be mono-, di- or tri-substituted by one or more of the groups C₁-C₄-alkyl, phenyl optionally substituted by C₁-C₄-alkyl or benzyl optionally substituted by C₁-C₄-alkyl or to which a benzene ring may optionally be fused via two adjacent carbon atoms;
R² denotes -C(=NH)NH₂ or -CH₂-NH₂;
R³ and R⁴ which may be identical or different, denote hydrogen, C₁-C₆-alkyl, which may be mono- or disubstituted by one or more of the groups COOH, COO-C₁-C₄-alkyl, C₃-C₆-cycloalkyl, -NH₂, -NH(C₁-C₄-alkyl), -N(C₁-C₄-alkyl)₂, -NHphenyl, -N(phenyl)₂, -NHbenzyl, -N(benzyl)₂, -NH-CO-(C₁-C₄-alkyl), -C(=NH)NH₂ or -NH-C(=NH)NH₂, or
phenyl-C₁-C₄-alkyl, wherein the C₁-C₄-alkylene bridge may optionally be substituted by phenyl, COOH or COO-C₁-C₄-alkyl and wherein the phenyl ring may optionally be mono-, di- or tri-substituted, directly or via a C₁-C₄-alkylene bridge, by one or more of the groups C₁-C₄-alkyl, C₁-C₄-alkoxy, CF₃, fluorine, chlorine, bromine, COOH, COO-C₁-C₄-alkyl, -NH₂, -NH(C₁-C₄-alkyl), -N(C₁-C₄-alkyl)₂, -NHphenyl, -N(phenyl)₂, -NHbenzyl, -N(benzyl)₂, -NH-CO-(C₁-C₄-alkyl), -C(=NH)NH₂ or -NH-C(=NH)NH₂, or
a 5-, 6- or 7- membered, saturated or unsaturated heterocyclic group linked directly or via a C₁-C₄-alkylene bridge, which may contain one, two, three or four heteroatoms selected from the group comprising oxygen, nitrogen or sulphur and which may optionally be mono-, di- or tri-substituted by one or more of the groups C₁-C₄-alkyl, phenyl or benzyl or to which a benzene ring may optionally be fused via two adjacent carbon atoms, or
C₃-C₈-cycloalkyl, naphthyl or phenyl, which may optionally be mono-, di- or tri-substituted by one or more of the groups C₁-C₄-alkyl, C₁-C₄-alkoxy, phenyloxy, benzyloxy, CF₃, fluorine, chlorine, bromine, COOH or COO-C₁-C₄-alkyl, or
R³ and R⁴ together with the nitrogen atom form a 5-, 6- or 7-membered, saturated or unsaturated heterocyclic group, which may contain one or two further heteroatoms selected from the group comprising oxygen, nitrogen or sulphur and which may optionally be substituted by one or more of the groups C₁-C₄-alkyl, C₅-C₆-cycloalkyl, benzyl, which may optionally be substituted by C₁-C₄-alkyl, pyridyl or phenyl, optionally substituted by C₁-C₄-alkyl, C₁-C₄-alkoxy or hydroxy,
optionally in the form of their tautomers, their racemates, their enantiomers, their diastereomers and mixtures thereof and optionally the pharmacologically acceptable acid addition salts thereof.

2. Aminocarbonyl-substituted benzimidazole derivatives of general formula (I) 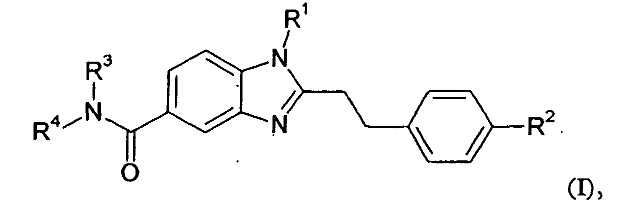 wherein
R¹ denotes C₁-C₁₀-alkyl, which may optionally be mono-, di- or tri-substituted by one or more of the groups C₁-C₄-alkoxy, phenoxy-, C₁-C₄-alkoxy-phenoxy, hydroxyphenoxy, C₃-C₆-cycloalkyl, -NH₂, -NH(C₁-C₄-alkyl), -N(C₁-C₄-alkyl)₂, -NH-CO-(C₁-C₄-alkyl), -CO-NH₂, -CO-NH-(C₁-C₄-alkyl) or -NH-CO-benzyl, or
phenyl-C ₁-C₄-alkyl, wherein the phenyl ring may optionally be mono-, di- or tri-substituted by one or more of the groups C₁-C₄-alkyl, CF₃, fluorine, chlorine, bromine, COOH or COO-C₁-C₄-alkyl, or
a 5 or 6 membered, saturated or unsaturated heterocyclic group linked via a single bond or via a C₁-C₄-alkylene bridge, which may contain one, two or three heteroatoms selected from the group comprising oxygen, nitrogen or sulphur and may optionally be mono-, di- or tri-substituted by one or more of the groups C₁-C₄-alkyl, phenyl optionally substituted by C₁-C₄-alkyl or benzyl optionally substituted by C₁-C₄-alkyl, or to which a benzene ring may optionally be fused via two adjacent carbon atoms;
R² denotes -C(=NH)NH₂ or -CH₂-NH₂;
R³ denotes C₁-C₆-alkyl, which is mono- or disubstituted by one or more of the groups -NH₂, -NH(C₁-C₄-alkyl), -N(C₁-C₄-alkyl)₂, NHphenyl, -N(phenyl)₂, -NHbenzyl, -N(benzyl)₂, -NH-CO-(C₁-C₄-alkyl), -C(=NH)NH₂ or -NH-C(=NH)NH₂, or benzyl, wherein the phenyl ring is mono- or disubstituted, directly or via a C₁-C₄-alkylene bridge, by one or more of the groups -NH₂, -NH(C₁-C₄-alkyl), -N(C₁-C₄-alkyl)₂, -NHphenyl, -N(phenyl)₂, -NHbenzyl, -N(benzyl)₂, -NH-CO-(C₁-C₄-alkyl), -C(=NH)NH₂ or -NH-C(=NH)NH₂, or
phenyl-C₂-C₄-alkyl, wherein the C₂-C₄-alkylene bridge may optionally be substituted by phenyl, COOH or COO-C₁-C₄-alkyl and wherein the phenyl ring may optionally be mono- or di-substituted, directly or via a C₁-C₄-alkylene bridge, by one or more of the groups -NH₂, -NH(C₁-C₄-alkyl), -N(C₁-C₄-alkyl)₂, -NHphenyl, -N(phenyl)₂, -NHbenzyl, -N(benzyl)₂, -NH-CO-(C₁-C₄-alkyl), -C(=NH)NH₂ or -NH-C(=NH)NH₂, or
a 5 or 6 membered, saturated or unsaturated heterocyclic group linked via a C₁-C₄-alkylene bridge, which may contain one, two or three heteroatoms selected from the group comprising oxygen, nitrogen or sulphur and which may optionally be mono-, di- or tri-substituted by one or more of the groups C₁-C₄-alkyl, phenyl or benzyl;
R⁴ denotes hydrogen, C₁-C₆-alkyl, which may be mono- or disubstituted by one or more of the groups COOH, COO-C₁-C₄-alkyl or C₃-C₆-cycloalkyl, or
phenyl-C₁-C₄-alkyl, wherein the C₁-C₄-alkylene bridge may optionally be substituted by phenyl and wherein the phenyl ring may optionally be mono-, di- or tri-substituted by one or more of the groups C₁-C₄-alkyl, C₁-C₄-alkoxy, CF₃, fluorine, chlorine, bromine, COOH or COO-C₁-C₄-alkyl, or
C₃-C₈-cycloalkyl, naphthyl or phenyl, which may optionally be mono-, di- or tri-substituted by one or more of the groups C₁-C₄-alkyl, C₁-C₄-alkoxy, phenyloxy, benzyloxy, CF₃, fluorine, chlorine, bromine, COOH or COO-C₁-C₄-alkyl, or
a 5-,6- or 7-membered, saturated or unsaturated heterocyclic group linked via a C₁-C₄-alkylene bridge, which contains a heteroatom selected from the group comprising oxygen, nitrogen or sulphur and which may optionally be mono-, di- or tri-substituted by one or more of the groups C₁-C₄-alkyl, phenyl or benzyl or to which a benzene ring may optionally be fused via two adjacent carbon atoms, or
R³ and R⁴ together with the nitrogen atom form a 5-, 6- or 7-membered, saturated or unsaturated heterocyclic group, which contains one or two further heteroatoms selected from the group comprising oxygen, nitrogen or sulphur and may optionally be substituted by one or more of the groups C₁-C₄-alkyl, benzyl, which is optionally C₁-C₄-alkyl-substituted, C₅-C₆-cycloalkyl, pyridyl or phenyl, which optionally bears a group selected from the group comprising C₁-C₄-alkyl, C₁-C₄-alkoxy or hydroxy,
optionally in the form of their tautomers, their racemates, their enantiomers, their diastereomers and mixtures thereof and optionally the pharmacologically acceptable acid addition salts thereof.

3. Compounds of general formula (I) according to Claim 2, wherein
R¹ denotes unsubstituted C₁-C₁₀-alkyl, or
C₁-C₄-alkyl mono- or disubstituted by C₁-C₄-alkoxy, phenoxy, C₁-C₄-alkoxy-phenoxy, hydroxyphenoxy, C₃-C₆-cycloalkyl, -NH₂, -NH(C₁-C₄-alkyl), -N(C₁-C₄-alkyl)₂, -NH-CO-(C₁-C₄-alkyl), -CO-NH₂, -CO-NH-(C₁-C₄-alkyl) or -NH-CO-benzyl, or
phenyl-C₁-C₃-alkyl, wherein the phenyl ring may optionally be mono- or disubstituted by C₁-C₄-alkyl, CF₃, fluorine, chlorine, bromine, COOH or COO-C₁-C₄-alkyl, or
a 5-, 6- or 7- membered, saturated or unsaturated heterocyclic group linked via a C₁-C₃-alkylene bridge, which may contain one or two heteroatoms selected from the group comprising oxygen, nitrogen or sulphur and may optionally be mono- or disubstituted by one or more of the groups methyl, ethyl, propyl, phenyl, methylphenyl or benzyl or to which a benzene ring may optionally be fused via two adjacent carbon atoms;
R² denotes -C(=NH)NH₂ or -CH₂-NH₂;
R³ denotes C₁-C₆-alkyl, which is mono- or disubstituted by one or more of the groups -NH₂, -NH(C₁-C₄-alkyl), -N(C₁-C₄-alkyl)₂, -NHphenyl, -N(phenyl)₂, -NHbenzyl, -N(benzyl)₂, -NH-CO-(C₁-C₄-alkyl), -C(=NH)NH₂ or -NH-C(=NH)NH₂, or
benzyl, wherein the phenyl ring is substituted directly or via a C₁-C₄-alkylene bridge by one of the groups -NH₂, -NH(C₁-C₄-alkyl), -N(C₁-C₄-alkyl)₂, -NHphenyl, -N(phenyl)₂, -NHbenzyl, -N(benzyl)₂, -NH-CO-(C₁-C₄-alkyl), -C(=NH)NH₂ or -NH-C(=NH)NH₂, or
phenyl-C₂-C₄-alkyl, wherein the C₂-C₄-alkylene bridge may optionally be substituted by phenyl, COOH or COO-C₁-C₄-alkyl and wherein the phenyl ring may be substituted directly or via a C₁-C₄-alkylene bridge by one of the groups -NH₂, -NH(C₁-C₄-alkyl), -N(C₁-C₄-alkyl)₂, -NHphenyl, -N(phenyl)₂, -NHbenzyl, -N(benzyl)₂, -C(=NH)NH₂ or -NH-C(=NH)NH₂, or
a 5-, 6- or 7-membered, saturated or unsaturated heterocyclic group linked via a C₁-C₄-alkylene bridge, which may contain one or two heteroatoms selected from the group comprising oxygen or nitrogen and may optionally be mono- or disubstituted by one or more of the groups methyl, ethyl, propyl, phenyl or benzyl;
R⁴ denotes hydrogen, C₁-C₄-alkyl, which may be substituted by one of the groups COOH, COO-C₁-C₄-alkyl or C₃-C₆-cycloalkyl, or
phenyl-C₁-C₄-alkyl, wherein the C₁-C₄-alkylene bridge may optionally be substituted by phenyl and wherein the phenyl ring may optionally be mono- or disubstituted by one or more of the groups C₁-C₄-alkyl,
C₁-C₄-alkoxy, CF₃, fluorine, chlorine, bromine, COOH or COO-C₁-C₄-alkyl, or
C₃-C₈-cycloalkyl, naphthyl or phenyl, which may optionally be mono- or disubstituted by one or more of the groups C₁-C₄-alkyl, C₁-C₄-alkoxy, phenyloxy, benzyloxy, CF₃, fluorine, chlorine, bromine, COOH or COO-C₁-C₄-alkyl, or
a 5-, 6- or 7-membered, saturated or unsaturated heterocyclic group linked via a C₁-C₄-alkylene bridge, which contains a heteroatom selected from the group comprising oxygen, nitrogen or sulphur and which may optionally be mono- or disubstituted by one or more of the groups methyl, ethyl, propyl, phenyl or benzyl or to which a benzene ring may optionally be fused via two adjacent carbon atoms, or
R³ and R⁴ together with the nitrogen atom form a 6- or 7- membered, saturated or unsaturated heterocyclic group, which contains one or two further heteroatoms selected from the group comprising oxygen or nitrogen and which may optionally be substituted by one or more of the groups methyl, ethyl, propyl, benzyl, cyclopentyl, cyclohexyl, pyridyl or phenyl, which optionally carries a group selected from the group comprising methyl, methoxy, ethoxy, propyloxy or hydroxy, optionally in the form of their tautomers, their racemates, their enantiomers, their diastereomers and mixtures thereof and optionally the pharmacologically acceptable acid addition salts thereof.

4. Compounds of general formula (I) according to Claim 2 or 3, wherein
R¹ denotes unsubstituted C₁-C₁₀-alkyl, or C₁-C₄-alkyl substituted by C₁-C₄-alkoxy, phenoxy, C₁-C₄-alkoxy-phenoxy, hydroxyphenoxy, C₃-C₆-cycloalkyl, -NH₂, -NH(C₁-C₄-alkyl), -N(C₁-C₄-alkyl)₂, -NH-CO-(C₁-C₄-alkyl), -CO-NH₂,-CO-NH-(C₁-C₄-alkyl) or -NH-CO-benzyl, or
phenyl-C₁-C₃-alkyl, wherein the phenyl ring may optionally be mono- or disubstituted by C₁-C₄-alkyl, CF₃, fluorine, chlorine, bromine, COOH or COO-C₁-C₄-alkyl, or
a heterocyclic group linked via a C₁-C₃-alkylene bridge, optionally mono- or disubstituted by one or more of the groups methyl, ethyl, propyl, phenyl, methylphenyl or benzyl, selected from the group comprising pyrrole, pyrroline, pyrrolidine, pyridine, piperidine, pyrimidine, piperazine, morpholine, thiomorpholine, imidazole, imidazoline, imidazolidine, pyrazole, pyrazoline, pyrazolidine, triazole, furan, tetrahydrofuran, α-pyran, γ-pyran, dioxolan, tetrahydropyran, dioxane, thiophene, dihydrothiophene, thiolan, dithiolan, oxazole, isoxazole, thiazole, isothiazole, oxadiazole, benzodioxole, benzimidazole, benzothiophene, benzofuran or indole;
R² denotes -C(=NH)NH₂ or -CH₂-NH₂;
R³ denotes C₁-C₃-alkyl, which is substituted by -NH₂, -NH(C₁-C₃-alkyl), -N(C₁-C₃-alkyl)₂, -NHphenyl, -N(phenyl)₂, -NHbenzyl, -N(benzyl)₂, -NH-CO-(C₁-C₃-alkyl), -C(=NH)NH₂ or -NH-C(=NH)NH₂, or
benzyl, wherein the phenyl ring is substituted directly or via a methylene bridge by one of the groups -NH₂, -NH(C₁-C₃-alkyl), -N(C₁-C₄-alkyl)₂, -NHphenyl, -N(phenyl)₂, -NHbenzyl, -N(benzyl)₂, -NH-CO-(C₁-C₃-alkyl), -C(=NH)NH₂ or -NH-C(=NH)NH₂, or
phenyl-C₂-C₃-alkyl, wherein the C₂-C₃-alkylene bridge may optionally be substituted by phenyl, COOH or COO-C₁-C₃-alkyl and wherein the phenyl ring may be substituted directly or via a methylene bridge by one of the groups -NH₂, -NH(C₁-C₃-alkyl), -N(C₁-C₃-alkyl)₂, -NHphenyl, -N(phenyl)₂, -NHbenzyl, -N(benzyl)₂, -C(=NH)NH₂ or -NH-C(=NH)NH₂, or
a heterocyclic group linked via a C₁-C₃-alkylene bridge, optionally mono- or disubstituted by one or more of the groups methyl, ethyl, propyl, phenyl or benzyl, selected from the group comprising pyrrole, pyrroline, pyrrolidine, pyridine, piperidine, pyrimidine, piperazine, morpholine, diazepan, imidazole, imidazoline, imidazolidine, pyrazole, pyrazoline, pyrazolidine, furan, tetrahydrofuran, α-pyran, γ-pyran, dioxolan, tetrahydropyran, dioxane, oxazole or isoxazole;
R⁴ denotes hydrogen, C₁-C₄-alkyl, which may be substituted by one of the groups COOH, COO-C₁-C₃-alkyl or C₃-C₆-cycloalkyl, or
phenyl-C₁-C₃-alkyl, wherein the C₁-C₃-alkylene bridge may optionally be substituted by phenyl and wherein the phenyl ring may optionally be substituted by C₁-C₃-alkyl, C₁-C₃-alkoxy, CF₃, fluorine, chlorine, bromine, COOH or COO-C₁-C₃-alkyl, or
C₃-C₈-cycloalkyl, naphthyl or phenyl, which may optionally be mono- or disubstituted by one or more of the groups C₁-C₃-alkyl, C₁-C₃-alkoxy, phenyloxy, benzyloxy, CF₃, fluorine, chlorine, bromine, COOH or COO-C₁-C₃-alkyl, or
a heterocyclic group linked via a C₁-C₃-alkylene bridge, optionally mono- or disubstituted by one or more of the groups methyl, ethyl, propyl, phenyl, methylphenyl or benzyl, selected from the group comprising pyrrole, pyrroline, pyrrolidine, pyridine, piperidine, pyrimidine, piperazine, morpholine, thiomorpholine, imidazole, imidazoline, imidazolidine, pyrazole, pyrazoline, pyrazolidine, triazole, furan, tetrahydrofuran, α-pyran, γ-pyran, dioxolan, tetrahydropyran, dioxane, thiophene, dihydrothiophene, thiolan, dithiolan, oxazole, isoxazole, thiazole, isothiazole, oxadiazole, benzodioxole, benzimidazole, benzothiophene, benzofuran or indole;
or
R³ and R⁴ together with the nitrogen atom form a 6- or 7- membered, saturated heterocyclic group, which contains one or two further nitrogen heteroatoms and which may optionally be substituted by one or more of the groups methyl, ethyl, propyl, benzyl, cyclopentyl, cyclohexyl, pyridyl
or phenyl, which optionally carries a group selected from the group comprising methyl, methoxy, ethoxy, propyloxy or hydroxy, optionally in the form of their tautomers, their racemates, their enantiomers, their diastereomers and mixtures thereof and optionally the pharmacologically acceptable acid addition salts thereof.

5. Compounds of general formula (I) according to one of claims 2-4, wherein
R¹ denotes methyl, ethyl, propyl, butyl, pentyl, hexyl, heptyl, octyl, nonyl or decyl,
or
a methyl, ethyl or propyl group which is substituted by methoxy, ethoxy, cyclopropyl, cyclopentyl, cyclohexyl, phenyl, methoxyphenoxy, -NH₂, -NH(C₁-C₄-alkyl), -N(C₁-C₄-alkyl)₂, -NH-CO-methyl, -CO-NH₂, -CO-NH-methyl or -NH-CO-benzyl, or
benzyl, which is mono- or disubstituted by methyl, ethyl, propyl, CF₃, fluorine, chlorine, bromine, COOH, COOMe or COOEt, or
phenylethyl, which is mono- or disubstituted by methyl, ethyl, propyl, CF₃, fluorine, chlorine, bromine, COOH, COOMe or COOEt, or
a heterocyclic group linked via a methylene, ethylene or propylene bridge, optionally mono- or disubstituted by one or more of the groups methyl, ethyl, propyl, phenyl, methylphenyl or benzyl, selected from the group comprising pyrrole, pyrrolidine, pyridine, piperidine, piperazine, morpholine, furan, tetrahydrofuran, thiophene, benzodioxole or benzimidazole;
R² denotes -C(=NH)NH₂ or -CH₂-NH₂;
R³ denotes a methyl, ethyl or propyl group which is substituted by -NH₂, -NH(C₁-C₃-alkyl), -N(C₁-C₃-alkyl)₂, -NHphenyl, -N(phenyl)₂, -NHbenzyl, -N(benzyl)₂, -NH-CO-(C₁-C₃-alkyl), -NH-CO-benzyl (?) or -C(=NH)NH₂, or
benzyl, which is substituted directly or via a methylene bridge by one of the groups -NH₂, -NH(C₁-C₃-alkyl), -N(C₁-C₄-alkyl)₂, -NHphenyl, -N(phenyl)₂, -NHbenzyl, -N(benzyl)₂, -NH-CO-(C₁-C₃-alkyl) or -C(=NH)NH₂, or
phenyl-C₂-C₃-alkyl, wherein the C₂-C₃-alkylene bridge may optionally be substituted by phenyl, COOH or COO-C₁-C₃-alkyl and wherein the phenyl ring may be substituted directly or via a methylene bridge by one of the groups -NH₂, -NH(C₁-C₃-alkyl), -N(C₁-C₃-alkyl)₂, -NHphenyl, -N(phenyl)₂, -NHbenzyl, -N(benzyl)₂ or -C(=NH)NH₂, or
a heterocyclic group linked via a methylene, ethylene or propylene bridge, optionally mono- or disubstituted by methyl, ethyl, propyl, phenyl or benzyl, selected from the group comprising pyrrole, pyrrolidine, pyridine, piperidine, piperazine, morpholine, diazepan, furan, tetrahydrofuran, thiophene, benzodioxole or benzimidazole;
R⁴ denotes hydrogen or a methyl, ethyl, propyl or butyl group which may be substituted by one of the groups COOH, COOMe, COOEt, cyclopropyl, cyclopentyl or cyclohexyl, or
benzyl, which may optionally be substituted by methyl, ethyl, propyl, methoxy, ethoxy, CF₃, fluorine, chlorine, bromine, COOH, COOMe or COOEt, or phenylethyl, phenylpropyl, diphenylpropyl;
cyclopropyl, cyclopentyl, cyclohexyl, cyclooctyl, naphthyl or phenyl, which may optionally be substituted by methyl, ethyl, propyl, methoxy, ethoxy, phenyloxy, benzyloxy, CF₃, fluorine, chlorine, bromine, COOH, COOMe or COOEt, or
a heterocyclic group linked via a methylene, ethylene or propylene bridge, optionally mono- or disubstituted by methyl, ethyl, propyl, phenyl or benzyl, selected from the group comprising pyrrole, pyrrolidine, pyridine, piperidine, piperazine, morpholine, furan, tetrahydrofuran, thiophene, quinoline, isoquinoline, benzodioxole or benzimidazole;
or
R³ and R⁴ together with the nitrogen atom form a piperazine or diazepan ring which may optionally be substituted by one of the groups methyl, ethyl, propyl, cyclopentyl, cyclohexyl, pyridyl, benzyl or phenyl, which optionally carries a group selected from the group comprising methyl, methoxy, ethoxy, propyloxy or hydroxy,
optionally in the form of their tautomers, their racemates, their enantiomers, their diastereomers and mixtures thereof and optionally the pharmacologically acceptable acid addition salts thereof.

6. Compounds of general formula (I) according to one of claims 2-5, wherein
R¹ denotes methyl, ethyl, propyl, pentyl or n-decyl,
or
a methyl, ethyl or propyl group which is substituted by methoxy, ethoxy, cyclopropyl, cyclopentyl, cyclohexyl, phenyl or methoxyphenoxy, or
benzyl, which is mono- or disubstituted by methyl, CF₃, COOH, COOMe or COOEt, or
a tetrahydrofuran linked via a methylene bridge;
R² denotes -C(=NH)NH₂ or -CH₂-NH₂;
R³ denotes an ethyl or propyl group which is substituted by -NH₂, -NHMe, -NMe₂, -NHEt, -NEt₂, -NHphenyl, -N(phenyl)₂, -NHbenzyl, -N(benzyl)₂ or -C(=NH)NH₂, or
benzyl which is substituted by one of the groups -NH₂, -CH₂-NH₂, -NMe₂, -NHMe, -NEt₂, -NHEt, -NH-CO-Me, -CH₂-NH-CO-Me or -C(=NH)NH₂, or
phenylethyl, wherein the ethylene bridge may optionally be substituted by COOH, COOMe or COOEt and wherein the phenyl ring is optionally substituted by one of the groups -NH₂, -CH₂-NH₂, -NMe₂, -NHMe, -NEt₂, -NHEt, -NH-CO-Me, -CH₂-NH-CO-Me or -C(=NH)NH₂, or
phenylpropyl, diphenylpropyl or pyridylmethyl;
R⁴ denotes hydrogen or a methyl, ethyl, propyl or butyl group, which may be substituted by one of the groups COOH, COOMe, COOEt, cyclopropyl, cyclopentyl or cyclohexyl, or
benzyl, which may optionally be substituted by methyl, ethyl, propyl, methoxy, ethoxy, CF₃, fluorine, chlorine, bromine, COOH, COOMe or COOEt, or phenyl-ethyl, phenylpropyl, diphenylpropyl, or
cyclopentyl, cyclohexyl, cyclooctyl, naphthyl or phenyl, which may optionally be substituted by methyl, ethyl, methoxy, ethoxy, phenyloxy or CF₃, or
a pyridine or quinoline linked via a methylene bridge,
or
R³ and R⁴ together with the nitrogen atom form a piperazine or diazepan ring, which may optionally be substituted by one of the groups cyclopentyl, cyclohexyl, pyridyl, benzyl or phenyl, which optionally carries one of the groups selected from the group comprising methyl, methoxy, ethoxy, propyloxy or hydroxy,
optionally in the form of their tautomers, their racemates, their enantiomers, their diastereomers and mixtures thereof and optionally the pharmacologically acceptable acid addition salts thereof.

7. Compounds of general formula (I) according to one of claims 2-6, wherein
R¹ denotes methyl, ethyl, propyl, pentyl, phenylethyl, phenylpropyl, cyclopropylmethyl, tetrahydrofuranylmethyl or benzyl, which is mono- or disubstituted by CF₃, COOH, COOMe or COOEt;
R² denotes -C(=NH)NH₂ or -CH₂-NH₂;
R³ denotes an ethyl or propyl group which is substituted by -NH₂, -NHMe, -NMe₂, -NHEt, -NEt₂, -NHphenyl, -N(phenyl)₂, -NHbenzyl, -N(benzyl)₂ or -C(=NH)NH₂,
or benzyl substituted by one of the groups -NH₂, -CH₂-NH₂, -NMe₂, -NHMe, -NEt₂; -NHEt, -NH-CO-Me, -CH₂-NH-CO-Me or -C(=NH)NH₂,
or phenylethyl, wherein the ethylene bridge is substituted by COOH, COOMe or COOEt and wherein the phenyl ring carries one of the groups -NH₂, -CH₂-NH₂, -NMe₂, -NHMe, -NEt₂, -NHEt, -NH-CO-Me, -CH₂-NH-CO-Me or -C(=NH)NH₂, or
phenylpropyl, diphenylpropyl or pyridylmethyl;
R⁴ denotes hydrogen or a methyl, ethyl, propyl or butyl group which may be substituted by one of the groups COOH, COOMe, COOEt or cyclohexyl, or
phenyl, which may optionally be substituted by methyl, ethyl, methoxy, ethoxy, phenyloxy or CF₃, or
benzyl, phenylethyl, phenylpropyl, diphenylpropyl, cyclohexyl, cyclooctyl or naphthyl, or
a pyridine or quinoline linked via a methylene bridge,
or
R³ and R⁴ together with the nitrogen atom form a piperazine or diazepan ring, which may optionally be substituted by one of the groups cyclopentyl, cyclohexyl, pyridyl, benzyl or phenyl, which optionally carries a group selected from the group comprising methyl, methoxy, ethoxy, propyloxy or hydroxy,
optionally in the form of their tautomers, their racemates, their enantiomers, their diastereomers and mixtures thereof and optionally the pharmacologically acceptable acid addition salts thereof.

8. Compounds of general formula (I) according to one of claims 2-7, wherein
R¹ denotes methyl;
R² denotes -C(=NH)NH₂ or -CH₂-NH₂;
R³ denotes ethyl, substituted by NH₂, -NMe₂, NHphenyl, -NHbenzyl, -N(benzyl)₂, pyrrolidine, piperidine, diazepan or -C(=NH)NH₂, benzyl substituted by one of the groups -CH₂-NH₂, -NMe₂ or -C(=NH)NH₂, phenylethyl, wherein the ethylene bridge is substituted by COOH, COOMe or COOEt and wherein the phenyl ring carries one of the groups -CH₂-NH-CO-Me or -C(=NH)NH₂, diphenylpropyl or pyridylmethyl;
R⁴ denotes hydrogen or a methyl or ethyl group which may optionally be substituted by one of the groups COOH or COOEt; propyl, butyl or phenyl, which may optionally be substituted by methyl, ethyl, methoxy, ethoxy, phenyloxy or CF_{3;} benzyl, phenylethyl, phenylpropyl, diphenylpropyl, cyclohexyl, cyclooctyl, naphthyl, pyridylmethyl or quinolinylmethyl
or
R³ and R⁴ together with the nitrogen atom form a piperazine or diazepan ring substituted by one of the groups benzyl, cyclopentyl, cyclohexyl or phenyl, which optionally carries a group selected from the group comprising methyl, ethoxy, propyloxy or hydroxy,
optionally in the form of their tautomers, their racemates, their enantiomers, their diastereomers and mixtures thereof and optionally the pharmacologically acceptable acid addition salts thereof.

9. Compounds of general formula (I) according to one of claims 2-8, wherein
R¹ denotes methyl;
R² denotes -C(=NH)NH₂;
R³ denotes ethyl, substituted by -NH₂, -NHphenyl, -NHbenzyl, -N(benzyl)₂, pyrrolidine, piperidine, diazepan or -C(=NH)NH₂ ; benzyl substituted by -C(=NH)NH₂, or diphenylpropyl;
R⁴ denotes hydrogen, methyl, propyl, butyl, benzyl or phenyl, which may optionally be substituted by ethyl or phenyloxy; phenylethyl, cyclohexyl or cyclooctyl, or
R³ and R⁴ together with the nitrogen atom form a piperazine ring which is substituted by a group selected from the group comprising cyclopentyl, cyclohexyl, phenyl, methylphenyl, ethoxyphenyl or propoxyphenyl, or a diazepan ring substituted by methylphenyl,
optionally in the form of their tautomers, their racemates, their enantiomers, their diastereomers and mixtures thereof and optionally the pharmacologically acceptable acid addition salts thereof.

10. Compounds of general formula (I) according to one of claims 2-9, wherein
R¹ denotes methyl;
R² denotes -C(=NH)NH₂;
R³ denotes ethyl, substituted by -NH₂, -NHbenzyl, -N(benzyl)₂, pyrrolidine, piperidine, diazepan or -C(=NH)NH₂, benzyl substituted by -C(=NH)NH₂ , or diphenylpropyl;
R⁴ denotes hydrogen, methyl, butyl or phenyl, which may optionally be substituted by ethyl or phenyloxy; phenylethyl, cyclohexyl or cyclooctyl,
optionally in the form of their tautomers, their racemates, their enantiomers, their diastereomers and mixtures thereof and optionally the pharmacologically acceptable acid addition salts thereof.

11. Prodrugs of general formula (II) 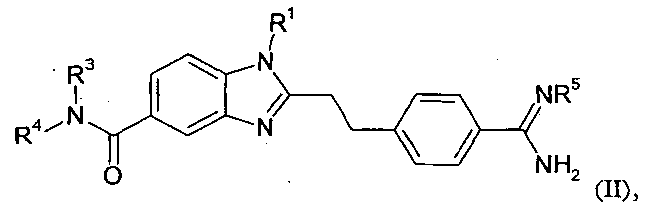 wherein
R¹ and R⁴ are defined as in claims 2 - 10 and
R³ may have the meanings given in claims 2 - 10 or denotes C₁-C₄-alkyl, which is substituted by a group selected from the group comprising-C(=NOH)NH₂, -C(=NCOO-C₁-C₄-alkyl)NH₂ or -C(=NCOO-C₁-C₄-alkyl-phenyl)NH₂;
R⁵ may denote hydroxy, -COO-C₁-C₈-alkyl or -COO-C₁-C₄-alkyl-phenyl, whilst in the abovementioned group the phenyl ring may be substituted by C₁-C₄-alkyl or C₁-C₄-alkoxy,
optionally in the form of their tautomers, their racemates, their enantiomers, their diastereomers and mixtures thereof and optionally the pharmacologically acceptable acid addition salts thereof.

12. Prodrugs of general formula (II) according to Claim 11, wherein
R¹ and R⁴ may have the meanings given in claims 2-10;
R³ may have the meanings given in claims 2-10
or denotes C₁-C₄-alkyl, which is substituted by a group selected from the group comprising -C(=NOH)NH₂, -C(=NCOOmethyl)NH₂, -C(=NCOOethyl)NH₂, -C(=NCOOpropylyl)NH₂ or -C(=NCOO-benzyl)NH₂;
R⁵ may denote hydroxy, methoxycarbonyl, ethoxycarbonyl, propyloxycarbonyl, butyloxycarbonyl, pentyloxycarbonyl, hexyloxycarbonyl, benzyloxycarbonyl,
optionally in the form of their tautomers, their racemates, their enantiomers, their diastereomers and mixtures thereof and optionally the pharmacologically acceptable acid addition salts thereof.

13. Intermediate products of general formula (III) 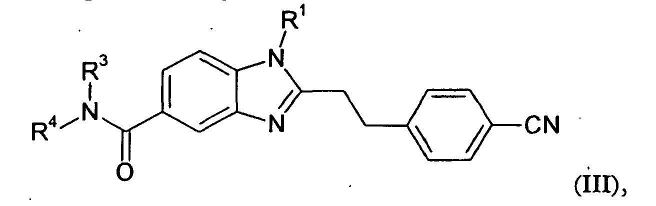 wherein the groups R¹, R³ and R⁴ may have the meanings given in claims 2-12.

14. Use of compounds of general formula (I) according to one of claims 2-10 for preparing a pharmaceutical composition for the prevention and/or treatment of diseases in which tryptase inhibitors may have a therapeutic value.

15. Use of prodrugs of general formula (II) according to one of claims 11 or 12 for preparing a pharmaceutical composition for the prevention and/or treatment of diseases in which tryptase inhibitors may have a therapeutic value.

16. Pharmaceutical composition, **characterised in that** it contains one or more compounds according to one of claims 2-12.

17. Process for preparing compounds of general formula (I) 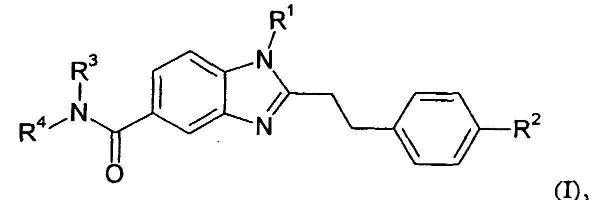 wherein the groups R¹, R², R³ and R⁴ may have the meanings given in claims 2-10, **characterised in that** a compound of general formula (III) 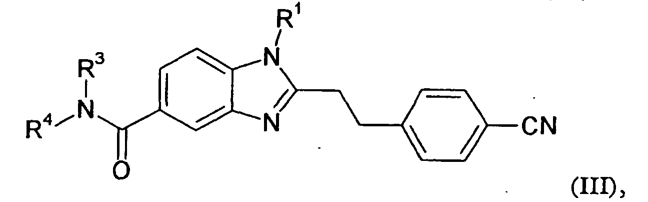 wherein the groups R¹, R³ and R⁴ may have the meanings given in claims 2-10, is converted directly into the compound of general formula (I).

18. Process for preparing compounds of general formula (I) 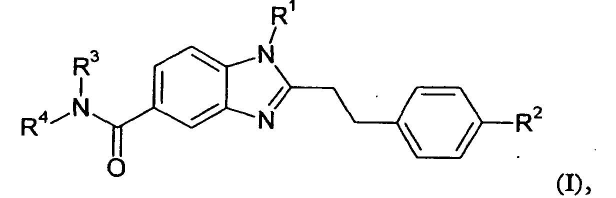 wherein the groups R¹, R², R³ and R⁴ may have the meanings given in claims 2-10, **characterised in that** a compound of general formula (II) 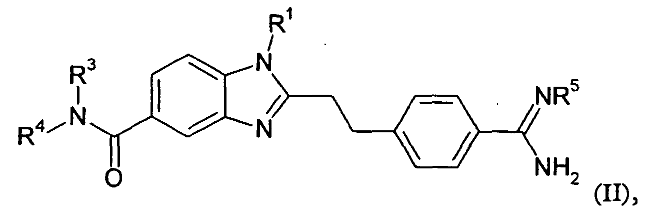 wherein the groups R¹ and R⁴ may have the meanings given in claims 2 to 10 and the groups R³ and R⁵ may have the meanings given in claims 11 and 12, is converted into a compound of general formula (I).

19. Process for preparing compounds of general formula (II) 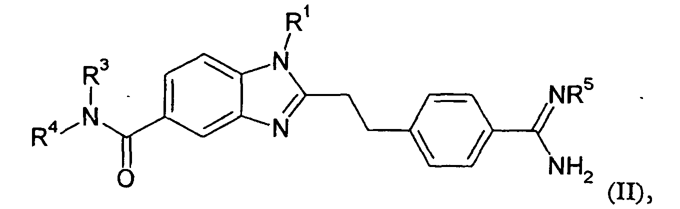 wherein the groups R¹ and R⁴ may have the meanings given in claims 2 to 10 and the groups R³ and R⁵ may have the meanings given in claims 11 and 12, **characterised in that** a compound of general formula (III) 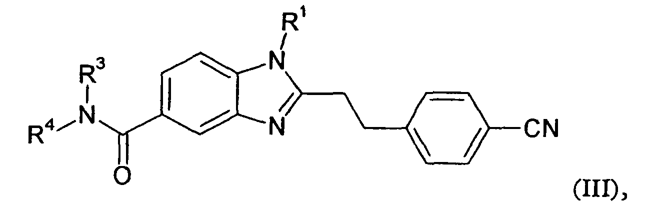 wherein the groups R¹, R³ and R⁴ may have the meanings given in claims 2-10, is converted into a compound of general formula (II).

20. Process for preparing compounds of general formula (I) 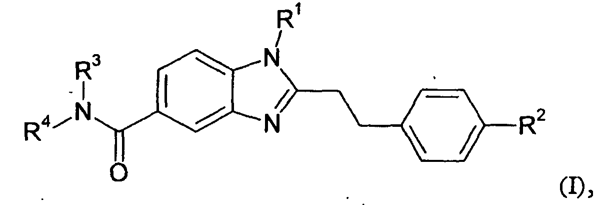 wherein the groups R¹, R², R³ and R⁴ may have the meanings given in claims 2-10, **characterised in that** the synthesis is carried out by reaction on a polymeric carrier (resin) and comprises the following steps:
- coupling a diamine to the polymeric carrier,
- reductive amination comprising reacting with an aldehyde R⁴-CHO,
- acylation with fluoro- or chloronitrobenzoyl chloride,
- nucleophilic substitution with an amine R¹-NH₂,
- reduction,
- oxidative cyclisation and
- cleaving the compounds of general formula (I) from the polymeric carrier.

## Revendications

1. Utilisation de composés de formule générale (I) 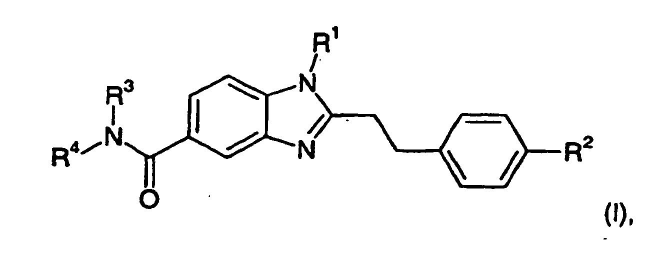 pour la production d'un médicament pour la prévention et/ou le traitement des maladies dans lesquelles les inhibiteurs de tryptase peuvent présenter une utilité thérapeutique, où
R¹ peut représenter C₁-C₁₀-alkyle, qui peut éventuellement être substitué une, deux ou trois fois par un ou plusieurs des groupes C₁-C₄-alcoxy, phénoxy, hydroxyphénoxy, C₁-C₄-alcoxy-phénoxy, C₃-C₆-cycloalkyle; -NH₂, -NH(C₁-C₄-alkyle), -N(C₁-C₄-alkyle)₂, -NH-CO-(C₁-C₄-alkyle), -CO-NH₂, -CO-NH-(C₁-C₄-alkyle) ou -NH-CO-benzyle, ou
phényl-C₁-C₄-alkyle, où le cycle phényle peut éventuellement être substitué une, deux ou trois fois par un ou plusieurs des groupements C₁-C₄-alkyle, CF₃, fluor, chlore, brome, COOH ou COO-C₁-C₄-alkyle, ou
un hétérocycle saturé ou insaturé, à 5 ou 6 chaînons, lié par une simple liaison ou par le biais d'un pont C₁-C₄-alkylène, qui peut contenir un, deux ou trois hétéroatomes choisis dans le groupe oxygène, azote ou soufre et qui peut éventuellement être substitué une, deux ou trois fois par un ou plusieurs des groupements C₁-C₄-alkyle, phényle éventuellement substitué par C₁-C₄-alkyle ou benzyle éventuellement substitué par C₁-C₄-alkyle ou auquel un cycle benzénique peut éventuellement être condensé par le biais de deux atomes de carbone voisins ; R² peut représenter -C(=NH)NH₂ ou -CH₂-NH₂ ;
R³ et R⁴,
identiques ou différents,
peuvent représenter l'hydrogène, C₁-C₆-alkyle, qui peut être substitué une ou deux fois par un ou plusieurs des groupes COOH, COO-C₁-C₄-alkyle, C₃-C₆-cycloalkyle, -NH₂, -NH(C₁-C₄-alkyle), -N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou -NH-C(=NH)NH₂, ou
phényl-C₁-C₄-alkyle, où le pont C₁-C₄-alkylène peut éventuellement être substitué par phényle, COOH ou COO-C₁-C₄-alkyle et où le cycle phényle peut éventuellement être substitué une, deux ou trois fois, directement ou par le biais d'un pont C₁-C₄-alkylène par un ou plusieurs des groupements C₁-C₄-alkyle, C₁-C₄-alcoxy, CF₃, fluor, chlore, brome, COOH, COO-C₁-C₄-alkyle, -NH₂, -NH(C₁-C₄-alkyle), -N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou -NH-C(=NH)NH₂, ou un hétérocycle saturé ou insaturé, à 5, 6 ou 7 chaînons, lié directement ou par le biais d'un pont C₁-C₄-alkylène, qui peut contenir un, deux, trois ou quatre hétéroatomes choisis dans le groupe oxygène, azote ou soufre et qui peut éventuellement être substitué une, deux ou trois fois par un ou plusieurs des groupements C₁-C₄-alkyle, phényle ou benzyle ou auquel un cycle benzénique peut éventuellement être condensé par le biais de deux atomes de carbone voisins,
ou
C₃-C₈-cycloalkyle, naphtyle ou phényle, qui peut éventuellement être substitué une, deux ou trois fois par un ou plusieurs des groupements C₁-C₄-alkyle, C₁-C₄-alcoxy, phényloxy, benzyloxy, CF₃, fluor, chlore, brome, COOH ou COO-C₁-C₄-alkyle, ou
R³ et R⁴ forment avec l'atome d'azote un hétérocycle saturé ou insaturé, à 5, 6 ou 7 chaînons, qui peut contenir un ou deux autres hétéroatomes choisis dans le groupe oxygène, azote ou soufre et qui peut éventuellement être substitué par un
ou plusieurs des groupements C₁-C₄-alkyle, C₅-C₆-cycloalkyle, benzyle, qui peut éventuellement être substitué par C₁-C₄-alkyle, pyridyle ou phényle, qui est éventuellement substitué par C₁-C₄-alkyle, C₁-C₄-alcoxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

2. Dérivés de benzimidazole aminocarbonyl-substitués de formule générale (I) 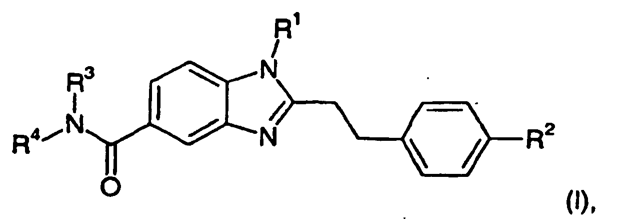 où
R¹ peut représenter C₁-C₁₀-alkyle, qui peut éventuellement être substitué une, deux ou trois fois par un ou plusieurs des groupes C₁-C₄-alcoxy, phénoxy, C₁-C₄-alcoxy-phénoxy, hydroxyphénoxy, C₃-C₆-cycloalkyle, -NH₂, -NH(C₁-C₄-alkyle), - N(C₁-C₄-alkyle)₂, -NH-CO-(C₁-C₄-alkyle), -CO-NH₂, -CO-NH-(C₁-C₄-alkyle) ou -NH-CO-benzyle, ou
phényl-C₁-C₄-alkyle, où le cycle phényle peut éventuellement être substitué une, deux ou trois fois par un ou plusieurs des groupements C₁-C₄-alkyle, CF₃, fluor, chlore, brome, COOH ou COO-C₁-C₄-alkyle, ou
un hétérocycle saturé ou insaturé, à 5 ou 6 chaînons, lié par une simple liaison ou par le biais d'un pont C₁-C₄-alkylène, qui peut contenir un, deux ou trois hétéroatomes choisis dans le groupe oxygène, azote ou soufre et qui peut éventuellement être substitué une, deux ou trois fois par un ou plusieurs des groupements C₁-C₄-alkyle, phényle éventuellement substitué par C₁-C₄-alkyle ou benzyle éventuellement substitué par C₁-C₄-alkyle ou auquel un cycle benzénique peut éventuellement être condensé par le biais de deux atomes de carbone voisins ;
R² peut représenter -C(=NH)NH₂ ou -CH₂-NH₂ ;
R³ peut représenter C₁-C₆-alkyle, qui peut être substitué une ou deux fois par un ou plusieurs des groupes -NH₂, -NH(C₁-C₄-alkyle), -N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou -NH-C(=NH)NH₂, ou
benzyle, où le cycle phényle est substitué une ou deux fois, directement ou par le biais d'un pont C₁-C₄-alkylène par un ou plusieurs des groupements -NH₂, -NH(C₁-C₄-alkyle), -N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou -NH-C(=NH)NH₂, ou phényl-C₂-C₄-alkyle, où le pont C₂-C₄-alkylène peut éventuellement être substitué par phényle, COOH ou COO-C₁-C₄-alkyle et où le cycle phényle peut éventuellement être substitué une ou deux fois, directement ou par le biais d'un pont C₁-C₄-alkylène par un ou plusieurs des groupes -NH₂, -NH(C₁-C₄-alkyle), - N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou NH-C(=NH)NH₂, ou
un hétérocycle saturé ou insaturé, à 5 ou 6 chaînons, lié par le biais d'un pont C₁-C₄-alkylène, qui peut contenir un, deux ou trois hétéroatomes choisis dans le groupe oxygène, azote ou soufre et qui peut éventuellement être substitué une, deux ou trois fois par un ou plusieurs des groupements C₁-C₄-alkyle, phényle ou benzyle ;
R⁴ peut représenter l'hydrogène, C₁-C₆-alkyle, qui peut être substitué une ou deux fois par un ou plusieurs des groupes COOH, COO-C₁-C₄-alkyle ou C₃-C₆-cycloalkyle, ou
phényl-C₁-C₄-alkyle, où le pont C₁-C₄-alkylène peut éventuellement être substitué par phényle et où le cycle phényle peut éventuellement être substitué une, deux ou trois fois par un ou plusieurs des groupements C₁-C₄-alkyle, C₁-C₄-alcoxy, CF₃, fluor, chlore, brome, COOH ou COO-C₁-C₄-alkyle, ou
C₃-C₈-cycloalkyle, naphtyle ou phényle, qui peut éventuellement être substitué une, deux ou trois fois par un ou plusieurs des groupements C₁-C₄-alkyle, C₁-C₄-alcoxy, phényloxy, benzyloxy, CF₃, fluor, chlore, brome, COOH ou COO-C₁-C₄-alkyle, ou
un hétérocycle saturé ou insaturé, à 5, 6 ou 7 chaînons, lié par le biais d'un pont C₁-C₄-alkylène, qui contient un hétéroatome choisi dans le groupe oxygène, azote
ou soufre et qui peut éventuellement être substitué une, deux ou trois fois par un
ou plusieurs des groupements C₁-C₄-alkyle, phényle ou benzyle ou auquel un cycle benzénique peut éventuellement être condensé par le biais de deux atomes de carbone voisins, ou
R³ et R⁴ forment avec l'atome d'azote un hétérocycle saturé ou insaturé, à 5, 6 ou 7 chaînons, qui contient un ou deux autres hétéroatomes choisis dans le groupe oxygène, azote ou soufre et qui peut éventuellement être substitué par un ou plusieurs des groupements C₁-C₄-alkyle, benzyle, qui est éventuellement substitué par C₁-C₄-alkyle, C₅-C₆-cycloalkyle, pyridyle ou phényle, qui porte éventuellement un groupement choisi dans le groupe C₁-C₄-alkyle, C₁-C₄-alcoxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

3. Composés de formule générale (I) selon la revendication 2 où
R¹ peut représenter C₁-C₁₀-alkyle non substitué ou C₁-C₄-alkyle substitué une ou deux fois par C₁-C₄-alcoxy, phénoxy, C₁-C₄-alcoxy-phénoxy, hydroxyphénoxy, C₃-C₆-cycloalkyle, -NH₂, -NH(C₁-C₄-alkyle), -N(C₁-C₄-alkyle)₂, -NH-CO-(C₁-C₄-alkyle), -CO-NH₂, -CO-NH-(C₁-C₄-alkyle) ou -NH-CO-benzyle, ou
phényl-C₁-C₃-alkyle, où le cycle phényle peut éventuellement être substitué une ou deux fois par C₁-C₄-alkyle, CF₃, fluor, chlore, brome, COOH ou COO-C₁-C₄-alkyle, ou
un hétérocycle saturé ou insaturé, à 5, 6 ou 7 chaînons, lié par le biais d'un pont C₁-C₃-alkylène, qui peut contenir un ou deux hétéroatomes choisis dans le groupe oxygène, azote ou soufre et qui peut éventuellement être substitué une ou deux fois par un ou plusieurs des groupements méthyle, éthyle, propyle, phényle, méthylphényle ou benzyle ou auquel un cycle benzénique peut éventuellement être condensé par le biais de deux atomes de carbone voisins ;
R² peut représenter -C(=NH)NH₂ ou -CH₂-NH₂ ;
R³ peut représenter C₁-C₆-alkyle, qui est substitué une ou deux fois par un ou plusieurs des groupes -NH₂, -NH(C₁-C₄-alkyle), -N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou -NH-C(=NH)NH₂, ou
benzyle, où le cycle phényle est substitué directement ou par le biais d'un pont C₁-C₄-alkylène par l'un des groupes -NH₂, -NH(C₁-C₄-alkyle), -N(C₁-C₄-alkyle)₂, -NHphényle, -N(phényle)₂, -NHbenzyle, -N(benzyle)₂, -NH-CO-(C₁-C₄-alkyle), -C(=NH)NH₂ ou -NH-C(=NH)NH₂, ou
phényl-C₂-C₄-alkyle, où le pont C₂-C₄-alkylène peut éventuellement être substitué par phényle, COOH ou COO-C₁-C₄-alkyle et où le cycle phényle peut être substitué directement ou par le biais d'un pont C₁-C₄-alkylène par l'un des groupes -NH₂, -NH(C₁-C₄-alkyle), -N(C₁-C₄-alkyle)₂, -NHphényle, -N(phényle)₂, -NHbenzyle, -N(benzyle)₂, -C(=NH)NH₂ ou -NH-C(=NH)NH₂, ou
un hétérocycle saturé ou insaturé, à 5, 6 ou 7 chaînons, lié par le biais d'un pont C₁-C₄-alkylène, qui peut contenir un ou deux hétéroatomes choisis dans le groupe oxygène, azote ou soufre et qui peut éventuellement être substitué une ou deux fois par un ou plusieurs des groupements méthyle, éthyle, propyle, phényle ou benzyle ;
R⁴ peut représenter l'hydrogène, C₁-C₄-alkyle, qui peut être substitué par l'un des groupes COOH, COO-C₁-C₄-alkyle ou C₃-C₆-cycloalkyle, ou
phényl-C₁-C₄-alkyle, où le pont C₁-C₄-alkylène peut éventuellement être substitué par phényle et où le cycle phényle peut éventuellement être substitué une ou deux fois par un ou plusieurs des groupements C₁-C₄-alkyle, C₁-C₄-alcoxy, CF₃, fluor, chlore, brome, COOH ou COO-C₁-C₄-alkyle, ou
C₃-C₈-cycloalkyle, naphtyle ou phényle, qui peut éventuellement être substitué une ou deux fois par un ou plusieurs des groupements C₁-C₄-alkyle, C₁-C₄-alcoxy, phényloxy, benzyloxy, CF₃, fluor, chlore, brome, COOH ou COO-C₁-C₄-alkyle, ou
un hétérocycle saturé ou insaturé, à 5, 6 ou 7 chaînons, lié par le biais d'un pont C₁-C₄-alkylène, qui contient un hétéroatome choisi dans le groupe oxygène, azote
ou soufre et qui peut éventuellement être substitué une ou deux fois par un ou plusieurs des groupements méthyle, éthyle, propyle, phényle ou benzyle ou auquel un cycle benzénique peut éventuellement être condensé par le biais de deux atomes de carbone voisins, ou
R³ et R⁴ forment avec l'atome d'azote un hétérocycle saturé ou insaturé, à 6 ou 7 chaînons, qui contient un ou deux autres hétéroatomes choisis dans le groupe oxygène ou azote et qui peut éventuellement être substitué par un ou plusieurs des groupements méthyle, éthyle, propyle, benzyle, cyclopentyle, cyclohexyle, pyridyle ou phényle, qui porte éventuellement un groupement choisi dans le groupe méthyle, méthoxy, éthoxy, propyloxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

4. Composés de formule générale (I) selon la revendication 2 ou 3 où
R¹ peut représenter C₁-C₁₀-alkyle non substitué ou C₁-C₄-alkyle substitué par C₁-C₄-alcoxy, phénoxy, C₁-C₄-alcoxy-phénoxy, hydroxyphénoxy, C₃-C₆-cycloalkyle, -NH₂, -NH(C₁-C₄-alkyle), -N(C₁-C₄-alkyle)₂, -NH-CO-(C₁-C₄-alkyle), -CO-NH₂, -CO-NH-(C₁-C₄-alkyle) ou NH-CO-benzyle, ou
phényl-C₁-C₃-alkyle, où le cycle phényle peut éventuellement être substitué une ou deux fois par C₁-C₄-alkyle, CF₃, fluor, chlore, brome, COOH ou COO-C₁-C₄-alkyle, ou
un hétérocycle lié par le biais d'un pont C₁-C₃-alkylène, éventuellement substitué une ou deux fois par un ou plusieurs des groupements méthyle, éthyle, propyle, phényle, méthylphényle ou benzyle, choisi dans le groupe pyrrole, pyrroline, pyrrolidine, pyridine, pipéridine, pyrimidine, pipérazine, morpholine, thiomorpholine, imidazole, imidazoline, imidazolidine, pyrazole, pyrazoline, pyrazolidine, triazole, furane, tétrahydrofurane, α-pyrane, γ-pyrane, dioxolane, tétrahydropyrane, dioxane, thiophène, dihydrothiophène, thiolane, dithiolane, oxazole, isoxazole, thiazole, isothiazole, oxadiazole, benzodioxole, benzimidazole, benzothiophène, benzofurane ou indole ;
R² peut représenter -C(=NH)NH₂ ou -CH₂-NH₂ ;
R³ peut représenter C₁-C₃-alkyle, qui est substitué par -NH₂, -NH(C₁-C₃-alkyle), -N(C₁-C₃-alkyle)₂, -NHphényle, -N(phényle)₂, -NHbenzyle, -N(benzyle)₂, -NH-CO-(C₁-C₃-alkyle), -C(=NH)NH₂ ou -NH-C(=NH)NH₂, ou
benzyle, où le cycle phényle est substitué directement ou par le biais d'un pont méthylène par l'un des groupes -NH₂, -NH(C₁-C₃-alkyle), -N(C₁-C₄-alkyle)₂, -NHphényle, -N(phényle)₂, -NHbenzyle, -N(benzyle)₂, -NH-CO-(C₁-C₃-alkyle), -C(=NH)NH₂ ou -NH-C(=NH)NH₂, ou
phényl-C₂-C₃-alkyle, où le pont C₂-C₃-alkylène peut éventuellement être substitué par phényle, COOH ou COO-C₁-C₃-alkyle et où le cycle phényle peut être substitué directement ou par le biais d'un pont méthylène par l'un des groupes -NH₂, -NH(C₁-C₃-alkyle), -N(C₁-C₃-alkyle)₂, -NHphényle, -N(phényle)₂, -NHbenzyle, -N(benzyle)₂, -C(=NH)NH₂ ou NH-C(=NH)NH₂, ou
un hétérocycle lié par le biais d'un pont C₁-C₃-alkylène, éventuellement substitué une ou deux fois par un ou plusieurs des groupements méthyle, éthyle, propyle, phényle ou benzyle, choisi dans le groupe pyrrole, pyrroline, pyrrolidine, pyridine, pipéridine, pyrimidine, pipérazine, morpholine, diazépane, imidazole, imidazoline, imidazolidine, pyrazole, pyrazoline, pyrazolidine, furane, tétrahydrofurane, α-pyrane, γ-pyrane, dioxolane, tétrahydropyrane, dioxane, oxazole ou isoxazole ;
R⁴ peut représenter l'hydrogène, C₁-C₄-alkyle, qui peut être substitué par l'un des groupes COOH, COO-C₁-C₃-alkyle ou C₃-C₆-cycloalkyle, ou
phényl-C₁-C₃-alkyle, où le pont C₁-C₃-alkylène peut éventuellement être substitué par phényle et où le cycle phényle peut éventuellement être substitué par C₁-C₃-alkyle, C₁-C₃-alcoxy, CF₃, fluor, chlore, brome, COOH ou COO-C₁-C₃-alkyle, ou C₃-C₈-cycloalkyle, naphtyle ou phényle, qui peut éventuellement être substitué une ou deux fois par un ou plusieurs des groupements C₁-C₃-alkyle, C₁-C₃-alcoxy, phényloxy, benzyloxy, CF₃, fluor, chlore, brome, COOH ou COO-C₁-C₃-alkyle, ou
un hétérocycle lié par le biais d'un pont C₁-C₃-alkylène, éventuellement substitué une ou deux fois par un ou plusieurs des groupements méthyle, éthyle, propyle, phényle, méthylphényle ou benzyle, choisi dans le groupe pyrrole, pyrroline, pyrrolidine, pyridine, pipéridine, pyrimidine, pipérazine, morpholine, thiomorpholine, imidazole, imidazoline, imidazolidine, pyrazole, pyrazoline, pyrazolidine, triazole, furané, tétrahydrofurane, α-pyrane, γ-pyrane, dioxolane, tétrahydropyrane, dioxane, thiophène, dihydrothiophène, thiolane, dithiolane, oxazole, isoxazole, thiazole, isothiazole, oxadiazole, benzodioxole, benzimidazole, benzothiophène, benzofurane ou indole ;
ou
R³ et R⁴ forment avec l'atome d'azote un hétérocycle saturé à 6 ou 7 chaînons, qui contient un ou deux autres hétéroatomes d'azote et qui peut éventuellement être substitué par un ou plusieurs des groupements méthyle, éthyle, propyle, benzyle, cyclopentyle, cyclohexyle, pyridyle ou phényle, qui porte éventuellement un groupement choisi dans le groupe méthyle, méthoxy, éthoxy, propyloxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

5. Composés de formule générale (I) selon l'une des revendications 2-4 où
R¹ peut représenter méthyle, éthyle, propyle, butyle, pentyle, hexyle, heptyle, octyle, nonyle ou décyle ou
un groupement méthyle, éthyle ou propyle qui est substitué par méthoxy, éthoxy, cyclopropyle, cyclopentyle, cyclohexyle, phényle, méthoxyphénoxy, -NH₂, -NH(C₁-C₄-alkyle), -N(C₁-C₄-alkyle)₂, -NH-CO-méthyle, -CO-NH₂, -CO-NH-méthyle ou -NH-CO-benzyle, ou
benzyle, qui est substitué une ou deux fois par méthyle, éthyle, propyle, CF₃, fluor, chlore, brome, COOH, COOMe ou COOEt, ou
phényléthyle qui est substitué une ou deux fois par méthyle, éthyle, propyle, CF₃, fluor, chlore, brome, COOH, COOMe ou COOEt, ou
un hétérocycle lié par le biais d'un pont méthylène, éthylène ou propylène, éventuellement substitué une ou deux fois par un ou plusieurs des groupements méthyle, éthyle, propyle, phényle, méthylphényle ou benzyle, choisi dans le groupe pyrrole, pyrrolidine, pyridine, pipéridine, pipérazine, morpholine, furane, tétrahydrofurane, thiophène, benzodioxole ou benzimidazole ;
R² peut représenter -C(=NH)NH₂ ou -CH₂-NH₂ ;
R³ peut représenter un groupement méthyle, éthyle ou propyle, qui est substitué par -NH₂, -NH(C₁-C₃-alkyle), -N(C₁-C₃-alkyle)₂, -NHphényle, -N(phényle)₂, -NHbenzyle, -N(benzyle)₂, -NH-CO-(C₁-C₃-alkyle), -NH-CO-benzyle (?) ou -C(=NH)NH₂, ou
benzyle, qui est substitué directement ou par le biais d'un pont méthylène par l'un des groupes -NH₂, -NH(C₁-C₃-alkyle), -N(C₁-C₄-alkyle)₂, -NHphényle, -N(phényle)₂, -NHbenzyle, -N(benzyle)₂, -NH-CO-(C₁-C₃-alkyle) ou -C(=NH)NH₂, ou
phényl-C₂-C₃-alkyle, où le pont C₂-C₃-alkylène peut éventuellement être substitué par phényle, COOH ou COO-C₁-C₃-alkyle et où le cycle phényle peut être substitué directement ou par le biais d'un pont méthylène par l'un des groupes -NH₂, -NH(C₁-C₃-alkyle), -N(C₁-C₃-alkyle)₂, -NHphényle, -N(phényle)₂, -NHbenzyle, -N(benzyle)₂ ou -C(=NH)NH₂, ou
un hétérocycle lié par le biais d'un pont méthylène, éthylène ou propylène, éventuellement substitué une ou deux fois par méthyle, éthyle, propyle, phényle ou benzyle, choisi dans le groupe pyrrole, pyrrolidine, pyridine, pipéridine, pipérazine, morpholine, diazépane, furane, tétrahydrofurane, thiophène, benzodioxole ou benzimidazole ;
R⁴ peut représenter l'hydrogène ou un groupement méthyle, éthyle, propyle ou butyle, qui peut être substitué par l'un des groupes COOH, COOMe, COOEt, cyclopropyle, cyclopentyle ou cyclohexyle, ou
benzyle, qui peut éventuellement être substitué par méthyle, éthyle, propyle, méthoxy, éthoxy, CF₃, fluor, chlore, brome, COOH; COOMe ou COOEt, ou phényléthyle, phénylpropyle, diphénylpropyle ;
cyclopropyle, cyclopentyle, cyclohexyle, cyclooctyle, naphtyle ou phényle, qui peut éventuellement être substitué par méthyle, éthyle, propyle, méthoxy, éthoxy, phényloxy, benzyloxy, CF₃, fluor, chlore, brome, COOH, COOMe ou COOEt, ou un hétérocycle lié par le biais d'un pont méthylène, éthylène ou propylène, éventuellement substitué une ou deux fois par méthyle, éthyle, propyle, phényle ou benzyle, choisi dans le groupe pyrrole, pyrrolidine, pyridine, pipéridine, pipérazine, morpholine, furane, tétrahydrofurane, thiophène, quinoléine, isoquinoléine, benzodioxole ou benzimidazole ;
ou
R³ et R⁴ forment avec l'atome d'azote un cycle pipérazine ou diazépane, qui peut éventuellement être substitué par l'un des groupements méthyle, éthyle, propyle, cyclopentyle, cyclohexyle, pyridyle, benzyle ou phényle, qui porte éventuellement un groupement choisi dans le groupe méthyle, méthoxy, éthoxy, propyloxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

6. Composés de formule générale (I) selon l'une des revendications 2-5 où
R¹ peut représenter méthyle, éthyle, propyle, pentyle ou n-décyle ou
un groupement méthyle, éthyle ou propyle qui est substitué par méthoxy, éthoxy, cyclopropyle, cyclopentyle, cyclohexyle, phényle ou méthoxyphénoxy, ou benzyle, qui est substitué une ou deux fois par méthyle, CF₃, COOH, COOMe ou COOEt, ou
un tétrahydrofurane lié par le biais d'un pont méthylène ;
R² peut représenter -C(=NH)NH₂ ou -CH₂-NH₂ ;
R³ peut représenter un groupement éthyle ou propyle, qui est substitué par -NH₂, -NHMe, -NMe₂, -NHEt, -NEt₂, -NHphényle, -N(phényle)₂, -NHbenzyle, -N(benzyle)₂ ou -C(=NH)NH₂, ou
benzyle, qui est substitué par l'un des groupes -NH₂, -CH₂-NH₂, -NMe₂, NHMe, -NEt₂, -NHEt, -NH-CO-Me, -CH₂-NH-CO-Me ou -C(=NH)NH₂, ou
phényléthyle, où le pont éthylène peut éventuellement être substitué par COOH, COOMe ou COOEt et où le cycle phényle est substitué par l'un des groupes -NH₂, -CH₂-NH₂, -NMe₂, -NHMe, -NEt₂, -NHEt, -NH-CO-Me, -CH₂-NH-CO-Me ou -C(=NH)NH₂, ou
phénylpropyle, diphénylpropyle ou pyridylméthyle ;
R⁴ peut représenter l'hydrogène ou un groupement méthyle, éthyle, propyle ou butyle, qui peut être substitué par l'un des groupes COOH, COOMe, COOEt, cyclopropyle, cyclopentyle ou cyclohexyle, ou
benzyle, qui peut éventuellement être substitué par méthyle, éthyle, propyle, méthoxy, éthoxy, CF₃, fluor, chlore, brome, COOH, COOMe ou COOEt, ou phényléthyle, phénylpropyle, diphénylpropyle, ou
cyclopentyle, cyclohexyle, cyclooctyle, naphtyle ou phényle, qui peut éventuellement être substitué par méthyle, éthyle, méthoxy, éthoxy, phényloxy ou CF₃, ou
une pyridine ou quinoléine liée par le biais d'un pont méthylène,
ou
R³ et R⁴ forment avec l'atome d'azote un cycle pipérazine ou diazépane, qui peut éventuellement être substitué par l'un des groupements cyclopentyle, cyclohexyle, pyridyle, benzyle ou phényle, qui porte éventuellement l'un des groupements choisis dans le groupe méthyle, méthoxy, éthoxy, propyloxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

7. Composés de formule générale (I) selon l'une des revendications 2-6 où
R¹ peut représenter méthyle, éthyle, propyle, pentyle, phényléthyle, phénylpropyle, cyclopropylméthyle, tétrahydrofuranylméthyle ou benzyle, qui est substitué une ou deux fois par CF₃, COOH, COOMe ou COOEt ;
R² peut représenter -C(=NH)NH₂ ou -CH₂-NH₂ ;
R³ peut représenter un groupement éthyle ou propyle, qui est substitué par -NH₂, -NHMe, -NMe₂, -NHEt, -NEt₂, -NHphényle, -N(phényle)₂, -NHbenzyle, -N(benzyle)₂ ou -C(=NH)NH₂, ou
benzyle, qui est substitué par l'un des groupes -NH₂, -CH₂-NH₂, -NMe₂, NHMe, -NEt₂, -NHEt, -NH-CO-Me, -CH₂-NH-CO-Me ou -C(=NH)NH₂, ou
phényléthyle, où le pont éthylène est substitué par COOH, COOMe ou COOEt et
où le cycle phényle porte l'un des groupes -NH₂, -CH₂-NH₂, -NMe₂, -NHMe, -NEt₂, -NHEt, -NH-CO-Me, -CH₂-NH-CO-Me ou -C(=NH)NH₂, ou phénylpropyle, diphénylpropyle ou pyridylméthyle ;
R⁴ peut représenter l'hydrogène ou un groupement méthyle, éthyle, propyle ou butyle, qui peut être substitué par l'un des groupes COOH, COOMe, COOEt ou cyclohexyle, ou
phényle, qui peut éventuellement être substitué par méthyle, éthyle, méthoxy, éthoxy, phényloxy ou CF₃, ou
benzyle, phényléthyle, phénylpropyle, diphénylpropyle, cyclohexyle, cyclooctyle ou naphtyle, ou
une pyridine ou quinoléine liée par le biais d'un pont méthylène,
ou
R³ et R⁴ forment avec l'atome d'azote un cycle pipérazine ou diazépane, qui peut éventuellement être substitué par l'un des groupements cyclopentyle, cyclohexyle, pyridyle, benzyle ou phényle, qui porte éventuellement un groupement choisi dans le groupe méthyle, méthoxy, éthoxy, propyloxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

8. Composés de formule générale (I) selon l'une des revendications 2-7 où
R¹ peut représenter méthyle ;
R² peut représenter -C(=NH)NH₂ ou -CH₂-NH₂ ;
R³ peut représenter éthyle, qui est substitué par -NH₂, -NMe₂, -NHphényle, -NHbenzyle, -N(benzyle)₂, pyrrolidine, pipéridine, diazépane ou -C(=NH)NH₂, benzyle, qui est substitué par l'un des groupes -CH₂-NH₂, -NMe₂ ou -C(=NH)NH₂,
phényléthyle, où le pont éthylène est substitué par COOH, COOMe ou COOEt et
où le cycle phényle porte l'un des groupes -CH₂-NH-CO-Me ou -C(=NH)NH₂, diphénylpropyle ou pyridylméthyle ;
R⁴ peut représenter l'hydrogène ou un groupement méthyle ou éthyle, qui peut éventuellement être substitué par l'un des groupes COOH ou COOEt, propyle, butyle ou phényle, qui peut éventuellement être substitué par méthyle, éthyle, méthoxy, éthoxy, phényloxy ou CF₃, benzyle, phényléthyle, phénylpropyle, diphénylpropyle, cyclohexyle, cyclooctyle, naphtyle, pyridylméthyle ou quinolinylméthyle
ou
R³ et R⁴ forment avec l'atome d'azote un cycle pipérazine ou diazépane, qui est substitué par l'un des groupements benzyle, cyclopentyle, cyclohexyle ou phényle, qui porte éventuellement un groupement choisi dans le groupe méthyle, éthoxy, propyloxy ou hydroxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

9. Composés de formule générale (I) selon l'une des revendications 2-8 où
R¹ peut représenter méthyle ;
R² peut représenter -C(=NH)NH₂ ;
R³ peut représenter éthyle, qui est substitué par -NH₂, -NHphényle, -NHbenzyle, -N(benzyle)₂, pyrrolidine, pipéridine, diazépane ou -C(=NH)NH₂,
benzyle, qui est substitué par -C(=NH)NH₂, ou diphénylpropyle ;
R⁴ peut représenter l'hydrogène, méthyle, propyle, butyle, benzyle ou phényle, qui peut éventuellement être substitué par éthyle ou phényloxy, phényléthyle, cyclohexyle ou cyclooctyle, ou
R³ et R⁴ forment avec l'atome d'azote un cycle pipérazine, qui est substitué par un groupement choisi dans le groupe cyclopentyle, cyclohexyle, phényle, méthylphényle, éthoxyphényle ou propoxyphényle,
ou un cycle diazépane, qui est substitué par méthylphényle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

10. Composés de formule générale (I) selon l'une des revendications 2-9 où
R¹ peut représenter méthyle ;
R² peut représenter -C(=NH)NH₂ ;
R³ peut représenter éthyle, qui est substitué par -NH₂, -NHbenzyle, -N(benzyle)₂, pyrrolidine, pipéridine, diazépane ou -C(=NH)NH₂, benzyle, qui est substitué par -C(=NH)NH₂, ou diphénylpropyle ;
R⁴ peut représenter l'hydrogène, méthyle, butyle ou phényle, qui peut éventuellement être substitué par éthyle ou phényloxy, phényléthyle, cyclohexyle ou cyclooctyle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

11. Promédicaments de formule générale (II) 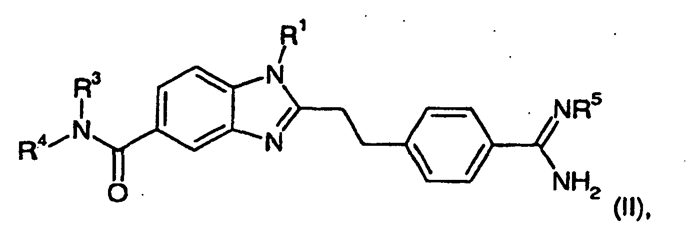 où
R¹ et R⁴ peuvent présenter les significations citées dans les revendications 2-10 ;
R³ peut présenter les significations citées dans les revendications 2-10 ou représente C₁-C₄-alkyle qui est substitué par un groupement choisi dans le groupe -C(=NOH)NH₂, -C(=NCOO-C₁-C₄-alkyl)NH₂ ou
-C(=NCOO- C₁-C₄-alkyl-phényl)NH₂ ;
R⁵ peut représenter hydroxy, -COO-C₁-C₈-alkyle ou -COO-C₁-C₄-alkyl-phényle,
où, dans le groupe cité précédemment, le cycle phényle peut être substitué dans chaque cas par C₁-C₄-alkyle ou C₁-C₄-alcoxy,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

12. Promédicaments de formule générale (II) selon la revendication 11, où
R¹ et R⁴ peuvent présenter les significations citées dans les revendications 2-10 ;
R³ peut présenter les significations citées dans les revendications 2-10 ou représente C₁-C₄-alkyle qui est substitué par un groupement choisi dans le groupe -C(=NOH)NH₂, -C(=NCOOméthyl)NH₂, -C(=NCOOéthyl)NH₂, -C(=NCOOpropylyl)NH₂ ou -C(=NCOO-benzyl)NH₂;
R⁵ peut représenter hydroxy, méthoxycarbonyle, éthoxycarbonyle, propyloxycarbonyle, butyloxycarbonyle, pentyloxycarbonyle, hexyloxycarbonyle, benzyloxycarbonyle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

13. Produits intermédiaires de formule générale (III) 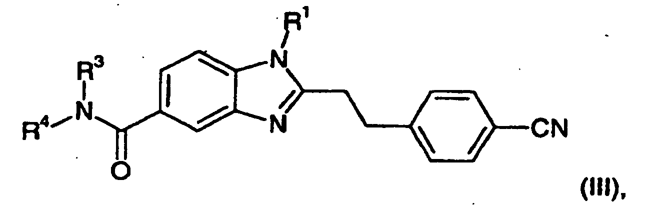 où les groupements R¹, R³ et R⁴ peuvent présenter les significations citées dans les revendications 2-12.

14. Utilisation de composés de formule générale (I) selon l'une des revendications 2-10 pour la production d'un médicament pour la prévention et/ou le traitement des maladies dans lesquelles les inhibiteurs de tryptase peuvent présenter une utilité thérapeutique.

15. Utilisation de promédicaments de formule générale (II) selon l'une des revendications 11 ou 12 pour la production d'un médicament pour la prévention et/ou le traitement des maladies dans lesquelles les inhibiteurs de tryptase peuvent présenter une utilité thérapeutique.

16. Composition pharmaceutique **caractérisée par** une teneur d'un ou plusieurs composés selon l'une des revendications 2-12.

17. Procédé de production de composés de formule générale (I) 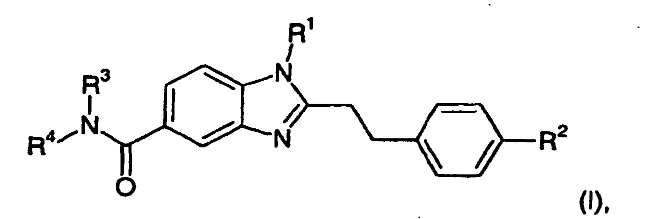 où les groupements R¹, R², R³ et R⁴ peuvent présenter les significations citées dans les revendications 2-10, **caractérisé en ce qu'**un composé de formule générale (III) 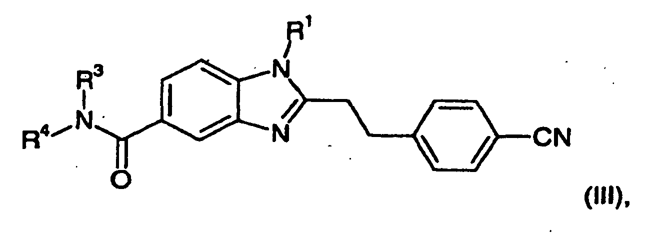 où les groupements R¹, R³ et R⁴ peuvent présenter les significations citées dans les revendications 2-10, est converti directement en les composés de formule générale (I).

18. Procédé de production de composés de formule générale (I) 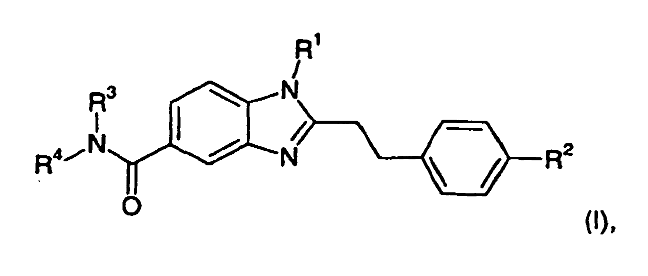 où les groupements R¹, R², R³ et R⁴ peuvent présenter les significations citées dans les revendications 2-10, **caractérisé en ce qu'**un composé de formule générale (II) 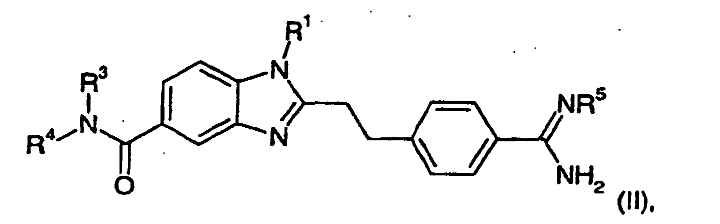 où les groupements R¹ et R⁴ peuvent présenter les significations citées dans les revendications 2-10 et les groupements R³ et R⁵ peuvent présenter les significations citées dans les revendications 11 et 12 est converti en les composés de formule générale (I).

19. Procédé de production de composés de formule générale (II) 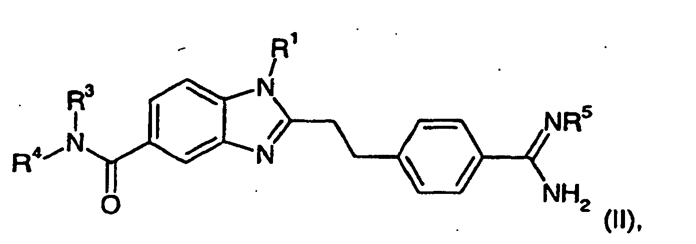 où les groupements R¹ et R⁴ peuvent présenter les significations citées dans les revendications 2-10 et les groupements R³ et R⁵ peuvent présenter les significations citées dans les revendications 11 et 12, **caractérisé en ce qu'**un composé de formule générale (III) 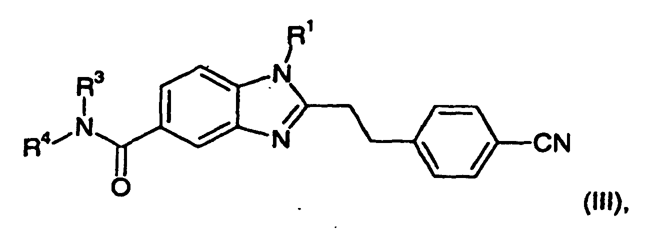 où les groupements R¹, R³ et R⁴ peuvent présenter les significations citées dans les revendications 2-10, est converti en un composé de formule générale (II).

20. Procédé de production de composés de formule générale (I) 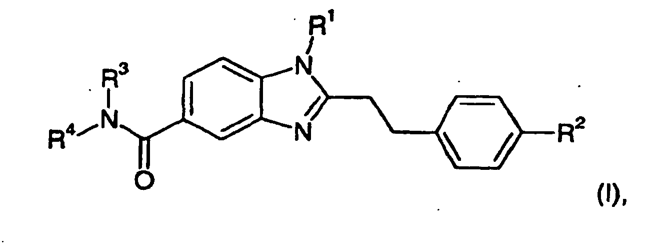 où les groupements R¹, R², R³ et R⁴ peuvent présenter les significations citées dans les revendications 2-10, **caractérisé en ce que** la synthèse a lieu par réaction sur un support polymère (résine) et comprend les étapes de synthèse suivantes :
- couplage d'une diamine au support polymère,
- amination réductive avec réaction avec un aldéhyde R⁴-CHO;
- acylation avec le chlorure de fluoro- ou chloronitrobenzoyle,
- substitution nucléophile avec une amine R¹-NH₂,
- réduction,
- cyclisation oxydative et
- clivage des composés de formule générale (I) du support polymère.
